# EUROPEAN PATENT SPECIFICATION

(11) **EP 2 970 403 B1**
(45) Date of publication and mention of the grant of the patent: **20.09.2017**
(21) Application number: 14722012.3
(22) Date of filing: 12.03.2014
(51) Int. Cl.: C07K 14/37, C12N 15/82

(54) **IDENTIFICATION OF P. PACHYRHIZI PROTEIN EFFECTORS AND THEIR USE IN PRODUCING ASIAN SOYBEAN RUST (ASR) RESISTANT PLANTS**
IDENTIFIZIERUNG VON P. PACHYRHIZI-PROTEINEFFEKTOREN UND DEREN VERWENDUNG ZUR HERSTELLUNG VON GEGENÜBER ASIATISCHEM SOJABOHNENROST (ASR) RESISTENTEN PFLANZEN
IDENTIFICATION D'EFFECTEURS PROTÉIQUES DE P. PACHYRHIZI ET LEUR UTILISATION DANS LE CADRE DE LA PRODUCTION DE PLANTES RÉSISTANTES À LA ROUILLE ASIATIQUE

(30) Priority: 13.03.2013 US 201313798408
(43) Date of publication of application: 20.01.2016
(60) Divisional application: 17184990.4
(73) Proprietor: E. I. du Pont de Nemours and Company, Wilmington, DE 19805 (US)
(72) Inventor: BROGLIE, Karen E., Landenberg, PA 19350 (US); RAIRDAN, Gregory J., Wilmington, DE 19806 (US)
(74) Representative: J A Kemp
(86) International application number: PCT/US2014/024286
(87) International publication number: WO 2014/165066

(56) References cited:
- WO-A1-2008/017706
- WO-A1-2013/001435
- WO-A2-2007/016680
- WO-A2-2011/137414
- DATABASE UniProt [Online] 24 March 2009 (2009-03-24), "SubName: Full=Putative uncharacterized protein {ECO:0000313|EMBL:EEF32134.1};", XP002761868, retrieved from EBI accession no. UNIPROT:B9SW95 Database accession no. B9SW95 & AGNES P CHAN ET AL: "Draft genome sequence of the oilseed species Ricinus communis", NATURE BIOTECHNOLOGY, GALE GROUP INC, US, vol. 28, no. 9, 1 September 2010 (2010-09-01), pages 951-959, XP002676754, ISSN: 1087-0156, DOI: 10.1038/NBT.1674 Retrieved from the Internet: URL:http://www.nature.com/nbt/journal/v28/ n9/full/nbt.1674.html> [retrieved on 2010-08-22]
- DATABASE UniProt [Online] 28 June 2011 (2011-06-28), "SubName: Full=Secreted protein {ECO:0000313|EMBL:EGG08429.1};", XP002761867, retrieved from EBI accession no. UNIPROT:F4RG77 Database accession no. F4RG77 & DUPLESSIS SEBASTIEN ET AL: "Obligate biotrophy features unraveled by the genomic analysis of rust fungi", PROCEEDINGS OF THE NATIONAL ACADEMY OF SCIENCES OF THE UNITED STATES OF AMERICA, vol. 108, no. 22, May 2011 (2011-05), pages 9166-9171, ISSN: 0027-8424
- DATABASE EMBL [Online] 1 January 2007 (2007-01-01), "JGI_ABWZ4110.fwd ABWZ Phakopsora pachyrhizi infected soybean leaf tissue 13-15 days post inoculation with TW72-1 urediniospores Glycine max cDNA clone ABWZ4110 5', mRNA sequence.", XP002726492, retrieved from EBI accession no. EM_EST:EH223337 Database accession no. EH223337
- STONE CHRISTINE L ET AL: "Gene expression and proteomic analysis of the formation of Phakopsora pachyrhizi appressoria", BMC GENOMICS, vol. 13, June 2012 (2012-06), XP002726493, ISSN: 1471-2164
- LUSTER DOUGLAS G ET AL: "Proteomic analysis of germinating urediniospores of Phakopsora pachyrhizi, causal agent of Asian soybean rust", PROTEOMICS, vol. 10, no. 19, October 2010 (2010-10), pages 3549-3557, XP002726494, ISSN: 1615-9853
- LUSTER DOUGLAS G ET AL: "Novel Phakopsora pachyrhizi Extracellular Proteins Are Ideal Targets for Immunological Diagnostic Assays", APPLIED AND ENVIRONMENTAL MICROBIOLOGY, vol. 78, no. 11, June 2012 (2012-06), pages 3890-3895, XP002726495,
- FANG ZHIWEI D ET AL: "Combinatorially Selected Peptides for Protection of Soybean Against Phakopsora pachyrhizi", PHYTOPATHOLOGY, vol. 100, no. 10, October 2010 (2010-10), pages 1111-1117, XP002726496, ISSN: 0031-949X
- WANG YUN ET AL: "Proteomic Analysis of Differentially Expressed Proteins in Resistant Soybean Leaves after Phakopsora pachyrhizi Infection", JOURNAL OF PHYTOPATHOLOGY (BERLIN), vol. 160, no. 10, October 2012 (2012-10), pages 554-560, XP002726497,
- DATABASE UniProt [Online] 24 March 2009 (2009-03-24), "SubName: Full=Putative uncharacterized protein {ECO:0000313|EMBL:EEF32134.1};", XP002761868, retrieved from EBI accession no. UNIPROT:B9SW95 Database accession no. B9SW95 & AGNES P CHAN ET AL: "Draft genome sequence of the oilseed species Ricinus communis", NATURE BIOTECHNOLOGY, GALE GROUP INC, US, vol. 28, no. 9, 1 September 2010 (2010-09-01), pages 951-959, XP002676754, ISSN: 1087-0156, DOI: 10.1038/NBT.1674 Retrieved from the Internet: URL:http://www.nature.com/nbt/journal/v28/ n9/full/nbt.1674.html> [retrieved on 2010-08-22]
- DATABASE UniProt [Online] 28 June 2011 (2011-06-28), "SubName: Full=Secreted protein {ECO:0000313|EMBL:EGG08429.1};", XP002761867, retrieved from EBI accession no. UNIPROT:F4RG77 Database accession no. F4RG77 & DUPLESSIS SEBASTIEN ET AL: "Obligate biotrophy features unraveled by the genomic analysis of rust fungi", PROCEEDINGS OF THE NATIONAL ACADEMY OF SCIENCES OF THE UNITED STATES OF AMERICA, vol. 108, no. 22, May 2011 (2011-05), pages 9166-9171, ISSN: 0027-8424

### SEQUENCE LISTING

<110> E.I. DuPont de Nemours & Company
   Reardon, Gregory
   Broglie, Karen
<120> Identification of P. pachyrhizi Protein Effectors and their use in Producing Asian Soybean Rust (ASR) Resistant Plants
<130> 4249WOPCT
<150> 13/798,408
   <151> 2013-03-13
<160> 378
<170> PatentIn version 3.5
<210> 1
   <211> 168
   <212> DNA
   <213> Phakopsora pachyrhizi
<220>
   <221> misc_feature
   <222> (1)..(168)
   <223> 01-c18
<400> 1
   atgatttcca aggttattta caactcgtta tattacctaa atgcttcgat ggtaatggtg       60
   gcggtcagtc ttcaaaattc ggcgcttcat aagagagctg tttcaagcag cgagccatgt      120
   tgttgcgctg ggtgtggaga gttcctttct caatgttctg ttatctga                   168
<210> 2
   <211> 55
   <212> PRT
   <213> Phakopsora pachyrhizi
<220>
   <221> VARIANT
   <222> (1)..(55)
   <223> 01-e12
<400> 2
   Met Ile Ser Lys Val Ile Tyr Asn Ser Leu Tyr Tyr Leu Asn Ala Ser
   1               5                   10                  15
   Met Val Met Val Ala Val Ser Leu Gln Asn Ser Ala Leu His Lys Arg
               20                  25                  30
   Ala Val Ser Ser Ser Glu Pro Cys Cys Cys Ala Gly Cys Gly Glu Phe
           35                  40                  45
   Leu Ser Gln Cys Ser Val Ile
       50                  55
<210> 3
   <211> 429
   <212> DNA
   <213> Phakopsora pachyrhizi
<220>
   <221> misc_feature
   <222> (1)..(429)
   <223> 01-e12
<400> 3
   atgcatgctg ctaatatgaa agtttcaacc cttatgatag tagtgttaag ttttatccag       60
   ctatattctt ttactttatc tcaaatcacc aatgggggca ttacgcagga tccaaatgat      120
   aaatacaggg ttcgatgttt agccgactct aaatcagggg atataatttc tgatgactgc      180
   tatgattcat tggatgactt ttcccaagat gatggaatta ttaaattcaa tgcaaaagga      240
   gaagataatg atgaagactc ttgtaggttg gagttagttg gttatcctaa tcaaatcacc      300
   aataatgatg aaatcaaagt gccaaaaaca tctgtgatca gcgcaatcaa ttttggctta      360
   gatggctgta aaaattcttc agccatagtt gaagcaacag gctttattct caacatttac      420
   aaaaattaa                                                              429
<210> 4
   <211> 142
   <212> PRT
   <213> Phakopsora pachyrhizi
<220>
   <221> VARIANT
   <222> (1)..(142)
   <223> 01-e12
<400> 4
   Met His Ala Ala Asn Met Lys Val Ser Thr Leu Met Ile Val Val Leu
   1               5                   10                  15
   Ser Phe Ile Gln Leu Tyr Ser Phe Thr Leu Ser Gln Ile Thr Asn Gly
               20                  25                  30
   Gly Ile Thr Gln Asp Pro Asn Asp Lys Tyr Arg Val Arg Cys Leu Ala
           35                  40                  45
   Asp Ser Lys Ser Gly Asp Ile Ile Ser Asp Asp Cys Tyr Asp Ser Leu
       50                  55                  60
   Asp Asp Phe Ser Gln Asp Asp Gly Ile Ile Lys Phe Asn Ala Lys Gly
   65                  70                  75                  80
   Glu Asp Asn Asp Glu Asp Ser Cys Arg Leu Glu Leu Val Gly Tyr Pro
                   85                  90                  95
   Asn Gln Ile Thr Asn Asn Asp Glu Ile Lys Val Pro Lys Thr Ser Val
               100                 105                 110
   Ile Ser Ala Ile Asn Phe Gly Leu Asp Gly Cys Lys Asn Ser Ser Ala
           115                 120                 125
   Ile Val Glu Ala Thr Gly Phe Ile Leu Asn Ile Tyr Lys Asn
       130                 135                 140
<210> 5
   <211> 294
   <212> DNA
   <213> Phakopsora pachyrhizi
<220>
   <221> misc_feature
   <222> (1)..(294)
   <223> 02-g20
<400> 5
   atgaccttct tcaacatgct cctccttctg tggagagtcc gtgtccccag gaaaacctcc       60
   aacatcatgt tcatgtccat caaaatgatt ttcaggaaaa ttggcatcct ccaccaaatt      120
   gcctgcatca tcttcatcag gcagcaggtg aggaaatcca gagctatcaa tcacagtccc      180
   tacatcatgt tcatcagaat gattctgagg aaaattaggg tcctcgggca catatcctac      240
   aacatgtcca tcaaaatcgt gctcggccat gagtatattt tctgcgaaaa ataa            294
<210> 6
   <211> 97
   <212> PRT
   <213> Phakopsora pachyrhizi
<220>
   <221> VARIANT
   <222> (1)..(97)
   <223> 02-g20
<400> 6
   Met Thr Phe Phe Asn Met Leu Leu Leu Leu Trp Arg Val Arg Val Pro
   1               5                   10                  15
   Arg Lys Thr Ser Asn Ile Met Phe Met Ser Ile Lys Met Ile Phe Arg
               20                  25                  30
   Lys Ile Gly Ile Leu His Gln Ile Ala Cys Ile Ile Phe Ile Arg Gln
           35                  40                  45
   Gln Val Arg Lys Ser Arg Ala Ile Asn His Ser Pro Tyr Ile Met Phe
       50                  55                  60
   Ile Arg Met Ile Leu Arg Lys Ile Arg Val Leu Gly His Ile Ser Tyr
   65                  70                  75                  80
   Asn Met Ser Ile Lys Ile Val Leu Gly His Glu Tyr Ile Phe Cys Glu
                   85                  90                  95
   Lys
<210> 7
   <211> 483
   <212> DNA
   <213> Phakopsora pachyrhizi
<220>
   <221> misc_feature
   <222> (1)..(483)
   <223> 04-a13
<400> 7
   atgatcttca aaccagtctt actacgtcag agcatggtta cagtctcagg acctagtcga      60
   ttgttgagat cgactatcag aagatcaacc atctcacatc tcaatcacac cccgatcatc     120
   tccaaacagt ggatccaatc agctttctac agtgctggtc atgatacagc tgatgaatcg     180
   tatccagcct tcaacgagag gtacactaag ttctttgagg gagttcaaga cctttttgag     240
   cttcaaagag gtttgaataa ctgctttgcc tatgacttgg ttcctgcggt ttcaactgta     300
   gaagcagccc tcaaggcggc caggcgtgtg aatgatctgc ctactgcgat tcgaatattt     360
   gaaggtctca aagaaaaggt tgagaacaag acacagtaca aggcatattt agaagagtta     420
   aaacctttga gagatgaact tggtgttcct accaaagaag aactatacgg tcaaactatt     480
   taa                                                                   483
<210> 8
   <211> 160
   <212> PRT
   <213> Phakopsora pachyrhizi
<220>
   <221> VARIANT
   <222> (1)..(160)
   <223> 04-a13
<400> 8
   Met Ile Phe Lys Pro Val Leu Leu Arg Gln Ser Met Val Thr Val Ser
   1 5 10 15
   Gly Pro Ser Arg Leu Leu Arg Ser Thr Ile Arg Arg Ser Thr Ile Ser
   20 25 30
   His Leu Asn His Thr Pro Ile Ile Ser Lys Gln Trp Ile Gln Ser Ala
   35 40 45
   Phe Tyr Ser Ala Gly His Asp Thr Ala Asp Glu Ser Tyr Pro Ala Phe
   50 55 60
   Asn Glu Arg Tyr Thr Lys Phe Phe Glu Gly Val Gln Asp Leu Phe Glu
   65 70 75 80
   Leu Gln Arg Gly Leu Asn Asn Cys Phe Ala Tyr Asp Leu Val Pro Ala
   85 90 95
   Val Ser Thr Val Glu Ala Ala Leu Lys Ala Ala Arg Arg Val Asn Asp
   100 105 110
   Leu Pro Thr Ala Ile Arg Ile Phe Glu Gly Leu Lys Glu Lys Val Glu
   115 120 125
   Asn Lys Thr Gln Tyr Lys Ala Tyr Leu Glu Glu Leu Lys Pro Leu Arg
   130 135 140
   Asp Glu Leu Gly Val Pro Thr Lys Glu Glu Leu Tyr Gly Gln Thr Ile
   145 150 155 160
<210> 9
   <211> 954
   <212> DNA
   <213> Phakopsora pachyrhizi
<220>
   <221> misc_feature
   <222> (1)..(954)
   <223> 04-f24
<400> 9
   atgaaaaata tttttagagg ttctttatat atatttttga cactccttaa tttgattgta 60
   agcctcgaag cagccttagt agcatttgac gaggaagtta gcttgttgag gaatacggat 120
   cgcataagcc aaactcaacc caagacttta gagctccaaa aatattcaaa ggcttataat 180
   ggtcctttga tatatgcccc atccagctct gtagaagtct tcaaaagtgc agctcagttg 240
   cacactagtc caaaatctga taaatctttt actcgagatt ggagaagtat ccacatgaat 300
   gatttattaa atccaaggat tactcaacct ataaaatcta aaaaagggtc tttactttta 360
   tacttattta gaaaaaaacc taaaactcaa tatgaggtta aaaagacatt agttctttcc 420
   gacaaaggtt ctaccgatac ttcatcttta ttcaataaag aatctttagc gctaagttac 480
   cccagaaaag aaaaaaattc cggaaactac ttagagggct tagagattga agttccaacg 540
   ctcagtgatg aagcattagt agatctaaga gactggaagc ggagcacatc agtgttatat 600
   tttatagaac agatcgagct aagaacaaat aaggttgtag atttactaaa aaggccagat 660
   ttaaatatag aagatgtaga gggtctctca aactttttga aagacttaaa cgttgatgag 720
   aatggagatt tgaaagacct cagggagggt gaaacgatct atctgatgat tcaagacgtt 780
   tggaaggctt caaagtctaa cttccctgcc gaggtaaaaa ctcaaatctt ttatagatac 840
   gagtatagac tcaaaaagat cttcgataag ctctcagagc aaaggttttt cattaaaaag 900
   ggtaaaaagg aaactctaat caaggaatca atggagatag ttctatacag gctg 954
<210> 10
   <211> 318
   <212> PRT
   <213> Phakopsora pachyrhizi
<220>
   <221> VARIANT
   <222> (1)..(318)
   <223> 04-f24
<400> 10
   Met Lys Asn Ile Phe Arg Gly Ser Leu Tyr Ile Phe Leu Thr Leu Leu
   1               5                   10                  15
   Asn Leu Ile Val Ser Leu Glu Ala Ala Leu Val Ala Phe Asp Glu Glu
               20                  25                  30
   Val Ser Leu Leu Arg Asn Thr Asp Arg Ile Ser Gln Thr Gln Pro Lys
           35                  40                  45
   Thr Leu Glu Leu Gln Lys Tyr Ser Lys Ala Tyr Asn Gly Pro Leu Ile
       50                  55                  60
   Tyr Ala Pro Ser Ser Ser Val Glu Val Phe Lys Ser Ala Ala Gln Leu
   65                  70                  75                  80
   His Thr Ser Pro Lys Ser Asp Lys Ser Phe Thr Arg Asp Trp Arg Ser
                   85                  90                  95
   Ile His Met Asn Asp Leu Leu Asn Pro Arg Ile Thr Gln Pro Ile Lys
               100                 105                 110
   Ser Lys Lys Gly Ser Leu Leu Leu Tyr Leu Phe Arg Lys Lys Pro Lys
           115                 120                 125
   Thr Gln Tyr Glu Val Lys Lys Thr Leu Val Leu Ser Asp Lys Gly Ser
       130                 135                 140
   Thr Asp Thr Ser Ser Leu Phe Asn Lys Glu Ser Leu Ala Leu Ser Tyr
   145                 150                 155                 160
   Pro Arg Lys Glu Lys Asn Ser Gly Asn Tyr Leu Glu Gly Leu Glu Ile
                   165                 170                 175
   Glu Val Pro Thr Leu Ser Asp Glu Ala Leu Val Asp Leu Arg Asp Trp
               180                 185                 190
   Lys Arg Ser Thr Ser Val Leu Tyr Phe Ile Glu Gln Ile Glu Leu Arg
           195                 200                 205
   Thr Asn Lys Val Val Asp Leu Leu Lys Arg Pro Asp Leu Asn Ile Glu
       210                 215                 220
   Asp Val Glu Gly Leu Ser Asn Phe Leu Lys Asp Leu Asn Val Asp Glu
   225                 230                 235                 240
   Asn Gly Asp Leu Lys Asp Leu Arg Glu Gly Glu Thr Ile Tyr Leu Met
                   245                 250                 255
   Ile Gln Asp Val Trp Lys Ala Ser Lys Ser Asn Phe Pro Ala Glu Val
               260                 265                 270
   Lys Thr Gln Ile Phe Tyr Arg Tyr Glu Tyr Arg Leu Lys Lys Ile Phe
           275                 280                 285
   Asp Lys Leu Ser Glu Gln Arg Phe Phe Ile Lys Lys Gly Lys Lys Glu
       290                 295                 300
   Thr Leu Ile Lys Glu Ser Met Glu Ile Val Leu Tyr Arg Leu
   305                 310                 315
<210> 11
   <211> 1065
   <212> DNA
   <213> Phakopsora pachyrhizi
<220>
   <221> misc_feature
   <222> (1)..(1065)
   <223> 04-i14
<400> 11
   atgaatcagc actatagaga aattatggtt ttcagaaaag cagttatata tctgtgctta      60
   ggagcactta aaatattatc taccaatgca gcgttttcaa agagtaacga tatcaaaact     120
   ttagatgata ttgctgaagg ctctatgcct gaaaaccctt ttttcagcca agataatttt     180
   atttcctgta ctaaaaatac atcgcaaatc gagaaacagt cacaagcaga aggttacatt     240
   aattttccag ataaaaatgt agttatcact cagtttgtcg ctaataacaa aagggatttc     300
   aaggaaacat tagagaatag agaggccaag ccagttcatt cacctgttaa cttagaagag     360
   cataaattaa cgcctagcat tgtaactgac caggttgatg atttaatgtt tttcgctact     420
   gagttggatg attttccaac ttatttgatg gaatttcatc cagctgagat acagcctaat     480
   tctgctactg cgtctgatgg tttggaactc agctatcatg actatcacct tcaggacaat     540
   gattttgggg aaaataattc ttggttgata aaccaatctg aaccattagg atttcaaagt     600
   agtactgtcc taaatggtgc ggcagaaatt ccagtcccag agcatcaaaa tttaggagct     660
   ccgagtctcg acaataatca gcttttctgt agcttatttg aagcggagcc cacagctgtc     720
   tcagaagaaa acacttacaa cgcttgggca agcaattttg gacctactct tcaggaggaa     780
   gcaagagaat cgtataaaga agggcaaatt tcaaatgaat caattgaaaa ccttcattca     840
   ctgaatttca attacgactc caatatttta aagattagac tttcaaaagc acaaaatgac     900
   gtatttgaat tagaagacct tccggtttta gaaaaacagc tatctcaaac attgagccca     960
   ggagaaaaat atataataga aaacgaatca ttacttaaaa gcaggcggag tcactcaaaa    1020
   gcccctggta gtaaaagtcg caagatcacg aaaaacacta aagat                    1065
<210> 12
   <211> 355
   <212> PRT
   <213> Phakopsora pachyrhizi
<220>
   <221> VARIANT
   <222> (1)..(355)
   <223> 04-i14
<400> 12
   Met Asn Gln His Tyr Arg Glu Ile Met Val Phe Arg Lys Ala Val Ile
   1               5                   10                  15
   Tyr Leu Cys Leu Gly Ala Leu Lys Ile Leu Ser Thr Asn Ala Ala Phe
               20                  25                  30
   Ser Lys Ser Asn Asp Ile Lys Thr Leu Asp Asp Ile Ala Glu Gly Ser
           35                  40                  45
   Met Pro Glu Asn Pro Phe Phe Ser Gln Asp Asn Phe Ile Ser Cys Thr
       50                  55                  60
   Lys Asn Thr Ser Gln Ile Glu Lys Gln Ser Gln Ala Glu Gly Tyr Ile
   65                  70                  75                  80
   Asn Phe Pro Asp Lys Asn Val Val Ile Thr Gln Phe Val Ala Asn Asn
                   85                  90                  95
   Lys Arg Asp Phe Lys Glu Thr Leu Glu Asn Arg Glu Ala Lys Pro Val
               100                 105                 110
   His Ser Pro Val Asn Leu Glu Glu His Lys Leu Thr Pro Ser Ile Val
           115                 120                 125
   Thr Asp Gln Val Asp Asp Leu Met Phe Phe Ala Thr Glu Leu Asp Asp
       130                 135                 140
   Phe Pro Thr Tyr Leu Met Glu Phe His Pro Ala Glu Ile Gln Pro Asn
   145                 150                 155                 160
   Ser Ala Thr Ala Ser Asp Gly Leu Glu Leu Ser Tyr His Asp Tyr His
                   165                 170                 175
   Leu Gln Asp Asn Asp Phe Gly Glu Asn Asn Ser Trp Leu Ile Asn Gln
               180                 185                 190
   Ser Glu Pro Leu Gly Phe Gln Ser Ser Thr Val Leu Asn Gly Ala Ala
           195                 200                 205
   Glu Ile Pro Val Pro Glu His Gln Asn Leu Gly Ala Pro Ser Leu Asp
       210                 215                 220
   Asn Asn Gln Leu Phe Cys Ser Leu Phe Glu Ala Glu Pro Thr Ala Val
   225                 230                 235                 240
   Ser Glu Glu Asn Thr Tyr Asn Ala Trp Ala Ser Asn Phe Gly Pro Thr
                   245                 250                 255
   Leu Gln Glu Glu Ala Arg Glu Ser Tyr Lys Glu Gly Gln Ile Ser Asn
               260                 265                 270
   Glu Ser Ile Glu Asn Leu His Ser Leu Asn Phe Asn Tyr Asp Ser Asn
           275                 280                 285
   Ile Leu Lys Ile Arg Leu Ser Lys Ala Gln Asn Asp Val Phe Glu Leu
       290                 295                 300
   Glu Asp Leu Pro Val Leu Glu Lys Gln Leu Ser Gln Thr Leu Ser Pro
   305                 310                 315                 320
   Gly Glu Lys Tyr Ile Ile Glu Asn Glu Ser Leu Leu Lys Ser Arg Arg
                   325                 330                 335
   Ser His Ser Lys Ala Pro Gly Ser Lys Ser Arg Lys Ile Thr Lys Asn
               340                 345                 350
   Thr Lys Asp
           355
<210> 13
   <211> 984
   <212> DNA
   <213> Phakopsora pachyrhizi
<220>
   <221> misc_feature
   <222> (1)..(984)
   <223> 04-L8
<400> 13
   atgaaaaaag cccagtgtac gataatatgc ctcgcactga gctttcaggt gtttctagaa      60
   gctgggtcaa cctatttgac cgagcccatg agaagtattg atggcgaggc cagcttcggc     120
   tcatgtagca cttcctggat gtctaatgct gggaacgcca aggccaatgg tgtaaatccg     180
   aatggtgtgc tggataaagt ggtataccct ttctctaaac aatgttactg gtctggcaag     240
   gacatgaaaa accttgagag ctttaatttt tgtggtaata aacaaattgc cgctcagtac     300
   agagacctta ttaacagcta catatcgcca aggcagataa tccttcataa tgaaaataaa     360
   gaaaaattag ctttattaaa cagaaattta aaagagctta gccctgaaga caaagaagca     420
   ctagaattat gggcaagcaa aaactttcag cctaacaacg tattaataga aaatgaaagc     480
   ccaaatgaat tccgaagtgc taaggctgct ttagaatgga atctaaagag ggcttctgct     540
   aagcaagatg tatggtggac caaagaggat cttcaacgtt gggctcatca cgattctgat     600
   gaaacagtta tcattaaatt tggtgatata ctaaagtttg atcagaaaag ggaggatttt     660
   gtgatcaaag atagtccagt acaattagct tcaagacttt tcagagcgct agataaatca     720
   actggtggta attcttttaa caagcgagtt gcttggccct ctgaagaaat tcaatccttg     780
   agattaatca aagccgaaga ctcaaacaaa tctttatttc atatgaggtt ggatcaaatg     840
   ttaaagaatt ataaaagatt taaagatcat aaaagtcaag aaaatggaaa ttttaaggaa     900
   ttcagtgatt ttattggttt atcaaaaact gataaaactg aacctcaaaa taaattacac     960
   caaaagaaca ttgaattaaa ataa                                            984
<210> 14
   <211> 327
   <212> PRT
   <213> Phakopsora pachyrhizi
<220>
   <221> VARIANT
   <222> (1)..(327)
   <223> 04-L8
<400> 14
   Met Lys Lys Ala Gln Cys Thr Ile Ile Cys Leu Ala Leu Ser Phe Gln
   1               5                   10                  15
   Val Phe Leu Glu Ala Gly Ser Thr Tyr Leu Thr Glu Pro Met Arg Ser
               20                  25                  30
   Ile Asp Gly Glu Ala Ser Phe Gly Ser Cys Ser Thr Ser Trp Met Ser
           35                  40                  45
   Asn Ala Gly Asn Ala Lys Ala Asn Gly Val Asn Pro Asn Gly Val Leu
       50                  55                  60
   Asp Lys Val Val Tyr Pro Phe Ser Lys Gln Cys Tyr Trp Ser Gly Lys
   65                  70                  75                  80
   Asp Met Lys Asn Leu Glu Ser Phe Asn Phe Cys Gly Asn Lys Gln Ile
                   85                  90                  95
   Ala Ala Gln Tyr Arg Asp Leu Ile Asn Ser Tyr Ile Ser Pro Arg Gln
               100                 105                 110
   Ile Ile Leu His Asn Glu Asn Lys Glu Lys Leu Ala Leu Leu Asn Arg
           115                 120                 125
   Asn Leu Lys Glu Leu Ser Pro Glu Asp Lys Glu Ala Leu Glu Leu Trp
       130                 135                 140
   Ala Ser Lys Asn Phe Gln Pro Asn Asn Val Leu Ile Glu Asn Glu Ser
   145                 150                 155                 160
   Pro Asn Glu Phe Arg Ser Ala Lys Ala Ala Leu Glu Trp Asn Leu Lys
                   165                 170                 175
   Arg Ala Ser Ala Lys Gln Asp Val Trp Trp Thr Lys Glu Asp Leu Gln
               180                 185                 190
   Arg Trp Ala His His Asp Ser Asp Glu Thr Val Ile Ile Lys Phe Gly
           195                 200                 205
   Asp Ile Leu Lys Phe Asp Gln Lys Arg Glu Asp Phe Val Ile Lys Asp
       210                 215                 220
   Ser Pro Val Gln Leu Ala Ser Arg Leu Phe Arg Ala Leu Asp Lys Ser
   225                 230                 235                 240
   Thr Gly Gly Asn Ser Phe Asn Lys Arg Val Ala Trp Pro Ser Glu Glu
                   245                 250                 255
   Ile Gln Ser Leu Arg Leu Ile Lys Ala Glu Asp Ser Asn Lys Ser Leu
               260                 265                 270
   Phe His Met Arg Leu Asp Gln Met Leu Lys Asn Tyr Lys Arg Phe Lys
           275                 280                 285
   Asp His Lys Ser Gln Glu Asn Gly Asn Phe Lys Glu Phe Ser Asp Phe
       290                 295                 300
   Ile Gly Leu Ser Lys Thr Asp Lys Thr Glu Pro Gln Asn Lys Leu His
   305                 310                 315                 320
   Gln Lys Asn Ile Glu Leu Lys
                   325
<210> 15
   <211> 1101
   <212> DNA
   <213> Phakopsora pachyrhizi
<220>
   <221> misc_feature
   <222> (1)..(1101)
   <223> 09-d10
<400> 15
   atggcttttt ggaagaatag ttgttttgta tttttaaata ccttagcaaa gttaacatgt      60
   ggaatgcttc cggaaggcaa ccaaataaaa cacctcagtg atgtaaccag tggttctcaa     120
   tttcttatcc cgtttgcagc agaggatagt ttggggacac aagcaaatga tgctctgcac     180
   gtagcaactt catcaaggaa tacccagaac acgccctgga cggcagatag ctcgattaag     240
   gaacacgtaa acatgggtga tataatgcat caagatgaag catttaggat taataaaata     300
   ttttctccat tacccctttt tccaattgct tacaacccgc ctggttataa tcaacacatg     360
   gttggtttta cttcaagtcc ttatttaaat ctagattatg ttacacatcc ttctatctta     420
   tcgcacccag ctgcgaataa acatgacttt gataataggg tggagaagag cttcgattat     480
   tttcaacaaa ttccaaaata caactttcaa gacattcaat acaggtttgc ggatcagcca     540
   cagaatagtg atgttaataa tcatcaaaaa aatttgaagg atgcagaagg aagtagtcag     600
   caattcattc attatcttac ctccgatgag ttttcagcca tttacccaaa tttagaacag     660
   ctacaggtat accaaagcca ccatattcca agcaatccaa atgagctaat cagaaatcaa     720
   ctttctccag ttaattcaga ggaattgagg ggcatcaaaa ataaagctat tagtcaatat     780
   tccgaaacta gaaaaggaaa agaaatgttg gaaagtagct tacaccatac agaccagttc     840
   aatcaattta ttcgaagcaa agaaaatgtt atacaccaag gcgaagaaaa cagcttggtt     900
   gtagcaccgc atgaatcatc tttatctcat ttacaaagtg atctgaatga atctgatgga     960
   aggagattca agaattccaa gaacggtgat aaaggtgatt cgcacagcca aatcgctact    1020
   tcatccagac gaataccacc gaaaaagcat cgattcaaaa cttctttaag atcactacca    1080
   gtcgatgaaa actcaaatga c                                              1101
<210> 16
   <211> 367
   <212> PRT
   <213> Phakopsora pachyrhizi
<220>
   <221> VARIANT
   <222> (1)..(367)
   <223> 09-d10
<400> 16
   Met Ala Phe Trp Lys Asn Ser Cys Phe Val Phe Leu Asn Thr Leu Ala
   1               5                   10                  15
   Lys Leu Thr Cys Gly Met Leu Pro Glu Gly Asn Gln Ile Lys His Leu
               20                  25                  30
   Ser Asp Val Thr Ser Gly Ser Gln Phe Leu Ile Pro Phe Ala Ala Glu
           35                  40                  45
   Asp Ser Leu Gly Thr Gln Ala Asn Asp Ala Leu His Val Ala Thr Ser
       50                  55                  60
   Ser Arg Asn Thr Gln Asn Thr Pro Trp Thr Ala Asp Ser Ser Ile Lys
   65                  70                  75                  80
   Glu His Val Asn Met Gly Asp Ile Met His Gln Asp Glu Ala Phe Arg
                   85                  90                  95
   Ile Asn Lys Ile Phe Ser Pro Leu Pro Leu Phe Pro Ile Ala Tyr Asn
               100                 105                 110
   Pro Pro Gly Tyr Asn Gln His Met Val Gly Phe Thr Ser Ser Pro Tyr
           115                 120                 125
   Leu Asn Leu Asp Tyr Val Thr His Pro Ser Ile Leu Ser His Pro Ala
       130                 135                 140
   Ala Asn Lys His Asp Phe Asp Asn Arg Val Glu Lys Ser Phe Asp Tyr
   145                 150                 155                 160
   Phe Gln Gln Ile Pro Lys Tyr Asn Phe Gln Asp Ile Gln Tyr Arg Phe
                   165                 170                 175
   Ala Asp Gln Pro Gln Asn Ser Asp Val Asn Asn His Gln Lys Asn Leu
               180                 185                 190
   Lys Asp Ala Glu Gly Ser Ser Gln Gln Phe Ile His Tyr Leu Thr Ser
           195                 200                 205
   Asp Glu Phe Ser Ala Ile Tyr Pro Asn Leu Glu Gln Leu Gln Val Tyr
       210                 215                 220
   Gln Ser His His Ile Pro Ser Asn Pro Asn Glu Leu Ile Arg Asn Gln
   225                 230                 235                 240
   Leu Ser Pro Val Asn Ser Glu Glu Leu Arg Gly Ile Lys Asn Lys Ala
                   245                 250                 255
   Ile Ser Gln Tyr Ser Glu Thr Arg Lys Gly Lys Glu Met Leu Glu Ser
               260                 265                 270
   Ser Leu His His Thr Asp Gln Phe Asn Gln Phe Ile Arg Ser Lys Glu
           275                 280                 285
   Asn Val Ile His Gln Gly Glu Glu Asn Ser Leu Val Val Ala Pro His
       290                 295                 300
   Glu Ser Ser Leu Ser His Leu Gln Ser Asp Leu Asn Glu Ser Asp Gly
   305                 310                 315                 320
   Arg Arg Phe Lys Asn Ser Lys Asn Gly Asp Lys Gly Asp Ser His Ser
                   325                 330                 335
   Gln Ile Ala Thr Ser Ser Arg Arg Ile Pro Pro Lys Lys His Arg Phe
               340                 345                 350
   Lys Thr Ser Leu Arg Ser Leu Pro Val Asp Glu Asn Ser Asn Asp
           355                 360                 365
<210> 17
   <211> 316
   <212> DNA
   <213> Phakopsora pachyrhizi
<220>
   <221> misc_feature
   <222> (1)..(316)
   <223> 09-j15
<400> 17
   aggatttcag caaaattttg ctgaaggaac tcttgagttt aaacttccta atcgttcttt        60
   aggtaataat caagctgcat tcaaaactct atcgtcgtac tccaaagaca acaacaactt       120
   ggaggcaggc ctcgtttggc cattaagtga accaaagatc acaatcatta aacttgacct       180
   caaacagtta ttagaagaat ttaaaacaaa attaacagaa gcgcttacga ttattgagga       240
   actgtgcagc tataaaccga acacttataa agaaatcagc aatcaaataa tacaagttta       300
   taaacatatt acccga                                                       316
<210> 18
   <211> 157
   <212> PRT
   <213> Phakopsora pachyrhizi
<220>
   <221> VARIANT
   <222> (1)..(157)
   <223> 09-j15
<400> 18
   Met His Leu His Ser Leu Thr Ile Lys Leu Ser Met Arg Leu Leu Tyr
   1               5                   10                  15
   Phe Cys Ser Ala Trp Phe Ser Leu Ile Phe Asn Asn Phe Ser Cys Phe
               20                  25                  30
   Asn Asp Phe Val Pro Ser Glu Phe Thr Arg Asp Leu Gln Leu Gly Lys
           35                  40                  45
   Gly Asn Phe Arg Gly Phe Gln Gln Asn Phe Ala Glu Gly Thr Leu Glu
       50                  55                  60
   Phe Lys Leu Pro Asn Arg Ser Leu Gly Asn Asn Gln Ala Ala Phe Lys
   65                  70                  75                  80
   Thr Leu Ser Ser Tyr Ser Lys Asp Asn Asn Asn Leu Glu Ala Gly Leu
                   85                  90                  95
   Val Trp Pro Leu Ser Glu Pro Lys Ile Thr Ile Ile Lys Leu Asp Leu
               100                 105                 110
   Lys Gln Leu Leu Glu Glu Phe Lys Thr Lys Leu Thr Glu Ala Leu Thr
           115                 120                 125
   Ile Ile Glu Glu Leu Cys Ser Tyr Lys Pro Asn Thr Tyr Lys Glu Ile
       130                 135                 140
   Ser Asn Gln Ile Ile Gln Val Tyr Lys His Ile Thr Arg
                   145                 150                 155
<210> 19
   <211> 183
   <212> DNA
   <213> Phakopsora pachyrhizi
<220>
   <221> misc_feature
   <222> (1)..(183)
   <223> 10-c2
<400> 19
   atgctggttc atgtaagatt tgtagcaatg aggctggtgc attttatctt tgttccacct        60
   agacgacaaa agaattcatt catgtactta caggcgacgc atgctaaatg cgcattttgt       120
   tattttctct ctaatgcttc ttctctttat caatattctc agaccttttt attttggtat        180
   taa                                                                     183
<210> 20
   <211> 60
   <212> PRT
   <213> Phakopsora pachyrhizi
<220>
   <221> VARIANT
   <222> (1)..(60)
   <223> 10-c2
<400> 20
   Met Leu Val His Val Arg Phe Val Ala Met Arg Leu Val His Phe Ile
   1               5                   10                  15
   Phe Val Pro Pro Arg Arg Gln Lys Asn Ser Phe Met Tyr Leu Gln Ala
               20                  25                  30
   Thr His Ala Lys Cys Ala Phe Cys Tyr Phe Leu Ser Asn Ala Ser Ser
           35                  40                  45
   Leu Tyr Gln Tyr Ser Gln Thr Phe Leu Phe Trp Tyr
       50                  55                  60
<210> 21
   <211> 258
   <212> DNA
   <213> Phakopsora pachyrhizi
<220>
   <221> misc_feature
   <222> (1)..(258)
   <223> 10-e18
<400> 21
   atgatttttt ggaaaatatt tatatgcttg tgcttagttg aatttaacat aatattaatt       60
   gttgcagcat tatcagaggg tagctctgtc aaaaccttag aggatataat tgagggctct      120
   aagttacaaa actctctggc gggccagcaa aaccttgctt ttcgttcaaa taacgcagat      180
   aacatcggga atcgattaca aggcaaacat caaattagct tcaccactag agatgtagat      240
   atcactccgt tccccacc                                                    258
<210> 22
   <211> 86
   <212> PRT
   <213> Phakopsora pachyrhizi
<220>
   <221> VARIANT
   <222> (1)..(86)
   <223> 10-e18
<400> 22
   Met Ile Phe Trp Lys Ile Phe Ile Cys Leu Cys Leu Val Glu Phe Asn
   1               5                   10                  15
   Ile Ile Leu Ile Val Ala Ala Leu Ser Glu Gly Ser Ser Val Lys Thr
               20                  25                  30
   Leu Glu Asp Ile Ile Glu Gly Ser Lys Leu Gln Asn Ser Leu Ala Gly
           35                  40                  45
   Gln Gln Asn Leu Ala Phe Arg Ser Asn Asn Ala Asp Asn Ile Gly Asn
       50                  55                  60
   Arg Leu Gln Gly Lys His Gln Ile Ser Phe Thr Thr Arg Asp Val Asp
   65                  70                  75                  80
   Ile Thr Pro Phe Pro Thr
                   85
<210> 23
   <211> 966
   <212> DNA
   <213> Phakopsora pachyrhizi
<220>
   <221> misc_feature
   <222> (1)..(966)
   <223> 10-g13
<400> 23
   atggtttttc taaaatgtat gacaattttc atctttggtc tcttggaatt aaaccaaata      60
   atttgtacag tcgaaccagc gcttttaaat tttaaccgga tcaagaaatc ttatgatgaa     120
   gctgatgtgc gagtgtttat gcttgactat gatggaacac taccaatagc ccctaaatcg     180
   gatgccaatt accttaggaa tcttataggt caacttgcaa aagatgataa gaatttagta     240
   tacataaaca ctgctcgtcc tatctactat gcaatcaaag aatttgaaaa tcttaaaaat     300
   gtaataattg ttggtgaact tgggttgtac caagcaaaag ctactgaaga agtactagaa     360
   gctgaaaaaa ataatcaatt ttggatagtc aataacgata ttgttcagtc tacaatcaaa     420
   agacttatta aatcaggcca aataaataat ctgactacat ttaagtgtag gatggttggt     480
   catggaactt ttggaggaat aattgtcaca gataagaatc tttctgtaaa tgaaaaagaa     540
   gaaatttatc agaaggttgt taaagagtta agagaagaga tcggtgatgt aaggtttcaa     600
   cctgctcagg gaacctcaaa tgataaatat cttactagta taggagttga tctatctgga     660
   gagaacaaat acagggttga tgtatctggt gaatttataa atataatacc taaggatcaa     720
   cacaagggga agtttgtgaa ggattttatg aaaaaatttg tcaaagagaa cagtggaaag     780
   aggatctttg gtctaagtat tggaaatgag gatgcagatg aggcaatgca ccttgtcatg     840
   aattcttatg gactatactc agcacttgtt ggtgatttag tagacttaca agaaaccttg     900
   gcaacttttc acctagcaaa tgaacaggaa actggacagc tcatcctaca gctttcaagt     960
   cactga                                                                966
<210> 24
   <211> 321
   <212> PRT
   <213> Phakopsora pachyrhizi
<220>
   <221> VARIANT
   <222> (1)..(321)
   <223> 10-g13
<400> 24
   Met Val Phe Leu Lys Cys Met Thr Ile Phe Ile Phe Gly Leu Leu Glu
   1               5                   10                  15
   Leu Asn Gln Ile Ile Cys Thr Val Glu Pro Ala Leu Leu Asn Phe Asn
               20                  25                  30
   Arg Ile Lys Lys Ser Tyr Asp Glu Ala Asp Val Arg Val Phe Met Leu
           35                  40                  45
   Asp Tyr Asp Gly Thr Leu Pro Ile Ala Pro Lys Ser Asp Ala Asn Tyr
       50                  55                  60
   Leu Arg Asn Leu Ile Gly Gln Leu Ala Lys Asp Asp Lys Asn Leu Val
   65                  70                  75                  80
   Tyr Ile Asn Thr Ala Arg Pro Ile Tyr Tyr Ala Ile Lys Glu Phe Glu
                   85                  90                  95
   Asn Leu Lys Asn Val Ile Ile Val Gly Glu Leu Gly Leu Tyr Gln Ala
               100                 105                 110
   Lys Ala Thr Glu Glu Val Leu Glu Ala Glu Lys Asn Asn Gln Phe Trp
           115                 120                 125
   Ile Val Asn Asn Asp Ile Val Gln Ser Thr Ile Lys Arg Leu Ile Lys
       130                 135                 140
   Ser Gly Gln Ile Asn Asn Leu Thr Thr Phe Lys Cys Arg Met Val Gly
   145                 150                 155                 160
   His Gly Thr Phe Gly Gly Ile Ile Val Thr Asp Lys Asn Leu Ser Val
                   165                 170                 175
   Asn Glu Lys Glu Glu Ile Tyr Gln Lys Val Val Lys Glu Leu Arg Glu
               180                 185                 190
   Glu Ile Gly Asp Val Arg Phe Gln Pro Ala Gln Gly Thr Ser Asn Asp
           195                 200                 205
   Lys Tyr Leu Thr Ser Ile Gly Val Asp Leu Ser Gly Glu Asn Lys Tyr
       210                 215                 220
   Arg Val Asp Val Ser Gly Glu Phe Ile Asn Ile Ile Pro Lys Asp Gln
   225                 230                 235                 240
   His Lys Gly Lys Phe Val Lys Asp Phe Met Lys Lys Phe Val Lys Glu
                   245                 250                 255
   Asn Ser Gly Lys Arg Ile Phe Gly Leu Ser Ile Gly Asn Glu Asp Ala
               260                 265                 270
   Asp Glu Ala Met His Leu Val Met Asn Ser Tyr Gly Leu Tyr Ser Ala
           275                 280                 285
   Leu Val Gly Asp Leu Val Asp Leu Gln Glu Thr Leu Ala Thr Phe His
       290                 295                 300
   Leu Ala Asn Glu Gln Glu Thr Gly Gln Leu Ile Leu Gln Leu Ser Ser
   305                 310                 315                 320
   His
<210> 25
   <211> 465
   <212> DNA
   <213> Phakopsora pachyrhizi
<220>
   <221> misc_feature
   <222> (1)..(465)
   <223> 12-L21
<400> 25
   atgcaatcca actttctcct cgctttttgc ttgattgctg ctgtctctgt tccagttagc      60
   agggctcacg gtgttattac ttcagttgag ggcgcaaatg gtcaaactgg atctgccttt     120
   ggaatggtag agtccactcc tcgtgatggc actcgaacga acccattcca aactgacagt     180
   tccatcattc gagaccgtga ggtttcaagc ggaaaggcgt ctgcttgtgg acgcactctt     240
   gcgggcggaa acaacgatat tgctagtgac atgtcatcgg ctgaatctgc cggattggcc     300
   agtattggac ctgacggaaa agtcagaatg actcttcacc aggtcaacgg tgacggtgga     360
   ggcccataca gctgtgaagt tgacaccacc gccactggtg acaacttcaa gaaaatgaac     420
   atcgacacaa acgttcctgg caagaacagt cgatcaaggg catag                     465
<210> 26
   <211> 154
   <212> PRT
   <213> Phakopsora pachyrhizi
<220>
   <221> VARIANT
   <222> (1)..(154)
   <223> 12-L21
<400> 26
   Met Gln Ser Asn Phe Leu Leu Ala Phe Cys Leu Ile Ala Ala Val Ser
   1               5                   10                  15
   Val Pro Val Ser Arg Ala His Gly Val Ile Thr Ser Val Glu Gly Ala
               20                  25                  30
   Asn Gly Gln Thr Gly Ser Ala Phe Gly Met Val Glu Ser Thr Pro Arg
           35                  40                  45
   Asp Gly Thr Arg Thr Asn Pro Phe Gln Thr Asp Ser Ser Ile Ile Arg
       50                  55                  60
   Asp Arg Glu Val Ser Ser Gly Lys Ala Ser Ala Cys Gly Arg Thr Leu
   65                  70                  75                  80
   Ala Gly Gly Asn Asn Asp Ile Ala Ser Asp Met Ser Ser Ala Glu Ser
                   85                  90                  95
   Ala Gly Leu Ala Ser Ile Gly Pro Asp Gly Lys Val Arg Met Thr Leu
               100                 105                 110
   His Gln Val Asn Gly Asp Gly Gly Gly Pro Tyr Ser Cys Glu Val Asp
           115                 120                 125
   Thr Thr Ala Thr Gly Asp Asn Phe Lys Lys Met Asn Ile Asp Thr Asn
       130                 135                 140
   Val Pro Gly Lys Asn Ser Arg Ser Arg Ala
   145                 150
<210> 27
   <211> 906
   <212> DNA
   <213> Phakopsora pachyrhizi
<220>
   <221> misc_feature
   <222> (1)..(906)
   <223> 15-d2
<400> 27
   atgtactcaa aaatctcaaa cctttctatt ctcttagtga cttcaagttt tattttctta      60
   aattgtgcag attgctttac cacaaatttt caatcaagga ctagtgagta caagcctgct     120
   tcaaactcag tgcagaacgt aacgataaaa gattctggag ataaaaaata ttttcctgtt     180
   cgttttgtca ctgtctacgc accggcctct caacatcaag caacgtcctc tgatcaaaac     240
   attagtcaag taaccaaagg tgaaaatgta acaagtaccc cttcaaatga gaagcagaga     300
   gatactgttt acgtcaacga atcagaagtt ggtaacttga ttccgatggc aacccaagga     360
   attcttgatg aagtctatgt gatgaataga gaattggatt ggaccaataa cgagtttccg     420
   atctacaact caacaggggg aatcgcttat acgattacca ataagataaa cggctctcag     480
   ttggcagagt cacagttcgc aatcatcggt cctgaccgtt ggttagttct aacttctgat     540
   acgaaatctg gtctctgtgg tttttcaaat gagtactctt cctccgacca cgttttatac     600
   agcttaagac caagactttt catgccagat cgttggtacc tatctggtga cttggtcagt     660
   ccactgaaag atcatgctta cgaatttcgc cggggtgctc taagttttga gggtgatata     720
   ctcactttgg gaactcatac cagacatgct aagatcagca atggtaaatt ggctcaaggg     780
   tggatcgata agaagattcc gggtggccgc acaatttccg tctttactga tggtactatt     840
   cctttgccaa acttaatttc tctgatagtg ataagcgtga caagaattaa aaaatgcggt     900
   ttttag                                                                906
<210> 28
   <211> 301
   <212> PRT
   <213> Phakopsora pachyrhizi
<220>
   <221> VARIANT
   <222> (1)..(301)
   <223> 15-d2
<400> 28
   Met Tyr Ser Lys Ile Ser Asn Leu Ser Ile Leu Leu Val Thr Ser Ser
   1               5                   10                  15
   Phe Ile Phe Leu Asn Cys Ala Asp Cys Phe Thr Thr Asn Phe Gln Ser
               20                  25                  30
   Arg Thr Ser Glu Tyr Lys Pro Ala Ser Asn Ser Val Gln Asn Val Thr
           35                  40                  45
   Ile Lys Asp Ser Gly Asp Lys Lys Tyr Phe Pro Val Arg Phe Val Thr
       50                  55                  60
   Val Tyr Ala Pro Ala Ser Gln His Gln Ala Thr Ser Ser Asp Gln Asn
   65                  70                  75                  80
   Ile Ser Gln Val Thr Lys Gly Glu Asn Val Thr Ser Thr Pro Ser Asn
                   85                  90                  95
   Glu Lys Gln Arg Asp Thr Val Tyr Val Asn Glu Ser Glu Val Gly Asn
               100                 105                 110
   Leu Ile Pro Met Ala Thr Gln Gly Ile Leu Asp Glu Val Tyr Val Met
           115                 120                 125
   Asn Arg Glu Leu Asp Trp Thr Asn Asn Glu Phe Pro Ile Tyr Asn Ser
       130                 135                 140
   Thr Gly Gly Ile Ala Tyr Thr Ile Thr Asn Lys Ile Asn Gly Ser Gln
   145                 150                 155                 160
   Leu Ala Glu Ser Gln Phe Ala Ile Ile Gly Pro Asp Arg Trp Leu Val
                   165                 170                 175
   Leu Thr Ser Asp Thr Lys Ser Gly Leu Cys Gly Phe Ser Asn Glu Tyr
               180                 185                 190
   Ser Ser Ser Asp His Val Leu Tyr Ser Leu Arg Pro Arg Leu Phe Met
           195                 200                 205
   Pro Asp Arg Trp Tyr Leu Ser Gly Asp Leu Val Ser Pro Leu Lys Asp
       210                 215                 220
   His Ala Tyr Glu Phe Arg Arg Gly Ala Leu Ser Phe Glu Gly Asp Ile
   225                 230                 235                 240
   Leu Thr Leu Gly Thr His Thr Arg His Ala Lys Ile Ser Asn Gly Lys
                   245                 250                 255
   Leu Ala Gln Gly Trp Ile Asp Lys Lys Ile Pro Gly Gly Arg Thr Ile
               260                 265                 270
   Ser Val Phe Thr Asp Gly Thr Ile Pro Leu Pro Asn Leu Ile Ser Leu
           275                 280                 285
   Ile Val Ile Ser Val Thr Arg Ile Lys Lys Cys Gly Phe
       290                 295                 300
<210> 29
   <211> 243
   <212> DNA
   <213> Phakopsora pachyrhizi
<220>
   <221> misc_feature
   <222> (1)..(243)
   <223> 15-i14
<400> 29
   atgttgttgc agaaaataat caccttaatc caaatagaaa gaatcatatt tatcaccaga      60
   cttaaaacat tcagcgaacc atatacaaaa cagaaattag ttatatatat tacaaatagc     120
   agcttaaatt ttaagcttgt gtcatgtcct gaacaatttc atttcatttg tcaacaaatg     180
   gagaatcaaa caagagctaa taagtatcta gaaaccttca atagagtaac aagccaaatt     240
   tga                                                                   243
<210> 30
   <211> 80
   <212> PRT
   <213> Phakopsora pachyrhizi
<220>
   <221> VARIANT
   <222> (1)..(80)
   <223> 15-i14
<400> 30
   Met Leu Leu Gln Lys Ile Ile Thr Leu Ile Gln Ile Glu Arg Ile Ile
   1               5                   10                  15
   Phe Ile Thr Arg Leu Lys Thr Phe Ser Glu Pro Tyr Thr Lys Gln Lys
               20                  25                  30
   Leu Val Ile Tyr Ile Thr Asn Ser Ser Leu Asn Phe Lys Leu Val Ser
           35                  40                  45
   Cys Pro Glu Gln Phe His Phe Ile Cys Gln Gln Met Glu Asn Gln Thr
       50                  55                  60
   Arg Ala Asn Lys Tyr Leu Glu Thr Phe Asn Arg Val Thr Ser Gln Ile
   65                  70                  75                  80
<210> 31
   <211> 529
   <212> DNA
   <213> Phakopsora pachyrhizi
<220>
   <221> misc_feature
   <222> (1)..(529)
   <223> 19-o21
<400> 31
   atggcaattg gggtttaaca cgatgatgat ctcacgcccc ttgtcgaccc gatcattgct      60
   ctatggtaca gtgatttcac caatatttat aaccattcta ttactgttcc tatccggact     120
   accgacggct gagaagccag ttcaagagtc aatctttatc aaatcttaca aagccaagcg     180
   atcgaactca caggaccagc agaacggaca ggggcagccg agcaatcagt cagtcaatcc     240
   tcaatttgga gaaaatgcga agaatgatga tggcggtgag agagtgtata gcggagtcca     300
   gaaggcatcg gatctaggag agggaaaagc agaggggcaa gtttggaagg actacaaaga     360
   ttatctggac caaacatcaa tcctcttacc gatccctcaa tcaatctaca ggaagattcc     420
   aacaagcata aagtctacgg ttcttttgga ctggcccatc tacagatttg atgaaaactc     480
   tgagagggct cagcatctga tcaaccagat taacgacgaa ataaattga                529
<210> 32
   <211> 174
   <212> PRT
   <213> Phakopsora pachyrhizi
<220>
   <221> VARIANT
   <222> (1)..(174)
   <223> 19-o21
<400> 32
   Met Ala Ile Gly Ser Ala Arg Asn Pro Gly Thr Pro Glu Ser Thr Arg
   1               5                   10                  15
   Ser Leu Leu Tyr Gly Thr Val Ile Ser Pro Ile Phe Ile Thr Ile Leu
               20                  25                  30
   Leu Leu Phe Leu Ser Gly Leu Pro Thr Ala Glu Lys Pro Val Gln Glu
           35                  40                  45
   Ser Ile Phe Ile Lys Ser Tyr Lys Ala Lys Arg Ser Asn Ser Gln Asp
       50                  55                  60
   Gln Gln Asn Gly Gln Gly Gln Pro Ser Asn Gln Ser Val Asn Pro Gln
   65                  70                  75                  80
   Phe Gly Glu Asn Ala Lys Asn Asp Asp Gly Gly Glu Arg Val Tyr Ser
                   85                  90                  95
   Gly Val Gln Lys Ala Ser Asp Leu Gly Glu Gly Lys Ala Glu Gly Gln
               100                 105                 110
   Val Trp Lys Asp Tyr Lys Asp Tyr Leu Asp Gln Thr Ser Ile Leu Leu
           115                 120                 125
   Pro Ile Pro Gln Ser Ile Tyr Arg Lys Ile Pro Thr Ser Ile Lys Ser
       130                 135                 140
   Thr Val Leu Leu Asp Trp Pro Ile Tyr Arg Phe Asp Glu Asn Ser Glu
   145                 150                 155                 160
   Arg Ala Gln His Leu Ile Asn Gln Ile Asn Asp Glu Ile Asn
                   165                 170
<210> 33
   <211> 876
   <212> DNA
   <213> Phakopsora pachyrhizi
<220>
   <221> misc_feature
   <222> (1)..(876)
   <223> 20-g1
<400> 33
   atgtatcact taacctttgt cctcttttca ctcttttttt tgtccgctaa cggagcttct     60
   acttcggagc caagcggaca gaccctagat tgctcccact acactggcgc gaacaccaaa    120
   gaagcaactt gtaacgaatt ccctggcaga atttgtcacg gaggttgcac tggagcagtt    180
   gtggccagta attgtacctt gaaccccggc gaagaaccaa aagatcaaac ctacaccata    240
   gcttttggaa aatcttcagc tacgatttca atctgtcgca atgagaaggg atcgtattct    300
   tgcacaggac ctatcaaagg aagcgcaaag tgctctgttt gtgttgaccc tccaacaggt    360
   tccgatcaaa gcccaaccac gcctgctcct gctccaggaa acaacggatc gacttcaaaa    420
   gaaggccaaa ctctacagtg cactcatttt actggtgcta atactcaaag cgcaacttgt    480
   aacgaggttc ctggcagagt ttgcaacaag ggttgtacct catcagtcgt tgccaccaag    540
   tgtacgttga atcctggtga ccaagagagt caacaaaact gtacccaagc tttcgggaaa    600
   tcctcagctg cgatatccat ttgcatcaat gacaaagggt cattttcatg cacgggaagc    660
   gtcagcggaa atgccacttg ctctggatgt accgatcaat catcatctgg ttcctctgaa    720
   taccctggtt cttctggaac caccaagccc cctactgacc ctacaggtgg tgaaaacaaa    780
   gaccaagaca agaaggccga agcaaccagc ttgcaattcg ccatgagctc tttctgctta    840
   gctttgtctc taatgattgg tgtcgccgtg ctctag                              876
<210> 34
   <211> 291
   <212> PRT
   <213> Phakopsora pachyrhizi
<220>
   <221> VARIANT
   <222> (1)..(291)
   <223> 20-g1
<400> 34
   Met Tyr His Leu Thr Phe Val Leu Phe Ser Leu Phe Phe Leu Ser Ala
   1               5                   10                  15
   Asn Gly Ala Ser Thr Ser Glu Pro Ser Gly Gln Thr Leu Asp Cys Ser
               20                  25                  30
   His Tyr Thr Gly Ala Asn Thr Lys Glu Ala Thr Cys Asn Glu Phe Pro
           35                  40                  45
   Gly Arg Ile Cys His Gly Gly Cys Thr Gly Ala Val Val Ala Ser Asn
       50                  55                  60
   Cys Thr Leu Asn Pro Gly Glu Glu Pro Lys Asp Gln Thr Tyr Thr Ile
   65                  70                  75                  80
   Ala Phe Gly Lys Ser Ser Ala Thr Ile Ser Ile Cys Arg Asn Glu Lys
                   85                  90                  95
   Gly Ser Tyr Ser Cys Thr Gly Pro Ile Lys Gly Ser Ala Lys Cys Ser
               100                 105                 110
   Val Cys Val Asp Pro Pro Thr Gly Ser Asp Gln Ser Pro Thr Thr Pro
           115                 120                 125
   Ala Pro Ala Pro Gly Asn Asn Gly Ser Thr Ser Lys Glu Gly Gln Thr
       130                 135                 140
   Leu Gln Cys Thr His Phe Thr Gly Ala Asn Thr Gln Ser Ala Thr Cys
   145                 150                 155                 160
   Asn Glu Val Pro Gly Arg Val Cys Asn Lys Gly Cys Thr Ser Ser Val
                   165                 170                 175
   Val Ala Thr Lys Cys Thr Leu Asn Pro Gly Asp Gln Glu Ser Gln Gln
               180                 185                 190
   Asn Cys Thr Gln Ala Phe Gly Lys Ser Ser Ala Ala Ile Ser Ile Cys
           195                 200                 205
   Ile Asn Asp Lys Gly Ser Phe Ser Cys Thr Gly Ser Val Ser Gly Asn
       210                 215                 220
   Ala Thr Cys Ser Gly Cys Thr Asp Gln Ser Ser Ser Gly Ser Ser Glu
   225                 230                 235                 240
   Tyr Pro Gly Ser Ser Gly Thr Thr Lys Pro Pro Thr Asp Pro Thr Gly
                   245                 250                 255
   Gly Glu Asn Lys Asp Gln Asp Lys Lys Ala Glu Ala Thr Ser Leu Gln
               260                 265                 270
   Phe Ala Met Ser Ser Phe Cys Leu Ala Leu Ser Leu Met Ile Gly Val
           275                 280                 285
   Ala Val Leu
       290
<210> 35
   <211> 270
   <212> DNA
   <213> Phakopsora pachyrhizi
<220>
   <221> misc_feature
   <222> (1)..(270)
   <223> 20-h7
<400> 35
   atgtgtggcc tgaaaatgag agctacatgt agatcggtta agttggggag ctgcaggtgt        60
   ttgggatgtg gaacaatgct caagtgtagg aggaattggt tgcccaatag gatgtgtcag       120
   ttgagaagta gaaggttgtg gcaaaatagt acctctacct ctccctcttc ccctacctct       180
   actaatccct ctaccagtac ctcttcctct accactcata ttctgattat ttgccattta       240
   tatgcatata aaaataatta cctaatgtag                                       270
<210> 36
   <211> 89
   <212> PRT
   <213> Phakopsora pachyrhizi
<220>
   <221> VARIANT
   <222> (1)..(89)
   <223> 20-h7
<400> 36
   Met Cys Gly Leu Lys Met Arg Ala Thr Cys Arg Ser Val Lys Leu Gly
   1               5                   10                  15
   Ser Cys Arg Cys Leu Gly Cys Gly Thr Met Leu Lys Cys Arg Arg Asn
               20                  25                  30
   Trp Leu Pro Asn Arg Met Cys Gln Leu Arg Ser Arg Arg Leu Trp Gln
           35                  40                  45
   Asn Ser Thr Ser Thr Ser Pro Ser Ser Pro Thr Ser Thr Asn Pro Ser
       50                  55                  60
   Thr Ser Thr Ser Ser Ser Thr Thr His Ile Leu Ile Ile Cys His Leu
   65                  70                  75                  80
   Tyr Ala Tyr Lys Asn Asn Tyr Leu Met
                   85
<210> 37
   <211> 996
   <212> DNA
   <213> Phakopsora pachyrhizi
<220>
   <221> misc_feature
   <222> (1)..(996)
   <223> 20-j15
<400> 37
   atggtttgtc aatgggagcc ttggactcag tacggtcatg atgccgtggt gcagtatgag        60
   ggttgcttct acaagatcat tcaacctcac aggtccgagc ccaactggca acccacaaac       120
   acacctgctc tatggtcgag agttcctgat gattgctgcc atcagtacag gtctggtgag       180
   aatcaccatg ggggccaaga gcataatcga ccacagtttg tagatcaatc aggtggtgct       240
   ccaggaggag gctaccaaga aaggccccaa ggcggtggtt ggggacaaga caacaacggt       300
   ggaaatcaac tacaagaaaa tcaaggctgg ctagacgaga aaagaaaaca tcaattggag       360
   attgggggtg ggattttggg cgctgtggct atcggagccg gcgcatatgc ttacagtaag       420
   cacaagagcg aagaaaagga agaggctaag aaacaagctt ggcaaaacga atctaatcat       480
   gaaacgtggt tgagaggtgc acatgcgcat actcaacatt acatgtctgg tggacaaagt      540
   ccaccagtct attgggtttt ggtcgacaga aatgatccga tacctaataa cgctatcgaa      600
   ggcggacgtg aaggcggtca ctcactttac attggtaggg tgttcttcaa gtccggtcta      660
   catatcggaa aagtgtctag ccatgtaggt ggtattgcta tcggatgggg tggcaaggag      720
   cacaatgact ttgataagtt tgaggttctc tgtggtgaca atcgtgccat tcgttggata      780
   aatcactcat cgcaccaagg aaatattact gtccaaggtt ggcagcccgt agaaggagga      840
   agagaggcag atggaagatt cttgtttgtt agtcaagtcc aacatgatgg tggtgttcat      900
   ccttgcaagg cccaagatac caccgatttt gcgatctttt cttacggagg taaagaacac      960
   acctccaaag aattcaacat cttggcttat gcgtga                                996
<210> 38
   <211> 331
   <212> PRT
   <213> Phakopsora pachyrhizi
<220>
   <221> VARIANT
   <222> (1)..(331)
   <223> 20-j15
<400> 38
   Met Val Cys Gln Trp Glu Pro Trp Thr Gln Tyr Gly His Asp Ala Val
   1               5                   10                  15
   Val Gln Tyr Glu Gly Cys Phe Tyr Lys Ile Ile Gln Pro His Arg Ser
               20                  25                  30
   Glu Pro Asn Trp Gln Pro Thr Asn Thr Pro Ala Leu Trp Ser Arg Val
           35                  40                  45
   Pro Asp Asp Cys Cys His Gln Tyr Arg Ser Gly Glu Asn His His Gly
       50                  55                  60
   Gly Gln Glu His Asn Arg Pro Gln Phe Val Asp Gln Ser Gly Gly Ala
   65                  70                  75                  80
   Pro Gly Gly Gly Tyr Gln Glu Arg Pro Gln Gly Gly Gly Trp Gly Gln
                   85                  90                  95
   Asp Asn Asn Gly Gly Asn Gln Leu Gln Glu Asn Gln Gly Trp Leu Asp
               100                 105                 110
   Glu Lys Arg Lys His Gln Leu Glu Ile Gly Gly Gly Ile Leu Gly Ala
           115                 120                 125
   Val Ala Ile Gly Ala Gly Ala Tyr Ala Tyr Ser Lys His Lys Ser Glu
       130                 135                 140
   Glu Lys Glu Glu Ala Lys Lys Gln Ala Trp Gln Asn Glu Ser Asn His
   145                 150                 155                 160
   Glu Thr Trp Leu Arg Gly Ala His Ala His Thr Gln His Tyr Met Ser
                   165                 170                 175
   Gly Gly Gln Ser Pro Pro Val Tyr Trp Val Leu Val Asp Arg Asn Asp
               180                 185                 190
   Pro Ile Pro Asn Asn Ala Ile Glu Gly Gly Arg Glu Gly Gly His Ser
           195                 200                 205
   Leu Tyr Ile Gly Arg Val Phe Phe Lys Ser Gly Leu His Ile Gly Lys
       210                 215                 220
   Val Ser Ser His Val Gly Gly Ile Ala Ile Gly Trp Gly Gly Lys Glu
   225                 230                 235                 240
   His Asn Asp Phe Asp Lys Phe Glu Val Leu Cys Gly Asp Asn Arg Ala
                   245                 250                 255
   Ile Arg Trp Ile Asn His Ser Ser His Gln Gly Asn Ile Thr Val Gln
               260                 265                 270
   Gly Trp Gln Pro Val Glu Gly Gly Arg Glu Ala Asp Gly Arg Phe Leu
           275                 280                 285
   Phe Val Ser Gln Val Gln His Asp Gly Gly Val His Pro Cys Lys Ala
       290                 295                 300
   Gln Asp Thr Thr Asp Phe Ala Ile Phe Ser Tyr Gly Gly Lys Glu His
   305                 310                 315                 320
   Thr Ser Lys Glu Phe Asn Ile Leu Ala Tyr Ala
                   325                 330
<210> 39
   <211> 255
   <212> DNA
   <213> Phakopsora pachyrhizi
<220>
   <221> misc_feature
   <222> (1)..(255)
   <223> 21-b5
<400> 39
   atgagctata ggctgagggt ccagggtttt ttggtgaaga tatcagccag ttggggggtt      60
   tttttttttt taaaaaaaaa aaaaaaaatt atacttttat ttcaaagtta tagaataatg     120
   atttgtgtaa taaaattctc atatgatatg ttagattttt ttactgaaaa tttttatcta     180
   tgtaaatttg agttgaaact agtgttatca cattctaata ttattaagtc taaggtgata     240
   aaaccagcca gatag                                                      255
<210> 40
   <211> 84
   <212> PRT
   <213> Phakopsora pachyrhizi
<220>
   <221> VARIANT
   <222> (1)..(84)
   <223> 21-b5
<400> 40
   Met Ser Tyr Arg Leu Arg Val Gln Gly Phe Leu Val Lys Ile Ser Ala
   1               5                   10                  15
   Ser Trp Gly Val Phe Phe Phe Leu Lys Lys Lys Lys Lys Ile Ile Leu
               20                  25                  30
   Leu Phe Gln Ser Tyr Arg Ile Met Ile Cys Val Ile Lys Phe Ser Tyr
           35                  40                  45
   Asp Met Leu Asp Phe Phe Thr Glu Asn Phe Tyr Leu Cys Lys Phe Glu
       50                  55                  60
   Leu Lys Leu Val Leu Ser His Ser Asn Ile Ile Lys Ser Lys Val Ile
   65                  70                  75                  80
   Lys Pro Ala Arg
<210> 41
   <211> 330
   <212> DNA
   <213> Phakopsora pachyrhizi
<220>
   <221> misc_feature
   <222> (1)..(330)
   <223> 21-k6
<400> 41
   atgttcgaat atcgaataaa attttcaaaa aaactgttcc tctttgtaat aagtagcttt      60
   attactctaa actttcaact attagaagct aagaaaccgg cccatggact tgcaaagatt     120
   ggcaatagct cagtaacagg atcgaagtta aaacctcctc gtcgagttgt accgctcgga     180
   attcttcccg tagtgacaca tactagcaca cccttagaaa gagtacaaaa ggcacaacac     240
   catcacgaat ttcacaggaa tagacctcaa aatagagatt ctacattaca ttctagtatc     300
   aagcacacca ggaaatctta ttctccctaa                                      330
<210> 42
   <211> 109
   <212> PRT
   <213> Phakopsora pachyrhizi
<220>
   <221> VARIANT
   <222> (1)..(109)
   <223> 21-k6
<400> 42
   Met Phe Glu Tyr Arg Ile Lys Phe Ser Lys Lys Leu Phe Leu Phe Val
   1               5                   10                  15
   Ile Ser Ser Phe Ile Thr Leu Asn Phe Gln Leu Leu Glu Ala Lys Lys
               20                  25                  30
   Pro Ala His Gly Leu Ala Lys Ile Gly Asn Ser Ser Val Thr Gly Ser
           35                  40                  45
   Lys Leu Lys Pro Pro Arg Arg Val Val Pro Leu Gly Ile Leu Pro Val
       50                  55                  60
   Val Thr His Thr Ser Thr Pro Leu Glu Arg Val Gln Lys Ala Gln His
   65                  70                  75                  80
   His His Glu Phe His Arg Asn Arg Pro Gln Asn Arg Asp Ser Thr Leu
                   85                  90                  95
   His Ser Ser Ile Lys His Thr Arg Lys Ser Tyr Ser Pro
               100                 105
<210> 43
   <211> 876
   <212> DNA
   <213> Phakopsora pachyrhizi
<220>
   <221> misc_feature
   <222> (1)..(876)
   <223> 30-L16
<400> 43
   atgttgcact tttctagttt aacttctcta ggaatttttg cgtgccaaac tgcttggctc      60
   gttgcagttt caaacgcctt cgtagcggaa catgcagtta tccaggacat aaccaatctt     120
   gcttttagtc ctaataagat tgtagataga acacttggac aaaaaaaccc aaattttgcc     180
   aagttcgtca atgcagaacc attactttat cgtgaaatgg ctcgcaaagc tggacgatct     240
   gttggaacag tcttaactga ctctcgagga accaatcttc ctctactcat ccgtgacctt     300
   cgagtcaccg actggattca tagcgatccg gtaattaagg gaatgccacc aaacaatcta     360
   cctcaaaatg ctattgaact tgcccagaac tccttcgcat ctcgtctagt ggacattcgg     420
   aacgcaaatg ataaagccaa gttcgaccag cacgcattaa tagcacgtaa ccacttagct     480
   aaggtactgt tttacatcat aagtgagatg catatatcta tgcttccgaa acttgatgta     540
   gtcaatgtag ctgcgtggca gaaagaaagg gcaggccgcg tcgctctttt gatgacacaa     600
   aatcctttcc tcaagcggct ttattccgtc caccaacaaa tcggaaatcc ggatcaatac     660
   attactcctg agactgcgga tgcagctttt atgaaacttt ttcctcatca aaattcaaac     720
   aaacagggtc atccttcaca atcggcaaca tctgaaaaaa tgaaaactca ccctgagggc     780
   gaaggaaact ttcctgccca tcgccctgtg gatgaaaata aagagggcat tgtccctgga     840
   actcacccta atgctgcaac tgtttcttct tcttaa                               876
<210> 44
   <211> 291
   <212> PRT
   <213> Phakopsora pachyrhizi
<220>
   <221> VARIANT
   <222> (1)..(291)
   <223> 30-L16
<400> 44
   Met Leu His Phe Ser Ser Leu Thr Ser Leu Gly Ile Phe Ala Cys Gln
   1               5                   10                  15
   Thr Ala Trp Leu Val Ala Val Ser Asn Ala Phe Val Ala Glu His Ala
               20                  25                  30
   Val Ile Gln Asp Ile Thr Asn Leu Ala Phe Ser Pro Asn Lys Ile Val
           35                  40                  45
   Asp Arg Thr Leu Gly Gln Lys Asn Pro Asn Phe Ala Lys Phe Val Asn
       50                  55                  60
   Ala Glu Pro Leu Leu Tyr Arg Glu Met Ala Arg Lys Ala Gly Arg Ser
   65                  70                  75                  80
   Val Gly Thr Val Leu Thr Asp Ser Arg Gly Thr Asn Leu Pro Leu Leu
                   85                  90                  95
   Ile Arg Asp Leu Arg Val Thr Asp Trp Ile His Ser Asp Pro Val Ile
               100                 105                 110
   Lys Gly Met Pro Pro Asn Asn Leu Pro Gln Asn Ala Ile Glu Leu Ala
           115                 120                 125
   Gln Asn Ser Phe Ala Ser Arg Leu Val Asp Ile Arg Asn Ala Asn Asp
       130                 135                 140
   Lys Ala Lys Phe Asp Gln His Ala Leu Ile Ala Arg Asn His Leu Ala
   145                 150                 155                 160
   Lys Val Leu Phe Tyr Ile Ile Ser Glu Met His Ile Ser Met Leu Pro
                   165                 170                 175
   Lys Leu Asp Val Val Asn Val Ala Ala Trp Gln Lys Glu Arg Ala Gly
               180                 185                 190
   Arg Val Ala Leu Leu Met Thr Gln Asn Pro Phe Leu Lys Arg Leu Tyr
           195                 200                 205
   Ser Val His Gln Gln Ile Gly Asn Pro Asp Gln Tyr Ile Thr Pro Glu
       210                 215                 220
   Thr Ala Asp Ala Ala Phe Met Lys Leu Phe Pro His Gln Asn Ser Asn
   225                 230                 235                 240
   Lys Gln Gly His Pro Ser Gln Ser Ala Thr Ser Glu Lys Met Lys Thr
                   245                 250                 255
   His Pro Glu Gly Glu Gly Asn Phe Pro Ala His Arg Pro Val Asp Glu
               260                 265                 270
   Asn Lys Glu Gly Ile Val Pro Gly Thr His Pro Asn Ala Ala Thr Val
           275                 280                 285
   Ser Ser Ser
       290
<210> 45
   <211> 936
   <212> DNA
   <213> Phakopsora pachyrhizi
<220>
   <221> misc_feature
   <222> (1)..(936)
   <223> 28-L20
<400> 45
   atgagaatca gtttatacac cctcatatgc gtttctttaa accttcatag cctaggttgt      60
   gttaattttc tccaaatttc ccacaatcca acatttacgt tgggtaataa tgaaattagt     120
   gttttgccac acgcggtaaa tcaaccttgg ttacgaagcc aacgcacgat aattactcca     180
   gagggtgagg ttactgtgaa tatgctgcgt ggccataatt ctgttgatca gaaatcgcta     240
   caaatgcaaa acgttataat accttataga gacttgatct gcagctatga aagatcagac     300
   tctttagcac tgagcaaatc tacgcaaggt caatacaaac aattagctca agagttagat     360
   aacttcaaga atgagaagga aggaaaatca atggaagctt tagaatcatt ggcgaaaaaa     420
   acttttaata aactaaagtt tcatcatcct aactcggacg gtgaaacttt tgaaaatgct     480
   aagctgatgg cggaatggac cataaataga gcagcttcaa agcaaggcgt ttggtggagc     540
   aaaaaggact tgacacgctg gaacaaaaat tcgattgata aaatgacagc tataaagatt     600
   ggcgatcttc tagactttga cattatgact tccaatcata agctctcatt aaccaacaaa     660
   ttaaaagatg atttgtctga agctttaaag tctctcaaac tagaaggact ttcgacggag     720
   ggaagccctc tactactttc accagttata attgatttac gtaagtttaa ggcaaacgat     780
   ggtcagactt tatttgacat ccgccttaga caatttcaag aactacagga gaaaccaagt     840
   agttcaatgc ggggggcaaa tgcgattctc aaacagcctc aaatcaatgg ggatgaccaa      900
   attataaaaa ctctatgctc ttatctatca acttag                               936
<210> 46
   <211> 311
   <212> PRT
   <213> Phakopsora pachyrhizi
<220>
   <221> VARIANT
   <222> (1)..(311)
   <223> 28-L20
<400> 46
   Met Arg Ile Ser Leu Tyr Thr Leu Ile Cys Val Ser Leu Asn Leu His
   1               5                   10                  15
   Ser Leu Gly Cys Val Asn Phe Leu Gln Ile Ser His Asn Pro Thr Phe
               20                  25                  30
   Thr Leu Gly Asn Asn Glu Ile Ser Val Leu Pro His Ala Val Asn Gln
           35                  40                  45
   Pro Trp Leu Arg Ser Gln Arg Thr Ile Ile Thr Pro Glu Gly Glu Val
       50                  55                  60
   Thr Val Asn Met Leu Arg Gly His Asn Ser Val Asp Gln Lys Ser Leu
   65                  70                  75                  80
   Gln Met Gln Asn Val Ile Ile Pro Tyr Arg Asp Leu Ile Cys Ser Tyr
                   85                  90                  95
   Glu Arg Ser Asp Ser Leu Ala Leu Ser Lys Ser Thr Gln Gly Gln Tyr
               100                 105                 110
   Lys Gln Leu Ala Gln Glu Leu Asp Asn Phe Lys Asn Glu Lys Glu Gly
           115                 120                 125
   Lys Ser Met Glu Ala Leu Glu Ser Leu Ala Lys Lys Thr Phe Asn Lys
       130                 135                 140
   Leu Lys Phe His His Pro Asn Ser Asp Gly Glu Thr Phe Glu Asn Ala
   145                 150                 155                 160
   Lys Leu Met Ala Glu Trp Thr Ile Asn Arg Ala Ala Ser Lys Gln Gly
                   165                 170                 175
   Val Trp Trp Ser Lys Lys Asp Leu Thr Arg Trp Asn Lys Asn Ser Ile
               180                 185                 190
   Asp Lys Met Thr Ala Ile Lys Ile Gly Asp Leu Leu Asp Phe Asp Ile
           195                 200                 205
   Met Thr Ser Asn His Lys Leu Ser Leu Thr Asn Lys Leu Lys Asp Asp
       210                 215                 220
   Leu Ser Glu Ala Leu Lys Ser Leu Lys Leu Glu Gly Leu Ser Thr Glu
   225                 230                 235                 240
   Gly Ser Pro Leu Leu Leu Ser Pro Val Ile Ile Asp Leu Arg Lys Phe
                   245                 250                 255
   Lys Ala Asn Asp Gly Gln Thr Leu Phe Asp Ile Arg Leu Arg Gln Phe
               260                 265                 270
   Gln Glu Leu Gln Glu Lys Pro Ser Ser Ser Met Arg Gly Ala Asn Ala
           275                 280                 285
   Ile Leu Lys Gln Pro Gln Ile Asn Gly Asp Asp Gln Ile Ile Lys Thr
       290                 295                 300
   Leu Cys Ser Tyr Leu Ser Thr
   305                 310
<210> 47
   <211> 1047
   <212> DNA
   <213> Phakopsora pachyrhizi
<220>
   <221> misc_feature
   <222> (1)..(1047)
   <223> 26-k11
<400> 47
   atgcactcga gagcatctta ttggtcgagc tttgccttag ctctagccac aataactatc      60
   ccctctcctg cgacgagcaa acaatgcgct gcggatccca ctaacacggg ttgcgttatg     120
   tgcccggaat ctgaactaat ggaatggtgg aacaaaggcc ttgaacacaa caaaaagtgc     180
   agcgatgttg atgatgttac caagaaggcg aaatgttact gcaaaggata cgctacctta     240
   gcctacgctt ggtctaaagg cagatgctgc actgaatact cccccacatt acaagcctcg     300
   gaaaatatct gcaatcttgc tacctatccc gaatatcata taaagatgca tttagatcat     360
   catttaaccg acgctaagcg agtgcaagac ggtatctggc acaatgactc tggtgttaaa     420
   gtgcccaaag tctacgagca cgttgatgtt tacaggggcg ataaccctga ggttggtgaa     480
   ggctactcag aaaaagacgc aaaagcccaa tcctctgaaa aaacttaccg ggaatccaag     540
   gttgttaaga ctcatcaatc cgcccccaat tctttggagg accaatccac gaaaaaggag     600
   ctatcttcat atagcgggca acaagctacc aattttgggt cgcaagcacc ccgggtcaat     660
   tttatgagcg aggggaagtc taatgtagaa aaacataaac cttcaagctg gacagagaaa     720
   cgtaataaaa agcatgttca gtataacccc tcgtcctgga gctaccctca tgctcgtctc     780
   tatgctggca ctaggcatca caaagctaga gtgagcaaaa agccccgcaa ttgtgtcaat     840
   gagcaggaga agtatcacaa ttatgttaaa tatcattcta agcgatccac tttggaaaca     900
   aagccaaccc taggcatcac gaaagcagag tcattaattg cgaagaaact gcaaaagcga     960
   ggtgtgattt gtgggataga ttttcaaaac gttccatatc ctaaatctgt acctacagct    1020
   gtgcttaagg tttatgctga actttga                                       1047
<210> 48
   <211> 348
   <212> PRT
   <213> Phakopsora pachyrhizi
<220>
   <221> VARIANT
   <222> (1)..(348)
   <223> 26-k11
<400> 48
   Met His Ser Arg Ala Ser Tyr Trp Ser Ser Phe Ala Leu Ala Leu Ala
   1               5                   10                  15
   Thr Ile Thr Ile Pro Ser Pro Ala Thr Ser Lys Gln Cys Ala Ala Asp
               20                  25                  30
   Pro Thr Asn Thr Gly Cys Val Met Cys Pro Glu Ser Glu Leu Met Glu
           35                  40                  45
   Trp Trp Asn Lys Gly Leu Glu His Asn Lys Lys Cys Ser Asp Val Asp
       50                  55                  60
   Asp Val Thr Lys Lys Ala Lys Cys Tyr Cys Lys Gly Tyr Ala Thr Leu
   65                  70                  75                  80
   Ala Tyr Ala Trp Ser Lys Gly Arg Cys Cys Thr Glu Tyr Ser Pro Thr
                   85                  90                  95
   Leu Gln Ala Ser Glu Asn Ile Cys Asn Leu Ala Thr Tyr Pro Glu Tyr
               100                 105                 110
   His Ile Lys Met His Leu Asp His His Leu Thr Asp Ala Lys Arg Val
           115                 120                 125
   Gln Asp Gly Ile Trp His Asn Asp Ser Gly Val Lys Val Pro Lys Val
       130                 135                 140
   Tyr Glu His Val Asp Val Tyr Arg Gly Asp Asn Pro Glu Val Gly Glu
   145                 150                 155                 160
   Gly Tyr Ser Glu Lys Asp Ala Lys Ala Gln Ser Ser Glu Lys Thr Tyr
                   165                 170                 175
   Arg Glu Ser Lys Val Val Lys Thr His Gln Ser Ala Pro Asn Ser Leu
               180                 185                 190
   Glu Asp Gln Ser Thr Lys Lys Glu Leu Ser Ser Tyr Ser Gly Gln Gln
           195                 200                 205
   Ala Thr Asn Phe Gly Ser Gln Ala Pro Arg Val Asn Phe Met Ser Glu
       210                 215                 220
   Gly Lys Ser Asn Val Glu Lys His Lys Pro Ser Ser Trp Thr Glu Lys
   225                 230                 235                 240
   Arg Asn Lys Lys His Val Gln Tyr Asn Pro Ser Ser Trp Ser Tyr Pro
                   245                 250                 255
   His Ala Arg Leu Tyr Ala Gly Thr Arg His His Lys Ala Arg Val Ser
               260                 265                 270
   Lys Lys Pro Arg Asn Cys Val Asn Glu Gln Glu Lys Tyr His Asn Tyr
           275                 280                 285
   Val Lys Tyr His Ser Lys Arg Ser Thr Leu Glu Thr Lys Pro Thr Leu
       290                 295                 300
   Gly Ile Thr Lys Ala Glu Ser Leu Ile Ala Lys Lys Leu Gln Lys Arg
   305                 310                 315                 320
   Gly Val Ile Cys Gly Ile Asp Phe Gln Asn Val Pro Tyr Pro Lys Ser
                   325                 330                 335
   Val Pro Thr Ala Val Leu Lys Val Tyr Ala Glu Leu
               340                 345
<210> 49
   <211> 312
   <212> DNA
   <213> Phakopsora pachyrhizi
<220>
   <221> misc_feature
   <222> (1)..(312)
   <223> 21-L24
<400> 49
   atggttggat gtgagagcgg tgcaacgaaa aagtctatga taatcaagat gattggattc       60
   ttgtgcattt ggtttcaact attagaaata gttttgactc aatctactgc tgcaactact      120
   agtgccactg agactaaaaa ttataatagt tattttaaaa ggcttgaaaa aagaagccca      180
   tcgaccagct ctggtcgaat tgagaaaaga catcgcagat gtggaccgcc atatggggga       240
   tattatggac ctggatatag tagtggttat tataactatg gctattactc tggatataag      300
   aaaaagcaat aa                                                          312
<210> 50
   <211> 103
   <212> PRT
   <213> Phakopsora pachyrhizi
<220>
   <221> VARIANT
   <222> (1)..(103)
   <223> 21-L24
<400> 50
   Met Val Gly Cys Glu Ser Gly Ala Thr Lys Lys Ser Met Ile Ile Lys
   1               5                   10                  15
   Met Ile Gly Phe Leu Cys Ile Trp Phe Gln Leu Leu Glu Ile Val Leu
               20                  25                  30
   Thr Gln Ser Thr Ala Ala Thr Thr Ser Ala Thr Glu Thr Lys Asn Tyr
           35                  40                  45
   Asn Ser Tyr Phe Lys Arg Leu Glu Lys Arg Ser Pro Ser Thr Ser Ser
       50                  55                  60
   Gly Arg Ile Glu Lys Arg His Arg Arg Cys Gly Pro Pro Tyr Gly Gly
   65                  70                  75                  80
   Tyr Tyr Gly Pro Gly Tyr Ser Ser Gly Tyr Tyr Asn Tyr Gly Tyr Tyr
                   85                  90                  95
   Ser Gly Tyr Lys Lys Lys Gln
               100
<210> 51
   <211> 174
   <212> DNA
   <213> Phakopsora pachyrhizi
<220>
   <221> misc_feature
   <222> (1)..(174)
   <223> 21-k22
<400> 51
   atgctttcgc ttcaagtatt gcgcctgtcg ttgctctatt gtttgagtga agatttacag       60
   ctaacggagt tgttaaaaaa ttgccatgtc ttttatttgg aagctatcaa caaactagaa      120
   agatacaaga agaaacaaaa gtattttaaa attcacttca aatatttatc ctga            174
<210> 52
   <211> 57
   <212> PRT
   <213> Phakopsora pachyrhizi
<220>
   <221> VARIANT
   <222> (1)..(57)
   <223> 21-k22
<400> 52
   Met Leu Ser Leu Gln Val Leu Arg Leu Ser Leu Leu Tyr Cys Leu Ser
   1               5                   10                  15
   Glu Asp Leu Gln Leu Thr Glu Leu Leu Lys Asn Cys His Val Phe Tyr
               20                  25                  30
   Leu Glu Ala Ile Asn Lys Leu Glu Arg Tyr Lys Lys Lys Gln Lys Tyr
           35                  40                  45
   Phe Lys Ile His Phe Lys Tyr Leu Ser
       50                  55
<210> 53
   <211> 636
   <212> DNA
   <213> Phakopsora pachyrhizi
<220>
   <221> misc_feature
   <222> (1)..(636)
   <223> 20-a22
<400> 53
   atgaatcacg ctttgatcat ctgtctactt ggacttgcct cctttggctt gatctcgggg      60
   cattttacca tggactatcc tccaacccga ggttttatgg aggatgacga ggataaattc     120
   tgtggaggat tccctaccaa tgctaccggt agacatccgt tcccgatatc cggtcctgct     180
   catgttgcgc tcatggctca ccacgggtcc cccaaaatca gtatttccat aagctttaag     240
   cacaatttta ctagtgaagc cgagtttaac ggcactgagt tgatatctaa cgctcctctg     300
   cctcatcctg gaaatgcttg ttttgaaatt gatctgtcga acgttactag taagatgacg     360
   cctaaacctt ccaacggaac attagcaact ctagaggtta aatatgatgc cggaggtgag     420
   gctttgtacc agtgttcaga yttagttctg gtccaggatg ctgcggtgac aaatcaaagc     480
   cttagcagct gctctgccgg ccgtggcagt aatggcggaa gcggctctga tgccaagcaa     540
   aatgctcaaa atagctctgc taactctttg tggttccaac ctactccgac tatgatctgc     600
   cttagtctaa ttcttttatc gctctccatt ctttaa                               636
<210> 54
   <211> 211
   <212> PRT
   <213> Phakopsora pachyrhizi
<220>
   <221> VARIANT
   <222> (1)..(211)
   <223> 20-a22
<400> 54
   Met Asn His Ala Leu Ile Ile Cys Leu Leu Gly Leu Ala Ser Phe Gly
   1               5                   10                  15
   Leu Ile Ser Gly His Phe Thr Met Asp Tyr Pro Pro Thr Arg Gly Phe
               20                  25                  30
   Met Glu Asp Asp Glu Asp Lys Phe Cys Gly Gly Phe Pro Thr Asn Ala
           35                  40                  45
   Thr Gly Arg His Pro Phe Pro Ile Ser Gly Pro Ala His Val Ala Leu
       50                  55                  60
   Met Ala His His Gly Ser Pro Lys Ile Ser Ile Ser Ile Ser Phe Lys
   65                  70                  75                  80
   His Asn Phe Thr Ser Glu Ala Glu Phe Asn Gly Thr Glu Leu Ile Ser
                   85                  90                  95
   Asn Ala Pro Leu Pro His Pro Gly Asn Ala Cys Phe Glu Ile Asp Leu
               100                 105                 110
   Ser Asn Val Thr Ser Lys Met Thr Pro Lys Pro Ser Asn Gly Thr Leu
           115                 120                 125
   Ala Thr Leu Glu Val Lys Tyr Asp Ala Gly Gly Glu Ala Leu Tyr Gln
       130                 135                 140
   Cys Ser Asp Leu Val Leu Val Gln Asp Ala Ala Val Thr Asn Gln Ser
   145                 150                 155                 160
   Leu Ser Ser Cys Ser Ala Gly Arg Gly Ser Asn Gly Gly Ser Gly Ser
                   165                 170                 175
   Asp Ala Lys Gln Asn Ala Gln Asn Ser Ser Ala Asn Ser Leu Trp Phe
               180                 185                 190
   Gln Pro Thr Pro Thr Met Ile Cys Leu Ser Leu Ile Leu Leu Ser Leu
           195                 200                 205
   Ser Ile Leu
       210
<210> 55
   <211> 639
   <212> DNA
   <213> Phakopsora pachyrhizi
<220>
   <221> misc_feature
   <222> (1)..(639)
   <223> 19-o13
<400> 55
   atggccaaaa ttttgtttca atttattttg ctaataattg gcttgttaag ttcgtgtggt      60
   agagcgatgg agtcggtgga actgcctaag gtttccaagc aagctaaagt ggcttccacc    120
   tccaccatcc ccgtggtggg cgttgagcta aagaacactg gccatttcac aaatgagatt    180
   cccactcaat cgttaaattt aaactataaa tcttttaaac aagaactggg aaaagtgcag    240
   gaaaggatta aaaagcatag ctttgaaacc caaagatatc taaagaatga ctttaatccc    300
   attcaaaatt tgatcgaaaa attattgaga gtagccgaag agtacaaaaa tggagaaaaa    360
   ataagctttg actcaaagaa gcatttgaca aacattagcg catcagatat ggtattgaat    420
   ggaatcttgg atgaagttag aaagtttctg tcrgatttat ataagatacc caatatacct    480
   tggaatactg gtatgagtcg gaaatgggta atcatgtcag gcatcttgca agtactagac    540
   ccactcataa aacatggctt tttaaacaaa gtgcaaatgc agcagtggtt tgaagattca    600
   gaagtcctcc gaaatttctg cctatgttct aagcaatga                            639
<210> 56
   <211> 312
   <212> PRT
   <213> Phakopsora pachyrhizi
<220>
   <221> VARIANT
   <222> (1)..(312)
   <223> 19-o13
<400> 56
   Met Ala Lys Ile Leu Phe Gln Phe Ile Leu Leu Ile Ile Gly Leu Leu
   1               5                   10                  15
   Ser Ser Cys Gly Arg Ala Met Glu Ser Val Glu Leu Pro Lys Val Ser
               20                  25                  30
   Lys Gln Ala Lys Val Ala Ser Thr Ser Thr Ile Pro Val Val Gly Val
           35                  40                  45
   Glu Leu Lys Asn Thr Gly His Phe Thr Asn Glu Ile Pro Thr Gln Ser
       50                  55                  60
   Leu Asn Leu Asn Tyr Lys Ser Phe Lys Gln Glu Leu Gly Lys Val Gln
   65                  70                  75                  80
   Glu Arg Ile Lys Lys His Ser Phe Glu Thr Gln Arg Tyr Leu Lys Asn
                   85                  90                  95
   Asp Phe Asn Pro Ile Gln Asn Leu Ile Glu Lys Leu Leu Arg Val Ala
               100                 105                 110
   Glu Glu Tyr Lys Asn Gly Glu Lys Ile Ser Phe Asp Ser Lys Lys His
           115                 120                 125
   Leu Thr Asn Ile Ser Ala Ser Asp Met Val Leu Asn Gly Ile Leu Asp
       130                 135                 140
   Glu Val Arg Lys Phe Leu Ser Asp Leu Tyr Lys Ile Pro Asn Ile Pro
   145                 150                 155                 160
   Trp Asn Thr Gly Met Ser Arg Lys Trp Val Ile Met Ser Gly Ile Leu
                   165                 170                 175
   Gln Val Leu Asp Pro Leu Ile Lys His Gly Phe Leu Asn Lys Val Gln
               180                 185                 190
   Met Gln Gln Trp Phe Glu Asp Ser Glu Val Leu Arg Asn Ser Ala Tyr
           195                 200                 205
   Val Leu Ser Asn Glu Phe Gly Leu His Ser Ser Tyr Trp Leu Pro Ser
       210                 215                 220
   Val Glu Lys Leu Ile Asn Leu Thr Ala Asp Glu Phe Leu Trp Leu Ser
   225                 230                 235                 240
   Leu Leu Asn Glu Gln Glu Ala Lys Asn Phe Leu Arg Gln Leu Ile Gly
                   245                 250                 255
   Phe Thr Tyr Asn Ser Asn Glu Ser Cys Asn Ile Ser His Pro Lys Phe
               260                 265                 270
   Thr Pro Ile Val Asn Leu Phe Ile Gly Asp Ser Gly Val Thr Lys Asp
           275                 280                 285
   Thr Phe Asn Ala Leu Ile Glu Ala Ile Asp Ser Ser Glu Lys Phe Ser
       290                 295                 300
   Glu Ser Thr Glu Gln Glu Ser Ser
   305                 310
<210> 57
   <211> 405
   <212> DNA
   <213> Phakopsora pachyrhizi
<220>
   <221> misc_feature
   <222> (1)..(405)
   <223> 19-L7
<400> 57
   atggagcaga agaaaagttt taaagcttta gttgttcttc tatttgccct gctcaatctc        60
   ttggtatcga tcagctctgc accgatgagc aagataacaa cagagacctc aaaaatctta       120
   aacccagaga ccaacctgag cttgatcgaa aaatctacgg aaaataatag agtcgaaggc       180
   ctgactgact taaaaagtgt cgaaaataat ccagcctcca aaacacaatc tgctccaagc       240
   tatcagccac tatcagtggt tgattcttca aaatcacagg gctcaactgc acctatccta       300
   tacaattatc agacgcctac tacacaaacg ttcacatacc aaaacccgga aatcccattg       360
   tatggcaaga ttaaatatgg aactaccagt agaacttata agtag                       405
<210> 58
   <211> 134
   <212> PRT
   <213> Phakopsora pachyrhizi
<220>
   <221> VARIANT
   <222> (1)..(134)
   <223> 19-L7
<400> 58
   Met Glu Gln Lys Lys Ser Phe Lys Ala Leu Val Val Leu Leu Phe Ala
   1               5                   10                  15
   Leu Leu Asn Leu Leu Val Ser Ile Ser Ser Ala Pro Met Ser Lys Ile
               20                  25                  30
   Thr Thr Glu Thr Ser Lys Ile Leu Asn Pro Glu Thr Asn Leu Ser Leu
           35                  40                  45
   Ile Glu Lys Ser Thr Glu Asn Asn Arg Val Glu Gly Leu Thr Asp Leu
       50                  55                  60
   Lys Ser Val Glu Asn Asn Pro Ala Ser Lys Thr Gln Ser Ala Pro Ser
   65                  70                  75                  80
   Tyr Gln Pro Leu Ser Val Val Asp Ser Ser Lys Ser Gln Gly Ser Thr
                   85                  90                  95
   Ala Pro Ile Leu Tyr Asn Tyr Gln Thr Pro Thr Thr Gln Thr Phe Thr
               100                 105                 110
   Tyr Gln Asn Pro Glu Ile Pro Leu Tyr Gly Lys Ile Lys Tyr Gly Thr
           115                 120                 125
   Thr Ser Arg Thr Tyr Lys
       130
<210> 59
   <211> 906
   <212> DNA
   <213> Phakopsora pachyrhizi
<220>
   <221> misc_feature
   <222> (1)..(906)
   <223> 21-c12
<400> 59
   atgaaaatcc ccatgatcta ccgttgcgca atcgcccttt tggccttttt tccaagcagc      60
   ttgaaggctt attttttgtt gaaaaaacct ttatcacttc tagagtcaga tgaaaaaata     120
   ttttattgca ctgcgcccat acaatctgga tacacagata ttctcaagta cttaccaata     180
   aacaaaaaac cagacccctc accttatgtc aaaacggaga actcggtcat aactcaaatg     240
   agcggagatg atgcatgtct aaaatatcga aaaaatttaa tatcctcccc gcctaaagat     300
   ctcgtaaatc taagggcata taaaacatta cttttgagat cagtttatat gaaccttgag     360
   ggcgtgttta agacatataa acataattct ggccctcaaa ccattttata tcatttgctt     420
   gaagcacgcc ctgcggcctg taggctctta caaataaacg aaaaccgggc ggaccttaag     480
   gctgacacag cacgaattat atgtgatcat ctggcaaaca taaaacatac agaagaagat     540
   tttttcgaat ttgccggctg gatgcacttt attgccatga ttatcgatat tggagatgtt     600
   gtgaaaatct cgccagaagc atttgcagcc gacattgctt ttcatgtcgg actcttgtcc     660
   cttgagcaga agtttaattc ggatccggac cggtccaaaa tcataaaagc tttgctaaca     720
   attcttgatg aacgtcgtca gaaatttcag cggtataaaa cctttcttaa gaattttttc     780
   aaagattatg atgaaaaact gccttggatg cgagaatatt attttccaaa aaacgggttg     840
   ccagaaaatt tttgtaagac tttcttccta tcgagcaatc tatgtcaaga taataaaata     900
   aaataa                                                                906
<210> 60
   <211> 301
   <212> PRT
   <213> Phakopsora pachyrhizi
<220>
   <221> VARIANT
   <222> (1)..(301)
   <223> 21-c12
<400> 60
   Met Lys Ile Pro Met Ile Tyr Arg Cys Ala Ile Ala Leu Leu Ala Phe
   1               5                   10                  15
   Phe Pro Ser Ser Leu Lys Ala Tyr Phe Leu Leu Lys Lys Pro Leu Ser
               20                  25                  30
   Leu Leu Glu Ser Asp Glu Lys Ile Phe Tyr Cys Thr Ala Pro Ile Gln
           35                  40                  45
   Ser Gly Tyr Thr Asp Ile Leu Lys Tyr Leu Pro Ile Asn Lys Lys Pro
       50                  55                  60
   Asp Pro Ser Pro Tyr Val Lys Thr Glu Asn Ser Val Ile Thr Gln Met
   65                  70                  75                  80
   Ser Gly Asp Asp Ala Cys Leu Lys Tyr Arg Lys Asn Leu Ile Ser Ser
                   85                  90                  95
   Pro Pro Lys Asp Leu Val Asn Leu Arg Ala Tyr Lys Thr Leu Leu Leu
               100                 105                 110
   Arg Ser Val Tyr Met Asn Leu Glu Gly Val Phe Lys Thr Tyr Lys His
           115                 120                 125
   Asn Ser Gly Pro Gln Thr Ile Leu Tyr His Leu Leu Glu Ala Arg Pro
       130                 135                 140
   Ala Ala Cys Arg Leu Leu Gln Ile Asn Glu Asn Arg Ala Asp Leu Lys
   145                 150                 155                 160
   Ala Asp Thr Ala Arg Ile Ile Cys Asp His Leu Ala Asn Ile Lys His
                   165                 170                 175
   Thr Glu Glu Asp Phe Phe Glu Phe Ala Gly Trp Met His Phe Ile Ala
               180                 185                 190
   Met Ile Ile Asp Ile Gly Asp Val Val Lys Ile Ser Pro Glu Ala Phe
           195                 200                 205
   Ala Ala Asp Ile Ala Phe His Val Gly Leu Leu Ser Leu Glu Gln Lys
       210                 215                 220
   Phe Asn Ser Asp Pro Asp Arg Ser Lys Ile Ile Lys Ala Leu Leu Thr
   225                 230                 235                 240
   Ile Leu Asp Glu Arg Arg Gln Lys Phe Gln Arg Tyr Lys Thr Phe Leu
                   245                 250                 255
   Lys Asn Phe Phe Lys Asp Tyr Asp Glu Lys Leu Pro Trp Met Arg Glu
               260                 265                 270
   Tyr Tyr Phe Pro Lys Asn Gly Leu Pro Glu Asn Phe Cys Lys Thr Phe
           275                 280                 285
   Phe Leu Ser Ser Asn Leu Cys Gln Asp Asn Lys Ile Lys
       290                 295                 300
<210> 61
   <211> 723
   <212> DNA
   <213> Phakopsora pachyrhizi
<220>
   <221> misc_feature
   <222> (1)..(723)
   <223> 16-n11
<400> 61
   atgaagatga atatgcttca ccattctttg ttgctcttca ctttcttctg tataaaactg      60
   ttgatttgct tacccatgaa tggtaagact ccagcagttg ttgccgatgc cgtttcagca     120
   attcaggaaa ccggaaaagt tgcaagcacc agcatagaag taccaagcgc aaatttgggt     180
   gcggaagtca aggaactttc agcgattgat gcgtctgtgt tcacaaatgt tcctgctatc     240
   aatccgaagg attcggccca agtagaaaat ctggggcaat caactttgca ggagaaagaa     300
   agcccagtta tattgaaggg tgatgtaaat catgagaaaa aaaatctaat aaaaaccgaa     360
   tctaaatctg cagatagtga aataaaaaaa gaagttgatg aaaaaccctt gaaagaaaaa     420
   tcacagggtg aagacggtcc tgatggccct gaaaaaaaaa acattgaggg cgaaggagga     480
   gatctgaaaa atgacaaaaa tttgaatcaa tcagaacaaa aaactaggtt gagcaggatt     540
   aagaactggt ttataaggca ataccttatg ttgaaaaaca atgtataccc taaaataaaa     600
   gaaaaaagtc aacaattttg ggcttggaca aagaaaaaat tttcaagagg caataacaag     660
   gtagctacaa atgaaattga attaaagcca tgttgccgag gaaaaaagac acatttcagc     720
   taa                                                                   723
<210> 62
   <211> 240
   <212> PRT
   <213> Phakopsora pachyrhizi
<220>
   <221> VARIANT
   <222> (1)..(240)
   <223> 16-n11
<400> 62
   Met Lys Met Asn Met Leu His His Ser Leu Leu Leu Phe Thr Phe Phe
   1               5                   10                  15
   Cys Ile Lys Leu Leu Ile Cys Leu Pro Met Asn Gly Lys Thr Pro Ala
               20                  25                  30
   Val Val Ala Asp Ala Val Ser Ala Ile Gln Glu Thr Gly Lys Val Ala
           35                  40                  45
   Ser Thr Ser Ile Glu Val Pro Ser Ala Asn Leu Gly Ala Glu Val Lys
       50                  55                  60
   Glu Leu Ser Ala Ile Asp Ala Ser Val Phe Thr Asn Val Pro Ala Ile
   65                  70                  75                  80
   Asn Pro Lys Asp Ser Ala Gln Val Glu Asn Leu Gly Gln Ser Thr Leu
                   85                  90                  95
   Gln Glu Lys Glu Ser Pro Val Ile Leu Lys Gly Asp Val Asn His Glu
               100                 105                 110
   Lys Lys Asn Leu Ile Lys Thr Glu Ser Lys Ser Ala Asp Ser Glu Ile
           115                 120                 125
   Lys Lys Glu Val Asp Glu Lys Pro Leu Lys Glu Lys Ser Gln Gly Glu
       130                 135                 140
   Asp Gly Pro Asp Gly Pro Glu Lys Lys Asn Ile Glu Gly Glu Gly Gly
   145                 150                 155                 160
   Asp Leu Lys Asn Asp Lys Asn Leu Asn Gln Ser Glu Gln Lys Thr Arg
                   165                 170                 175
   Leu Ser Arg Ile Lys Asn Trp Phe Ile Arg Gln Tyr Leu Met Leu Lys
               180                 185                 190
   Asn Asn Val Tyr Pro Lys Ile Lys Glu Lys Ser Gln Gln Phe Trp Ala
           195                 200                 205
   Trp Thr Lys Lys Lys Phe Ser Arg Gly Asn Asn Lys Val Ala Thr Asn
       210                 215                 220
   Glu Ile Glu Leu Lys Pro Cys Cys Arg Gly Lys Lys Thr His Phe Ser
   225                 230                 235                 240
<210> 63
   <211> 654
   <212> DNA
   <213> Phakopsora pachyrhizi
<220>
   <221> misc_feature
   <222> (1)..(654)
   <223> 15-m3
<400> 63
   atgaagcttt tcagctgtac aaatgagtgg aacattttgc cctttgtatt attaagcgcg       60
   tggtatacca gaaccgttct tggaatgcat tttgccgaaa gctcaaagga aaatattatc     120
   agctctgcaa agggtctact acaggaacag cctccggaaa atataccaat tacacaagga     180
   attagcatta ggactgaggg acaagattct ctttcgataa atgcagaagt aaaagagccc     240
   aaatggcatt cggtttctca aaaggatgaa aatttccccg atgtaagaac ttggaaagag     300
   attttgtatg gtagaacgaa tgcacacaat tatggttatt cagatgatga aataacgcct     360
   gaaataaggc aaatactgaa aaatcgcctt ccaaagtttc tcccaccccc agcagaccca     420
   aaactcgaaa ggatcaaaga taacttcgaa tttttaatga gaaacaatga tcccgctcga     480
   aattataatt taaattttca aataaataca catcttaggg atctgcttga caattttaaa     540
   gtgaatgctc agttaccaac cgaattggat gagcaattta gtgatgtaat aaaatttctc     600
   aaagattcaa cttttaagaa tgataacgaa aaacatgtaa ttgagaatat tcac           654
<210> 64
   <211> 218
   <212> PRT
   <213> Phakopsora pachyrhizi
<220>
   <221> VARIANT
   <222> (1)..(218)
   <223> 15-m3
<400> 64
   Met Lys Leu Phe Ser Cys Thr Asn Glu Trp Asn Ile Leu Pro Phe Val
   1               5                   10                  15
   Leu Leu Ser Ala Trp Tyr Thr Arg Thr Val Leu Gly Met His Phe Ala
               20                  25                  30
   Glu Ser Ser Lys Glu Asn Ile Ile Ser Ser Ala Lys Gly Leu Leu Gln
           35                  40                  45
   Glu Gln Pro Pro Glu Asn Ile Pro Ile Thr Gln Gly Ile Ser Ile Arg
       50                  55                  60
   Thr Glu Gly Gln Asp Ser Leu Ser Ile Asn Ala Glu Val Lys Glu Pro
   65                  70                  75                  80
   Lys Trp His Ser Val Ser Gln Lys Asp Glu Asn Phe Pro Asp Val Arg
                   85                  90                  95
   Thr Trp Lys Glu Ile Leu Tyr Gly Arg Thr Asn Ala His Asn Tyr Gly
               100                 105                 110
   Tyr Ser Asp Asp Glu Ile Thr Pro Glu Ile Arg Gln Ile Leu Lys Asn
           115                 120                 125
   Arg Leu Pro Lys Phe Leu Pro Pro Pro Ala Asp Pro Lys Leu Glu Arg
       130                 135                 140
   Ile Lys Asp Asn Phe Glu Phe Leu Met Arg Asn Asn Asp Pro Ala Arg
   145                 150                 155                 160
   Asn Tyr Asn Leu Asn Phe Gln Ile Asn Thr His Leu Arg Asp Leu Leu
                   165                 170                 175
   Asp Asn Phe Lys Val Asn Ala Gln Leu Pro Thr Glu Leu Asp Glu Gln
               180                 185                 190
   Phe Ser Asp Val Ile Lys Phe Leu Lys Asp Ser Thr Phe Lys Asn Asp
           195                 200                 205
   Asn Glu Lys His Val Ile Glu Asn Ile His
       210                 215
<210> 65
   <211> 486
   <212> DNA
   <213> Phakopsora pachyrhizi
<220>
   <221> misc_feature
   <222> (1)..(486)
   <223> 27-e12
<400> 65
   atgaagatga atatgcttca ccattctttg ttgctcttca ctttcttctg tataaaactg      60
   ttgatttgct tacccatgaa tggtaagact ccagcagttg ttgccgatgc cgtttcagca     120
   attcaggaaa ccggaaaagt tgcaagtacc agcatagaag taccaagcgc aaatttgggt     180
   gcggaagtca aggaactttc agcgattgat gcgtctgtgt tcacaaatgt tcctgctatc     240
   aatccgaagg attcggccca agtagaaaat ctggggcaat caactttgca ggagaaagaa     300
   agcccagtta tattgaaggg tgatgtaaat catgagaaaa aaaatctaat aaaaaccgaa     360
   tctaaatctg cagatagtga aataaaaaaa gaagttgatg aaaaaccctt gaaagaaaaa     420
   tcacagggtg aagacggtcc tgatggccct gaaaaaaaaa cattgagggc gaaggaggag     480
   atctga                                                                486
<210> 66
   <211> 161
   <212> PRT
   <213> Phakopsora pachyrhizi
<220>
   <221> VARIANT
   <222> (1)..(161)
   <223> 27-e12
<400> 66
   Met Lys Met Asn Met Leu His His Ser Leu Leu Leu Phe Thr Phe Phe
   1               5                   10                  15
   Cys Ile Lys Leu Leu Ile Cys Leu Pro Met Asn Gly Lys Thr Pro Ala
               20                  25                  30
   Val Val Ala Asp Ala Val Ser Ala Ile Gln Glu Thr Gly Lys Val Ala
           35                  40                  45
   Ser Thr Ser Ile Glu Val Pro Ser Ala Asn Leu Gly Ala Glu Val Lys
       50                  55                  60
   Glu Leu Ser Ala Ile Asp Ala Ser Val Phe Thr Asn Val Pro Ala Ile
   65                  70                  75                  80
   Asn Pro Lys Asp Ser Ala Gln Val Glu Asn Leu Gly Gln Ser Thr Leu
                   85                  90                  95
   Gln Glu Lys Glu Ser Pro Val Ile Leu Lys Gly Asp Val Asn His Glu
               100                 105                 110
   Lys Lys Asn Leu Ile Lys Thr Glu Ser Lys Ser Ala Asp Ser Glu Ile
           115                 120                 125
   Lys Lys Glu Val Asp Glu Lys Pro Leu Lys Glu Lys Ser Gln Gly Glu
       130                 135                 140
   Asp Gly Pro Asp Gly Pro Glu Lys Lys Thr Leu Arg Ala Lys Glu Glu
   145                 150                 155                 160
   Ile
<210> 67
   <211> 552
   <212> DNA
   <213> Phakopsora pachyrhizi
<220>
   <221> misc_feature
   <222> (1)..(552)
   <223> 24-i9
<400> 67
   atgctgttct gcaaattttt attaattttt tcgttgatgt gctatggcgc tctagctgct      60
   ccgataatga gagttttttg ggcggctttt ccacttttaa atcttccagt cttcaaattc     120
   ctcattcacc cccaagagtc tttgaagtta cttcttggtt tagagagagg ayttcaggaa     180
   attaaagatc tagaattgag taagagtgct tttagacttc aaaagttgat tttaggtgat     240
   cagaaagtgt ttctgaagga aattgacggc aaaattgaaa acgaagcaac aaccttcgca     300
   aatgtcatga gacttgataa taaaaagcag tttgaaaaca tacccttcag actacctcta     360
   aatacaaagg atttgcatga aagtatttat aattctttag tagaagtagt taatgatcct     420
   aaaattattg gcctccaaag gattcccaac ccaaaattga cgcaaaaacg tttaagtgaa     480
   ttgaaaccca tatcggagcc cattgcagat attaatatac ttcgtactgc agtggcgcat     540
   tcaaaattat aa                                                         552
<210> 68
   <211> 182
   <212> PRT
   <213> Phakopsora pachyrhizi
<220>
   <221> VARIANT
   <222> (1)..(182)
   <223> 24-i9
<400> 68
   Met Leu Phe Cys Lys Phe Leu Leu Ile Phe Ser Leu Met Cys Tyr Gly
   1               5                   10                  15
   Ala Leu Ala Ala Pro Ile Met Arg Val Phe Trp Ala Ala Phe Pro Leu
               20                  25                  30
   Leu Asn Leu Pro Val Phe Lys Phe Leu Ile His Pro Gln Glu Ser Leu
           35                  40                  45
   Lys Leu Leu Leu Gly Leu Glu Arg Gly Gln Glu Ile Lys Asp Leu Glu
       50                  55                  60
   Leu Ser Lys Ser Ala Phe Arg Leu Gln Lys Leu Ile Leu Gly Asp Gln
   65                  70                  75                  80
   Lys Val Phe Leu Lys Glu Ile Asp Gly Lys Ile Glu Asn Glu Ala Thr
                   85                  90                  95
   Thr Phe Ala Asn Val Met Arg Leu Asp Asn Lys Lys Gln Phe Glu Asn
               100                 105                 110
   Ile Pro Phe Arg Leu Pro Leu Asn Thr Lys Asp Leu His Glu Ser Ile
           115                 120                 125
   Tyr Asn Ser Leu Val Glu Val Val Asn Asp Pro Lys Ile Ile Gly Leu
       130                 135                 140
   Gln Arg Ile Pro Asn Pro Lys Leu Thr Gln Lys Arg Leu Ser Glu Leu
   145                 150                 155                 160
   Lys Pro Ile Ser Glu Pro Ile Ala Asp Ile Asn Ile Leu Arg Thr Ala
                   165                 170                 175
   Val Ala His Ser Lys Leu
               180
<210> 69
   <211> 1074
   <212> DNA
   <213> Phakopsora pachyrhizi
<220>
   <221> misc_feature
   <222> (1)..(1074)
   <223> 23-m13
<400> 69
   atgctatcgc ttttgaaact ttgttgtttc ttgactttta tttacagctg ggggtctgca      60
   aggccaatgt ttaaactatg tcggggtatg gattataaat tatgcatagt cgagaacgcg     120
   ggcaatctgg gtataaaccc aagtaaggac accggaagtt ttatgaaatt tatgactgct     180
   aattcgccag atgcgttcag ctccagtagc tcggcttcaa gcgttgtttc taaaaaagta     240
   agggcaggct ctacgaaaaa acagagaaat attccaagca atttgttaac tcaaggtgag     300
   cagctagaga gcagaagaga aattaacatt ccaagggcgc cccaaaaatt aaaaaccaaa     360
   gaaaaaaact tgaatttaag ttcagaaaaa atcaacccga aatctctcag ggtttcggaa     420
   aattcaactg ctgatcgaca ttctagatcc ctaccgcctg ggaaatcttc tcctttggta     480
   accaacaaca acaataaaaa atcaaaatca ttaccaaagt caaagccgaa caagagttta     540
   acaaaagttt atgagactca ttatgcaaaa agtcccaccc gagttcctat aaaagccccc     600
   atgataaacc agattttgcc accaaaacgc aaaatattta tctcacctac tttaactagc     660
   ggcaataaac ctgctgctga gcttaaatct ccagaaaact caaaacgctt ctctaccatt     720
   aaagataaaa aactatcccc gccggtctct tttgactcta aatccccaga aaactcgaga     780
   cgcttctcta ccaataaaga taaaagtcta tccccgccgg tcacttttga ctccacaaaa     840
   attccgcatt ctaagcagac taatacaaat atccttgtta aacctcataa cttacctaat     900
   aatgacatca aagactctcg tgaagtcaat cgattgacaa aagaaaaaaa tattggagaa     960
   gtatccttca gaattcatcc agatgatgat gaatctttta agtttccaaa tttgaaagaa    1020
   acacatatag gtaataaaaa tcacttttgg aatttcaatg aagaaaatca ataa          1074
<210> 70
   <211> 357
   <212> PRT
   <213> Phakopsora pachyrhizi
<220>
   <221> VARIANT
   <222> (1)..(357)
   <223> 23-m13
<400> 70
   Met Leu Ser Leu Leu Lys Leu Cys Cys Phe Leu Thr Phe Ile Tyr Ser
   1               5                   10                  15
   Trp Gly Ser Ala Arg Pro Met Phe Lys Leu Cys Arg Gly Met Asp Tyr
               20                  25                  30
   Lys Leu Cys Ile Val Glu Asn Ala Gly Asn Leu Gly Ile Asn Pro Ser
           35                  40                  45
   Lys Asp Thr Gly Ser Phe Met Lys Phe Met Thr Ala Asn Ser Pro Asp
       50                  55                  60
   Ala Phe Ser Ser Ser Ser Ser Ala Ser Ser Val Val Ser Lys Lys Val
   65                  70                  75                  80
   Arg Ala Gly Ser Thr Lys Lys Gln Arg Asn Ile Pro Ser Asn Leu Leu
                   85                  90                  95
   Thr Gln Gly Glu Gln Leu Glu Ser Arg Arg Glu Ile Asn Ile Pro Arg
               100                 105                 110
   Ala Pro Gln Lys Leu Lys Thr Lys Glu Lys Asn Leu Asn Leu Ser Ser
           115                 120                 125
   Glu Lys Ile Asn Pro Lys Ser Leu Arg Val Ser Glu Asn Ser Thr Ala
       130                 135                 140
   Asp Arg His Ser Arg Ser Leu Pro Pro Gly Lys Ser Ser Pro Leu Val
   145                 150                 155                 160
   Thr Asn Asn Asn Asn Lys Lys Ser Lys Ser Leu Pro Lys Ser Lys Pro
                   165                 170                 175
   Asn Lys Ser Leu Thr Lys Val Tyr Glu Thr His Tyr Ala Lys Ser Pro
               180                 185                 190
   Thr Arg Val Pro Ile Lys Ala Pro Met Ile Asn Gln Ile Leu Pro Pro
           195                 200                 205
   Lys Arg Lys Ile Phe Ile Ser Pro Thr Leu Thr Ser Gly Asn Lys Pro
       210                 215                 220
   Ala Ala Glu Leu Lys Ser Pro Glu Asn Ser Lys Arg Phe Ser Thr Ile
   225                 230                 235                 240
   Lys Asp Lys Lys Leu Ser Pro Pro Val Ser Phe Asp Ser Lys Ser Pro
                   245                 250                 255
   Glu Asn Ser Arg Arg Phe Ser Thr Asn Lys Asp Lys Ser Leu Ser Pro
               260                 265                 270
   Pro Val Thr Phe Asp Ser Thr Lys Ile Pro His Ser Lys Gln Thr Asn
           275                 280                 285
   Thr Asn Ile Leu Val Lys Pro His Asn Leu Pro Asn Asn Asp Ile Lys
       290                 295                 300
   Asp Ser Arg Glu Val Asn Arg Leu Thr Lys Glu Lys Asn Ile Gly Glu
   305                 310                 315                 320
   Val Ser Phe Arg Ile His Pro Asp Asp Asp Glu Ser Phe Lys Phe Pro
                   325                 330                 335
   Asn Leu Lys Glu Thr His Ile Gly Asn Lys Asn His Phe Trp Asn Phe
               340                 345                 350
   Asn Glu Glu Asn Gln
           355
<210> 71
   <211> 627
   <212> DNA
   <213> Phakopsora pachyrhizi
<220>
   <221> misc_feature
   <222> (1)..(627)
   <223> 02-f1 (01-G4)
<400> 71
   atgcgttcac ttatcgttcc caccctttta tgcgtgctat ttattcacaa atcttgcgca      60
   accgttcaca ctcagtgcta caactatttt ttacaaaagg atggatgcgt tttctctgca     120
   gctgatgaca gaaatcgatg ttctgcagat cccaagccca gtacagccgt tggagtggtt     180
   caagaaagca acaaaaatgt taaaagacac actctggcac gccgttatga tactacttta     240
   ccatctcctt ctatacaggg tgaaggaatt tgcggacact atgacacagc aacagctgag     300
   ggagccagtc tttgggttgg tcctaaccca ggtagcacaa gacctgagga ggcaggctgg     360
   cttaacaggg gaaagacatc taattgcaat aagagattat atgtaataaa ccctcgcaca     420
   ggaaaaaccg tctacgtaaa ggtcatagat ggccgcgatt ttcagacaac acagcctgat     480
   gtgggatgct tccagattgc actcacccaa aagaccattc ttgagcttga tccgactgat     540
   gaagaaaagg caaagggtgc cataggatct ctcacatggg acttcgacaa cctgcatgga     600
   attagttctc agcaaggtcc tgtatga                                         627
<210> 72
   <211> 208
   <212> PRT
   <213> Phakopsora pachyrhizi
<220>
   <221> VARIANT
   <222> (1)..(208)
   <223> 02-f1 (01-G4)
<400> 72
   Met Arg Ser Leu Ile Val Pro Thr Leu Leu Cys Val Leu Phe Ile His
   1               5                   10                  15
   Lys Ser Cys Ala Thr Val His Thr Gln Cys Tyr Asn Tyr Phe Leu Gln
               20                  25                  30
   Lys Asp Gly Cys Val Phe Ser Ala Ala Asp Asp Arg Asn Arg Cys Ser
           35                  40                  45
   Ala Asp Pro Lys Pro Ser Thr Ala Val Gly Val Val Gln Glu Ser Asn
       50                  55                  60
   Lys Asn Val Lys Arg His Thr Leu Ala Arg Arg Tyr Asp Thr Thr Leu
   65                  70                  75                  80
   Pro Ser Pro Ser Ile Gln Gly Glu Gly Ile Cys Gly His Tyr Asp Thr
                   85                  90                  95
   Ala Thr Ala Glu Gly Ala Ser Leu Trp Val Gly Pro Asn Pro Gly Ser
               100                 105                 110
   Thr Arg Pro Glu Glu Ala Gly Trp Leu Asn Arg Gly Lys Thr Ser Asn
           115                 120                 125
   Cys Asn Lys Arg Leu Tyr Val Ile Asn Pro Arg Thr Gly Lys Thr Val
       130                 135                 140
   Tyr Val Lys Val Ile Asp Gly Arg Asp Phe Gln Thr Thr Gln Pro Asp
   145                 150                 155                 160
   Val Gly Cys Phe Gln Ile Ala Leu Thr Gln Lys Thr Ile Leu Glu Leu
                   165                 170                 175
   Asp Pro Thr Asp Glu Glu Lys Ala Lys Gly Ala Ile Gly Ser Leu Thr
               180                 185                 190
   Trp Asp Phe Asp Asn Leu His Gly Ile Ser Ser Gln Gln Gly Pro Val
           195                 200                 205
<210> 73
   <211> 597
   <212> DNA
   <213> Phakopsora pachyrhizi
<220>
   <221> misc_feature
   <222> (1)..(597)
   <223> 02-c11
<400> 73
   atgtatcgat ttccactcat ttcccttgcc atttatgctt cagtcgtata tggtcaagga     60
   actaccgtca ctcctactac gaacgtcaac ccaaacacat caggtaatcc gccaaaccaa    120
   cccactaatg gtacccctac caatccggca actcctacgg catctcctac gacgactcct    180
   actcccaaaa ttcttgctgt taattgtaca cagtcttact ttgagatcac tgatgattca    240
   atgaacggaa cttctatcgg tgcatgtacg aagattggag acggagaaaa aggcggttcc    300
   ttctgccaaa ccaaagagtg ttttggcttt gccacttgcc aagattgtaa cctagcaacc    360
   cctggtccta ataatacgac tgtaatctca aacaccacta tcaaagcagt accctgcaac    420
   aagggctact atatccctgg accaggagat aacgttactg tgacttattg ctccgcccaa    480
   aacaatgcga atccggatga ctacatttgc aagggcccat gcacaagttt tacagcgtgt    540
   ggagattgta tcaatataaa cgatccagcg attgcgggcc tgaaagatac tccttaa       597
<210> 74
   <211> 198
   <212> PRT
   <213> Phakopsora pachyrhizi
<220>
   <221> VARIANT
   <222> (1)..(198)
   <223> 02-c11
<400> 74
   Met Tyr Arg Phe Pro Leu Ile Ser Leu Ala Ile Tyr Ala Ser Val Val
   1               5                   10                  15
   Tyr Gly Gln Gly Thr Thr Val Thr Pro Thr Thr Asn Val Asn Pro Asn
               20                  25                  30
   Thr Ser Gly Asn Pro Pro Asn Gln Pro Thr Asn Gly Thr Pro Thr Asn
           35                  40                  45
   Pro Ala Thr Pro Thr Ala Ser Pro Thr Thr Thr Pro Thr Pro Lys Ile
       50                  55                  60
   Leu Ala Val Asn Cys Thr Gln Ser Tyr Phe Glu Ile Thr Asp Asp Ser
   65                  70                  75                  80
   Met Asn Gly Thr Ser Ile Gly Ala Cys Thr Lys Ile Gly Asp Gly Glu
                   85                  90                  95
   Lys Gly Gly Ser Phe Cys Gln Thr Lys Glu Cys Phe Gly Phe Ala Thr
               100                 105                 110
   Cys Gln Asp Cys Asn Leu Ala Thr Pro Gly Pro Asn Asn Thr Thr Val
           115                 120                 125
   Ile Ser Asn Thr Thr Ile Lys Ala Val Pro Cys Asn Lys Gly Tyr Tyr
       130                 135                 140
   Ile Pro Gly Pro Gly Asp Asn Val Thr Val Thr Tyr Cys Ser Ala Gln
   145                 150                 155                 160
   Asn Asn Ala Asn Pro Asp Asp Tyr Ile Cys Lys Gly Pro Cys Thr Ser
                   165                 170                 175
   Phe Thr Ala Cys Gly Asp Cys Ile Asn Ile Asn Asp Pro Ala Ile Ala
               180                 185                 190
   Gly Leu Lys Asp Thr Pro
           195
<210> 75
   <211> 318
   <212> DNA
   <213> Phakopsora pachyrhizi
<220>
   <221> misc_feature
   <222> (1)..(318)
   <223> 02-p12
<400> 75
   atgaagttaa tgcagctaat attctatatg ctcaacatac tagcactcac gatggcccat      60
   aactgttgtg agctgtcaaa gatgccaact cttcctgaga taggcgtgat gtcctcgaag     120
   cctgttagac caaagccaat ccaatccacg gcatcgaaag gcacaaagtt cttgcctact     180
   acgctctgta tcatcaagtg caagtttaac atctgtctgg ctcatgctaa tgtcataaac     240
   tccgtacaaa ttggtaatcg gatgagggag agtaagggag ctgtcaactt gaatgctcca     300
   cgccaccatt cattttaa                                                   318
<210> 76
   <211> 105
   <212> PRT
   <213> Phakopsora pachyrhizi
<220>
   <221> VARIANT
   <222> (1)..(105)
   <223> 02-p12
<400> 76
   Met Lys Leu Met Gln Leu Ile Phe Tyr Met Leu Asn Ile Leu Ala Leu
   1               5                   10                  15
   Thr Met Ala His Asn Cys Cys Glu Leu Ser Lys Met Pro Thr Leu Pro
               20                  25                  30
   Glu Ile Gly Val Met Ser Ser Lys Pro Val Arg Pro Lys Pro Ile Gln
           35                  40                  45
   Ser Thr Ala Ser Lys Gly Thr Lys Phe Leu Pro Thr Thr Leu Cys Ile
       50                  55                  60
   Ile Lys Cys Lys Phe Asn Ile Cys Leu Ala His Ala Asn Val Ile Asn
   65                  70                  75                  80
   Ser Val Gln Ile Gly Asn Arg Met Arg Glu Ser Lys Gly Ala Val Asn
                   85                  90                  95
   Leu Asn Ala Pro Arg His His Ser Phe
               100                 105
<210> 77
   <211> 588
   <212> DNA
   <213> Phakopsora pachyrhizi
<220>
   <221> misc_feature
   <222> (1)..(588)
   <223> 03-c8
<400> 77
   atgaaaagag aaattaaatt gatattattg attgctgtaa ctttactaag tgcacccttt      60
   gtagtgaaca atttgccgtt taatagcaac tcactaatct ctgttagtga atgtagcaac     120
   aaaacccctc tacttgaaaa cagggtcgcg gagattgggt ttaaacgccc agagatactc     180
   gacggtcagg agaaagagtt tttttcccaa cagaggattg gttgcttacc aaacattttt     240
   aggaaggcta taggatcatg ttgctttggg aacgaggaaa ataaaaatat aaaatatagg     300
   cttctgaagc cagagtattc ttcttgggat gaaataaggt atatctttga tccaaaattc     360
   ctacttgttc atcgaaattc tgataatttt ttggagaaca ttgatttaga aaaatttgag     420
   aaaaagcttc ttggtaatcc ggacggaaat caagcacatt tgtttagtcg tccaaagttt     480
   gcagaccttg aaaacgcttg ggtgaaatat ctcagacaag tagttctatt agtcgtagag     540
   ttagggagct tatgttacat tggccagcgt ttttgcggaa gggtttga                  588
<210> 78
   <211> 195
   <212> PRT
   <213> Phakopsora pachyrhizi
<220>
   <221> VARIANT
   <222> (1)..(195)
   <223> 03-c8
<400> 78
   Met Lys Arg Glu Ile Lys Leu Ile Leu Leu Ile Ala Val Thr Leu Leu
   1               5                   10                  15
   Ser Ala Pro Phe Val Val Asn Asn Leu Pro Phe Asn Ser Asn Ser Leu
               20                  25                  30
   Ile Ser Val Ser Glu Cys Ser Asn Lys Thr Pro Leu Leu Glu Asn Arg
           35                  40                  45
   Val Ala Glu Ile Gly Phe Lys Arg Pro Glu Ile Leu Asp Gly Gln Glu
       50                  55                  60
   Lys Glu Phe Phe Ser Gln Gln Arg Ile Gly Cys Leu Pro Asn Ile Phe
   65                  70                  75                  80
   Arg Lys Ala Ile Gly Ser Cys Cys Phe Gly Asn Glu Glu Asn Lys Asn
                   85                  90                  95
   Ile Lys Tyr Arg Leu Leu Lys Pro Glu Tyr Ser Ser Trp Asp Glu Ile
               100                 105                 110
   Arg Tyr Ile Phe Asp Pro Lys Phe Leu Leu Val His Arg Asn Ser Asp
           115                 120                 125
   Asn Phe Leu Glu Asn Ile Asp Leu Glu Lys Phe Glu Lys Lys Leu Leu
       130                 135                 140
   Gly Asn Pro Asp Gly Asn Gln Ala His Leu Phe Ser Arg Pro Lys Phe
   145                 150                 155                 160
   Ala Asp Leu Glu Asn Ala Trp Val Lys Tyr Leu Arg Gln Val Val Leu
                   165                 170                 175
   Leu Val Val Glu Leu Gly Ser Leu Cys Tyr Ile Gly Gln Arg Phe Cys
               180                 185                 190
   Gly Arg Val
           195
<210> 79
   <211> 444
   <212> DNA
   <213> Phakopsora pachyrhizi
<220>
   <221> misc_feature
   <222> (1)..(444)
   <223> 02-a6
<400> 79
   atgctgctag gtctacattt ttcctccctt atgaattgga ctacaataat tctgtccaca     60
   actctgcttc ttagactgtc taaatgcgaa cttttcgatg ccagtttggc agtaagaggg    120
   atcactcatg gcactccaag tttaggtcat gattctaaga ctcttcatat cacctcacag    180
   ggagggctca atagtccatc tcaccatgtg aagcagcttt caactaaagc cagaaaaccc    240
   cctaaggttc caaatgcaaa agattccggt tgggaaatta ttgacccaaa acttccagag    300
   tgggaactga ttgaaaagcc aaaagtaaaa aaaaaactcc ccaaaattta tgtcccagag    360
   gccaagtttt attcttctga tggtagttca gaaaccatca atttcataga agatcattat    420
   gcgattaaag aatctacacc gtaa                                           444
<210> 80
   <211> 147
   <212> PRT
   <213> Phakopsora pachyrhizi
<220>
   <221> VARIANT
   <222> (1)..(147)
   <223> 02-a6
<400> 80
   Met Leu Leu Gly Leu His Phe Ser Ser Leu Met Asn Trp Thr Thr Ile
   1               5                   10                  15
   Ile Leu Ser Thr Thr Leu Leu Leu Arg Leu Ser Lys Cys Glu Leu Phe
               20                  25                  30
   Asp Ala Ser Leu Ala Val Arg Gly Ile Thr His Gly Thr Pro Ser Leu
           35                  40                  45
   Gly His Asp Ser Lys Thr Leu His Ile Thr Ser Gln Gly Gly Leu Asn
       50                  55                  60
   Ser Pro Ser His His Val Lys Gln Leu Ser Thr Lys Ala Arg Lys Pro
   65                  70                  75                  80
   Pro Lys Val Pro Asn Ala Lys Asp Ser Gly Trp Glu Ile Ile Asp Pro
                   85                  90                  95
   Lys Leu Pro Glu Trp Glu Leu Ile Glu Lys Pro Lys Val Lys Lys Lys
               100                 105                 110
   Leu Pro Lys Ile Tyr Val Pro Glu Ala Lys Phe Tyr Ser Ser Asp Gly
           115                 120                 125
   Ser Ser Glu Thr Ile Asn Phe Ile Glu Asp His Tyr Ala Ile Lys Glu
       130                 135                 140
   Ser Thr Pro
   145
<210> 81
   <211> 231
   <212> DNA
   <213> Phakopsora pachyrhizi
<220>
   <221> misc_feature
   <222> (1)..(231)
   <223> 02-c24
<400> 81
   atggctggaa aagagattta tgaaccaaga aagtcaatga tgattaaaat gattgggttc      60
   ttatgcattt ggtttcaact attaggagta gtttttacac aatctactgc tgcaactaca     120
   agtgccacta aggccaaaac ttatgatact tatttcaaga ggcttgcaaa aagaagccca     180
   ttgactaact ctggccgaat tgagagaagg cataaatgtg gaccgccata g              231
<210> 82
   <211> 76
   <212> PRT
   <213> Phakopsora pachyrhizi
<220>
   <221> VARIANT
   <222> (1)..(76)
   <223> 02-c24
<400> 82
   Met Ala Gly Lys Glu Ile Tyr Glu Pro Arg Lys Ser Met Met Ile Lys
   1               5                   10                  15
   Met Ile Gly Phe Leu Cys Ile Trp Phe Gln Leu Leu Gly Val Val Phe
               20                  25                  30
   Thr Gln Ser Thr Ala Ala Thr Thr Ser Ala Thr Lys Ala Lys Thr Tyr
           35                  40                  45
   Asp Thr Tyr Phe Lys Arg Leu Ala Lys Arg Ser Pro Leu Thr Asn Ser
       50                  55                  60
   Gly Arg Ile Glu Arg Arg His Lys Cys Gly Pro Pro
   65                  70                  75
<210> 83
   <211> 1389
   <212> DNA
   <213> Phakopsora pachyrhizi
<220>
   <221> misc_feature
   <222> (1)..(1389)
   <223> 02-h8
<400> 83
   atgcaaaata aattccagcc attcaagctt ttgttagctt tgagtttgtt cattgcaggc      60
   tcatcagcaa accaagatga tggctccgca atcactgtga attacagcga gaacaaagct     120
   agcacatctg cttctgtaga aactcgatcc atcggtgtac aattcaaccc agggtacaaa     180
   agttccaata gcacggttgt ccccaccaag aacaaaaaaa caggaaacgg gagggtcagc     240
   gcatattttc ctagctacaa cgcagatcat gattctgtct ctaaaatcag atatgatctt     300
   tacgatgatt taatcttctt cgtggccact acaacgtcga acttcaccat tggccttggt     360
   aatctcaccc aaaacgaatg ggattcctta gcttatgaat ttgttaacag aagcaaggaa     420
   gctggagttt ctccgtcatg cagtattgga ggctggacag gatctgttta tttctctgcg     480
   ctcgcatcta ccgctgagaa tcgaaccacc tttgcaaatt ctgccattaa ttttgctaag     540
   aaatatggct tcgagggtat tgacattgat tgggagtatc cacttatcca aggaatcgga     600
   tgcaatatta ttcaagattc tgatgcagaa aattatcagt tgctcttgaa ggaaattaaa     660
   agaatctggc ctgagggaaa gcttagcaca gccgtaagca tcgcaggaat tcgtgcgtcg     720
   gactacagtg cccttcctgc tgccaattta accaccttag caagtgtcgt tgacattttg     780
   aaaattatgg catacgatgt ctatggaggc tggtcaatta cgactggacc acatgctccg     840
   ttaagaagca catgtgcgga ccccaatgat aatttgtcgg ttgaaaccgc tatcgacgtt     900
   tatatccggc aaggctttag cccgagtcaa ctttccctag gattgcctgg ttacggtaga     960
   agctggctgt tagagagtcc aacccttgtt cccaagactg ttcaaaacta cacaagttat    1020
   tactatcaaa atttcactgg attgcctcaa ggcgggaact ttgatgataa acctggagta    1080
   gttgacgttt gtggtcagac atctactagc tggggcggta ctatccttgt ctcagaattg    1140
   gtttcgcgtg gttatttgaa cgaagacgaa actaaggcag gttctggatt tgttcgatac    1200
   tatgacgagt gcagtggaca gcctttcatt gccaatggca cccatttgat ttcctacgac    1260
   gatacccaaa gtacattgca aaaagtaaaa tacgcaaaat cgcgaaacat atcacatatc    1320
   tatttctttg actcatttgg cccaacagac tctactgtta aggctgcaag agaagcatta    1380
   ctagcataa                                                            1389
<210> 84
   <211> 462
   <212> PRT
   <213> Phakopsora pachyrhizi
<220>
   <221> VARIANT
   <222> (1)..(462)
   <223> 02-h8
<400> 84
   Met Gln Asn Lys Phe Gln Pro Phe Lys Leu Leu Leu Ala Leu Ser Leu
   1               5                   10                  15
   Phe Ile Ala Gly Ser Ser Ala Asn Gln Asp Asp Gly Ser Ala Ile Thr
               20                  25                  30
   Val Asn Tyr Ser Glu Asn Lys Ala Ser Thr Ser Ala Ser Val Glu Thr
           35                  40                  45
   Arg Ser Ile Gly Val Gln Phe Asn Pro Gly Tyr Lys Ser Ser Asn Ser
       50                  55                  60
   Thr Val Val Pro Thr Lys Asn Lys Lys Thr Gly Asn Gly Arg Val Ser
   65                  70                  75                  80
   Ala Tyr Phe Pro Ser Tyr Asn Ala Asp His Asp Ser Val Ser Lys Ile
                   85                  90                  95
   Arg Tyr Asp Leu Tyr Asp Asp Leu Ile Phe Phe Val Ala Thr Thr Thr
               100                 105                 110
   Ser Asn Phe Thr Ile Gly Leu Gly Asn Leu Thr Gln Asn Glu Trp Asp
           115                 120                 125
   Ser Leu Ala Tyr Glu Phe Val Asn Arg Ser Lys Glu Ala Gly Val Ser
       130                 135                 140
   Pro Ser Cys Ser Ile Gly Gly Trp Thr Gly Ser Val Tyr Phe Ser Ala
   145                 150                 155                 160
   Leu Ala Ser Thr Ala Glu Asn Arg Thr Thr Phe Ala Asn Ser Ala Ile
                   165                 170                 175
   Asn Phe Ala Lys Lys Tyr Gly Phe Glu Gly Ile Asp Ile Asp Trp Glu
               180                 185                 190
   Tyr Pro Leu Ile Gln Gly Ile Gly Cys Asn Ile Ile Gln Asp Ser Asp
           195                 200                 205
   Ala Glu Asn Tyr Gln Leu Leu Leu Lys Glu Ile Lys Arg Ile Trp Pro
       210                 215                 220
   Glu Gly Lys Leu Ser Thr Ala Val Ser Ile Ala Gly Ile Arg Ala Ser
   225                 230                 235                 240
   Asp Tyr Ser Ala Leu Pro Ala Ala Asn Leu Thr Thr Leu Ala Ser Val
                   245                 250                 255
   Val Asp Ile Leu Lys Ile Met Ala Tyr Asp Val Tyr Gly Gly Trp Ser
               260                 265                 270
   Ile Thr Thr Gly Pro His Ala Pro Leu Arg Ser Thr Cys Ala Asp Pro
           275                 280                 285
   Asn Asp Asn Leu Ser Val Glu Thr Ala Ile Asp Val Tyr Ile Arg Gln
       290                 295                 300
   Gly Phe Ser Pro Ser Gln Leu Ser Leu Gly Leu Pro Gly Tyr Gly Arg
   305                 310                 315                 320
   Ser Trp Leu Leu Glu Ser Pro Thr Leu Val Pro Lys Thr Val Gln Asn
                   325                 330                 335
   Tyr Thr Ser Tyr Tyr Tyr Gln Asn Phe Thr Gly Leu Pro Gln Gly Gly
               340                 345                 350
   Asn Phe Asp Asp Lys Pro Gly Val Val Asp Val Cys Gly Gln Thr Ser
           355                 360                 365
   Thr Ser Trp Gly Gly Thr Ile Leu Val Ser Glu Leu Val Ser Arg Gly
       370                 375                 380
   Tyr Leu Asn Glu Asp Glu Thr Lys Ala Gly Ser Gly Phe Val Arg Tyr
   385                 390                 395                 400
   Tyr Asp Glu Cys Ser Gly Gln Pro Phe Ile Ala Asn Gly Thr His Leu
                   405                 410                 415
   Ile Ser Tyr Asp Asp Thr Gln Ser Thr Leu Gln Lys Val Lys Tyr Ala
               420                 425                 430
   Lys Ser Arg Asn Ile Ser His Ile Tyr Phe Phe Asp Ser Phe Gly Pro
           435                 440                 445
   Thr Asp Ser Thr Val Lys Ala Ala Arg Glu Ala Leu Leu Ala
       450                 455                 460
<210> 85
   <211> 615
   <212> DNA
   <213> Phakopsora pachyrhizi
<220>
   <221> misc_feature
   <222> (1)..(615)
   <223> 04-c1
<400> 85
   atgatgagag ctagcaagtc aatcgttttt atttcagcat gtttttacct gttttctgga       60
   gaaacacttg cagcctcagc tttaaaagga gtcgaaactg aggccaacag tattcctgat      120
   gcggcaaagt ttggcgagag tctttcaatc aagacagata gtctttccga gctgccgtta      180
   aaatcgtctc ctacaattcc aagtacaccg tctggtttag atggcaatcc tcaaagttca      240
   ctgctagata aaagtgcaat gaaagaaatt gatgcttcag aatcagcaga aaaaataaag      300
   agccatcctt ttacatctgg tatctctgcc aaaaggccta caaagtcgct tgacgcgtca      360
   gcaacaggct ccgacggact agttcaacag gtgcacgtcg gtgattcgca aatagctgtt      420
   gatgcgaaaa cggagattcc acccgcagcc gcttatgaac acaataaagt aactcggaaa      480
   tgtaaccatg atcatgatca tgatcaccac catcaccatc accatcatca tggccctggt      540
   gacgatcatg atcatgatca tcatcaccat caccatcacc atcatggtca tgatcatgac      600
   catgaccatg cataa                                                       615
<210> 86
   <211> 204
   <212> PRT
   <213> Phakopsora pachyrhizi
<220>
   <221> VARIANT
   <222> (1)..(204)
   <223> 04-c1
<400> 86
   Met Met Arg Ala Ser Lys Ser Ile Val Phe Ile Ser Ala Cys Phe Tyr
   1               5                   10                  15
   Leu Phe Ser Gly Glu Thr Leu Ala Ala Ser Ala Leu Lys Gly Val Glu
               20                  25                  30
   Thr Glu Ala Asn Ser Ile Pro Asp Ala Ala Lys Phe Gly Glu Ser Leu
           35                  40                  45
   Ser Ile Lys Thr Asp Ser Leu Ser Glu Leu Pro Leu Lys Ser Ser Pro
       50                  55                  60
   Thr Ile Pro Ser Thr Pro Ser Gly Leu Asp Gly Asn Pro Gln Ser Ser
   65                  70                  75                  80
   Leu Leu Asp Lys Ser Ala Met Lys Glu Ile Asp Ala Ser Glu Ser Ala
                   85                  90                  95
   Glu Lys Ile Lys Ser His Pro Phe Thr Ser Gly Ile Ser Ala Lys Arg
               100                 105                 110
   Pro Thr Lys Ser Leu Asp Ala Ser Ala Thr Gly Ser Asp Gly Leu Val
           115                 120                 125
   Gln Gln Val His Val Gly Asp Ser Gln Ile Ala Val Asp Ala Lys Thr
       130                 135                 140
   Glu Ile Pro Pro Ala Ala Ala Tyr Glu His Asn Lys Val Thr Arg Lys
   145                 150                 155                 160
   Cys Asn His Asp His Asp His Asp His His His His His His His His
                   165                 170                 175
   His Gly Pro Gly Asp Asp His Asp His Asp His His His His His His
               180                 185                 190
   His His His Gly His Asp His Asp His Asp His Ala
           195                 200
<210> 87
   <211> 549
   <212> DNA
   <213> Phakopsora pachyrhizi
<220>
   <221> misc_feature
   <222> (1)..(549)
   <223> 04-d11
<400> 87
   atgcaaggtt tttttcttgt ttccattgca gttcaggttg ctctcgtagt tgctcaagga      60
   aacaccccca ccaaagttac tgctcagcct caggtaaaca actcgtctgc tccggcaaac     120
   gcatctgcta ttgatctgcc aaaaataata gctgttaatt gtacacaagc ctacatcgaa     180
   ataactgaca gctcaattaa cggaactact attggcgctt gcaaaaggtt tgaagattct     240
   gaaggatctt tttgcaagcc tgatgattgc attggcagtg ccacttgctt atcctgcaaa     300
   aaagttaccg ttggtgccac tgcgaatagc accgttgtct cgaactcaac agtagactct     360
   gtggtgtgcc gcaaggacta cttctttcca ggaccgggtt ctaaatccaa ccagtccctt     420
   tgtacggatg agaaggatga taactacatc tgcagtggtg tttgcacaag ttttgctagc     480
   tgctctcatt gcgtcagcgc taaagaccca gcacttgcaa aattaaaaga gaagtctcca     540
   aaaaattaa                                                             549
<210> 88
   <211> 182
   <212> PRT
   <213> Phakopsora pachyrhizi
<220>
   <221> VARIANT
   <222> (1)..(182)
   <223> 04-d11
<400> 88
   Met Gln Gly Phe Phe Leu Val Ser Ile Ala Val Gln Val Ala Leu Val
   1               5                   10                  15
   Val Ala Gln Gly Asn Thr Pro Thr Lys Val Thr Ala Gln Pro Gln Val
               20                  25                  30
   Asn Asn Ser Ser Ala Pro Ala Asn Ala Ser Ala Ile Asp Leu Pro Lys
           35                  40                  45
   Ile Ile Ala Val Asn Cys Thr Gln Ala Tyr Ile Glu Ile Thr Asp Ser
       50                  55                  60
   Ser Ile Asn Gly Thr Thr Ile Gly Ala Cys Lys Arg Phe Glu Asp Ser
   65                  70                  75                  80
   Glu Gly Ser Phe Cys Lys Pro Asp Asp Cys Ile Gly Ser Ala Thr Cys
                   85                  90                  95
   Leu Ser Cys Lys Lys Val Thr Val Gly Ala Thr Ala Asn Ser Thr Val
               100                 105                 110
   Val Ser Asn Ser Thr Val Asp Ser Val Val Cys Arg Lys Asp Tyr Phe
           115                 120                 125
   Phe Pro Gly Pro Gly Ser Lys Ser Asn Gln Ser Leu Cys Thr Asp Glu
       130                 135                 140
   Lys Asp Asp Asn Tyr Ile Cys Ser Gly Val Cys Thr Ser Phe Ala Ser
   145                 150                 155                 160
   Cys Ser His Cys Val Ser Ala Lys Asp Pro Ala Leu Ala Lys Leu Lys
                   165                 170                 175
   Glu Lys Ser Pro Lys Asn
               180
<210> 89
   <211> 1326
   <212> DNA
   <213> Phakopsora pachyrhizi
<220>
   <221> misc_feature
   <222> (1)..(1326)
   <223> 27i10
<400> 89
   atggttctcc gtacttctgt tcttgtgatt gcaatcgcag cagtaaacct gtgctttttc      60
   cagacaaatg ctacagatat ccttggaaga gataatcaac tcatccgtcg gcagacactc     120
   agtccaccgc cagcgagact caatacaaca gaatgcccgg atctacctat aagtccccaa     180
   acctggaagt ctctgaatct cgatgattac ttaaagcaat acccaggtgg tgttaacata     240
   tcacttcagg aatacgcatt tgcacatcat gcccaaaact tcatctgcgg tgtaggagaa     300
   gggtgcaacg ccggccagct ttgcaatcca atcacagcac ccgactggta tgtcttatat     360
   gccacccaag agtggaacgc aatgcagaac gcaatctata ctgcagtagg gtttgccgta     420
   tcaatggtcc aagctactgc tgcagctatg gtcactgact tctatccacc agagcataaa     480
   tcggtactat acaagctcaa tgacttgttt gtaatgctta gtgctgtagc attcactgta     540
   gcagttttat cattgctact tgactggcca gtggccgtat ttgtaggagc ggcaattggt      600
   ggttctttgg ccgccggtac ttccgcagtc ttaactggag tcaatcttgc aaattcttgg      660
   aatataaagc cagacggatt tactaagtgg tctaattacg cctactacct atcccaatgg      720
   caatcaaaag tccaagacga gctggctaac aatgctgcat cagtgatttc agccggaatt      780
   tcaagcactg ctggaatttc tgaagcactt aaggggggga atttccttac cgatgtccaa      840
   attaggccaa cctctgaaat tgaagatgag ataaaatata caatgagtgc ccgaatttta      900
   gttgatatta ttcgtaacca gggtggttac gttacttatg gaagtgaccc gtgcgacggg      960
   aaaggaccga atggggcatg ggatggcgaa gacgtgattt ctttctgcaa aaacggaact     1020
   atgatgaaca ttgttagagc aaagggaaac aagacgaaga aaaagtggta caatgcccgc     1080
   cttatagctt caaagtacgg cctcactgct gaatatctta ctactcaatc tgtggaatgc     1140
   caaaagaaat ataagacttt tggatacgat ccttacagga atggatctct tcctaaatca     1200
   gcatccgaag aatgtattgt taatttacct gtctgcgatt gcactagtcc agaaataaaa     1260
   cgtgctagaa agaaaggaca cacaacaact gttgcatgcc gtgaggttgg gaagcttcct     1320
   atctga                                                                1326
<210> 90
   <211> 441
   <212> PRT
   <213> Phakopsora pachyrhizi
<220>
   <221> VARIANT
   <222> (1)..(441)
   <223> 27i10
<400> 90
   Met Val Leu Arg Thr Ser Val Leu Val Ile Ala Ile Ala Ala Val Asn
   1               5                   10                  15
   Leu Cys Phe Phe Gln Thr Asn Ala Thr Asp Ile Leu Gly Arg Asp Asn
               20                  25                  30
   Gln Leu Ile Arg Arg Gln Thr Leu Ser Pro Pro Pro Ala Arg Leu Asn
           35                  40                  45
   Thr Thr Glu Cys Pro Asp Leu Pro Ile Ser Pro Gln Thr Trp Lys Ser
       50                  55                  60
   Leu Asn Leu Asp Asp Tyr Leu Lys Gln Tyr Pro Gly Gly Val Asn Ile
   65                  70                  75                  80
   Ser Leu Gln Glu Tyr Ala Phe Ala His His Ala Gln Asn Phe Ile Cys
                   85                  90                  95
   Gly Val Gly Glu Gly Cys Asn Ala Gly Gln Leu Cys Asn Pro Ile Thr
               100                 105                 110
   Ala Pro Asp Trp Tyr Val Leu Tyr Ala Thr Gln Glu Trp Asn Ala Met
           115                 120                 125
   Gln Asn Ala Ile Tyr Thr Ala Val Gly Phe Ala Val Ser Met Val Gln
       130                 135                 140
   Ala Thr Ala Ala Ala Met Val Thr Asp Phe Tyr Pro Pro Glu His Lys
   145                 150                 155                 160
   Ser Val Leu Tyr Lys Leu Asn Asp Leu Phe Val Met Leu Ser Ala Val
                   165                 170                 175
   Ala Phe Thr Val Ala Val Leu Ser Leu Leu Leu Asp Trp Pro Val Ala
               180                 185                 190
   Val Phe Val Gly Ala Ala Ile Gly Gly Ser Leu Ala Ala Gly Thr Ser
           195                 200                 205
   Ala Val Leu Thr Gly Val Asn Leu Ala Asn Ser Trp Asn Ile Lys Pro
       210                 215                 220
   Asp Gly Phe Thr Lys Trp Ser Asn Tyr Ala Tyr Tyr Leu Ser Gln Trp
   225                 230                 235                 240
   Gln Ser Lys Val Gln Asp Glu Leu Ala Asn Asn Ala Ala Ser Val Ile
                   245                 250                 255
   Ser Ala Gly Ile Ser Ser Thr Ala Gly Ile Ser Glu Ala Leu Lys Gly
               260                 265                 270
   Gly Asn Phe Leu Thr Asp Val Gln Ile Arg Pro Thr Ser Glu Ile Glu
           275                 280                 285
   Asp Glu Ile Lys Tyr Thr Met Ser Ala Arg Ile Leu Val Asp Ile Ile
       290                 295                 300
   Arg Asn Gln Gly Gly Tyr Val Thr Tyr Gly Ser Asp Pro Cys Asp Gly
   305                 310                 315                 320
   Lys Gly Pro Asn Gly Ala Trp Asp Gly Glu Asp Val Ile Ser Phe Cys
                   325                 330                 335
   Lys Asn Gly Thr Met Met Asn Ile Val Arg Ala Lys Gly Asn Lys Thr
                   340                 345                 350
   Lys Lys Lys Trp Tyr Asn Ala Arg Leu Ile Ala Ser Lys Tyr Gly Leu
           355                 360                 365
   Thr Ala Glu Tyr Leu Thr Thr Gln Ser Val Glu Cys Gln Lys Lys Tyr
       370                 375                 380
   Lys Thr Phe Gly Tyr Asp Pro Tyr Arg Asn Gly Ser Leu Pro Lys Ser
   385                 390                 395                 400
   Ala Ser Glu Glu Cys Ile Val Asn Leu Pro Val Cys Asp Cys Thr Ser
                   405                 410                 415
   Pro Glu Ile Lys Arg Ala Arg Lys Lys Gly His Thr Thr Thr Val Ala
               420                 425                 430
   Cys Arg Glu Val Gly Lys Leu Pro Ile
           435                 440
<210> 91
   <211> 219
   <212> DNA
   <213> Phakopsora pachyrhizi
<220>
   <221> misc_feature
   <222> (1)..(219)
   <223> 17j13
<400> 91
   atgatcgttc gaactttttt taatgctctc atagcaggtg ggatagccgg tacaaccgtc       60
   gatctggtct tctaccctct ggataccatc aagactaggc tacaatcatc gcagggtttt      120
   ctgaattccg gcggtctgag aggagtttac aaagggttgg gtagcgttgc tgtcggtagt      180
   gctcctggtg gttgctgccc tattttttac tacttatga                              219
<210> 92
   <211> 72
   <212> PRT
   <213> Phakopsora pachyrhizi
<220>
   <221> VARIANT
   <222> (1)..(72)
   <223> 17j13
<400> 92
   Met Ile Val Arg Thr Phe Phe Asn Ala Leu Ile Ala Gly Gly Ile Ala
   1               5                   10                  15
   Gly Thr Thr Val Asp Leu Val Phe Tyr Pro Leu Asp Thr Ile Lys Thr
               20                  25                  30
   Arg Leu Gln Ser Ser Gln Gly Phe Leu Asn Ser Gly Gly Leu Arg Gly
           35                  40                  45
   Val Tyr Lys Gly Leu Gly Ser Val Ala Val Gly Ser Ala Pro Gly Gly
       50                  55                  60
   Cys Cys Pro Ile Phe Tyr Tyr Leu
   65                  70
<210> 93
   <211> 606
   <212> DNA
   <213> Phakopsora pachyrhizi
<220>
   <221> misc_feature
   <222> (1)..(606)
   <223> 17k20
<400> 93
   atgcaattcc ttaccctagt cactcttttg atcacatctc agctggcatc gtcggtgcca      60
   cttgttgagc gagctgaaac tcgcacctct gcagagcatg tgaatcaaaa aggattcttt     120
   ggaggactac cttgcgcacc tatcggtgga atgcttcctc ctccagtggg cggtttcttg     180
   cccccaccac caattggcgg gtttcttcct ccaccaccaa tcggaggctt cggaggcttc     240
   ggtggcctag gtggctttgg cctaccccct ccaccaattg gcggattcgg tggtcttgga     300
   ttcggtggac taccacctcc cccgatcgga ggtctaggct ttggtggctt acctccccct     360
   atgattggtg gttttggagg tctaggaggt ggaattggcg gtatgggtgg tttcggtgga     420
   atkggtggtg gtggtttcgg tggtgccagc ggattcaacc gaaactcttt cagctctgtt     480
   caatctggct cttcatctaa cggattcaaa gctggtggtg gccaatttgg cggagctggt     540
   ggtttaggtg gaattggtgg aatgggtggt ttcttgaaaa acaacaaaga gaagagcgaa     600
   gcataa                                                                606
<210> 94
   <211> 201
   <212> PRT
   <213> Phakopsora pachyrhizi
<220>
   <221> VARIANT
   <222> (1)..(201)
   <223> 17k20
<400> 94
   Met Gln Phe Leu Thr Leu Val Thr Leu Leu Ile Thr Ser Gln Leu Ala
   1               5                   10                  15
   Ser Ser Val Pro Leu Val Glu Arg Ala Glu Thr Arg Thr Ser Ala Glu
               20                  25                  30
   His Val Asn Gln Lys Gly Phe Phe Gly Gly Leu Pro Cys Ala Pro Ile
           35                  40                  45
   Gly Gly Met Leu Pro Pro Pro Val Gly Gly Phe Leu Pro Pro Pro Pro
       50                  55                  60
   Ile Gly Gly Phe Leu Pro Pro Pro Pro Ile Gly Gly Phe Gly Gly Phe
   65                  70                  75                  80
   Gly Gly Leu Gly Gly Phe Gly Leu Pro Pro Pro Pro Ile Gly Gly Phe
                   85                  90                  95
   Gly Gly Leu Gly Phe Gly Gly Leu Pro Pro Pro Pro Ile Gly Gly Leu
               100                 105                 110
   Gly Phe Gly Gly Leu Pro Pro Pro Met Ile Gly Gly Phe Gly Gly Leu
           115                 120                 125
   Gly Gly Gly Ile Gly Gly Met Gly Gly Phe Gly Gly Met Gly Gly Gly
       130                 135                 140
   Gly Phe Gly Gly Ala Ser Gly Phe Asn Arg Asn Ser Phe Ser Ser Val
   145                 150                 155                 160
   Gln Ser Gly Ser Ser Ser Asn Gly Phe Lys Ala Gly Gly Gly Gln Phe
                   165                 170                 175
   Gly Gly Ala Gly Gly Leu Gly Gly Ile Gly Gly Met Gly Gly Phe Leu
               180                 185                 190
   Lys Asn Asn Lys Glu Lys Ser Glu Ala
           195                 200
<210> 95
   <211> 663
   <212> DNA
   <213> Phakopsora pachyrhizi
<220>
   <221> misc_feature
   <222> (1)..(663)
   <223> 15L1
<400> 95
   atgaaagtaa aactgttttc attgaagatc attataagtc ttgctacttt atctcgtgcg      60
   acgttttttg attccaagaa agcattatta tctgtgaagg actctgagcc atctttctca      120
   cagagtggcg aacaaagctt gcaacattgg aactctcaag tggggtcttc taaatcacca     180
   accgccaact tattcccaag agaatcagat tatattccac aagccaatca acagcaactt     240
   ggggagaagc gaagcaggga aggtgttctc ttagacgcgt tcactaaaga ccctgaatcc     300
   cgtcctgctc tacacctagg tctctcacaa tcagcattcc ctgaaactga aaatcaaaca     360
   tcagagatta aaaagcttcg tcttagcagt ctttctacac ctgagcatgg actaaaacat     420
   tcggacattg tctttgaatc agctcataat gagcctggat cacataatga tctgagtcta     480
   gatcttctac gatcggatat ggagaaaaga catagttcta ccagtgatac tctcgatcaa     540
   ggactaaaaa atccgcaatt tagttctaca tcctcaagta aagatcgaga agtagataat     600
   caccacattg atgctgcgca cttcgatttt tttaaagtct atgaaaaaat ccaccttgat     660
   aaa                                                                   663
<210> 96
   <211> 221
   <212> PRT
   <213> Phakopsora pachyrhizi
<220>
   <221> VARIANT
   <222> (1)..(221)
   <223> 15L1
<400> 96
   Met Lys Val Lys Leu Phe Ser Leu Lys Ile Ile Ile Ser Leu Ala Thr
   1               5                   10                  15
   Leu Ser Arg Ala Thr Phe Phe Asp Ser Lys Lys Ala Leu Leu Ser Val
               20                  25                  30
   Lys Asp Ser Glu Pro Ser Phe Ser Gln Ser Gly Glu Gln Ser Leu Gln
           35                  40                  45
   His Trp Asn Ser Gln Val Gly Ser Ser Lys Ser Pro Thr Ala Asn Leu
       50                  55                  60
   Phe Pro Arg Glu Ser Asp Tyr Ile Pro Gln Ala Asn Gln Gln Gln Leu
   65                  70                  75                  80
   Gly Glu Lys Arg Ser Arg Glu Gly Val Leu Leu Asp Ala Phe Thr Lys
                   85                  90                  95
   Asp Pro Glu Ser Arg Pro Ala Leu His Leu Gly Leu Ser Gln Ser Ala
               100                 105                 110
   Phe Pro Glu Thr Glu Asn Gln Thr Ser Glu Ile Lys Lys Leu Arg Leu
           115                 120                 125
   Ser Ser Leu Ser Thr Pro Glu His Gly Leu Lys His Ser Asp Ile Val
       130                 135                 140
   Phe Glu Ser Ala His Asn Glu Pro Gly Ser His Asn Asp Leu Ser Leu
   145                 150                 155                 160
   Asp Leu Leu Arg Ser Asp Met Glu Lys Arg His Ser Ser Thr Ser Asp
                   165                 170                 175
   Thr Leu Asp Gln Gly Leu Lys Asn Pro Gln Phe Ser Ser Thr Ser Ser
               180                 185                 190
   Ser Lys Asp Arg Glu Val Asp Asn His His Ile Asp Ala Ala His Phe
           195                 200                 205
   Asp Phe Phe Lys Val Tyr Glu Lys Ile His Leu Asp Lys
       210                 215                 220
<210> 97
   <211> 810
   <212> DNA
   <213> Phakopsora pachyrhizi
<220>
   <221> misc_feature
   <222> (1)..(810)
   <223> 30d13
<400> 97
   atggctttga cctcggaggg taaccatgcg atcgctatca agatcctcaa cgtcttagtc       60
   ttcttcttct ttttcggttc taacgtctat agctctcttg gtggtccttc gaccggttac      120
   tattctcaga aggaaaccta cattactcct gcccctgaaa cgttttggat atggacggtc      180
   atcaacctcc ttttccttgg ctttgtaatc tttcaattct ttgaagcggg cacaaaggcc      240
   attgttgacg ttgtcagttg gcgttttgcg gcgattggag ttttacaatc gatctggatt      300
   catctatccg ttggccatca ttacatctta gcctttgtat tctctctgat tgtggcatca      360
   cttgtctcac atgtttattg ggacctgaaa tcctctgact tgaaatccaa ggccgagtta      420
   attttcgttc atctaccatt cagcctctta catgcctacc tggtattttt gttggtttta      480
   agtgctttta cagcttttgg agttgataag gcagaacatc cagccggtat catcacccag      540
   atccttgtat caatcgcact agtagcactt gctttaaccg gaatgggata tgccttccac      600
   agcgatcaag gcgacatagc cggcgctgtc gtcatcgcac ttgagcttgc cggtgtattt      660
   gctcgtcaga gtaagcctga taccattcac tgggttgctt tcgtgtcttt cttggtcact      720
   ttggttgcgg ttctcaaggc aatttacttt actgttaagg gcggaagaat ccgtctcgag      780
   gattctgagc gcgcaccatt gattggctga                                      810
<210> 98
   <211> 269
   <212> PRT
   <213> Phakopsora pachyrhizi
<220>
   <221> VARIANT
   <222> (1)..(269)
   <223> 30d13
<400> 98
   Met Ala Leu Thr Ser Glu Gly Asn His Ala Ile Ala Ile Lys Ile Leu
   1               5                   10                  15
   Asn Val Leu Val Phe Phe Phe Phe Phe Gly Ser Asn Val Tyr Ser Ser
               20                  25                  30
   Leu Gly Gly Pro Ser Thr Gly Tyr Tyr Ser Gln Lys Glu Thr Tyr Ile
           35                  40                  45
   Thr Pro Ala Pro Glu Thr Phe Trp Ile Trp Thr Val Ile Asn Leu Leu
       50                  55                  60
   Phe Leu Gly Phe Val Ile Phe Gln Phe Phe Glu Ala Gly Thr Lys Ala
   65                  70                  75                  80
   Ile Val Asp Val Val Ser Trp Arg Phe Ala Ala Ile Gly Val Leu Gln
                   85                  90                  95
   Ser Ile Trp Ile His Leu Ser Val Gly His His Tyr Ile Leu Ala Phe
               100                 105                 110
   Val Phe Ser Leu Ile Val Ala Ser Leu Val Ser His Val Tyr Trp Asp
           115                 120                 125
   Leu Lys Ser Ser Asp Leu Lys Ser Lys Ala Glu Leu Ile Phe Val His
       130                 135                 140
   Leu Pro Phe Ser Leu Leu His Ala Tyr Leu Val Phe Leu Leu Val Leu
   145                 150                 155                 160
   Ser Ala Phe Thr Ala Phe Gly Val Asp Lys Ala Glu His Pro Ala Gly
                   165                 170                 175
   Ile Ile Thr Gln Ile Leu Val Ser Ile Ala Leu Val Ala Leu Ala Leu
               180                 185                 190
   Thr Gly Met Gly Tyr Ala Phe His Ser Asp Gln Gly Asp Ile Ala Gly
           195                 200                 205
   Ala Val Val Ile Ala Leu Glu Leu Ala Gly Val Phe Ala Arg Gln Ser
       210                 215                 220
   Lys Pro Asp Thr Ile His Trp Val Ala Phe Val Ser Phe Leu Val Thr
   225                 230                 235                 240
   Leu Val Ala Val Leu Lys Ala Ile Tyr Phe Thr Val Lys Gly Gly Arg
                   245                 250                 255
   Ile Arg Leu Glu Asp Ser Glu Arg Ala Pro Leu Ile Gly
               260                 265
<210> 99
   <211> 813
   <212> DNA
   <213> Phakopsora pachyrhizi
<220>
   <221> misc_feature
   <222> (1)..(813)
   <223> HESP-735
<400> 99
   atgaaccgaa ttaggatagc catcgtctta gcttctttaa gcttggcgaa ctgccaattt        60
   ttatttgggg atcctgcagc agatgctcag aacagctcaa caaatcaggc taattcaaca       120
   ggctctaaac tttcacccac tcaaagcagt gaccaaactg ttcctttgac ttattcctca       180
   aattccagtg atacaaactc attgaactca gctaatggca cagctactaa tagctctatc       240
   ctaacaggcg gcacgaacgc ttttgacaat actggttcta atagttccat caaaacaaac       300
   agcagcagcc tttttaacgg tactgctacc aacagctcta ttcaatccac agggctcatt       360
   ggtagctata atgggacaat ctcaccctcc aaaaacactt caatgttttc agaagtctac       420
   actttagact acagtattaa atgggatgaa agtgatttca atgtctatgg ccaaaatggt       480
   aaagttgaat ataccattag taacaaagtg gagggagtca acatgtcaaa gaaagaattt       540
   gttgtgaaag aagccaccga tggacaagca agagttagaa ttgacgccaa taataaattc       600
   tgtggatttg gtaaaactta cacatcagat gatggggcaa gctttacaat cgacccacgc       660
   atgtttttac ctgatcgctg gtttatcagg caaagcaatg ttacgtacgt ttttaaacgc       720
   ttcgccatga gtcttaatgg agatatcttg gatgtcgaaa acaagcgcct tgtagcgcag       780
   gtcaaagtcg acaaagctaa tactacgagt taa                                    813
<210> 100
   <211> 270
   <212> PRT
   <213> Phakopsora pachyrhizi
<220>
   <221> VARIANT
   <222> (1)..(270)
   <223> HESP-735
<400> 100
   Met Asn Arg Ile Arg Ile Ala Ile Val Leu Ala Ser Leu Ser Leu Ala
   1               5                   10                  15
   Asn Cys Gln Phe Leu Phe Gly Asp Pro Ala Ala Asp Ala Gln Asn Ser
               20                  25                  30
   Ser Thr Asn Gln Ala Asn Ser Thr Gly Ser Lys Leu Ser Pro Thr Gln
           35                  40                  45
   Ser Ser Asp Gln Thr Val Pro Leu Thr Tyr Ser Ser Asn Ser Ser Asp
       50                  55                  60
   Thr Asn Ser Leu Asn Ser Ala Asn Gly Thr Ala Thr Asn Ser Ser Ile
   65                  70                  75                  80
   Leu Thr Gly Gly Thr Asn Ala Phe Asp Asn Thr Gly Ser Asn Ser Ser
                   85                  90                  95
   Ile Lys Thr Asn Ser Ser Ser Leu Phe Asn Gly Thr Ala Thr Asn Ser
               100                 105                 110
   Ser Ile Gln Ser Thr Gly Leu Ile Gly Ser Tyr Asn Gly Thr Ile Ser
           115                 120                 125
   Pro Ser Lys Asn Thr Ser Met Phe Ser Glu Val Tyr Thr Leu Asp Tyr
       130                 135                 140
   Ser Ile Lys Trp Asp Glu Ser Asp Phe Asn Val Tyr Gly Gln Asn Gly
   145                 150                 155                 160
   Lys Val Glu Tyr Thr Ile Ser Asn Lys Val Glu Gly Val Asn Met Ser
                   165                 170                 175
   Lys Lys Glu Phe Val Val Lys Glu Ala Thr Asp Gly Gln Ala Arg Val
               180                 185                 190
   Arg Ile Asp Ala Asn Asn Lys Phe Cys Gly Phe Gly Lys Thr Tyr Thr
           195                 200                 205
   Ser Asp Asp Gly Ala Ser Phe Thr Ile Asp Pro Arg Met Phe Leu Pro
       210                 215                 220
   Asp Arg Trp Phe Ile Arg Gln Ser Asn Val Thr Tyr Val Phe Lys Arg
   225                 230                 235                 240
   Phe Ala Met Ser Leu Asn Gly Asp Ile Leu Asp Val Glu Asn Lys Arg
                   245                 250                 255
   Leu Val Ala Gln Val Lys Val Asp Lys Ala Asn Thr Thr Ser
               260                 265                 270
<210> 101
   <211> 885
   <212> DNA
   <213> Phakopsora pachyrhizi
<220>
   <221> misc_feature
   <222> (1)..(885)
   <223> 04-m23
<400> 101
   atgaagcttt tcagctgtac aaatgagtgg aacattttgc cctttgtatt attaagcgcg      60
   tggtatacca gaaccgttct tggaatgcat tttgccgaaa gctcaaagga aaatattatc     120
   agctctgcaa agggtctact acaggaacag cctccggaaa atatactaat tacaaaaggt     180
   cgcttaggcc ctaaaccaga tgactcagca tttactttta aaagtggaaa acgttataca     240
   cacgtagagg aacaaaaaac tagtgatatt cctgatccta cacgagtgcc aaaatttgat     300
   agaattgaag aaaagatctc aatgctatac aagcaaaaca attttaaaag gaacatgcat     360
   ctggacgata ttgttaggaa agaccttcta gtttatctca acaatatcaa aaataaagca     420
   ataaaagata atctgccaac cttggatgcc gaattaggca caatatttac acttcttgct     480
   ttaaaattga ggggcatttc agataataat ataacattaa aaatttctgc tacttttgaa     540
   actcttagtg aaaaaatttt aagcaaaata ttttcaaaag tcgaattgaa agaagatgat     600
   ctattgaaat acaaaatatt gagtgaaatg gttacagtgc tgatggctct ctttgccaat     660
   cgagattatt tagcaattca gtcattagtt gaaggtaaaa acgttatcag gctcctagct     720
   agcgaattaa attttataac tagaaataaa ttagaattac tggtattaga taaatcttat     780
   attgattttt tcttgaagtc taaagcaacg gaaaatttcc ataagatctt gaattatctc     840
   aagagtgaaa aaactcaaga aaagttacat agtttgttag agtaa                     885
<210> 102
   <211> 294
   <212> PRT
   <213> Phakopsora pachyrhizi
<220>
   <221> VARIANT
   <222> (1)..(294)
   <223> 04-m23
<400> 102
   Met Lys Leu Phe Ser Cys Thr Asn Glu Trp Asn Ile Leu Pro Phe Val
   1               5                   10                  15
   Leu Leu Ser Ala Trp Tyr Thr Arg Thr Val Leu Gly Met His Phe Ala
               20                  25                  30
   Glu Ser Ser Lys Glu Asn Ile Ile Ser Ser Ala Lys Gly Leu Leu Gln
           35                  40                  45
   Glu Gln Pro Pro Glu Asn Ile Leu Ile Thr Lys Gly Arg Leu Gly Pro
       50                  55                  60
   Lys Pro Asp Asp Ser Ala Phe Thr Phe Lys Ser Gly Lys Arg Tyr Thr
   65                  70                  75                  80
   His Val Glu Glu Gln Lys Thr Ser Asp Ile Pro Asp Pro Thr Arg Val
                   85                  90                  95
   Pro Lys Phe Asp Arg Ile Glu Glu Lys Ile Ser Met Leu Tyr Lys Gln
               100                 105                 110
   Asn Asn Phe Lys Arg Asn Met His Leu Asp Asp Ile Val Arg Lys Asp
           115                 120                 125
   Leu Leu Val Tyr Leu Asn Asn Ile Lys Asn Lys Ala Ile Lys Asp Asn
       130                 135                 140
   Leu Pro Thr Leu Asp Ala Glu Leu Gly Thr Ile Phe Thr Leu Leu Ala
   145                 150                 155                 160
   Leu Lys Leu Arg Gly Ile Ser Asp Asn Asn Ile Thr Leu Lys Ile Ser
                   165                 170                 175
   Ala Thr Phe Glu Thr Leu Ser Glu Lys Ile Leu Ser Lys Ile Phe Ser
               180                 185                 190
   Lys Val Glu Leu Lys Glu Asp Asp Leu Leu Lys Tyr Lys Ile Leu Ser
           195                 200                 205
   Glu Met Val Thr Val Leu Met Ala Leu Phe Ala Asn Arg Asp Tyr Leu
       210                 215                 220
   Ala Ile Gln Ser Leu Val Glu Gly Lys Asn Val Ile Arg Leu Leu Ala
   225                 230                 235                 240
   Ser Glu Leu Asn Phe Ile Thr Arg Asn Lys Leu Glu Leu Leu Val Leu
                   245                 250                 255
   Asp Lys Ser Tyr Ile Asp Phe Phe Leu Lys Ser Lys Ala Thr Glu Asn
               260                 265                 270
   Phe His Lys Ile Leu Asn Tyr Leu Lys Ser Glu Lys Thr Gln Glu Lys
           275                 280                 285
   Leu His Ser Leu Leu Glu
       290
<210> 103
   <211> 351
   <212> DNA
   <213> Phakopsora pachyrhizi
<220>
   <221> misc_feature
   <222> (1)..(351)
   <223> 13o11
<400> 103
   atggcaactt tcttggcaaa gaacgtagcc cttgtacctc tcttcgttgc cgtggggtta       60
   ggtctaggag gtggaatagg atttggaatc cattatttaa agaataacca agacgtcgtt      120
   ttaagaaaaa agtcaaaccc agacccatgg aacaaagttc cacaggacaa caacacgaag      180
   ttgttctctt ttaatccgga cttctggcga gctagagccc aattgactga tccaagatta      240
   agttttatgg aaagtaaacc agagaatgag agaactttac atgaacaagc catggtcgaa      300
   agggctaagc agatcagact gaatgacaag gaacgtacca ttcacagttg a               351
<210> 104
   <211> 116
   <212> PRT
   <213> Phakopsora pachyrhizi
<220>
   <221> VARIANT
   <222> (1)..(116)
   <223> 13o11
<400> 104
   Met Ala Thr Phe Leu Ala Lys Asn Val Ala Leu Val Pro Leu Phe Val
   1               5                   10                  15
   Ala Val Gly Leu Gly Leu Gly Gly Gly Ile Gly Phe Gly Ile His Tyr
               20                  25                  30
   Leu Lys Asn Asn Gln Asp Val Val Leu Arg Lys Lys Ser Asn Pro Asp
           35                  40                  45
   Pro Trp Asn Lys Val Pro Gln Asp Asn Asn Thr Lys Leu Phe Ser Phe
       50                  55                  60
   Asn Pro Asp Phe Trp Arg Ala Arg Ala Gln Leu Thr Asp Pro Arg Leu
   65                  70                  75                  80
   Ser Phe Met Glu Ser Lys Pro Glu Asn Glu Arg Thr Leu His Glu Gln
                   85                  90                  95
   Ala Met Val Glu Arg Ala Lys Gln Ile Arg Leu Asn Asp Lys Glu Arg
               100                 105                 110
   Thr Ile His Ser
           115
<210> 105
   <211> 1602
   <212> DNA
   <213> Phakopsora pachyrhizi
<220>
   <221> misc_feature
   <222> (1)..(1602)
   <223> 14c16
<400> 105
   atggcgcttc gtcaatttaa cacatgcttt gttctctatt gcactacgtt gatcttattg      60
   tcaacgatcg ttatgagtgg catggtaccg aaatctgaag gcgctgaaaa aataattggc     120
   gacggcgtca aaattgaaga cgtcacgttt aaagatatca acgaagcatc ctctggcggc     180
   tggagggagt accaggaggg ttttttagca tctggcagtc ctgccacgag ttatgatgat     240
   aatactaaaa ccacaagaca tattcctata cgcgaatttc ctctatcaaa gccaagaaaa     300
   tcgttgggga ccgcgaaaca tatggctgat caatttttcg aagctaaaag tcgccaatta     360
   agaagttgta cccaactgct tgccaagctt caaaattcaa gagaagaaat ccaaaactct     420
   atagataaag aagtttttaa caatctacag agcttgcttg cgcagctaaa tgattataca     480
   gacaaatact taaacgaact gaaacgccgt aagagtgcta atcttttatt gaatgaaaga     540
   tttgtaaatt tgattttgga agactttgcc aaagtcttcg agcacatatc tcaacatttt     600
   tatccagctt ttaagataga gaattcgcca gcaacatctt ttacgtttca tatcattaat     660
   tggcttgccg aaaatgtgca agatatgcac ttaattttgt tcaaactatt aaatagggaa     720
   aagatgatgg aaggactaga gtggtacact tcttctcaga ttattcttaa aaggggctat     780
   attgattttt ttaaaacgga tgatataaaa aactatttgc tgaatcatca agacttaaga     840
   gaaataaaga acatattaaa atatctaggg gaaaaacatt ggaaaaagct tgaacttggg     900
   ttcattaagt ctcatctgaa cccatcattt aatatggaaa caaatcgccc aatggtaaat     960
   ttaaacactg ttaacgattt taatgaaatt ctaaaatcga tagagaatgc ccagagcttt    1020
   aatcctgaat ggctgaaaaa actaagtatg gattcgaaag agaaaaacgt tgctttctat    1080
   ttcacagaga agatatttgt gcttcatatc ctaagttatt gccaaaaata cttcggaact    1140
   ataaaagagg accagctgat gatgcaaaaa gtagattatt tcaagagaat tgttgagctg    1200
   tatagaaaac agttagagat gtatgaagag ctcgtagatt ctggaaattt aagcagaatt    1260
   atactttcag aaaaattttc ggttatgaat cacattcttg cactaaaacc atcggaaata    1320
   gagaaaagtt ttccggtaga cgtggtctta ataggcgact ccaagtttga attccttaaa    1380
   acacgtaata ctttaggtga acattggaag tctagatata caaaccagtg cagcatttta    1440
   gagacagctc aaaaatatat acaagatcat cattacttaa ttcaaatcaa cataaatgga    1500
   atgacattta acctcaggtc aaagcccact ctatccgaac aattttatga tgaatttttt    1560
   tctttaagta aagtttacac ggagcttacc aaatatttat ag                       1602
<210> 106
   <211> 533
   <212> PRT
   <213> Phakopsora pachyrhizi
<220>
   <221> VARIANT
   <222> (1)..(533)
   <223> 14c16
<400> 106
   Met Ala Leu Arg Gln Phe Asn Thr Cys Phe Val Leu Tyr Cys Thr Thr
   1               5                   10                  15
   Leu Ile Leu Leu Ser Thr Ile Val Met Ser Gly Met Val Pro Lys Ser
               20                  25                  30
   Glu Gly Ala Glu Lys Ile Ile Gly Asp Gly Val Lys Ile Glu Asp Val
           35                  40                  45
   Thr Phe Lys Asp Ile Asn Glu Ala Ser Ser Gly Gly Trp Arg Glu Tyr
       50                  55                  60
   Gln Glu Gly Phe Leu Ala Ser Gly Ser Pro Ala Thr Ser Tyr Asp Asp
   65                  70                  75                  80
   Asn Thr Lys Thr Thr Arg His Ile Pro Ile Arg Glu Phe Pro Leu Ser
                   85                  90                  95
   Lys Pro Arg Lys Ser Leu Gly Thr Ala Lys His Met Ala Asp Gln Phe
               100                 105                 110
   Phe Glu Ala Lys Ser Arg Gln Leu Arg Ser Cys Thr Gln Leu Leu Ala
           115                 120                 125
   Lys Leu Gln Asn Ser Arg Glu Glu Ile Gln Asn Ser Ile Asp Lys Glu
       130                 135                 140
   Val Phe Asn Asn Leu Gln Ser Leu Leu Ala Gln Leu Asn Asp Tyr Thr
   145                 150                 155                 160
   Asp Lys Tyr Leu Asn Glu Leu Lys Arg Arg Lys Ser Ala Asn Leu Leu
                           165                 170                 175
   Leu Asn Glu Arg Phe Val Asn Leu Ile Leu Glu Asp Phe Ala Lys Val
                   180                 185                 190
   Phe Glu His Ile Ser Gln His Phe Tyr Pro Ala Phe Lys Ile Glu Asn
           195                 200                 205
   Ser Pro Ala Thr Ser Phe Thr Phe His Ile Ile Asn Trp Leu Ala Glu
       210                 215                 220
   Asn Val Gln Asp Met His Leu Ile Leu Phe Lys Leu Leu Asn Arg Glu
   225                 230                 235                 240
   Lys Met Met Glu Gly Leu Glu Trp Tyr Thr Ser Ser Gln Ile Ile Leu
                   245                 250                 255
   Lys Arg Gly Tyr Ile Asp Phe Phe Lys Thr Asp Asp Ile Lys Asn Tyr
               260                 265                 270
   Leu Leu Asn His Gln Asp Leu Arg Glu Ile Lys Asn Ile Leu Lys Tyr
           275                 280                 285
   Leu Gly Glu Lys His Trp Lys Lys Leu Glu Leu Gly Phe Ile Lys Ser
       290                 295                 300
   His Leu Asn Pro Ser Phe Asn Met Glu Thr Asn Arg Pro Met Val Asn
   305                 310                 315                 320
   Leu Asn Thr Val Asn Asp Phe Asn Glu Ile Leu Lys Ser Ile Glu Asn
                   325                 330                 335
   Ala Gln Ser Phe Asn Pro Glu Trp Leu Lys Lys Leu Ser Met Asp Ser
               340                 345                 350
   Lys Glu Lys Asn Val Ala Phe Tyr Phe Thr Glu Lys Ile Phe Val Leu
           355                 360                 365
   His Ile Leu Ser Tyr Cys Gln Lys Tyr Phe Gly Thr Ile Lys Glu Asp
       370                 375                 380
   Gln Leu Met Met Gln Lys Val Asp Tyr Phe Lys Arg Ile Val Glu Leu
   385                 390                 395                 400
   Tyr Arg Lys Gln Leu Glu Met Tyr Glu Glu Leu Val Asp Ser Gly Asn
                   405                 410                 415
   Leu Ser Arg Ile Ile Leu Ser Glu Lys Phe Ser Val Met Asn His Ile
               420                 425                 430
   Leu Ala Leu Lys Pro Ser Glu Ile Glu Lys Ser Phe Pro Val Asp Val
           435                 440                 445
   Val Leu Ile Gly Asp Ser Lys Phe Glu Phe Leu Lys Thr Arg Asn Thr
       450                 455                 460
   Leu Gly Glu His Trp Lys Ser Arg Tyr Thr Asn Gln Cys Ser Ile Leu
   465                 470                 475                 480
   Glu Thr Ala Gln Lys Tyr Ile Gln Asp His His Tyr Leu Ile Gln Ile
                   485                 490                 495
   Asn Ile Asn Gly Met Thr Phe Asn Leu Arg Ser Lys Pro Thr Leu Ser
               500                 505                 510
   Glu Gln Phe Tyr Asp Glu Phe Phe Ser Leu Ser Lys Val Tyr Thr Glu
           515                 520                 525
   Leu Thr Lys Tyr Leu
       530
<210> 107
   <211> 396
   <212> DNA
   <213> Phakopsora pachyrhizi
<220>
   <221> misc_feature
   <222> (1)..(396)
   <223> 15o4
<400> 107
   atgaaacatg atcaaagtta tagagctcta gtttttcttt tgtttgcctt gctaaatctc       60
   ttggtatcaa tcagttcagc accaatgggc aagataacaa cagaagcttc aaaaatctcc      120
   accccagtag ctgaactgaa caaatttgaa ggcatgagag acgtaaaaat tgttgaaaac      180
   aatccaagaa ttgaaacaca aggaacagct tccaaccacc agacaccagt agactttgaa      240
   tcttccaaaa cagatgactc aattggtcca cttctggtca atcatcaaac atctttaagc      300
   aaaactttta caaacaaaaa ctcattgatt aaacaaccat caccaggatt aaaactggat      360
   caactcttag atcgtataac aatgagcatt atttaa                                396
<210> 108
   <211> 131
   <212> PRT
   <213> Phakopsora pachyrhizi
<220>
   <221> VARIANT
   <222> (1)..(131)
   <223> 15o4
<400> 108
   Met Lys His Asp Gln Ser Tyr Arg Ala Leu Val Phe Leu Leu Phe Ala
   1               5                   10                  15
   Leu Leu Asn Leu Leu Val Ser Ile Ser Ser Ala Pro Met Gly Lys Ile
               20                  25                  30
   Thr Thr Glu Ala Ser Lys Ile Ser Thr Pro Val Ala Glu Leu Asn Lys
           35                  40                  45
   Phe Glu Gly Met Arg Asp Val Lys Ile Val Glu Asn Asn Pro Arg Ile
       50                  55                  60
   Glu Thr Gln Gly Thr Ala Ser Asn His Gln Thr Pro Val Asp Phe Glu
   65                  70                  75                  80
   Ser Ser Lys Thr Asp Asp Ser Ile Gly Pro Leu Leu Val Asn His Gln
                   85                  90                  95
   Thr Ser Leu Ser Lys Thr Phe Thr Asn Lys Asn Ser Leu Ile Lys Gln
               100                 105                 110
   Pro Ser Pro Gly Leu Lys Leu Asp Gln Leu Leu Asp Arg Ile Thr Met
           115                 120                 125
   Ser Ile Ile
       130
<210> 109
   <211> 972
   <212> DNA
   <213> Phakopsora pachyrhizi
<220>
   <221> misc_feature
   <222> (1)..(972)
   <223> 16a8
<400> 109
   atgttatggg gaaaaaatat ctatcgactg tcatgcatct cctcgggaat gagcctcttc      60
   tctaatctca tgtatatatc tttcatttac tctatatctg caaaaaattc agattctgat     120
   gcccaaggat tggtacatcc aatccgtgac cctcgtttgc tttctggagt caggcctgga     180
   attacctcga gccaaggcac tagcttgcaa gggagttatt accttagaaa ttcttatcta     240
   cgtcacaacc ttaaagaaaa ccaaactggt gttcccttgt tccttacgat ttctgtaact     300
   gacaccaatt catgttcccc gattgaaaat gctctcgtgg agctgtgggg cgcaaacaat     360
   catgggctgt actcaggctt tagcaaaagt ggcagccctt ctagtgactg ctcctcttgg     420
   ctgagaggcg caatggagac agattctgag ggtttggcta agtttgaaac cttataccct     480
   ggtcaagaag aaaaccgatc tttgcacttg tatgcgatta ttcgaacgga ttggtacgag     540
   cacagcaaga accaaacagt ggattcggat tctagacata atgcagctgc catcgttcaa     600
   atttttttcc cagacagttt gaatcaccaa gtcttgaata gaactgatta taaaattaca     660
   gggagacaat ttgttaagaa caagcaggac ttcattttct cttctggtca aggagtgtta     720
   aaggctaaag ctgaacttcc taaaagcagc atctgtggtg gtgttcaggc tcacgtacag     780
   atctctgtgg atgcccatgc atcacagaat attactgtca ctgaatctcc ttatcagtgt     840
   aagaccaatt gcaaagctta ccaaaacacc actgagcaaa caactacaat ttcagcaact     900
   tcaaaacaca agccacattt tataccgctt gcagtaatct tttttgcctc tacttatgtt     960
   ttctatggct ga                                                         972
<210> 110
   <211> 323
   <212> PRT
   <213> Phakopsora pachyrhizi
<220>
   <221> VARIANT
   <222> (1)..(323)
   <223> 16a8
<400> 110
   Met Leu Trp Gly Lys Asn Ile Tyr Arg Leu Ser Cys Ile Ser Ser Gly
   1               5                   10                  15
   Met Ser Leu Phe Ser Asn Leu Met Tyr Ile Ser Phe Ile Tyr Ser Ile
               20                  25                  30
   Ser Ala Lys Asn Ser Asp Ser Asp Ala Gln Gly Leu Val His Pro Ile
           35                  40                  45
   Arg Asp Pro Arg Leu Leu Ser Gly Val Arg Pro Gly Ile Thr Ser Ser
       50                  55                  60
   Gln Gly Thr Ser Leu Gln Gly Ser Tyr Tyr Leu Arg Asn Ser Tyr Leu
   65                  70                  75                  80
   Arg His Asn Leu Lys Glu Asn Gln Thr Gly Val Pro Leu Phe Leu Thr
                   85                  90                  95
   Ile Ser Val Thr Asp Thr Asn Ser Cys Ser Pro Ile Glu Asn Ala Leu
               100                 105                 110
   Val Glu Leu Trp Gly Ala Asn Asn His Gly Leu Tyr Ser Gly Phe Ser
           115                 120                 125
   Lys Ser Gly Ser Pro Ser Ser Asp Cys Ser Ser Trp Leu Arg Gly Ala
       130                 135                 140
   Met Glu Thr Asp Ser Glu Gly Leu Ala Lys Phe Glu Thr Leu Tyr Pro
   145                 150                 155                 160
   Gly Gln Glu Glu Asn Arg Ser Leu His Leu Tyr Ala Ile Ile Arg Thr
                   165                 170                 175
   Asp Trp Tyr Glu His Ser Lys Asn Gln Thr Val Asp Ser Asp Ser Arg
               180                 185                 190
   His Asn Ala Ala Ala Ile Val Gln Ile Phe Phe Pro Asp Ser Leu Asn
           195                 200                 205
   His Gln Val Leu Asn Arg Thr Asp Tyr Lys Ile Thr Gly Arg Gln Phe
       210                 215                 220
   Val Lys Asn Lys Gln Asp Phe Ile Phe Ser Ser Gly Gln Gly Val Leu
   225                 230                 235                 240
   Lys Ala Lys Ala Glu Leu Pro Lys Ser Ser Ile Cys Gly Gly Val Gln
                   245                 250                 255
   Ala His Val Gln Ile Ser Val Asp Ala His Ala Ser Gln Asn Ile Thr
               260                 265                 270
   Val Thr Glu Ser Pro Tyr Gln Cys Lys Thr Asn Cys Lys Ala Tyr Gln
           275                 280                 285
   Asn Thr Thr Glu Gln Thr Thr Thr Ile Ser Ala Thr Ser Lys His Lys
       290                 295                 300
   Pro His Phe Ile Pro Leu Ala Val Ile Phe Phe Ala Ser Thr Tyr Val
   305                 310                 315                 320
   Phe Tyr Gly
<210> 111
   <211> 360
   <212> DNA
   <213> Phakopsora pachyrhizi
<220>
   <221> misc_feature
   <222> (1)..(360)
   <223> 22-e10
<400> 111
   atgcagtcct ttatcttgtc agccattgtc tttatggtaa ttgctcaatt gactgttgcg      60
   tccccatttt tcggtggagg tttaggcggc ctaggaggtg ccggtggttt tggtggtgct     120
   catggtggtg gtgcatccgg ttacaaccac aattccttta gctcctccca aagtggctcg     180
   tcgtcttccg gtttcagcgg aggacaaggt ggctctgtca gaggtggtgg tatcggcgga      240
   atgggtggta ttggcggtat tggtcccatc ggtggacttg gaccattcgg ccccggaatt      300
   ggcggacttg gtggaccatt cctcaaggat aatgaacaca aaaacaagaa tgttcaataa      360
<210> 112
   <211> 119
   <212> PRT
   <213> Phakopsora pachyrhizi
<220>
   <221> VARIANT
   <222> (1)..(119)
   <223> 22-e10
<400> 112
   Met Gln Ser Phe Ile Leu Ser Ala Ile Val Phe Met Val Ile Ala Gln
   1               5                   10                  15
   Leu Thr Val Ala Ser Pro Phe Phe Gly Gly Gly Leu Gly Gly Leu Gly
               20                  25                  30
   Gly Ala Gly Gly Phe Gly Gly Ala His Gly Gly Gly Ala Ser Gly Tyr
           35                  40                  45
   Asn His Asn Ser Phe Ser Ser Ser Gln Ser Gly Ser Ser Ser Ser Gly
       50                  55                  60
   Phe Ser Gly Gly Gln Gly Gly Ser Val Arg Gly Gly Gly Ile Gly Gly
   65                  70                  75                  80
   Met Gly Gly Ile Gly Gly Ile Gly Pro Ile Gly Gly Leu Gly Pro Phe
                   85                  90                  95
   Gly Pro Gly Ile Gly Gly Leu Gly Gly Pro Phe Leu Lys Asp Asn Glu
               100                 105                 110
   His Lys Asn Lys Asn Val Gln
           115
<210> 113
   <211> 429
   <212> DNA
   <213> Phakopsora pachyrhizi
<220>
   <221> misc_feature
   <222> (1)..(429)
   <223> 14-a18
<400> 113
   atgtttgttt ccctcaaggc cataatttcg accacaatcg ctacccttgc ggtgatcgga       60
   gtcgtttcag ggcagaatat tactatagtc tctccacaga gcgggcaagt catcagggca      120
   ggtgatgtgt taacagttga actagcccaa caatctacca catcatcggt ccaaagcatc      180
   gcattcagca ccggtttcag ttcaaggccc acagatacta tttccttggg cagaccttta      240
   ggcggaaccc agtttttgac tgccagagga aacataacct ttcaaattaa tgtgcccaaa      300
   gcaagtgaat ttattgacgg gatttccaat gtcccttacg aattcaaagt agcccattac      360
   tatttccttg gtgccacctc tactcccacg attgatatag ccagcgttcc agttagcgta      420
   caacaataa                                                              429
<210> 114
   <211> 142
   <212> PRT
   <213> Phakopsora pachyrhizi
<220>
   <221> VARIANT
   <222> (1)..(142)
   <223> 14-a18
<400> 114
   Met Phe Val Ser Leu Lys Ala Ile Ile Ser Thr Thr Ile Ala Thr Leu
   1               5                   10                  15
   Ala Val Ile Gly Val Val Ser Gly Gln Asn Ile Thr Ile Val Ser Pro
               20                  25                  30
   Gln Ser Gly Gln Val Ile Arg Ala Gly Asp Val Leu Thr Val Glu Leu
           35                  40                  45
   Ala Gln Gln Ser Thr Thr Ser Ser Val Gln Ser Ile Ala Phe Ser Thr
       50                  55                  60
   Gly Phe Ser Ser Arg Pro Thr Asp Thr Ile Ser Leu Gly Arg Pro Leu
   65                  70                  75                  80
   Gly Gly Thr Gln Phe Leu Thr Ala Arg Gly Asn Ile Thr Phe Gln Ile
                   85                  90                  95
   Asn Val Pro Lys Ala Ser Glu Phe Ile Asp Gly Ile Ser Asn Val Pro
               100                 105                 110
   Tyr Glu Phe Lys Val Ala His Tyr Tyr Phe Leu Gly Ala Thr Ser Thr
           115                 120                 125
   Pro Thr Ile Asp Ile Ala Ser Val Pro Val Ser Val Gln Gln
       130                 135                 140
<210> 115
   <211> 657
   <212> DNA
   <213> Phakopsora pachyrhizi
<220>
   <221> misc_feature
   <222> (1)..(657)
   <223> 02-d23
<400> 115
   atgctttttg ctacactaat agccgtttgc ttacttgctc tgggaggcaa agctgagagt      60
   gatgtccaaa gtgataccgc atctaaactt cagcgacgag gacatgatga ctctctacct     120
   cctgtgacat ttattatgag agatagcaac gagcatgtag gcggaaagct gttgatctac     180
   aattctgatg gaacactagc tttcaccttt cgcagagcag ttttgaactc tgatggcctt     240
   tccaacgttg aagtcagaga tgtgcgaaat aatttttcga taaacttaga atctaacgat     300
   gacacctgct ttaaaaagtc tcactatgtt gaaagagaaa aaaatctggg tcaattcaaa     360
   attgatccac gaggtgcaaa agcagacaga tggcatttca ctaggaaaac tccacacgga     420
   gatttcaagt atgactttca tcgcaagtat ttttcgaagg acggtaatat ttacataaaa     480
   gacacccatg tgcgagtggc ttccctgaca tccgaaattc gacatgaagc ttggctccaa     540
   ccggggaaac atggtgttcc aacattctca ttgcatcttc aatataacac tgagcctatc     600
   ttttttgtag ctttgatggg cttagatctc accagagtag acacatgtgg actttga        657
<210> 116
   <211> 218
   <212> PRT
   <213> Phakopsora pachyrhizi
<220>
   <221> VARIANT
   <222> (1)..(218)
   <223> 02-d23
<400> 116
   Met Leu Phe Ala Thr Leu Ile Ala Val Cys Leu Leu Ala Leu Gly Gly
   1               5                   10                  15
   Lys Ala Glu Ser Asp Val Gln Ser Asp Thr Ala Ser Lys Leu Gln Arg
               20                  25                  30
   Arg Gly His Asp Asp Ser Leu Pro Pro Val Thr Phe Ile Met Arg Asp
           35                  40                  45
   Ser Asn Glu His Val Gly Gly Lys Leu Leu Ile Tyr Asn Ser Asp Gly
       50                  55                  60
   Thr Leu Ala Phe Thr Phe Arg Arg Ala Val Leu Asn Ser Asp Gly Leu
   65                  70                  75                  80
   Ser Asn Val Glu Val Arg Asp Val Arg Asn Asn Phe Ser Ile Asn Leu
                   85                  90                  95
   Glu Ser Asn Asp Asp Thr Cys Phe Lys Lys Ser His Tyr Val Glu Arg
               100                 105                 110
   Glu Lys Asn Leu Gly Gln Phe Lys Ile Asp Pro Arg Gly Ala Lys Ala
           115                 120                 125
   Asp Arg Trp His Phe Thr Arg Lys Thr Pro His Gly Asp Phe Lys Tyr
       130                 135                 140
   Asp Phe His Arg Lys Tyr Phe Ser Lys Asp Gly Asn Ile Tyr Ile Lys
   145                 150                 155                 160
   Asp Thr His Val Arg Val Ala Ser Leu Thr Ser Glu Ile Arg His Glu
                   165                 170                 175
   Ala Trp Leu Gln Pro Gly Lys His Gly Val Pro Thr Phe Ser Leu His
               180                 185                 190
   Leu Gln Tyr Asn Thr Glu Pro Ile Phe Phe Val Ala Leu Met Gly Leu
           195                 200                 205
   Asp Leu Thr Arg Val Asp Thr Cys Gly Leu
       210                 215
<210> 117
   <211> 381
   <212> DNA
   <213> Phakopsora pachyrhizi
<220>
   <221> misc_feature
   <222> (1)..(381)
   <223> 02-L20
<400> 117
   atgagggcaa tcttaatatt cctgattgga ttctgctttt tatacaacgg cgtcaggtcc      60
   atcgaatcca taataaaaaa agcacaaagc gaaataaaaa gtctagggag ctatggaaaa     120
   tcagaagctg tcaagcagcc acttactcag gcaagctctc atctaaatgg tttgcgagga     180
   gcgaaagggc aaaaaaaggc agcagtgaaa caattgatcc aagagcaatt gaacaattat     240
   agtgaaaatc tatataatga atgtaaaata aaaacttctg aaaaaagttc aagtaaagaa     300
   gaaatacgta ctttactttc agataagaag aaggtagaca aagcaattga aaatataatg     360
   aaagggcttg aaaaagctta a                                               381
<210> 118
   <211> 126
   <212> PRT
   <213> Phakopsora pachyrhizi
<220>
   <221> VARIANT
   <222> (1)..(126)
   <223> 02-L20
<400> 118
   Met Arg Ala Ile Leu Ile Phe Leu Ile Gly Phe Cys Phe Leu Tyr Asn
   1               5                   10                  15
   Gly Val Arg Ser Ile Glu Ser Ile Ile Lys Lys Ala Gln Ser Glu Ile
               20                  25                  30
   Lys Ser Leu Gly Ser Tyr Gly Lys Ser Glu Ala Val Lys Gln Pro Leu
           35                  40                  45
   Thr Gln Ala Ser Ser His Leu Asn Gly Leu Arg Gly Ala Lys Gly Gln
       50                  55                  60
   Lys Lys Ala Ala Val Lys Gln Leu Ile Gln Glu Gln Leu Asn Asn Tyr
   65                  70                  75                  80
   Ser Glu Asn Leu Tyr Asn Glu Cys Lys Ile Lys Thr Ser Glu Lys Ser
                   85                  90                  95
   Ser Ser Lys Glu Glu Ile Arg Thr Leu Leu Ser Asp Lys Lys Lys Val
               100                 105                 110
   Asp Lys Ala Ile Glu Asn Ile Met Lys Gly Leu Glu Lys Ala
           115                 120                 125
<210> 119
   <211> 579
   <212> DNA
   <213> Phakopsora pachyrhizi
<220>
   <221> misc_feature
   <222> (1)..(579)
   <223> 03-c18
<400> 119
   atgaggttaa tccctgtctt aattttgctt tttgcctctg cgctcaatgc ctcaccatgt      60
   gataaatcta aagttgcgaa aggcgaagat gtcactatga gcgtcatatt ttcagagacc     120
   tccatcgcat acgcatcagg gctcaccgtc atccaggaaa gttgccataa cggagaatat     180
   gagaaagttg aacaatctac tcttactatt caccagcaaa tgacgataat cacagagact     240
   gtgaaaagta aagactttaa caaagattct gcggttcaat acgagggaac tgtagcatct     300
   tacatcgtag actatagcaa agtagtacag actgttgttg attatccaga aactgaacct     360
   tgccatagta ccttggtaga gattcatcaa aaaattcaaa ctgttattaa tacgtacgcc     420
   tccgaatata acatttctct caagaaggaa gtagacaggc aaggtggaat tgatcccaaa     480
   tctctagaaa aactgaatct caaatttgat tttcacgaag cgcgtacgca tcaagaaaat     540
   gaagtcgatg aatatcaaat atctgaatac aagaattaa                             579
<210> 120
   <211> 192
   <212> PRT
   <213> Phakopsora pachyrhizi
<220>
   <221> VARIANT
   <222> (1)..(192)
   <223> 03-c18
<400> 120
   Met Arg Leu Ile Pro Val Leu Ile Leu Leu Phe Ala Ser Ala Leu Asn
   1               5                   10                  15
   Ala Ser Pro Cys Asp Lys Ser Lys Val Ala Lys Gly Glu Asp Val Thr
               20                  25                  30
   Met Ser Val Ile Phe Ser Glu Thr Ser Ile Ala Tyr Ala Ser Gly Leu
           35                  40                  45
   Thr Val Ile Gln Glu Ser Cys His Asn Gly Glu Tyr Glu Lys Val Glu
       50                  55                  60
   Gln Ser Thr Leu Thr Ile His Gln Gln Met Thr Ile Ile Thr Glu Thr
   65                  70                  75                  80
   Val Lys Ser Lys Asp Phe Asn Lys Asp Ser Ala Val Gln Tyr Glu Gly
                   85                  90                  95
   Thr Val Ala Ser Tyr Ile Val Asp Tyr Ser Lys Val Val Gln Thr Val
               100                 105                 110
   Val Asp Tyr Pro Glu Thr Glu Pro Cys His Ser Thr Leu Val Glu Ile
           115                 120                 125
   His Gln Lys Ile Gln Thr Val Ile Asn Thr Tyr Ala Ser Glu Tyr Asn
       130                 135                 140
   Ile Ser Leu Lys Lys Glu Val Asp Arg Gln Gly Gly Ile Asp Pro Lys
   145                 150                 155                 160
   Ser Leu Glu Lys Leu Asn Leu Lys Phe Asp Phe His Glu Ala Arg Thr
                   165                 170                 175
   His Gln Glu Asn Glu Val Asp Glu Tyr Gln Ile Ser Glu Tyr Lys Asn
               180                 185                 190
<210> 121
   <211> 885
   <212> DNA
   <213> Phakopsora pachyrhizi
<220>
   <221> misc_feature
   <222> (1)..(885)
   <223> 04-a4
<400> 121
   atgaactcca agtctaccct cgcgatactt gtcgtggtca ccgccagttt gttaccatac        60
   agctggggat ttaatcagga tagctcagaa actcaaacat ttgagcatcc ctcctccgcg       120
   aaggaatcac aatcttcatt cagttatgag agtaatgaaa gcaagtcatc ttactcaagc       180
   aatcaaatat cgacaaaaaa tcaatatggg cctagtagta gcttaggagg aagcactgaa       240
   gacggctttg atcctgaatt cgatccaaca gaggaagaca caattacagc agagactcca       300
   actaaaacaa tcttttgtcc aggaccaatt aataccggaa cgggagaggg tgtatggata       360
   gatggacatt gtgaaattat gtgcttcaac aatctcgtac tggatggaga tcgatgtacc     420
   tgccccccaa cctatcactt tgaccacaaa aatgtgaaat gcgtttgcag acctccactc     480
   tgtgaacaag gcggtaagtg tatcttgaag ccatcgcagt atcctggcgt ccacaactct       540
   gcccatagaa agaggtcaat gcctgcgcag cttcgattaa ccccacaagt ttataatgga       600
   aatcatgctc gaacatcttt cgatgataag cactgcattt caaatgagat cgcctgtcgc       660
   attggtagta tgactggtgg agttcaatgc gttgatccca caagtgacct ggaacactgt       720
   ggggggtgct ccaacaccac agaaggcata aactgcaatc aaattcccgg ggtagataac       780
   gctggatgca atcagtcaaa atgtgtcatt ttttcttgca aagatgggca cagactggtg       840
   aataacgtat gcgtgaaagc gctaaataac aaaagaaggc tgtaa                      885
<210> 122
   <211> 294
   <212> PRT
   <213> Phakopsora pachyrhizi
<220>
   <221> VARIANT
   <222> (1)..(294)
   <223> 04-a4
<400> 122
   Met Asn Ser Lys Ser Thr Leu Ala Ile Leu Val Val Val Thr Ala Ser
   1               5                   10                  15
   Leu Leu Pro Tyr Ser Trp Gly Phe Asn Gln Asp Ser Ser Glu Thr Gln
               20                  25                  30
   Thr Phe Glu His Pro Ser Ser Ala Lys Glu Ser Gln Ser Ser Phe Ser
           35                  40                  45
   Tyr Glu Ser Asn Glu Ser Lys Ser Ser Tyr Ser Ser Asn Gln Ile Ser
       50                  55                  60
   Thr Lys Asn Gln Tyr Gly Pro Ser Ser Ser Leu Gly Gly Ser Thr Glu
   65                  70                  75                  80
   Asp Gly Phe Asp Pro Glu Phe Asp Pro Thr Glu Glu Asp Thr Ile Thr
                   85                  90                  95
   Ala Glu Thr Pro Thr Lys Thr Ile Phe Cys Pro Gly Pro Ile Asn Thr
               100                 105                 110
   Gly Thr Gly Glu Gly Val Trp Ile Asp Gly His Cys Glu Ile Met Cys
           115                 120                 125
   Phe Asn Asn Leu Val Leu Asp Gly Asp Arg Cys Thr Cys Pro Pro Thr
       130                 135                 140
   Tyr His Phe Asp His Lys Asn Val Lys Cys Val Cys Arg Pro Pro Leu
   145                 150                 155                 160
   Cys Glu Gln Gly Gly Lys Cys Ile Leu Lys Pro Ser Gln Tyr Pro Gly
                   165                 170                 175
   Val His Asn Ser Ala His Arg Lys Arg Ser Met Pro Ala Gln Leu Arg
               180                 185                 190
   Leu Thr Pro Gln Val Tyr Asn Gly Asn His Ala Arg Thr Ser Phe Asp
           195                 200                 205
   Asp Lys His Cys Ile Ser Asn Glu Ile Ala Cys Arg Ile Gly Ser Met
       210                 215                 220
   Thr Gly Gly Val Gln Cys Val Asp Pro Thr Ser Asp Leu Glu His Cys
   225                 230                 235                 240
   Gly Gly Cys Ser Asn Thr Thr Glu Gly Ile Asn Cys Asn Gln Ile Pro
                   245                 250                 255
   Gly Val Asp Asn Ala Gly Cys Asn Gln Ser Lys Cys Val Ile Phe Ser
               260                 265                 270
   Cys Lys Asp Gly His Arg Leu Val Asn Asn Val Cys Val Lys Ala Leu
           275                 280                 285
   Asn Asn Lys Arg Arg Leu
       290
<210> 123
   <211> 642
   <212> DNA
   <213> Phakopsora pachyrhizi
<220>
   <221> misc_feature
   <222> (1)..(642)
   <223> 12d21
<400> 123
   atggtttact caaaggcaac cttcaccctg gtctcactcc tacctatgat cctggcccag       60
   cagagcatca acaacagtga taccacaacc acggctgcta ccccggccag tcccaccaac      120
   agcagcagct ctaccactac ttccactacc acctctaaca ctgccagtgg agctgctcag      180
   tccttccctg cgccaacagt ctcaggaaca tcctgtggta cagcccagca gaactttaac      240
   cagtgtgtta ccaaagtatc aaaggatatc tcctcatgtc cctctaccga taacacctgt      300
   ctttgccaaa cgtatgcaaa cctagcctac tgttacaacg cctgtccaga tctggcatca      360
   tcaggggccg gatacctgca gcagtccaca gtgaactgtg atgcagcagg tatcaagcca      420
   aacgctactt ccaacgtcac caccacccct gtcacctcca ccactaacag gaacaccacc      480
   aacacctcac ccatctctaa cacaaacaag aacaattcta cctcgggcaa cactgctgct      540
   acatttgctg ctggaaactc caaggcaagt gggattgagg ccccgttact gagcgttgct      600
   gttgttgggc tgtgtggtat cattgctagc ctatttgcat ga                          642
<210> 124
   <211> 213
   <212> PRT
   <213> Phakopsora pachyrhizi
<220>
   <221> VARIANT
   <222> (1)..(213)
   <223> 12d21
<400> 124
   Met Val Tyr Ser Lys Ala Thr Phe Thr Leu Val Ser Leu Leu Pro Met
   1               5                   10                  15
   Ile Leu Ala Gln Gln Ser Ile Asn Asn Ser Asp Thr Thr Thr Thr Ala
               20                  25                  30
   Ala Thr Pro Ala Ser Pro Thr Asn Ser Ser Ser Ser Thr Thr Thr Ser
           35                  40                  45
   Thr Thr Thr Ser Asn Thr Ala Ser Gly Ala Ala Gln Ser Phe Pro Ala
       50                  55                  60
   Pro Thr Val Ser Gly Thr Ser Cys Gly Thr Ala Gln Gln Asn Phe Asn
   65                  70                  75                  80
   Gln Cys Val Thr Lys Val Ser Lys Asp Ile Ser Ser Cys Pro Ser Thr
                   85                  90                  95
   Asp Asn Thr Cys Leu Cys Gln Thr Tyr Ala Asn Leu Ala Tyr Cys Tyr
               100                 105                 110
   Asn Ala Cys Pro Asp Leu Ala Ser Ser Gly Ala Gly Tyr Leu Gln Gln
           115                 120                 125
   Ser Thr Val Asn Cys Asp Ala Ala Gly Ile Lys Pro Asn Ala Thr Ser
       130                 135                 140
   Asn Val Thr Thr Thr Pro Val Thr Ser Thr Thr Asn Arg Asn Thr Thr
   145                 150                 155                 160
   Asn Thr Ser Pro Ile Ser Asn Thr Asn Lys Asn Asn Ser Thr Ser Gly
                   165                 170                 175
   Asn Thr Ala Ala Thr Phe Ala Ala Gly Asn Ser Lys Ala Ser Gly Ile
               180                 185                 190
   Glu Ala Pro Leu Leu Ser Val Ala Val Val Gly Leu Cys Gly Ile Ile
           195                 200                 205
   Ala Ser Leu Phe Ala
       210
<210> 125
   <211> 465
   <212> DNA
   <213> Phakopsora pachyrhizi
<220>
   <221> misc_feature
   <222> (1)..(465)
   <223> 12L21
<400> 125
   atgcaatcca actttctcct cgctttttgc ttgattgctg ctgtctctgt tccagttagc       60
   agggctcacg gtgttattac ttcagttgag ggcgcaaatg gtcaaactgg atctgccttt      120
   ggaatggtag agtccactcc tcgtgatggc actcgaacga acccattcca aactgacagt      180
   tccatcattc gagaccgtga ggtttcaagc ggaaaggcgt ctgcttgtgg acgcactctt      240
   gcgggcggaa acaacgatat tgctagtgac atgtcatcgg ctgaatctgc cggattggcc      300
   agtattggac ctgacggaaa agtcagaatg actcttcacc aggtcaacgg tgacggtgga      360
   ggcccataca gctgtgaagt tgacaccacc gccactggtg acaacttcaa gaaaatgaac      420
   atcgacacaa acgttcctgg caagaacagt cgatcaaggg catag                      465
<210> 126
   <211> 154
   <212> PRT
   <213> Phakopsora pachyrhizi
<220>
   <221> VARIANT
   <222> (1)..(154)
   <223> 12L21
<400> 126
   Met Gln Ser Asn Phe Leu Leu Ala Phe Cys Leu Ile Ala Ala Val Ser
   1               5                   10                  15
   Val Pro Val Ser Arg Ala His Gly Val Ile Thr Ser Val Glu Gly Ala
               20                  25                  30
   Asn Gly Gln Thr Gly Ser Ala Phe Gly Met Val Glu Ser Thr Pro Arg
           35                  40                  45
   Asp Gly Thr Arg Thr Asn Pro Phe Gln Thr Asp Ser Ser Ile Ile Arg
       50                  55                  60
   Asp Arg Glu Val Ser Ser Gly Lys Ala Ser Ala Cys Gly Arg Thr Leu
   65                  70                  75                  80
   Ala Gly Gly Asn Asn Asp Ile Ala Ser Asp Met Ser Ser Ala Glu Ser
                   85                  90                  95
   Ala Gly Leu Ala Ser Ile Gly Pro Asp Gly Lys Val Arg Met Thr Leu
               100                 105                 110
   His Gln Val Asn Gly Asp Gly Gly Gly Pro Tyr Ser Cys Glu Val Asp
           115                 120                 125
   Thr Thr Ala Thr Gly Asp Asn Phe Lys Lys Met Asn Ile Asp Thr Asn
       130                 135                 140
   Val Pro Gly Lys Asn Ser Arg Ser Arg Ala
   145                 150
<210> 127
   <211> 486
   <212> DNA
   <213> Phakopsora pachyrhizi
<220>
   <221> misc_feature
   <222> (1)..(486)
   <223> 15m10
<400> 127
   atgcttttca actcaaacgt gatcctcttt gcattaactt ttgctctggg atcgttatac       60
   cagggatcag aagctcagga tctggccgga ctacctaact gtggtcagaa ctgtctattg      120
   acagccttta cgacatcgag cgatggttgc acacagaccg actttgcatg tctttgcaag      180
   tcgcaaaagt ttactggtac atcaaattcg tgctactcct cgtcctgtac ctccactgaa      240
   gcagcgactg ccaagagctg gggtgccaag  acttgtgctt ctgtaggagt taatgtgacg      300
   gccacccttg gaaacaccac ttccgttgga actgtcatcg gaaatggaac taatcctaac      360
   accaactcta cgcttcataa tggcacaact gcctctacac atgcacagaa ctctggaggc      420
   ctcatcaacg tgcaacccat ctttttcatg tctttagtct ctgtcctggc tgcagtcttc      480
   atgtga                                                                 486
<210> 128
   <211> 161
   <212> PRT
   <213> Phakopsora pachyrhizi
<220>
   <221> VARIANT
   <222> (1)..(161)
   <223> 15m10
<400> 128
   Met Leu Phe Asn Ser Asn Val Ile Leu Phe Ala Leu Thr Phe Ala Leu
   1               5                   10                  15
   Gly Ser Leu Tyr Gln Gly Ser Glu Ala Gln Asp Leu Ala Gly Leu Pro
               20                  25                  30
   Asn Cys Gly Gln Asn Cys Leu Leu Thr Ala Phe Thr Thr Ser Ser Asp
           35                  40                  45
   Gly Cys Thr Gln Thr Asp Phe Ala Cys Leu Cys Lys Ser Gln Lys Phe
       50                  55                  60
   Thr Gly Thr Ser Asn Ser Cys Tyr Ser Ser Ser Cys Thr Ser Thr Glu
   65                  70                  75                  80
   Ala Ala Thr Ala Lys Ser Trp Gly Ala Lys Thr Cys Ala Ser Val Gly
                   85                  90                  95
   Val Asn Val Thr Ala Thr Leu Gly Asn Thr Thr Ser Val Gly Thr Val
               100                 105                 110
   Ile Gly Asn Gly Thr Asn Pro Asn Thr Asn Ser Thr Leu His Asn Gly
           115                 120                 125
   Thr Thr Ala Ser Thr His Ala Gln Asn Ser Gly Gly Leu Ile Asn Val
       130                 135                 140
   Gln Pro Ile Phe Phe Met Ser Leu Val Ser Val Leu Ala Ala Val Phe
   145                 150                 155                 160
   Met
<210> 129
   <211> 591
   <212> DNA
   <213> Phakopsora pachyrhizi
<220>
   <221> misc_feature
   <222> (1)..(591)
   <223> 24o10
<400> 129
   atgattcatc tattgcttaa aaaatttgtt tgcgcattat gtttattcct gatacgagga       60
   aacccttcta agtcagcctt aactagaatt gccaatggag gcgcagttaa aggactctcg      120
   actccagatt gggtactaga tttagctact aaaaaacatc ccacaaattt attagctgct      180
   aatgagctca aattttcaga gacggtcact aggccacctc caatacaaga tatgaatttc      240
   aggcaccctt cacctgagca aacagaaata ataaaaaact ttatcagaaa acacccaagt      300
   ggccaaacct atagactaga tgagaaaggg aactttatag atgtaccaaa ggagaattgg      360
   acgttaaaat actactgtgg tagggtaatc gtggtagacg atcacggcaa tgagctatat      420
   aatactggct gttgttattg caatgcgtct tcacagacca atttaaactc agcagagata      480
   ttggtaaaca aatgtgtata tggatttgga aaaattgctt tagagataat ttctgcactc      540
   cttggccatg aagacaggaa tcggtctagc aaaaggtatg gtggaaagta a               591
<210> 130
   <211> 196
   <212> PRT
   <213> Phakopsora pachyrhizi
<220>
   <221> VARIANT
   <222> (1)..(196)
   <223> 24o10
<400> 130
   Met Ile His Leu Leu Leu Lys Lys Phe Val Cys Ala Leu Cys Leu Phe
   1               5                   10                  15
   Leu Ile Arg Gly Asn Pro Ser Lys Ser Ala Leu Thr Arg Ile Ala Asn
               20                  25                  30
   Gly Gly Ala Val Lys Gly Leu Ser Thr Pro Asp Trp Val Leu Asp Leu
           35                  40                  45
   Ala Thr Lys Lys His Pro Thr Asn Leu Leu Ala Ala Asn Glu Leu Lys
       50                  55                  60
   Phe Ser Glu Thr Val Thr Arg Pro Pro Pro Ile Gln Asp Met Asn Phe
   65                  70                  75                  80
   Arg His Pro Ser Pro Glu Gln Thr Glu Ile Ile Lys Asn Phe Ile Arg
                   85                  90                  95
   Lys His Pro Ser Gly Gln Thr Tyr Arg Leu Asp Glu Lys Gly Asn Phe
               100                 105                 110
   Ile Asp Val Pro Lys Glu Asn Trp Thr Leu Lys Tyr Tyr Cys Gly Arg
           115                 120                 125
   Val Ile Val Val Asp Asp His Gly Asn Glu Leu Tyr Asn Thr Gly Cys
       130                 135                 140
   Cys Tyr Cys Asn Ala Ser Ser Gln Thr Asn Leu Asn Ser Ala Glu Ile
   145                 150                 155                 160
   Leu Val Asn Lys Cys Val Tyr Gly Phe Gly Lys Ile Ala Leu Glu Ile
                   165                 170                 175
   Ile Ser Ala Leu Leu Gly His Glu Asp Arg Asn Arg Ser Ser Lys Arg
               180                 185                 190
   Tyr Gly Gly Lys
           195
<210> 131
   <211> 486
   <212> DNA
   <213> Phakopsora pachyrhizi
<220>
   <221> misc_feature
   <222> (1)..(486)
   <223> 19n19
<400> 131
   atgatgacaa agtttaacac gatgatgatc tcacgcccct tatccctcct gctaggtatt      60
   atccttctga caaccatcgt atccatcaga gcttctgaga aggagaaact tcagatcggg     120
   gtcaaacata aaccgagctc atgtccaatc aaatctcaga agaatgatga attatcgatg     180
   cattataccg gaacgctcaa gtcagatgga agcgtttttg attcgtctgt ccaaaggaat     240
   gaaccgtttg ttttcactct tggattaggc caagtaataa agggatggga ccaaggtcta     300
   ttagatatgt gcattggaga aaagaggaag ttggtcatac cttcaaactt ggcgtatggt     360
   gataggggag ctggtggaaa aattcccggc ggtgcgacgc tgatctttga ggttgaactc     420
   ttggatattc ttaatcgaaa agcacctgcc tcggaagatc aaaaagagca taaagatgaa     480
   ctttaa                                                                486
<210> 132
   <211> 161
   <212> PRT
   <213> Phakopsora pachyrhizi
<220>
   <221> VARIANT
   <222> (1)..(161)
   <223> 19n19
<400> 132
   Met Met Thr Lys Phe Asn Thr Met Met Ile Ser Arg Pro Leu Ser Leu
   1               5                   10                  15
   Leu Leu Gly Ile Ile Leu Leu Thr Thr Ile Val Ser Ile Arg Ala Ser
               20                  25                  30
   Glu Lys Glu Lys Leu Gln Ile Gly Val Lys His Lys Pro Ser Ser Cys
           35                  40                  45
   Pro Ile Lys Ser Gln Lys Asn Asp Glu Leu Ser Met His Tyr Thr Gly
       50                  55                  60
   Thr Leu Lys Ser Asp Gly Ser Val Phe Asp Ser Ser Val Gln Arg Asn
   65                  70                  75                  80
   Glu Pro Phe Val Phe Thr Leu Gly Leu Gly Gln Val Ile Lys Gly Trp
                   85                  90                  95
   Asp Gln Gly Leu Leu Asp Met Cys Ile Gly Glu Lys Arg Lys Leu Val
               100                 105                 110
   Ile Pro Ser Asn Leu Ala Tyr Gly Asp Arg Gly Ala Gly Gly Lys Ile
           115                 120                 125
   Pro Gly Gly Ala Thr Leu Ile Phe Glu Val Glu Leu Leu Asp Ile Leu
       130                 135                 140
   Asn Arg Lys Ala Pro Ala Ser Glu Asp Gln Lys Glu His Lys Asp Glu
   145                 150                 155                 160
   Leu
<210> 133
   <211> 222
   <212> DNA
   <213> Phakopsora pachyrhizi
<220>
   <221> misc_feature
   <222> (1)..(222)
   <223> taa07765.01_phapa
<400> 133
   atgcgctgct ttatctttgc tattgctttt attgctgctg ctcagtgtgt cctgggagca       60
   ggagagtcta cttctaaaaa attgacccgc agagctcttg tcgacgttga aatccttaac      120
   agaggtagac gtccatgcaa caccggcaat tcacttattg aagttgacat cctaaacagc      180
   tatctgaaaa atgcttctaa agctgctaag aacgcccaat ag                         222
<210> 134
   <211> 73
   <212> PRT
   <213> Phakopsora pachyrhizi
<220>
   <221> VARIANT
   <222> (1)..(73)
   <223> taa07765.01_phapa
<400> 134
   Met Arg Cys Phe Ile Phe Ala Ile Ala Phe Ile Ala Ala Ala Gln Cys
   1               5                   10                  15
   Val Leu Gly Ala Gly Glu Ser Thr Ser Lys Lys Leu Thr Arg Arg Ala
               20                  25                  30
   Leu Val Asp Val Glu Ile Leu Asn Arg Gly Arg Arg Pro Cys Asn Thr
           35                  40                  45
   Gly Asn Ser Leu Ile Glu Val Asp Ile Leu Asn Ser Tyr Leu Lys Asn
       50                  55                  60
   Ala Ser Lys Ala Ala Lys Asn Ala Gln
   65                  70
<210> 135
   <211> 294
   <212> DNA
   <213> Phakopsora pachyrhizi
<220>
   <221> misc_feature
   <222> (1)..(294)
   <223> ta06712.01_phapa
<400> 135
   atgcaagtat ttatctactc aatattcatt gttgccctgg ctcagctaat tggtgctgca       60
   ccattcattg gcggcatcgg tggcatggga ggagtcggtg gcggattcgc aggaggagca      120
   tctggattcc ataaacactc gttttcctcc gttcaccatt cccattcctc atcaggattc      180
   accgcaggtc atggaggcgc tcacggcggt ttcggtcatg gtggtattgg aggtattggg      240
   ggagtgggcc ctttccttaa ggatagcaaa cacaaaaaca aaaattcaca ataa            294
<210> 136
   <211> 97
   <212> PRT
   <213> Phakopsora pachyrhizi
<220>
   <221> VARIANT
   <222> (1)..(97)
   <223> ta06712.01_phapa
<400> 136
   Met Gln Val Phe Ile Tyr Ser Ile Phe Ile Val Ala Leu Ala Gln Leu
   1               5                   10                  15
   Ile Gly Ala Ala Pro Phe Ile Gly Gly Ile Gly Gly Met Gly Gly Val
               20                  25                  30
   Gly Gly Gly Phe Ala Gly Gly Ala Ser Gly Phe His Lys His Ser Phe
           35                  40                  45
   Ser Ser Val His His Ser His Ser Ser Ser Gly Phe Thr Ala Gly His
       50                  55                  60
   Gly Gly Ala His Gly Gly Phe Gly His Gly Gly Ile Gly Gly Ile Gly
   65                  70                  75                  80
   Gly Val Gly Pro Phe Leu Lys Asp Ser Lys His Lys Asn Lys Asn Ser
                   85                  90                  95
   Gln
<210> 137
   <211> 492
   <212> DNA
   <213> Phakopsora pachyrhizi
<220>
   <221> misc_feature
   <222> (1)..(492)
   <223> ta09985.01_phapa
<400> 137
   atgatctaca ccggtctcgt tatttttgtg cttgccctct ccggcaaagt attttctact        60
   ggcaccgctc aacaaggctc ctctaactca atgagctgga ctgctgacac taatacaacc       120
   atgaaaccta ctgacgctgg cgccatgaac accacttcaa tccccaccat ggttcctggc       180
   gacatgtcaa ccggaaagtg catgtgccct gccccagtta cttgcgatgc tggcccttca       240
   aaagaaccaa acttacctac aataaatgtt ccaccacctt caacccatgt ccccgctggc       300
   aacacaaact ccacccaggg ccatcagcca ggaaattacc caggcagtgg tgccactccc       360
   cctgtcacaa accccccaac cccagatacc cctgttactg ataacaactc tacgagcggt       420
   gttgccggac tgcctaactc ggtttccatt gccgggttca ttgctgtggc tgcctcagct       480
   ttagtgatct ga                                                           492
<210> 138
   <211> 163
   <212> PRT
   <213> Phakopsora pachyrhizi
<220>
   <221> VARIANT
   <222> (1)..(163)
   <223> ta09985.01_phapa
<400> 138
   Met Ile Tyr Thr Gly Leu Val Ile Phe Val Leu Ala Leu Ser Gly Lys
   1               5                   10                  15
   Val Phe Ser Thr Gly Thr Ala Gln Gln Gly Ser Ser Asn Ser Met Ser
               20                  25                  30
   Trp Thr Ala Asp Thr Asn Thr Thr Met Lys Pro Thr Asp Ala Gly Ala
           35                  40                  45
   Met Asn Thr Thr Ser Ile Pro Thr Met Val Pro Gly Asp Met Ser Thr
       50                  55                  60
   Gly Lys Cys Met Cys Pro Ala Pro Val Thr Cys Asp Ala Gly Pro Ser
   65                  70                  75                  80
   Lys Glu Pro Asn Leu Pro Thr Ile Asn Val Pro Pro Pro Ser Thr His
                   85                  90                  95
   Val Pro Ala Gly Asn Thr Asn Ser Thr Gln Gly His Gln Pro Gly Asn
               100                 105                 110
   Tyr Pro Gly Ser Gly Ala Thr Pro Pro Val Thr Asn Pro Pro Thr Pro
           115                 120                 125
   Asp Thr Pro Val Thr Asp Asn Asn Ser Thr Ser Gly Val Ala Gly Leu
       130                 135                 140
   Pro Asn Ser Val Ser Ile Ala Gly Phe Ile Ala Val Ala Ala Ser Ala
   145                 150                 155                 160
   Leu Val Ile
<210> 139
   <211> 303
   <212> DNA
   <213> Phakopsora pachyrhizi
<220>
   <221> misc_feature
   <222> (1)..(303)
   <223> ta08989.02_phapa
<400> 139
   atgaggttca cttatcaagc gttttttgct ttttttttgg taatctcatt ggtcccagct      60
   gttcccgaag aattgagccc taataatggg gaacttatcc aagctaaaga tgtttcaagt    120
   gccaaaacaa aggatagctc aagcgagaaa tttatcgctc cccccggcta cggatggcga    180
   ggtggaggac cttggagagg accatggggc catggcggac gatggggaaa tggtggacgt    240
   tggggttggg gtccaggtgg tggctggcgc ggaccgggat ggcatggtca ctggcgcgag    300
   tga                                                                  303
<210> 140
   <211> 100
   <212> PRT
   <213> Phakopsora pachyrhizi
<220>
   <221> VARIANT
   <222> (1)..(100)
   <223> ta08989.02_phapa
<400> 140
   Met Arg Phe Thr Tyr Gln Ala Phe Phe Ala Phe Phe Leu Val Ile Ser
   1               5                   10                  15
   Leu Val Pro Ala Val Pro Glu Glu Leu Ser Pro Asn Asn Gly Glu Leu
               20                  25                  30
   Ile Gln Ala Lys Asp Val Ser Ser Ala Lys Thr Lys Asp Ser Ser Ser
           35                  40                  45
   Glu Lys Phe Ile Ala Pro Pro Gly Tyr Gly Trp Arg Gly Gly Gly Pro
       50                  55                  60
   Trp Arg Gly Pro Trp Gly His Gly Gly Arg Trp Gly Asn Gly Gly Arg
   65                  70                  75                  80
   Trp Gly Trp Gly Pro Gly Gly Gly Trp Arg Gly Pro Gly Trp His Gly
                   85                  90                  95
   His Trp Arg Glu
               100
<210> 141
   <211> 375
   <212> DNA
   <213> Phakopsora pachyrhizi
<220>
   <221> misc_feature
   <222> (1)..(375)
   <223> ta10092.01_phapa
<400> 141
   atgaaggtac ttatattctt cgcactaatt gctactatct gcttaccatt ggtagaaaat      60
   agatgccgta ccactggagc ctcatttgga tcatctccga acagggcttt tgctactcag     120
   ctattacctg acgtatgtag agaattccaa ggcacctatg ggcccaacca ggacaagacc     180
   atatgtagaa acggtagaga cagagatacc agcttcagat actacataaa acacgtatct     240
   ggtgggtata ggaacattga ctccactgag tgcacagacg gtctcaacaa agagattgta     300
   aattgcgata gaggtggaaa aacagcttat ggtaactggg aatacagtgt ggacccgaat     360
   aaaggaaatt gctaa                                                      375
<210> 142
   <211> 124
   <212> PRT
   <213> Phakopsora pachyrhizi
<220>
   <221> VARIANT
   <222> (1)..(124)
   <223> ta10092.01_phapa
<400> 142
   Met Lys Val Leu Ile Phe Phe Ala Leu Ile Ala Thr Ile Cys Leu Pro
   1               5                   10                  15
   Leu Val Glu Asn Arg Cys Arg Thr Thr Gly Ala Ser Phe Gly Ser Ser
               20                  25                  30
   Pro Asn Arg Ala Phe Ala Thr Gln Leu Leu Pro Asp Val Cys Arg Glu
           35                  40                  45
   Phe Gln Gly Thr Tyr Gly Pro Asn Gln Asp Lys Thr Ile Cys Arg Asn
       50                  55                  60
   Gly Arg Asp Arg Asp Thr Ser Phe Arg Tyr Tyr Ile Lys His Val Ser
   65                  70                  75                  80
   Gly Gly Tyr Arg Asn Ile Asp Ser Thr Glu Cys Thr Asp Gly Leu Asn
                   85                  90                  95
   Lys Glu Ile Val Asn Cys Asp Arg Gly Gly Lys Thr Ala Tyr Gly Asn
               100                 105                 110
   Trp Glu Tyr Ser Val Asp Pro Asn Lys Gly Asn Cys
           115                 120
<210> 143
   <211> 366
   <212> DNA
   <213> Phakopsora pachyrhizi
<220>
   <221> misc_feature
   <222> (1)..(366)
   <223> ta03571.01_phapa
<400> 143
   atgttccacg acttgaacta ctttgcacct gcccttattg ccttcgcctg tcttcttgag 60
   cttagtgctg ctcaagaaca gaggtcctgt agtttctaca ctggtgccaa taccacttcc 120
   gccacctgca acgagcaacc aaatgttgta tgcaccaagg gctgcactgg gcccttcgtc 180
   actgcaactg aatgtacgcc ggttaatgag tcggaagaag ctattgcgag cactcaggtg 240
   tgcagctttg gttttggtcg gaacacagct gctgcaaagg cctgcataaa tgaattaggt 300
   acttttagat gcactggtca aaccacaggc tcagctacct gtgacggatg tcaagctagg 360
   tcctaa 366
<210> 144
   <211> 121
   <212> PRT
   <213> Phakopsora pachyrhizi
<220>
   <221> VARIANT
   <222> (1)..(121)
   <223> ta03571.01_phapa
<400> 144
   Met Phe His Asp Leu Asn Tyr Phe Ala Pro Ala Leu Ile Ala Phe Ala
   1               5                   10                  15
   Cys Leu Leu Glu Leu Ser Ala Ala Gln Glu Gln Arg Ser Cys Ser Phe
               20                  25                  30
   Tyr Thr Gly Ala Asn Thr Thr Ser Ala Thr Cys Asn Glu Gln Pro Asn
           35                  40                  45
   Val Val Cys Thr Lys Gly Cys Thr Gly Pro Phe Val Thr Ala Thr Glu
       50                  55                  60
   Cys Thr Pro Val Asn Glu Ser Glu Glu Ala Ile Ala Ser Thr Gln Val
   65                  70                  75                  80
   Cys Ser Phe Gly Phe Gly Arg Asn Thr Ala Ala Ala Lys Ala Cys Ile
                   85                  90                  95
   Asn Glu Leu Gly Thr Phe Arg Cys Thr Gly Gln Thr Thr Gly Ser Ala
               100                 105                 110
   Thr Cys Asp Gly Cys Gln Ala Arg Ser
           115                 120
<210> 145
   <211> 567
   <212> DNA
   <213> Phakopsora pachyrhizi
<220>
   <221> misc_feature
   <222> (1)..(567)
   <223> ta05395.01_phapa
<400> 145
   atgttcggtc ttaagactat catcgttgcc atcgctgcca tcctacaagt tgcggaggct      60
   atcgacgact caggttgtgc ttccaagtgt atggctgtaa aattaaccga agccgctgtc     120
   tggttcggtc aaggcaatct ctcaacctac tgccaacatc ctgaattcat caccgcatac     180
   gattcgtgct taggcgataa ctgcaccagt cgcgatgagc tagaagtagc caagagaaat     240
   ggccgcgctg cttgcgctgc tgctagtatc aattccgttg caaataatgt ttcatccatt     300
   ggacgcaaca actctccagc agatatgcct gctggtgcag ggaaccgaag caccggttct     360
   ccttcaccta cagtcccaac caacacaagc gttaacatca attctacctt actaacacct     420
   aattctactg taaacgccac aaacagccga ttttccaacg caccattccc tgcgatttcg     480
   gctgcggcaa acagcacggc ctcaacaata acttgttcat caatgataat tggtttaact     540
   tctgctctgg ttttagcaag cctgtaa                                         567
<210> 146
   <211> 188
   <212> PRT
   <213> Phakopsora pachyrhizi
<220>
   <221> VARIANT
   <222> (1)..(188)
   <223> ta05395.01_phapa
<400> 146
   Met Phe Gly Leu Lys Thr Ile Ile Val Ala Ile Ala Ala Ile Leu Gln
   1               5                   10                  15
   Val Ala Glu Ala Ile Asp Asp Ser Gly Cys Ala Ser Lys Cys Met Ala
               20                  25                  30
   Val Lys Leu Thr Glu Ala Ala Val Trp Phe Gly Gln Gly Asn Leu Ser
           35                  40                  45
   Thr Tyr Cys Gln His Pro Glu Phe Ile Thr Ala Tyr Asp Ser Cys Leu
       50                  55                  60
   Gly Asp Asn Cys Thr Ser Arg Asp Glu Leu Glu Val Ala Lys Arg Asn
   65                  70                  75                  80
   Gly Arg Ala Ala Cys Ala Ala Ala Ser Ile Asn Ser Val Ala Asn Asn
                   85                  90                  95
   Val Ser Ser Ile Gly Arg Asn Asn Ser Pro Ala Asp Met Pro Ala Gly
               100                 105                 110
   Ala Gly Asn Arg Ser Thr Gly Ser Pro Ser Pro Thr Val Pro Thr Asn
           115                 120                 125
   Thr Ser Val Asn Ile Asn Ser Thr Leu Leu Thr Pro Asn Ser Thr Val
       130                 135                 140
   Asn Ala Thr Asn Ser Arg Phe Ser Asn Ala Pro Phe Pro Ala Ile Ser
   145                 150                 155                 160
   Ala Ala Ala Asn Ser Thr Ala Ser Thr Ile Thr Cys Ser Ser Met Ile
                   165                 170                 175
   Ile Gly Leu Thr Ser Ala Leu Val Leu Ala Ser Leu
               180                 185
<210> 147
   <211> 192
   <212> DNA
   <213> Phakopsora pachyrhizi
<220>
   <221> misc_feature
   <222> (1)..(192)
   <223> ta01387.04_phapa
<400> 147
   atgcattcat ttatttcgtc aaccctagtt ttaatcgtag cccagcttgt ctcagctgcc        60
   ctcttgctga aagatcggag gtgcaatcat ctgtcggcca ctcagaacaa aaatttgcag       120
   gactcggtgg ccttccgtgt cctcctgtcg gtggaatctt gcctcctgtt ggtggaatct       180
   tgccccctgt ag                                                           192
<210> 148
   <211> 63
   <212> PRT
   <213> Phakopsora pachyrhizi
<220>
   <221> VARIANT
   <222> (1)..(63)
   <223> ta01387.04_phapa
<400> 148
   Met His Ser Phe Ile Ser Ser Thr Leu Val Leu Ile Val Ala Gln Leu
   1               5                   10                  15
   Val Ser Ala Ala Leu Leu Leu Lys Asp Arg Arg Cys Asn His Leu Ser
               20                  25                  30
   Ala Thr Gln Asn Lys Asn Leu Gln Asp Ser Val Ala Phe Arg Val Leu
           35                  40                  45
   Leu Ser Val Glu Ser Cys Leu Leu Leu Val Glu Ser Cys Pro Leu
       50                  55                  60
<210> 149
   <211> 375
   <212> DNA
   <213> Phakopsora pachyrhizi
<220>
   <221> misc_feature
   <222> (1)..(375)
   <223> ta00470.01_phapa
<400> 149
   atgaggataa ttgccttttt cgcactactt gctgccatct gcctcccatt tgtagagaac      60
   agatgccata gaagtggagc cacattcgga tcgtctccca atagagctta tgctactcag     120
   ttattgcctg gcgtatgcaa agaattccaa ggcacctatg gaccaaatca ggacaagtcc     180
   gtttgtagaa acggcagaga tagagatacc agcttcagat actatatcaa acacgtctca     240
   gatgggtata ggagcattgg cccaactgag tgtacagatg gcctcaacaa agagatagtc     300
   aattgcgata gaggtggaaa aacggcttat ggcaactggg aatacaatgt tgaccctaac     360
   aaaggaaggt gctag                                                      375
<210> 150
   <211> 124
   <212> PRT
   <213> Phakopsora pachyrhizi
<220>
   <221> VARIANT
   <222> (1)..(124)
   <223> ta00470.01_phapa
<400> 150
   Met Arg Ile Ile Ala Phe Phe Ala Leu Leu Ala Ala Ile Cys Leu Pro
   1               5                   10                  15
   Phe Val Glu Asn Arg Cys His Arg Ser Gly Ala Thr Phe Gly Ser Ser
               20                  25                  30
   Pro Asn Arg Ala Tyr Ala Thr Gln Leu Leu Pro Gly Val Cys Lys Glu
           35                  40                  45
   Phe Gln Gly Thr Tyr Gly Pro Asn Gln Asp Lys Ser Val Cys Arg Asn
       50                  55                  60
   Gly Arg Asp Arg Asp Thr Ser Phe Arg Tyr Tyr Ile Lys His Val Ser
   65                  70                  75                  80
   Asp Gly Tyr Arg Ser Ile Gly Pro Thr Glu Cys Thr Asp Gly Leu Asn
                   85                  90                  95
   Lys Glu Ile Val Asn Cys Asp Arg Gly Gly Lys Thr Ala Tyr Gly Asn
               100                 105                 110
   Trp Glu Tyr Asn Val Asp Pro Asn Lys Gly Arg Cys
           115                 120
<210> 151
   <211> 465
   <212> DNA
   <213> Phakopsora pachyrhizi
<220>
   <221> misc_feature
   <222> (1)..(465)
   <223> ta08619.01_phapa
<400> 151
   atgcttaatc tatcattctc aggcctcttg atctttgcct gtgttctttt tcagtttacg      60
   tctatatcag caaagaattt aactgctact gctgtaaaaa atgctctgta ttcttcttgt     120
   gaaaagtctg ccttagccaa gccctgtggt ggggccctgg ataagctata tgacactgga     180
   cgatttaaaa gtttcacgat cgagaaacaa gtacactttg ataaaacttc aaattgcaaa     240
   ttcacctggt ggactgatac acaagttgct gctctaaatg tttccaaaac ttcagttcaa     300
   aatgcactat tggagatcga aagcgaatgt gtagctaacg gcctttcacc aacccaacac     360
   acgaacacct ccagtagcgc agatgtaccc actggaaaag gagtctatgt tggaactctt     420
   gaagcgaagc aatacgtagt cttagagctg gagttgtttt cttag                     465
<210> 152
   <211> 154
   <212> PRT
   <213> Phakopsora pachyrhizi
<220>
   <221> VARIANT
   <222> (1)..(154)
   <223> ta08619.01_phapa
<400> 152
   Met Leu Asn Leu Ser Phe Ser Gly Leu Leu Ile Phe Ala Cys Val Leu
   1               5                   10                  15
   Phe Gln Phe Thr Ser Ile Ser Ala Lys Asn Leu Thr Ala Thr Ala Val
               20                  25                  30
   Lys Asn Ala Leu Tyr Ser Ser Cys Glu Lys Ser Ala Leu Ala Lys Pro
           35                  40                  45
   Cys Gly Gly Ala Leu Asp Lys Leu Tyr Asp Thr Gly Arg Phe Lys Ser
       50                  55                  60
   Phe Thr Ile Glu Lys Gln Val His Phe Asp Lys Thr Ser Asn Cys Lys
   65                  70                  75                  80
   Phe Thr Trp Trp Thr Asp Thr Gln Val Ala Ala Leu Asn Val Ser Lys
                   85                  90                  95
   Thr Ser Val Gln Asn Ala Leu Leu Glu Ile Glu Ser Glu Cys Val Ala
               100                 105                 110
   Asn Gly Leu Ser Pro Thr Gln His Thr Asn Thr Ser Ser Ser Ala Asp
           115                 120                 125
   Val Pro Thr Gly Lys Gly Val Tyr Val Gly Thr Leu Glu Ala Lys Gln
       130                 135                 140
   Tyr Val Val Leu Glu Leu Glu Leu Phe Ser
   145                 150
<210> 153
   <211> 432
   <212> DNA
   <213> Phakopsora pachyrhizi
<220>
   <221> misc_feature
   <222> (1)..(432)
   <223> ta10247.01_phapa
<400> 153
   atgatgtcat tactatcttt ttccttggca ttcgttttat ttttaaatct tacggttact      60
   atcgattgca taaaaatcaa taatggagta aaggttgact gcaatctgcg tgccgagcca     120
   aaatattatg aaagaggcga tcctgattca ccgtatatga gatgcttcga tcaacaaaca     180
   tccaatatgc acaaatgtga aattgaaagc tgctctgata atccagtctg cgataattgc     240
   ttgatcacaa gcagtctacc accaaggcaa gtaactaacg gtctgacttg cctaaaatca     300
   tatatcattc ctcaaagcaa tagcaatcta agaaatttga gagaatcagt tttctgtgac      360
   actgattcag gaagttatac atgcgcagga ccctgcaggt cattcaaata ttgcagaaac     420
   tgtgtccctt ga                                                         432
<210> 154
   <211> 120
   <212> PRT
   <213> Phakopsora pachyrhizi
<220>
   <221> VARIANT
   <222> (1)..(120)
   <223> ta10247.01_phapa
<400> 154
   Met Met Ser Leu Leu Ser Phe Ser Leu Ala Phe Val Leu Phe Leu Asn
   1               5                   10                  15
   Leu Thr Val Thr Ile Asp Cys Ile Lys Ile Asn Asn Gly Val Lys Val
               20                  25                  30
   Asp Cys Asn Leu Arg Ala Glu Pro Lys Tyr Tyr Glu Arg Gly Asp Pro
           35                  40                  45
   Asp Ser Pro Tyr Met Arg Cys Phe Asp Gln Gln Thr Ser Asn Met His
       50                  55                  60
   Lys Cys Glu Ile Glu Ser Cys Ser Asp Asn Pro Val Cys Asp Asn Cys
   65                  70                  75                  80
   Leu Ile Thr Ser Ser Leu Pro Pro Arg Gln Val Thr Asn Gly Leu Thr
                   85                  90                  95
   Cys Leu Lys Ser Tyr Ile Ile Pro Gln Ser Asn Ser Asn Leu Arg Asn
               100                 105                 110
   Leu Arg Glu Ser Val Phe Cys Asp
           115                 120
<210> 155
   <211> 276
   <212> DNA
   <213> Phakopsora pachyrhizi
<220>
   <221> misc_feature
   <222> (1)..(276)
   <223> ta03856.01_phapa
<400> 155
   atgaggttta acaatttcca cagattctct tcgttcgaag cttcgaaggt taaattactt      60
   ttcatcgtat gcgctttatc cattagcctg accagctctt tacaattaaa cattttgaag     120
   ggtaccgaac ctggttgcgg ttcccttcct gagaataata tgattaatat gcaattatca     180
   caatattcgg atgcgcgaga tcctggtaaa ggtagttctc ctaagaccaa taaggaatgg     240
   ccatactctg atgataatca aaccgattta aagtga                               276
<210> 156
   <211> 91
   <212> PRT
   <213> Phakopsora pachyrhizi
<220>
   <221> VARIANT
   <222> (1)..(91)
   <223> ta03856.01_phapa
<400> 156
   Met Arg Phe Asn Asn Phe His Arg Phe Ser Ser Phe Glu Ala Ser Lys
   1               5                   10                  15
   Val Lys Leu Leu Phe Ile Val Cys Ala Leu Ser Ile Ser Leu Thr Ser
               20                  25                  30
   Ser Leu Gln Leu Asn Ile Leu Lys Gly Thr Glu Pro Gly Cys Gly Ser
           35                  40                  45
   Leu Pro Glu Asn Asn Met Ile Asn Met Gln Leu Ser Gln Tyr Ser Asp
       50                  55                  60
   Ala Arg Asp Pro Gly Lys Gly Ser Ser Pro Lys Thr Asn Lys Glu Trp
   65                  70                  75                  80
   Pro Tyr Ser Asp Asp Asn Gln Thr Asp Leu Lys
                   85                  90
<210> 157
   <211> 462
   <212> DNA
   <213> Phakopsora pachyrhizi
<220>
   <221> misc_feature
   <222> (1)..(462)
   <223> ta10287.01_phapa
<400> 157
   atgattgtga tgatttctag aacccttcca gttgttttct gtcttatttt attgatctca      60
   aacattagta ctcgtacagc tcaaccaggt gacatggtaa agagagccaa cccagatgct     120
   ttgtttaccg attgtaaaga cgccgttata aaagaaggct gtaacacggc tctggacgaa     180
   ctttggaata acggaaaaat taaaaggttt acaaccgaac atcaatttgt cacccatcca     240
   agcggttgca aaattacttg gtggtctgac ggtggcgtgg ttcgcgcagg ggataatcaa     300
   aaaggttcat tgcagaatgc tttacagcaa attgatgatg cttgtacttc atccggcctt     360
   tcatctgtct atgactcaaa taaaaaggga aacggtgctt acgttggtaa actgcttgga     420
   aaccaatact tggtcatcat ggcagagact ggtgcacagt ag                        462
<210> 158
   <211> 153
   <212> PRT
   <213> Phakopsora pachyrhizi
<220>
   <221> VARIANT
   <222> (1)..(153)
   <223> ta10287.01_phapa
<400> 158
   Met Ile Val Met Ile Ser Arg Thr Leu Pro Val Val Phe Cys Leu Ile
   1               5                   10                  15
   Leu Leu Ile Ser Asn Ile Ser Thr Arg Thr Ala Gln Pro Gly Asp Met
               20                  25                  30
   Val Lys Arg Ala Asn Pro Asp Ala Leu Phe Thr Asp Cys Lys Asp Ala
           35                  40                  45
   Val Ile Lys Glu Gly Cys Asn Thr Ala Leu Asp Glu Leu Trp Asn Asn
       50                  55                  60
   Gly Lys Ile Lys Arg Phe Thr Thr Glu His Gln Phe Val Thr His Pro
   65                  70                  75                  80
   Ser Gly Cys Lys Ile Thr Trp Trp Ser Asp Gly Gly Val Val Arg Ala
                   85                  90                  95
   Gly Asp Asn Gln Lys Gly Ser Leu Gln Asn Ala Leu Gln Gln Ile Asp
               100                 105                 110
   Asp Ala Cys Thr Ser Ser Gly Leu Ser Ser Val Tyr Asp Ser Asn Lys
           115                 120                 125
   Lys Gly Asn Gly Ala Tyr Val Gly Lys Leu Leu Gly Asn Gln Tyr Leu
       130                 135                 140
   Val Ile Met Ala Glu Thr Gly Ala Gln
   145                 150
<210> 159
   <211> 315
   <212> DNA
   <213> Phakopsora pachyrhizi
<220>
   <221> misc_feature
   <222> (1)..(315)
   <223> ta08674.01_phapa
<400> 159
   atgaacttct gcaagtttgc taccttaacc tttgcggcct ttctaggaat ctcactcttc       60
   caactatctg ctgctgtagc tgtaccattt caaaaaagcg cgctggaaaa ctactcagat      120
   acaatgaatg gagctgcaaa ccatcacgtc gacaaacgat cttcatcctc gttgctccca      180
   caaactcaat caaaatcttc accaagctta cagaaacgat ttatatatac gagacctatt      240
   gtacctttgg caccagtcag aagatgtata caatactctc ctatcactgg cctttgtgtt      300
   ctttattcct tctag                                                       315
<210> 160
   <211> 104
   <212> PRT
   <213> Phakopsora pachyrhizi
<220>
   <221> VARIANT
   <222> (1)..(104)
   <223> ta08674.01_phapa
<400> 160
   Met Asn Phe Cys Lys Phe Ala Thr Leu Thr Phe Ala Ala Phe Leu Gly
   1               5                   10                  15
   Ile Ser Leu Phe Gln Leu Ser Ala Ala Val Ala Val Pro Phe Gln Lys
               20                  25                  30
   Ser Ala Leu Glu Asn Tyr Ser Asp Thr Met Asn Gly Ala Ala Asn His
           35                  40                  45
   His Val Asp Lys Arg Ser Ser Ser Ser Leu Leu Pro Gln Thr Gln Ser
       50                  55                  60
   Lys Ser Ser Pro Ser Leu Gln Lys Arg Phe Ile Tyr Thr Arg Pro Ile
   65                  70                  75                  80
   Val Pro Leu Ala Pro Val Arg Arg Cys Ile Gln Tyr Ser Pro Ile Thr
                   85                  90                  95
   Gly Leu Cys Val Leu Tyr Ser Phe
               100
<210> 161
   <211> 249
   <212> DNA
   <213> Phakopsora pachyrhizi
<220>
   <221> misc_feature
   <222> (1)..(249)
   <223> ta08831.01_phapa
<400> 161
   atgaacatca tcagctcaac ctacttagct gttgcagcta ttctgattgc caaaaacaat      60
   tttgccggcg ccgccagcct aaaccctagg gatgaggttc cgaatttggc tcatatcaga     120
   tatgaagcaa ggtcagttgg gaagtacaat caccttgagg cccgcaaaaa gaggccaaaa     180
   aataaaactg gcgccaataa tgtaaactct gcaaacaaca aaaacaccag cgataatacg     240
   ggaagataa                                                             249
<210> 162
   <211> 82
   <212> PRT
   <213> Phakopsora pachyrhizi
<220>
   <221> VARIANT
   <222> (1)..(82)
   <223> ta08831.01_phapa
<400> 162
   Met Asn Ile Ile Ser Ser Thr Tyr Leu Ala Val Ala Ala Ile Leu Ile
   1               5                   10                  15
   Ala Lys Asn Asn Phe Ala Gly Ala Ala Ser Leu Asn Pro Arg Asp Glu
               20                  25                  30
   Val Pro Asn Leu Ala His Ile Arg Tyr Glu Ala Arg Ser Val Gly Lys
           35                  40                  45
   Tyr Asn His Leu Glu Ala Arg Lys Lys Arg Pro Lys Asn Lys Thr Gly
       50                  55                  60
   Ala Asn Asn Val Asn Ser Ala Asn Asn Lys Asn Thr Ser Asp Asn Thr
   65                  70                  75                  80
   Gly Arg
<210> 163
   <211> 366
   <212> DNA
   <213> Phakopsora pachyrhizi
<220>
   <221> misc_feature
   <222> (1)..(366)
   <223> ta10737.01_phapa
<400> 163
   atgttcttcg caaagaaatt tttgctttca atttgcatga tgggccttat aagtatggcc      60
   tgtgcacaaa cttcgccatt caaggtttca tgccaaggag attctggaat caaattagac     120
   gattgcacaa ctgcaattgc caattttgac ggtaagctta ctgacgttgg attcaacact     180
   caaggtgact ggagacagtt tggaagctgc agagtctata tcaggaaaaa ggttgctgct     240
   tcaaataaat caaccataaa ccgaggctac tttatcaact ttgttaaggc agctttggaa     300
   ggttgctctg gcggtactgg tgctgcggaa aattcagact actacgtttc gattataagc     360
   aaatag                                                                366
<210> 164
   <211> 121
   <212> PRT
   <213> Phakopsora pachyrhizi
<220>
   <221> VARIANT
   <222> (1)..(121)
   <223> ta10737.01_phapa
<400> 164
   Met Phe Phe Ala Lys Lys Phe Leu Leu Ser Ile Cys Met Met Gly Leu
   1               5                   10                  15
   Ile Ser Met Ala Cys Ala Gln Thr Ser Pro Phe Lys Val Ser Cys Gln
               20                  25                  30
   Gly Asp Ser Gly Ile Lys Leu Asp Asp Cys Thr Thr Ala Ile Ala Asn
           35                  40                  45
   Phe Asp Gly Lys Leu Thr Asp Val Gly Phe Asn Thr Gln Gly Asp Trp
       50                  55                  60
   Arg Gln Phe Gly Ser Cys Arg Val Tyr Ile Arg Lys Lys Val Ala Ala
   65                  70                  75                  80
   Ser Asn Lys Ser Thr Ile Asn Arg Gly Tyr Phe Ile Asn Phe Val Lys
                   85                  90                  95
   Ala Ala Leu Glu Gly Cys Ser Gly Gly Thr Gly Ala Ala Glu Asn Ser
               100                 105                 110
   Asp Tyr Tyr Val Ser Ile Ile Ser Lys
           115                 120
<210> 165
   <211> 486
   <212> DNA
   <213> Phakopsora pachyrhizi
<220>
   <221> misc_feature
   <222> (1)..(486)
   <223> ta02458.01_phapa
<400> 165
   atgaactctt tgaaagcatt ttcgctcttt gctgtcctta tcctgacatt aggaagtcaa       60
   aattcttcgc aaagagaatt gggaagctca aatcttttgg ccgtctgcgg agagaagtat      120
   gaaaaggcga gctcagtgtt tgcctgcagc caaggaagat taagatgtaa cgcggagcct      180
   ggagaaactg gaaaattttt ctgcacaggc gatggtaaat tgacgaaggt ggagggagaa      240
   aaggcttcca agtgtggctt caacccagag tgtgaaaaac aagggaactc agaaagtgct      300
   gagagtatgg attctgctaa caatttggcc aaaaggggga ataaaaagaa caagggtggt      360
   gcaaccccac aacagaaaca gccacagcaa cagcagcaac aacaacaaca gcaacaacaa      420
   aatcaattta taaacaagga ggattctgga gcgagcaaag cgcctgatcc aatcctagtt      480
   gagtaa                                                                 486
<210> 166
   <211> 161
   <212> PRT
   <213> Phakopsora pachyrhizi
<220>
   <221> VARIANT
   <222> (1)..(161)
   <223> ta02458.01_phapa
<400> 166
   Met Asn Ser Leu Lys Ala Phe Ser Leu Phe Ala Val Leu Ile Leu Thr
   1               5                   10                  15
   Leu Gly Ser Gln Asn Ser Ser Gln Arg Glu Leu Gly Ser Ser Asn Leu
               20                  25                  30
   Leu Ala Val Cys Gly Glu Lys Tyr Glu Lys Ala Ser Ser Val Phe Ala
           35                  40                  45
   Cys Ser Gln Gly Arg Leu Arg Cys Asn Ala Glu Pro Gly Glu Thr Gly
       50                  55                  60
   Lys Phe Phe Cys Thr Gly Asp Gly Lys Leu Thr Lys Val Glu Gly Glu
   65                  70                  75                  80
   Lys Ala Ser Lys Cys Gly Phe Asn Pro Glu Cys Glu Lys Gln Gly Asn
                   85                  90                  95
   Ser Glu Ser Ala Glu Ser Met Asp Ser Ala Asn Asn Leu Ala Lys Arg
               100                 105                 110
   Gly Asn Lys Lys Asn Lys Gly Gly Ala Thr Pro Gln Gln Lys Gln Pro
           115                 120                 125
   Gln Gln Gln Gln Gln Gln Gln Gln Gln Gln Gln Gln Asn Gln Phe Ile
       130                 135                 140
   Asn Lys Glu Asp Ser Gly Ala Ser Lys Ala Pro Asp Pro Ile Leu Val
   145                 150                 155                 160
   Glu
<210> 167
   <211> 366
   <212> DNA
   <213> Phakopsora pachyrhizi
<220>
   <221> misc_feature
   <222> (1)..(366)
   <223> ta09953.01_phapa
<400> 167
   atgcataaac gtattatata tatatccttt ggtaatccaa atattatttt ggttcttctc       60
   acaagttttc tatcttttta taattttaat gctgcacaat catcaaatct ggcggttcaa      120
   caaactgagt ccacaaagaa atcaattgat tatgttaaac atagaggaca aattcctagt      180
   gaggatgcta ctagactgca ggaaagattt tttccacgag ctgcagacgc aaccaaagtt      240
   gaaaagctcg atagctccat aaaaataaaa gggaaacgga tagaaaaagg tacaaaagtc      300
   aacggaagga aaaaaccggt agaaacaaat attaaacttg ctcatagttt ttcagtctca      360
   aattaa                                                                 366
<210> 168
   <211> 121
   <212> PRT
   <213> Phakopsora pachyrhizi
<220>
   <221> VARIANT
   <222> (1)..(121)
   <223> ta09953.01_phapa
<400> 168
   Met His Lys Arg Ile Ile Tyr Ile Ser Phe Gly Asn Pro Asn Ile Ile
   1               5                   10                  15
   Leu Val Leu Leu Thr Ser Phe Leu Ser Phe Tyr Asn Phe Asn Ala Ala
               20                  25                  30
   Gln Ser Ser Asn Leu Ala Val Gln Gln Thr Glu Ser Thr Lys Lys Ser
           35                  40                  45
   Ile Asp Tyr Val Lys His Arg Gly Gln Ile Pro Ser Glu Asp Ala Thr
       50                  55                  60
   Arg Leu Gln Glu Arg Phe Phe Pro Arg Ala Ala Asp Ala Thr Lys Val
   65                  70                  75                  80
   Glu Lys Leu Asp Ser Ser Ile Lys Ile Lys Gly Lys Arg Ile Glu Lys
                   85                  90                  95
   Gly Thr Lys Val Asn Gly Arg Lys Lys Pro Val Glu Thr Asn Ile Lys
               100                 105                 110
   Leu Ala His Ser Phe Ser Val Ser Asn
           115                 120
<210> 169
   <211> 441
   <212> DNA
   <213> Phakopsora pachyrhizi
<220>
   <221> misc_feature
   <222> (1)..(441)
   <223> ta00392.02_phapa
<400> 169
   atgttcagca ttaaaatctt cattgccctc gttttaatag cacagctgag ccaggcaagc       60
   gtcatttcta gccatgactc aggagtgtca acggttaaaa atcattggaa aaagaagaaa      120
   ccatgcacca agaggcccgt aaaagtaata cccccaccac cacctatagg tcgaccacca      180
   atacctcacg ttggtgttgt ggctggcggt atacctcttg gaggcgttcc tggagtgcca      240
   ccggttgggg gcttactacg ttgtgacggt ccgggatgtc cagcaggcgg tttttgtggt      300
   ccaggagctt gcagtggatg tgttagaaca actttcggtt ttagttgtgg cagtgtatct      360
   tcgtttacta ctacgttccc cggaggtttc atgacttcat cctcttctag tagttcttct      420
   caatcatcct ttccttttta a                                                441
<210> 170
   <211> 146
   <212> PRT
   <213> Phakopsora pachyrhizi
<220>
   <221> VARIANT
   <222> (1)..(146)
   <223> ta00392.02_phapa
<400> 170
   Met Phe Ser Ile Lys Ile Phe Ile Ala Leu Val Leu Ile Ala Gln Leu
   1               5                   10                  15
   Ser Gln Ala Ser Val Ile Ser Ser His Asp Ser Gly Val Ser Thr Val
               20                  25                  30
   Lys Asn His Trp Lys Lys Lys Lys Pro Cys Thr Lys Arg Pro Val Lys
           35                  40                  45
   Val Ile Pro Pro Pro Pro Pro Ile Gly Arg Pro Pro Ile Pro His Val
       50                  55                  60
   Gly Val Val Ala Gly Gly Ile Pro Leu Gly Gly Val Pro Gly Val Pro
   65                  70                  75                  80
   Pro Val Gly Gly Leu Leu Arg Cys Asp Gly Pro Gly Cys Pro Ala Gly
                   85                  90                  95
   Gly Phe Cys Gly Pro Gly Ala Cys Ser Gly Cys Val Arg Thr Thr Phe
               100                 105                 110
   Gly Phe Ser Cys Gly Ser Val Ser Ser Phe Thr Thr Thr Phe Pro Gly
           115                 120                 125
   Gly Phe Met Thr Ser Ser Ser Ser Ser Ser Ser Ser Gln Ser Ser Phe
       130                 135                 140
   Pro Phe
   145
<210> 171
   <211> 492
   <212> DNA
   <213> Phakopsora pachyrhizi
<220>
   <221> misc_feature
   <222> (1)..(492)
   <223> ta09632.01_phapa
<400> 171
   atggctaagc ggttattatc attctactac tttttaacct gttttgcgaa tattttgtcc      60
   actccaaagc tgcagccttt ggtatgcccg aatgattggt cacttgatcc aatatcaagt     120
   tcccaaaata ctaggcgtaa tgtttactgc tatgatcacc aattcttgtg cgaagaaagc     180
   gattgttatg agtatccagt ttgcagctct tgcactagtg ataaagggaa attagccaag     240
   acagttgcat gcaaaaatga ataccatgct tccgatccaa caactgacca acaagcaatc     300
   tgtgtcagca gcgatgagac cttttggcaa tgctctggcc cttgcgaagg atacctaaaa     360
   tgcataaatt tcagtcgtaa aggccctttc atcaaaagcc aactccgtaa aaggggcaat     420
   ccgtttgcgg ttgcgatgaa tgggagtcca ggcggaacct cgagtaattt tgattctgtc     480
   ttatcctttt ag                                                         492
<210> 172
   <211> 163
   <212> PRT
   <213> Phakopsora pachyrhizi
<220>
   <221> VARIANT
   <222> (1)..(163)
   <223> ta09632.01_phapa
<400> 172
   Met Ala Lys Arg Leu Leu Ser Phe Tyr Tyr Phe Leu Thr Cys Phe Ala
   1               5                   10                  15
   Asn Ile Leu Ser Thr Pro Lys Leu Gln Pro Leu Val Cys Pro Asn Asp
               20                  25                  30
   Trp Ser Leu Asp Pro Ile Ser Ser Ser Gln Asn Thr Arg Arg Asn Val
           35                  40                  45
   Tyr Cys Tyr Asp His Gln Phe Leu Cys Glu Glu Ser Asp Cys Tyr Glu
       50                  55                  60
   Tyr Pro Val Cys Ser Ser Cys Thr Ser Asp Lys Gly Lys Leu Ala Lys
   65                  70                  75                  80
   Thr Val Ala Cys Lys Asn Glu Tyr His Ala Ser Asp Pro Thr Thr Asp
                   85                  90                  95
   Gln Gln Ala Ile Cys Val Ser Ser Asp Glu Thr Phe Trp Gln Cys Ser
               100                 105                 110
   Gly Pro Cys Glu Gly Tyr Leu Lys Cys Ile Asn Phe Ser Arg Lys Gly
           115                 120                 125
   Pro Phe Ile Lys Ser Gln Leu Arg Lys Arg Gly Asn Pro Phe Ala Val
       130                 135                 140
   Ala Met Asn Gly Ser Pro Gly Gly Thr Ser Ser Asn Phe Asp Ser Val
   145                 150                 155                 160
   Leu Ser Phe
<210> 173
   <211> 531
   <212> DNA
   <213> Phakopsora pachyrhizi
<220>
   <221> misc_feature
   <222> (1)..(531)
   <223> ta01929.01_phapa
<400> 173
   atgtatattt cctcattgct agcagttcta tcttttgctt gctgtgttct tgcccaaacg      60
   aatgtcaccg gcccaaacaa tcaaacaatg cctgctgttt ttgcaatgaa ttgtactaac     120
   acctacttgc ctgtctcaga gcgccgatta gcgcaactgg cctctaacac ttccctaact     180
   cccgaggaaa ctaacaaact ttccgataat ttcactcagg ccatttgtca aaacaccact     240
   ttagatcttt gcttttgcga tatcacctct tgtggctctg gtgccattgg aaaaaattgc     300
   gttaaactga attccacaac tggtgatcca ctcgaaaaaa atcaaacccc actgccaaca     360
   gcgaattgct cagctagtct tgtttacaac cctaaagctc aatcggaagc tgacaaggcg     420
   aacatgtgca tgaatgccga aggcgatttt tttgcttgtg aatcttacag tggcagtact     480
   atgtgcagca tgtgtgtttc gtcttctgac cccactctta cgcaagttta g              531
<210> 174
   <211> 176
   <212> PRT
   <213> Phakopsora pachyrhizi
<220>
   <221> VARIANT
   <222> (1)..(176)
   <223> ta01929.01_phapa
<400> 174
   Met Tyr Ile Ser Ser Leu Leu Ala Val Leu Ser Phe Ala Cys Cys Val
   1               5                   10                  15
   Leu Ala Gln Thr Asn Val Thr Gly Pro Asn Asn Gln Thr Met Pro Ala
               20                  25                  30
   Val Phe Ala Met Asn Cys Thr Asn Thr Tyr Leu Pro Val Ser Glu Arg
           35                  40                  45
   Arg Leu Ala Gln Leu Ala Ser Asn Thr Ser Leu Thr Pro Glu Glu Thr
       50                  55                  60
   Asn Lys Leu Ser Asp Asn Phe Thr Gln Ala Ile Cys Gln Asn Thr Thr
   65                  70                  75                  80
   Leu Asp Leu Cys Phe Cys Asp Ile Thr Ser Cys Gly Ser Gly Ala Ile
                   85                  90                  95
   Gly Lys Asn Cys Val Lys Leu Asn Ser Thr Thr Gly Asp Pro Leu Glu
               100                 105                 110
   Lys Asn Gln Thr Pro Leu Pro Thr Ala Asn Cys Ser Ala Ser Leu Val
           115                 120                 125
   Tyr Asn Pro Lys Ala Gln Ser Glu Ala Asp Lys Ala Asn Met Cys Met
       130                 135                 140
   Asn Ala Glu Gly Asp Phe Phe Ala Cys Glu Ser Tyr Ser Gly Ser Thr
   145                 150                 155                 160
   Met Cys Ser Met Cys Val Ser Ser Ser Asp Pro Thr Leu Thr Gln Val
                   165                 170                 175
<210> 175
   <211> 423
   <212> DNA
   <213> Phakopsora pachyrhizi
<220>
   <221> misc_feature
   <222> (1)..(423)
   <223> ta00836.01_phapa
<400> 175
   atggcgttta attttcagac tttgttcact gcactcatag taattgccat ggttttagct      60
   ggttttgtgg ttgcagcata ctgcatctgg agcaaggtct tgcacaccga gaaaaatagt     120
   atcacagcct accaaaaggc attagcaaaa gctacaagca cagaaatcaa gtataagaag     180
   aagcatgaag acgtagctga aggtcataaa gatagcactt gccaatgttt taaaaaccag     240
   tccacggccg tgaattcggg cgcaaaacta agattttcag gattctcacg tccacaatgc     300
   aaccccaatt ttagccgcat taaccgtatt aatccgcaat ttcccacaac atcaaaaatc     360
   atgacgaaac cacaaccgcg accgcaattt acaaccttga cacctgtgcc tgggagactt     420
   tga                                                                   423
<210> 176
   <211> 140
   <212> PRT
   <213> Phakopsora pachyrhizi
<220>
   <221> VARIANT
   <222> (1)..(140)
   <223> ta00836.01_phapa
<400> 176
   Met Ala Phe Asn Phe Gln Thr Leu Phe Thr Ala Leu Ile Val Ile Ala
   1               5                   10                  15
   Met Val Leu Ala Gly Phe Val Val Ala Ala Tyr Cys Ile Trp Ser Lys
               20                  25                  30
   Val Leu His Thr Glu Lys Asn Ser Ile Thr Ala Tyr Gln Lys Ala Leu
           35                  40                  45
   Ala Lys Ala Thr Ser Thr Glu Ile Lys Tyr Lys Lys Lys His Glu Asp
       50                  55                  60
   Val Ala Glu Gly His Lys Asp Ser Thr Cys Gln Cys Phe Lys Asn Gln
   65                  70                  75                  80
   Ser Thr Ala Val Asn Ser Gly Ala Lys Leu Arg Phe Ser Gly Phe Ser
                   85                  90                  95
   Arg Pro Gln Cys Asn Pro Asn Phe Ser Arg Ile Asn Arg Ile Asn Pro
               100                 105                 110
   Gln Phe Pro Thr Thr Ser Lys Ile Met Thr Lys Pro Gln Pro Arg Pro
           115                 120                 125
   Gln Phe Thr Thr Leu Thr Pro Val Pro Gly Arg Leu
       130                 135                 140
<210> 177
   <211> 267
   <212> DNA
   <213> Phakopsora pachyrhizi
<220>
   <221> misc_feature
   <222> (1)..(267)
   <223> ta02276.02_phapa
<400> 177
   atgagaggaa ccatcatttg ggtcgcggct attggtcttg ctatcataag tttggctgat       60
   caagtaaaat ccgcgccttc agctcaggtc tctgagtttg gcaaagaccc aacaaaacta      120
   ccatcatcac tccaaaaacg tagtccaaga ccagaaccaa agaataagga tggaaagact      180
   aaaaagacta aaaagaccaa aaagaaaaag aaaaagggcc actcacacgg gggtgatgat      240
   gatgatgatg atgatgatga tgattag                                          267
<210> 178
   <211> 88
   <212> PRT
   <213> Phakopsora pachyrhizi
<220>
   <221> VARIANT
   <222> (1)..(88)
   <223> ta02276.02_phapa
<400> 178
   Met Arg Gly Thr Ile Ile Trp Val Ala Ala Ile Gly Leu Ala Ile Ile
   1               5                   10                  15
   Ser Leu Ala Asp Gln Val Lys Ser Ala Pro Ser Ala Gln Val Ser Glu
               20                  25                  30
   Phe Gly Lys Asp Pro Thr Lys Leu Pro Ser Ser Leu Gln Lys Arg Ser
           35                  40                  45
   Pro Arg Pro Glu Pro Lys Asn Lys Asp Gly Lys Thr Lys Lys Thr Lys
       50                  55                  60
   Lys Thr Lys Lys Lys Lys Lys Lys Gly His Ser His Gly Gly Asp Asp
   65                  70                  75                  80
   Asp Asp Asp Asp Asp Asp Asp Asp
                           85
<210> 179
   <211> 387
   <212> DNA
   <213> Phakopsora pachyrhizi
<220>
   <221> misc_feature
   <222> (1)..(387)
   <223> ta09592.01_phapa
<400> 179
   atgaatcgtt tcatgatact agcttttgtg ctagttgaac ttttgctctt aacctcaaaa        60
   gctcaagctt tatcatcaaa ctgtgaagaa gcttactttg agtctaacgt tccgaaagtt       120
   gcaggagtcc cgcaggcagg ctgtaaatct agctcctcaa aggaaggatt cttatgctct       180
   caaaatagct gcagcggcct taagtcctgc acccaatgta aacgctatat gccttcacca       240
   acagctggaa agagcggtac tttcgttggg aacataattc ctatgagcat taactgcaag       300
   accgactatg ttcttgtgaa caagatacaa atgtgctcaa acgctaacaa tgccggtacg       360
   gatgcgttgg agcttgtacc ggtatga                                           387
<210> 180
   <211> 128
   <212> PRT
   <213> Phakopsora pachyrhizi
<220>
   <221> VARIANT
   <222> (1)..(128)
   <223> ta09592.01_phapa
<400> 180
   Met Asn Arg Phe Met Ile Leu Ala Phe Val Leu Val Glu Leu Leu Leu
   1               5                   10                  15
   Leu Thr Ser Lys Ala Gln Ala Leu Ser Ser Asn Cys Glu Glu Ala Tyr
               20                  25                  30
   Phe Glu Ser Asn Val Pro Lys Val Ala Gly Val Pro Gln Ala Gly Cys
           35                  40                  45
   Lys Ser Ser Ser Ser Lys Glu Gly Phe Leu Cys Ser Gln Asn Ser Cys
       50                  55                  60
   Ser Gly Leu Lys Ser Cys Thr Gln Cys Lys Arg Tyr Met Pro Ser Pro
   65                  70                  75                  80
   Thr Ala Gly Lys Ser Gly Thr Phe Val Gly Asn Ile Ile Pro Met Ser
                   85                  90                  95
   Ile Asn Cys Lys Thr Asp Tyr Val Leu Val Asn Lys Ile Gln Met Cys
               100                 105                 110
   Ser Asn Ala Asn Asn Ala Gly Thr Asp Ala Leu Glu Leu Val Pro Val
           115                 120                 125
<210> 181
   <211> 201
   <212> DNA
   <213> Phakopsora pachyrhizi
<220>
   <221> misc_feature
   <222> (1)..(201)
   <223> ta01647.01_phapa
<400> 181
   atgatgcatc tacgtcattt tttcattttg tttcgagtat ttatatttat atatatactc      60
   tctgacttca ttctcccctc cattatattc ccatgttcaa gatttttaac gaaatggaaa     120
   tatgtaagtc ccatgcgaag aaagcagaag attctaaaaa aaatggagga attgaggggg     180
   atggagtacg ttgtagggta g                                               201
<210> 182
   <211> 66
   <212> PRT
   <213> Phakopsora pachyrhizi
<220>
   <221> VARIANT
   <222> (1)..(66)
   <223> ta01647.01_phapa
<400> 182
   Met Met His Leu Arg His Phe Phe Ile Leu Phe Arg Val Phe Ile Phe
   1               5                   10                  15
   Ile Tyr Ile Leu Ser Asp Phe Ile Leu Pro Ser Ile Ile Phe Pro Cys
               20                  25                  30
   Ser Arg Phe Leu Thr Lys Trp Lys Tyr Val Ser Pro Met Arg Arg Lys
           35                  40                  45
   Gln Lys Ile Leu Lys Lys Met Glu Glu Leu Arg Gly Met Glu Tyr Val
       50                  55                  60
   Val Gly
   65
<210> 183
   <211> 414
   <212> DNA
   <213> Phakopsora pachyrhizi
<220>
   <221> misc_feature
   <222> (1)..(414)
   <223> ta10088.01_phapa
<400> 183
   atgattaaac attgcgcttt cattttaatg ggcctaatgg cgtttgtgtc cttatcattt      60
   gcacagctac taccaagcgc ctacagctgt agcagggttg atctttacaa acctccacta     120
   agacgtgaag attgcagaga ctccctaaat ttgtttccag ttccaaacgg cgataatgtt     180
   atttaccttg gaagtggcaa ttggcgaggt tgtggaagct gcaaggtgac tatttacaat     240
   aggggatcaa gagaatctcg tgtgactgcg ccaaaaggct gggcagctac ggcagtacac     300
   caagctttcg accattgtga aggaaagccg ggatctgcta ctattggaga tgatgggaaa     360
   attattgcaa aaatcgatta cggaaattct ggacaaggag attgtcctcc ctaa           414
<210> 184
   <211> 137
   <212> PRT
   <213> Phakopsora pachyrhizi
<220>
   <221> VARIANT
   <222> (1)..(137)
   <223> tal0088.01_phapa
<400> 184
   Met Ile Lys His Cys Ala Phe Ile Leu Met Gly Leu Met Ala Phe Val
   1               5                   10                  15
   Ser Leu Ser Phe Ala Gln Leu Leu Pro Ser Ala Tyr Ser Cys Ser Arg
               20                  25                  30
   Val Asp Leu Tyr Lys Pro Pro Leu Arg Arg Glu Asp Cys Arg Asp Ser
           35                  40                  45
   Leu Asn Leu Phe Pro Val Pro Asn Gly Asp Asn Val Ile Tyr Leu Gly
       50                  55                  60
   Ser Gly Asn Trp Arg Gly Cys Gly Ser Cys Lys Val Thr Ile Tyr Asn
   65                  70                  75                  80
   Arg Gly Ser Arg Glu Ser Arg Val Thr Ala Pro Lys Gly Trp Ala Ala
                   85                  90                  95
   Thr Ala Val His Gln Ala Phe Asp His Cys Glu Gly Lys Pro Gly Ser
               100                 105                 110
   Ala Thr Ile Gly Asp Asp Gly Lys Ile Ile Ala Lys Ile Asp Tyr Gly
               115                 120                 125
   Asn Ser Gly Gln Gly Asp Cys Pro Pro
       130                 135
<210> 185
   <211> 588
   <212> DNA
   <213> Phakopsora pachyrhizi
<220>
   <221> misc_feature
   <222> (1)..(588)
   <223> ta08848.01_phapa
<400> 185
   atgataaaat cacccttgaa atttatttct tctttcactt taattttctt cctacataga      60
   gcggttcatt cggctattat agaaacttct gcccagaggg agctgccaag gctggctcga     120
   atctcgaacc ctacgaatgt agatgagggc tttaaaagca cgcggttatt tgctttagat     180
   tcgtcatgct catttccaga aaagaataaa ataaataaaa atagagtcgg tatggtggtt     240
   gagtcagaaa aaagttttaa gaaaaattta gctctagata aacttgaacc aaatagaatt     300
   gagaatcaca ttcttgaaaa taatatcaag gctaaccccg gttccacgac tatgagtgat     360
   aagagctccg aggtcttgag cccaggccca ggtaattcca aagtgaatcg tggccaaaat     420
   tataagacac agtcggaaga aaccatagaa gatttaatcc cagtgaaaaa gcagagcgaa     480
   caaggactag aaaattcttg ggatgcgccg aaaatacaga aatcatttag gagaagtaag     540
   agtcgtcttg gtaaaaaacc ggaatttttg catgaaaaaa attcctga                  588
<210> 186
   <211> 195
   <212> PRT
   <213> Phakopsora pachyrhizi
<220>
   <221> VARIANT
   <222> (1)..(195)
   <223> ta08848.01_phapa
<400> 186
   Met Ile Lys Ser Pro Leu Lys Phe Ile Ser Ser Phe Thr Leu Ile Phe
   1               5                   10                  15
   Phe Leu His Arg Ala Val His Ser Ala Ile Ile Glu Thr Ser Ala Gln
               20                  25                  30
   Arg Glu Leu Pro Arg Leu Ala Arg Ile Ser Asn Pro Thr Asn Val Asp
           35                  40                  45
   Glu Gly Phe Lys Ser Thr Arg Leu Phe Ala Leu Asp Ser Ser Cys Ser
       50                  55                  60
   Phe Pro Glu Lys Asn Lys Ile Asn Lys Asn Arg Val Gly Met Val Val
   65                  70                  75                  80
   Glu Ser Glu Lys Ser Phe Lys Lys Asn Leu Ala Leu Asp Lys Leu Glu
                   85                  90                  95
   Pro Asn Arg Ile Glu Asn His Ile Leu Glu Asn Asn Ile Lys Ala Asn
               100                 105                 110
   Pro Gly Ser Thr Thr Met Ser Asp Lys Ser Ser Glu Val Leu Ser Pro
           115                 120                 125
   Gly Pro Gly Asn Ser Lys Val Asn Arg Gly Gln Asn Tyr Lys Thr Gln
       130                 135                 140
   Ser Glu Glu Thr Ile Glu Asp Leu Ile Pro Val Lys Lys Gln Ser Glu
   145                 150                 155                 160
   Gln Gly Leu Glu Asn Ser Trp Asp Ala Pro Lys Ile Gln Lys Ser Phe
                   165                 170                 175
   Arg Arg Ser Lys Ser Arg Leu Gly Lys Lys Pro Glu Phe Leu His Glu
               180                 185                 190
   Lys Asn Ser
           195
<210> 187
   <211> 435
   <212> DNA
   <213> Phakopsora pachyrhizi
<220>
   <221> misc_feature
   <222> (1)..(435)
   <223> ta06926.01_phapa
<400> 187
   atgaaaaatt ttaagcaatt gaactggata attctgggat tattgatttc attatcctat      60
   gaaatcagaa gctcagtcac gcaaaacatt tatgaaaccg aaattaatgt tctaagagcc     120
   agacaggcag gccctcctac aattgacaca tcaactctgc gggaatgcaa aaacatgaag     180
   atcgttggtg gcggtggttt caatggacga aaagcttctg actttacatt tagaccagag     240
   gactctaaga ttttcaagca aaaagaagca cagaatctcg gaataatctg taatgccatg     300
   tgcaatgatt tgaagaatgt ttgcaaattc aaagataagg acgaagtagt tgtaaattgt     360
   aataagatca aaaattttat cggaactact aaagataaag gcaagatgga tctttggaac     420
   tcgttgttcg tttga                                                      435
<210> 188
   <211> 144
   <212> PRT
   <213> Phakopsora pachyrhizi
<220>
   <221> VARIANT
   <222> (1)..(144)
   <223> ta06926.01_phapa
<400> 188
   Met Lys Asn Phe Lys Gln Leu Asn Trp Ile Ile Leu Gly Leu Leu Ile
   1               5                   10                  15
   Ser Leu Ser Tyr Glu Ile Arg Ser Ser Val Thr Gln Asn Ile Tyr Glu
               20                  25                  30
   Thr Glu Ile Asn Val Leu Arg Ala Arg Gln Ala Gly Pro Pro Thr Ile
           35                  40                  45
   Asp Thr Ser Thr Leu Arg Glu Cys Lys Asn Met Lys Ile Val Gly Gly
       50                  55                  60
   Gly Gly Phe Asn Gly Arg Lys Ala Ser Asp Phe Thr Phe Arg Pro Glu
   65                  70                  75                  80
   Asp Ser Lys Ile Phe Lys Gln Lys Glu Ala Gln Asn Leu Gly Ile Ile
                   85                  90                  95
   Cys Asn Ala Met Cys Asn Asp Leu Lys Asn Val Cys Lys Phe Lys Asp
               100                 105                 110
   Lys Asp Glu Val Val Val Asn Cys Asn Lys Ile Lys Asn Phe Ile Gly
           115                 120                 125
   Thr Thr Lys Asp Lys Gly Lys Met Asp Leu Trp Asn Ser Leu Phe Val
       130                 135                 140
<210> 189
   <211> 261
   <212> DNA
   <213> Phakopsora pachyrhizi
<220>
   <221> misc_feature
   <222> (1)..(261)
   <223> ta09197.01_phapa
<400> 189
   atgtttatta aatcatattg ttttcttctt ggtctcacgc ttttcctttc atgtctttcg      60
   actactataa ctggagagtt aaacaatatg gatgaccaaa tactagcagc taaatttcga     120
   gcaaggcgtt ctattgaaac aatcaaagaa aatagtccta taaaacaaga acggaaaaaa     180
   cttcgtaatg aaccagtaaa cacgctagtc gcttatccag tgagggatcc caattgcgtt     240
   gatgatgatg ctccaagata g                                               261
<210> 190
   <211> 86
   <212> PRT
   <213> Phakopsora pachyrhizi
<220>
   <221> VARIANT
   <222> (1)..(86)
   <223> ta09197.01_phapa
<400> 190
   Met Phe Ile Lys Ser Tyr Cys Phe Leu Leu Gly Leu Thr Leu Phe Leu
   1               5                   10                  15
   Ser Cys Leu Ser Thr Thr Ile Thr Gly Glu Leu Asn Asn Met Asp Asp
               20                  25                  30
   Gln Ile Leu Ala Ala Lys Phe Arg Ala Arg Arg Ser Ile Glu Thr Ile
           35                  40                  45
   Lys Glu Asn Ser Pro Ile Lys Gln Glu Arg Lys Lys Leu Arg Asn Glu
       50                  55                  60
   Pro Val Asn Thr Leu Val Ala Tyr Pro Val Arg Asp Pro Asn Cys Val
   65                  70                  75                  80
   Asp Asp Asp Ala Pro Arg
                   85
<210> 191
   <211> 324
   <212> DNA
   <213> Phakopsora pachyrhizi
<220>
   <221> misc_feature
   <222> (1)..(324)
   <223> ta09320.01_phapa
<400> 191
   atgtactcta aagtgcttat gatttatcta tttttcattg cgatcgcaat tcttgatttc      60
   acaggtgctt tgaccacata cggtgaaaat gataagaaca tgcgcaaaag agcgatagag     120
   gctagaagcg cagtacatgg aagcaattcg aaggaaacac cagaattttc attcccacca     180
   cccgagttta tatataagaa agactgttgg aaataccaat ctgattgtaa ggcagtactt     240
   aaagaacata aatgtccact gtactgttca gtaatttgtt cgtattgctc tggagatggt     300
   acagaatatg atccaaaagt ataa                                            324
<210> 192
   <211> 107
   <212> PRT
   <213> Phakopsora pachyrhizi
<220>
   <221> VARIANT
   <222> (1)..(107)
   <223> ta09320.01_phapa
<400> 192
   Met Tyr Ser Lys Val Leu Met Ile Tyr Leu Phe Phe Ile Ala Ile Ala
   1               5                   10                  15
   Ile Leu Asp Phe Thr Gly Ala Leu Thr Thr Tyr Gly Glu Asn Asp Lys
               20                  25                  30
   Asn Met Arg Lys Arg Ala Ile Glu Ala Arg Ser Ala Val His Gly Ser
           35                  40                  45
   Asn Ser Lys Glu Thr Pro Glu Phe Ser Phe Pro Pro Pro Glu Phe Ile
       50                  55                  60
   Tyr Lys Lys Asp Cys Trp Lys Tyr Gln Ser Asp Cys Lys Ala Val Leu
   65                  70                  75                  80
   Lys Glu His Lys Cys Pro Leu Tyr Cys Ser Val Ile Cys Ser Tyr Cys
                   85                  90                  95
   Ser Gly Asp Gly Thr Glu Tyr Asp Pro Lys Val
                       100                 105
<210> 193
   <211> 474
   <212> DNA
   <213> Phakopsora pachyrhizi
<220>
   <221> misc_feature
   <222> (1)..(474)
   <223> ta01319.01_phapa
<400> 193
   atgtgttcca ttactaattc tgcatttata atctttgtcg tcgtgctctc ttttttttct        60
   actgaagcta gtccagccgg cattaatgtg ggggacaaaa gcgatcgagc caagtgcaag      120
   ggaaatggtg ttcagaaagt ctgtcaacca attttagaaa aattgtggtc ctctggaaag      180
   attaaagtct tcaaacccga gcagcaaaag gagaatgacg caagctgttc aatatcgtgg      240
   aaaagtcttg acggcaataa agaaattaaa tcagaaaatt ccttgtttaa cgtcttcaag      300
   aaaattgatg atgtttgtaa aaacaagacg gaatctgcaa gctctacttg gaatccaaat      360
   attacccaat atggaaacct ttcagatggt aaaaactatt ccggaattgg ttacttctta      420
   ggaaaagttg aacagaacac tgtttttatc acagccttgg ctgtaaaacc ataa            474
<210> 194
   <211> 157
   <212> PRT
   <213> Phakopsora pachyrhizi
<220>
   <221> VARIANT
   <222> (1)..(157)
   <223> ta01319.01_phapa
<400> 194
   Met Cys Ser Ile Thr Asn Ser Ala Phe Ile Ile Phe Val Val Val Leu
   1               5                   10                  15
   Ser Phe Phe Ser Thr Glu Ala Ser Pro Ala Gly Ile Asn Val Gly Asp
               20                  25                  30
   Lys Ser Asp Arg Ala Lys Cys Lys Gly Asn Gly Val Gln Lys Val Cys
           35                  40                  45
   Gln Pro Ile Leu Glu Lys Leu Trp Ser Ser Gly Lys Ile Lys Val Phe
       50                  55                  60
   Lys Pro Glu Gln Gln Lys Glu Asn Asp Ala Ser Cys Ser Ile Ser Trp
   65                  70                  75                  80
   Lys Ser Leu Asp Gly Asn Lys Glu Ile Lys Ser Glu Asn Ser Leu Phe
                   85                  90                  95
   Asn Val Phe Lys Lys Ile Asp Asp Val Cys Lys Asn Lys Thr Glu Ser
               100                 105                 110
   Ala Ser Ser Thr Trp Asn Pro Asn Ile Thr Gln Tyr Gly Asn Leu Ser
           115                 120                 125
   Asp Gly Lys Asn Tyr Ser Gly Ile Gly Tyr Phe Leu Gly Lys Val Glu
       130                 135                 140
   Gln Asn Thr Val Phe Ile Thr Ala Leu Ala Val Lys Pro
   145                 150                 155
<210> 195
   <211> 441
   <212> DNA
   <213> Phakopsora pachyrhizi
<220>
   <221> misc_feature
   <222> (1)..(441)
   <223> ta05216.01_phapa
<400> 195
   atgaatttca ttgcccttaa atcttttgct cttgcgttgg ttctatttct attcaattca 60
   catcaggcta aatgcttccc aaatccacgc tccccccact caagctctga gagaaagagc 120
   gtcagcctta gaggatttcc tgaggaagaa tcggtttccg cctctaagag cggagtcttg 180
   agaccaagca tcatccttaa aagtttaaat atggctcaga aaattagaac caggccaatt 240
   aaagctcgag agttcccaag taccccgagg gtgcctgagg gagacacgga ggacgattcc 300
   aaagctctat cgtctcacaa agtggcatat tcacccaaga ttgttttaaa gcaattcacc 360
   aattcgattc cttacttagc agagccagtt gtagaatcag acccacgagt caaaccaaga 420
   aaagctttac cacgcgaata a 441
<210> 196
   <211> 146
   <212> PRT
   <213> Phakopsora pachyrhizi
<220>
   <221> VARIANT
   <222> (1)..(146)
   <223> ta05216.01_phapa
<400> 196
   Met Asn Phe Ile Ala Leu Lys Ser Phe Ala Leu Ala Leu Val Leu Phe
   1               5                   10                  15
   Leu Phe Asn Ser His Gln Ala Lys Cys Phe Pro Asn Pro Arg Ser Pro
               20                  25                  30
   His Ser Ser Ser Glu Arg Lys Ser Val Ser Leu Arg Gly Phe Pro Glu
           35                  40                  45
   Glu Glu Ser Val Ser Ala Ser Lys Ser Gly Val Leu Arg Pro Ser Ile
       50                  55                  60
   Ile Leu Lys Ser Leu Asn Met Ala Gln Lys Ile Arg Thr Arg Pro Ile
   65                  70                  75                  80
   Lys Ala Arg Glu Phe Pro Ser Thr Pro Arg Val Pro Glu Gly Asp Thr
                   85                  90                  95
   Glu Asp Asp Ser Lys Ala Leu Ser Ser His Lys Val Ala Tyr Ser Pro
               100                 105                 110
   Lys Ile Val Leu Lys Gln Phe Thr Asn Ser Ile Pro Tyr Leu Ala Glu
           115                 120                 125
   Pro Val Val Glu Ser Asp Pro Arg Val Lys Pro Arg Lys Ala Leu Pro
       130                 135                 140
   Arg Glu
   145
<210> 197
   <211> 405
   <212> DNA
   <213> Phakopsora pachyrhizi
<220>
   <221> misc_feature
   <222> (1)..(405)
   <223> ta00624.01_phapa
<400> 197
   atgatgttca cagttcttaa ggtagtacca tcgatgttcc tgatcctaca aactcttctg      60
   gccttactag caacgagtct gaatatcggc atggcaaagg atataccacc ggcctcagtc     120
   tgttatagtt attcgaattc ggataacgat cgtgccactt gtcacggtgc cacctgcccc     180
   gatggttgtt cctggccctt tataactgcc caaaactgtg ttccaactgg accaacttca     240
   tctaaatcta ccacccagac ctgtcatatt gggtattgga aagatggaac tgacggcata     300
   tgcactaata ggttaagaga aatgtatcgc tgcagtggta aaactactgg agtggctttt     360
   tgcacaggct gtaagaatta tactagcaag aaacgaatta attga                     405
<210> 198
   <211> 134
   <212> PRT
   <213> Phakopsora pachyrhizi
<220>
   <221> VARIANT
   <222> (1)..(134)
   <223> ta00624.01_phapa
<400> 198
   Met Met Phe Thr Val Leu Lys Val Val Pro Ser Met Phe Leu Ile Leu
   1               5                   10                  15
   Gln Thr Leu Leu Ala Leu Leu Ala Thr Ser Leu Asn Ile Gly Met Ala
               20                  25                  30
   Lys Asp Ile Pro Pro Ala Ser Val Cys Tyr Ser Tyr Ser Asn Ser Asp
           35                  40                  45
   Asn Asp Arg Ala Thr Cys His Gly Ala Thr Cys Pro Asp Gly Cys Ser
       50                  55                  60
   Trp Pro Phe Ile Thr Ala Gln Asn Cys Val Pro Thr Gly Pro Thr Ser
   65                  70                  75                  80
   Ser Lys Ser Thr Thr Gln Thr Cys His Ile Gly Tyr Trp Lys Asp Gly
                   85                  90                  95
   Thr Asp Gly Ile Cys Thr Asn Arg Leu Arg Glu Met Tyr Arg Cys Ser
               100                 105                 110
   Gly Lys Thr Thr Gly Val Ala Phe Cys Thr Gly Cys Lys Asn Tyr Thr
           115                 120                 125
   Ser Lys Lys Arg Ile Asn
       130
<210> 199
   <211> 471
   <212> DNA
   <213> Phakopsora pachyrhizi
<220>
   <221> misc_feature
   <222> (1)..(471)
   <223> ta00870.01_phapa
<400> 199
   atgaggtact ctacttttct atcttgttct ttagccatat tttttttgct tgctgtcaat      60
   gtttctcctc aaacacctgc gccaggcggt cctcccacta gcacaggagg ccctccgact     120
   ggcactggaa gctctcctgc tggcccaggg ggtggctcga aggctccggg aggtgagggc     180
   aaaagtcccg atgccaaagg tcccgatgct aagacctctg aaggcaaagg cccccaaggc     240
   aaaagtcccg actcaaaagc cccgggagat gcaaaaagtc ccgggcttgg agactcgaag     300
   tcaggttgta cttgtcctgg cgagcagcca cctccaacca cacagaagcc accagaggat     360
   acttctcctt acccaaacgg cgctacaagc tatttgaagc cattctcaac ttcaacaaca     420
   ctatgttttg ctacaaatct gtttgcaatt cttacagtct tatatactta g              471
<210> 200
   <211> 156
   <212> PRT
   <213> Phakopsora pachyrhizi
<220>
   <221> VARIANT
   <222> (1)..(156)
   <223> ta00870.01_phapa
<400> 200
   Met Arg Tyr Ser Thr Phe Leu Ser Cys Ser Leu Ala Ile Phe Phe Leu
   1               5                   10                  15
   Leu Ala Val Asn Val Ser Pro Gln Thr Pro Ala Pro Gly Gly Pro Pro
               20                  25                  30
   Thr Ser Thr Gly Gly Pro Pro Thr Gly Thr Gly Ser Ser Pro Ala Gly
           35                  40                  45
   Pro Gly Gly Gly Ser Lys Ala Pro Gly Gly Glu Gly Lys Ser Pro Asp
       50                  55                  60
   Ala Lys Gly Pro Asp Ala Lys Thr Ser Glu Gly Lys Gly Pro Gln Gly
   65                  70                  75                  80
   Lys Ser Pro Asp Ser Lys Ala Pro Gly Asp Ala Lys Ser Pro Gly Leu
                   85                  90                  95
   Gly Asp Ser Lys Ser Gly Cys Thr Cys Pro Gly Glu Gln Pro Pro Pro
               100                 105                 110
   Thr Thr Gln Lys Pro Pro Glu Asp Thr Ser Pro Tyr Pro Asn Gly Ala
           115                 120                 125
   Thr Ser Tyr Leu Lys Pro Phe Ser Thr Ser Thr Thr Leu Cys Phe Ala
       130                 135                 140
   Thr Asn Leu Phe Ala Ile Leu Thr Val Leu Tyr Thr
   145                 150                 155
<210> 201
   <211> 519
   <212> DNA
   <213> Phakopsora pachyrhizi
<220>
   <221> misc_feature
   <222> (1)..(519)
   <223> ta08469.01_phapa
<400> 201
   atggcgaaat gttctttaag aatttggatt ttttttctag aaattttaac cctcttcctc      60
   ttaactgcag agtctgagga tcgacaagcc aattctgctg cggttaataa tgtctgcgac     120
   cgttcaatag cttgctctgt ttttgaagaa ggttggccta agaacaaatg gtttgaatgc     180
   ggatctactc gggtttcccc tgcagcattc gcgacattgc gccagtcaat ttcgattgga     240
   aatcctaaaa aaactcaagt ggacagcacg gatactactg aacatcatgt aaattgtgcc     300
   accttggatg cgagctacag cagacattct tactacaatg gcagatttac agtaaattat     360
   gccataaaag gagaatgtga ttgcaaagcg tccagatcag gtaaaagaag aaatcctatc     420
   tgttccaatg cgttatctct gagcaattct gcctgtaatt tggctcactt taagttttgc     480
   tcattgaaag aaaagaatac tctttgtgca acagtttaa                            519
<210> 202
   <211> 172
   <212> PRT
   <213> Phakopsora pachyrhizi
<220>
   <221> VARIANT
   <222> (1)..(172)
   <223> ta08469.01_phapa
<400> 202
   Met Ala Lys Cys Ser Leu Arg Ile Trp Ile Phe Phe Leu Glu Ile Leu
   1               5                   10                  15
   Thr Leu Phe Leu Leu Thr Ala Glu Ser Glu Asp Arg Gln Ala Asn Ser
               20                  25                  30
   Ala Ala Val Asn Asn Val Cys Asp Arg Ser Ile Ala Cys Ser Val Phe
           35                  40                  45
   Glu Glu Gly Trp Pro Lys Asn Lys Trp Phe Glu Cys Gly Ser Thr Arg
       50                  55                  60
   Val Ser Pro Ala Ala Phe Ala Thr Leu Arg Gln Ser Ile Ser Ile Gly
   65                  70                  75                  80
   Asn Pro Lys Lys Thr Gln Val Asp Ser Thr Asp Thr Thr Glu His His
                   85                  90                  95
   Val Asn Cys Ala Thr Leu Asp Ala Ser Tyr Ser Arg His Ser Tyr Tyr
               100                 105                 110
   Asn Gly Arg Phe Thr Val Asn Tyr Ala Ile Lys Gly Glu Cys Asp Cys
           115                 120                 125
   Lys Ala Ser Arg Ser Gly Lys Arg Arg Asn Pro Ile Cys Ser Asn Ala
       130                 135                 140
   Leu Ser Leu Ser Asn Ser Ala Cys Asn Leu Ala His Phe Lys Phe Cys
   145                 150                 155                 160
   Ser Leu Lys Glu Lys Asn Thr Leu Cys Ala Thr Val
                   165                 170
<210> 203
   <211> 381
   <212> DNA
   <213> Phakopsora pachyrhizi
<220>
   <221> misc_feature
   <222> (1)..(381)
   <223> tal0748.01_phapa
<400> 203
   atgtcgttcg caaaacaggt tcttctctca atttgcttct tcgctgtttt cagcaagata        60
   aacgctttga cggtagcaca accctcagtt cggtgcttgg gggataccaa tcctgctgcc       120
   attaaagcgg aagattgtga taatgccatg tctcagttta gtgtggattc taacggctac       180
   attcagtacg atccccaagg agaaaaaaga gattttggga gttgtagaat tcagattcaa       240
   agtacttcag ctgcatcaaa cttagcaact attaatcctg aagtcttgaa gatatatata       300
   tcaactggtt taacatcatg caacggagga acatcagcca ttgagccaca aggcctagtt       360
   gtgaatgtta ttaagctcta a                                                  381
<210> 204
   <211> 126
   <212> PRT
   <213> Phakopsora pachyrhizi
<220>
   <221> VARIANT
   <222> (1)..(126)
   <223> tal0748.01_phapa
<400> 204
   Met Ser Phe Ala Lys Gln Val Leu Leu Ser Ile Cys Phe Phe Ala Val
   1               5                   10                  15
   Phe Ser Lys Ile Asn Ala Leu Thr Val Ala Gln Pro Ser Val Arg Cys
               20                  25                  30
   Leu Gly Asp Thr Asn Pro Ala Ala Ile Lys Ala Glu Asp Cys Asp Asn
           35                  40                  45
   Ala Met Ser Gln Phe Ser Val Asp Ser Asn Gly Tyr Ile Gln Tyr Asp
       50                  55                  60
   Pro Gln Gly Glu Lys Arg Asp Phe Gly Ser Cys Arg Ile Gln Ile Gln
   65                  70                  75                  80
   Ser Thr Ser Ala Ala Ser Asn Leu Ala Thr Ile Asn Pro Glu Val Leu
                   85                  90                  95
   Lys Ile Tyr Ile Ser Thr Gly Leu Thr Ser Cys Asn Gly Gly Thr Ser
               100                 105                 110
   Ala Ile Glu Pro Gln Gly Leu Val Val Asn Val Ile Lys Leu
           115                 120                 125
<210> 205
   <211> 222
   <212> DNA
   <213> Phakopsora pachyrhizi
<220>
   <221> misc_feature
   <222> (1)..(222)
   <223> ta08283.01_phapa
<400> 205
   atgaacatca tcagctcgat ctacctcgct gttgcagcta ttttgatttc tcaaaacaat        60
   ttcaccagcg ccgcaaatct taatacccgg gacgaggttc cgaaacctgt tcatttcaga       120
   tatgaagcca gatctgttga gcggtacaat caccttgagg ctcgtagaaa gaggcggaaa       180
   ccacaaaact tcaatggaaa ctcagttaat actggtaatt ag                          222
<210> 206
   <211> 73
   <212> PRT
   <213> Phakopsora pachyrhizi
<220>
   <221> VARIANT
   <222> (1)..(73)
   <223> ta08283.01_phapa
<400> 206
   Met Asn Ile Ile Ser Ser Ile Tyr Leu Ala Val Ala Ala Ile Leu Ile
   1               5                   10                  15
   Ser Gln Asn Asn Phe Thr Ser Ala Ala Asn Leu Asn Thr Arg Asp Glu
               20                  25                  30
   Val Pro Lys Pro Val His Phe Arg Tyr Glu Ala Arg Ser Val Glu Arg
           35                  40                  45
   Tyr Asn His Leu Glu Ala Arg Arg Lys Arg Arg Lys Pro Gln Asn Phe
       50                  55                  60
   Asn Gly Asn Ser Val Asn Thr Gly Asn
   65                  70
<210> 207
   <211> 450
   <212> DNA
   <213> Phakopsora pachyrhizi
<220>
   <221> misc_feature
   <222> (1)..(450)
   <223> ta00466.01_phapa
<400> 207
   atgttttcca gagctcttcc cgttgttctt tgccttatgt tcatgatttc aaacatcagt      60
   gctcttgcaa ctcacacgag taaactagag aaaagaaaca ccccagacgg tttgtttacc     120
   gattgtagaa acgctgttat taaacaaggc tgtaacgccg ctctggacga tctttggaac     180
   aacggaagaa ttaaaaggtt tactaccgaa catcaatttg tcaaccatcc aagtggttgc     240
   aaacttactt ggtggtctga cggtggcgta actcgccccg ctgatgacca aaaaggcaca     300
   ttgcagaatg ctttacagca aatcgacgat gcttgtactt catccggtct atcatctgta     360
   tatgactcaa atagaaaagg aaatggtgtt tacgtcggaa aactgtttaa caaccaatac     420
   gtggttttga tggcacagac aggtgtatag                                      450
<210> 208
   <211> 149
   <212> PRT
   <213> Phakopsora pachyrhizi
<220>
   <221> VARIANT
   <222> (1)..(149)
   <223> ta00466.01_phapa
<400> 208
   Met Phe Ser Arg Ala Leu Pro Val Val Leu Cys Leu Met Phe Met Ile
   1               5                   10                  15
   Ser Asn Ile Ser Ala Leu Ala Thr His Thr Ser Lys Leu Glu Lys Arg
               20                  25                  30
   Asn Thr Pro Asp Gly Leu Phe Thr Asp Cys Arg Asn Ala Val Ile Lys
           35                  40                  45
   Gln Gly Cys Asn Ala Ala Leu Asp Asp Leu Trp Asn Asn Gly Arg Ile
       50                  55                  60
   Lys Arg Phe Thr Thr Glu His Gln Phe Val Asn His Pro Ser Gly Cys
   65                  70                  75                  80
   Lys Leu Thr Trp Trp Ser Asp Gly Gly Val Thr Arg Pro Ala Asp Asp
                   85                  90                  95
   Gln Lys Gly Thr Leu Gln Asn Ala Leu Gln Gln Ile Asp Asp Ala Cys
               100                 105                 110
   Thr Ser Ser Gly Leu Ser Ser Val Tyr Asp Ser Asn Arg Lys Gly Asn
           115                 120                 125
   Gly Val Tyr Val Gly Lys Leu Phe Asn Asn Gln Tyr Val Val Leu Met
       130                 135                 140
   Ala Gln Thr Gly Val
   145
<210> 209
   <211> 219
   <212> DNA
   <213> Phakopsora pachyrhizi
<220>
   <221> misc_feature
   <222> (1) .. (219)
   <223> ta10007.01_phapa
<400> 209
   atgcgcagct ttgtttcttg tgccttgctt atcgtatttt tccagctttc tataagtacg       60
   cctattattg agagatctga ggttaggctt ccacagagca atcgacagag aaacttcttc      120
   acgtcgtcgt tcccccgcca ccagctccct tacaccctca tccaccgccg ccaccaccac      180
   cacctgttgc acatcaccct ccacgttctc ttgggatga                             219
<210> 210
   <211> 72
   <212> PRT
   <213> Phakopsora pachyrhizi
<220>
   <221> VARIANT
   <222> (1)..(72)
   <223> ta10007.01_phapa
<400> 210
   Met Arg Ser Phe Val Ser Cys Ala Leu Leu Ile Val Phe Phe Gln Leu
   1               5                   10                  15
   Ser Ile Ser Thr Pro Ile Ile Glu Arg Ser Glu Val Arg Leu Pro Gln
               20                  25                  30
   Ser Asn Arg Gln Arg Asn Phe Phe Thr Ser Ser Phe Pro Arg His Gln
           35                  40                  45
   Leu Pro Tyr Thr Leu Ile His Arg Arg His His His His Leu Leu His
       50                  55                  60
   Ile Thr Leu His Val Leu Leu Gly
   65                  70
<210> 211
   <211> 510
   <212> DNA
   <213> Phakopsora pachyrhizi
<220>
   <221> misc_feature
   <222> (1)..(510)
   <223> ta07166.01_phapa
<400> 211
   atgagttacc agaggatgtt tctgatgttt caactggtga ttctggcagt gatgatgaat       60
   gatcagtcga actctgcacg agcaacgtta cttattcagg atgagccctt acatctgatc      120
   aagactcagt ttgagatcaa acctcagaag ctcatcacag tgatcaggca gccaacccag      180
   caggaagctt tggatctcag caggattggt caatcaactg gtactactac tactaccggt      240
   gttgatggat ctatcataag ggtcagaccg tcatccagag ggatgatgtt caggttcgag      300
   gatggtgttg gtggtggtgt tgagtcagac tctgggattg gtggaatgct gagtaagttt      360
   ctcagtagga tcagtagacc gagtgtatca tctggtagta gtgttgaatc taaccttgga      420
   gggtttgatc ttggttctat gttgaagagt gtaaagagct ctataagtga tgggatgact      480
   actgtcagaa gattagtcgt agtctattaa                                       510
<210> 212
   <211> 169
   <212> PRT
   <213> Phakopsora pachyrhizi
<220>
   <221> VARIANT
   <222> (1)..(169)
   <223> ta07166.01_phapa
<400> 212
   Met Ser Tyr Gln Arg Met Phe Leu Met Phe Gln Leu Val Ile Leu Ala
   1               5                   10                  15
   Val Met Met Asn Asp Gln Ser Asn Ser Ala Arg Ala Thr Leu Leu Ile
               20                  25                  30
   Gln Asp Glu Pro Leu His Leu Ile Lys Thr Gln Phe Glu Ile Lys Pro
           35                  40                  45
   Gln Lys Leu Ile Thr Val Ile Arg Gln Pro Thr Gln Gln Glu Ala Leu
       50                  55                  60
   Asp Leu Ser Arg Ile Gly Gln Ser Thr Gly Thr Thr Thr Thr Thr Gly
   65                  70                  75                  80
   Val Asp Gly Ser Ile Ile Arg Val Arg Pro Ser Ser Arg Gly Met Met
                   85                  90                  95
   Phe Arg Phe Glu Asp Gly Val Gly Gly Gly Val Glu Ser Asp Ser Gly
               100                 105                 110
   Ile Gly Gly Met Leu Ser Lys Phe Leu Ser Arg Ile Ser Arg Pro Ser
           115                 120                 125
   Val Ser Ser Gly Ser Ser Val Glu Ser Asn Leu Gly Gly Phe Asp Leu
       130                 135                 140
   Gly Ser Met Leu Lys Ser Val Lys Ser Ser Ile Ser Asp Gly Met Thr
   145                 150                 155                 160
   Thr Val Arg Arg Leu Val Val Val Tyr
                   165
<210> 213
   <211> 402
   <212> DNA
   <213> Phakopsora pachyrhizi
<220>
   <221> misc_feature
   <222> (1)..(402)
   <223> ta05900.01_phapa
<400> 213
   atgttcaaca aatcagcttt cgcagttact atcttctcac ttctctgtct cacgaatttt      60
   tacaaagtga ctgtggcatc aatttcagaa acccaggtct gttcctatta cagcgattcg     120
   tacggcaaca aatctacctg taacgagcaa cctaatgttg tttgtactgg gggatgccac     180
   ggaccctacg ttgtggcgaa taactgcatg ttgcaagatg gcactgcgag tggcacttca     240
   atcacctata acacaactca ggtatgcaca aagggctttg gacgaaacac ggcggcagcc     300
   aaggcttgtc tcaatgagat cggacctttc acttgcactg gtcccacaaa cggtgcagca     360
   gtgtgctacg gatgcaaaag caacacaatt caagatccat aa                        402
<210> 214
   <211> 133
   <212> PRT
   <213> Phakopsora pachyrhizi
<220>
   <221> VARIANT
   <222> (1)..(133)
   <223> ta05900.01_phapa
<400> 214
   Met Phe Asn Lys Ser Ala Phe Ala Val Thr Ile Phe Ser Leu Leu Cys
   1               5                   10                  15
   Leu Thr Asn Phe Tyr Lys Val Thr Val Ala Ser Ile Ser Glu Thr Gln
               20                  25                  30
   Val Cys Ser Tyr Tyr Ser Asp Ser Tyr Gly Asn Lys Ser Thr Cys Asn
           35                  40                  45
   Glu Gln Pro Asn Val Val Cys Thr Gly Gly Cys His Gly Pro Tyr Val
       50                  55                  60
   Val Ala Asn Asn Cys Met Leu Gln Asp Gly Thr Ala Ser Gly Thr Ser
   65                  70                  75                  80
   Ile Thr Tyr Asn Thr Thr Gln Val Cys Thr Lys Gly Phe Gly Arg Asn
                   85                  90                  95
   Thr Ala Ala Ala Lys Ala Cys Leu Asn Glu Ile Gly Pro Phe Thr Cys
               100                 105                 110
   Thr Gly Pro Thr Asn Gly Ala Ala Val Cys Tyr Gly Cys Lys Ser Asn
           115                 120                 125
   Thr Ile Gln Asp Pro
       130
<210> 215
   <211> 456
   <212> DNA
   <213> Phakopsora pachyrhizi
<220>
   <221> misc_feature
   <222> (1)..(456)
   <223> ta00694.01_phapa
<400> 215
   atgttttcca gagccctacc agccatcctc tgtgttcttt ttttaatttc aggaattagc      60
   gcccttgcct cccagacaag taaattgata aaaaggaata ttgcagatgg tttgtatacc     120
   acatgtcaaa acactgttgt aaaacatggc tgtaacaatc ttttggatca aatctggaat     180
   agcgggcaga tcaaaaggtt tactacaaag catcaatttg ttgttgacag tgctgcaggg     240
   tgcaaaatta cttggtgggc cgacagtggc gttgttcgcg ccaatgataa tcaaaaaagt     300
   tcattgcaac aagctttaca acaaattgat gacgcctgta ccgcgtctgg catatccacc     360
   gtttacgaca gaagcaagaa gggtgcaggt gtttatgttg gaagactgtt taacgaccag     420
   tatgtggcaa ttatggcaga gactggagta cagtag                               456
<210> 216
   <211> 151
   <212> PRT
   <213> Phakopsora pachyrhizi
<220>
   <221> VARIANT
   <222> (1)..(151)
   <223> ta00694.01_phapa
<400> 216
   Met Phe Ser Arg Ala Leu Pro Ala Ile Leu Cys Val Leu Phe Leu Ile
   1               5                   10                  15
   Ser Gly Ile Ser Ala Leu Ala Ser Gln Thr Ser Lys Leu Ile Lys Arg
               20                  25                  30
   Asn Ile Ala Asp Gly Leu Tyr Thr Thr Cys Gln Asn Thr Val Val Lys
           35                  40                  45
   His Gly Cys Asn Asn Leu Leu Asp Gln Ile Trp Asn Ser Gly Gln Ile
       50                  55                  60
   Lys Arg Phe Thr Thr Lys His Gln Phe Val Val Asp Ser Ala Ala Gly
   65                  70                  75                  80
   Cys Lys Ile Thr Trp Trp Ala Asp Ser Gly Val Val Arg Ala Asn Asp
                   85                  90                  95
   Asn Gln Lys Ser Ser Leu Gln Gln Ala Leu Gln Gln Ile Asp Asp Ala
               100                 105                 110
   Cys Thr Ala Ser Gly Ile Ser Thr Val Tyr Asp Arg Ser Lys Lys Gly
           115                 120                 125
   Ala Gly Val Tyr Val Gly Arg Leu Phe Asn Asp Gln Tyr Val Ala Ile
       130                 135                 140
   Met Ala Glu Thr Gly Val Gln
                       145                 150
<210> 217
   <211> 243
   <212> DNA
   <213> Phakopsora pachyrhizi
<220>
   <221> misc_feature
   <222> (1)..(243)
   <223> ta10860.01_phapa
<400> 217
   atgttaggtc taacaaatct tattaacttt gccttaatct ctgctgtctt cttaggctta      60
   gtatcatcaa taagcatacc gccaacggtt agtgaaagtt tagacagcgg ccttggagtc     120
   cgatcgcttc ctcggaataa cgagggcccc aatcccagct atgacaaacg ccaggttagg     180
   cctcttccgt tcatcaagtc tctccctcca acagagaacg tcgaacaaga agataccgcc     240
   tag                                                                  243
<210> 218
   <211> 80
   <212> PRT
   <213> Phakopsora pachyrhizi
<220>
   <221> VARIANT
   <222> (1)..(80)
   <223> ta10860.01_phapa
<400> 218
   Met Leu Gly Leu Thr Asn Leu Ile Asn Phe Ala Leu Ile Ser Ala Val
   1               5                   10                  15
   Phe Leu Gly Leu Val Ser Ser Ile Ser Ile Pro Pro Thr Val Ser Glu
               20                  25                  30
   Ser Leu Asp Ser Gly Leu Gly Val Arg Ser Leu Pro Arg Asn Asn Glu
           35                  40                  45
   Gly Pro Asn Pro Ser Tyr Asp Lys Arg Gln Val Arg Pro Leu Pro Phe
       50                  55                  60
   Ile Lys Ser Leu Pro Pro Thr Glu Asn Val Glu Gln Glu Asp Thr Ala
   65                  70                  75                  80
<210> 219
   <211> 480
   <212> DNA
   <213> Phakopsora pachyrhizi
<220>
   <221> misc_feature
   <222> (1)..(480)
   <223> ta09285.01_phapa
<400> 219
   atgggttata ctcgggtttt gatttttttg atcatctttc aagtctttta tcttgccttg      60
   ggagattcat tcgacaaaaa tatctatact cgtcaaaact ctctctctac ttgcgcaaga     120
   gactgctata caaatgcaac tgcaaacacc ggtgctttag gaacttgctc ccagacggat     180
   aacctttgct tatgcaggag ggatgagttt ggcaatagcg taaaggactg ttgggataaa     240
   tgtacggaca ttgaggaagc tgctgccaaa acttggttcg aaacagaatg cgctgcccat     300
   ggggttgctg tgagatttaa caatgttaca agtgccgttt cagagacagc atcttctgtg     360
   gcaaatacaa tcaacaacgc acgaaattcg cagcctaaga ctgtatcaat ttcagtggca     420
   ttagcatttt gttctcttat gttatacatt ttatttcacc tctctgtaga tctttcctag     480
<210> 220
   <211> 159
   <212> PRT
   <213> Phakopsora pachyrhizi
<220>
   <221> VARIANT
   <222> (1)..(159)
   <223> ta09285.01_phapa
<400> 220
   Met Gly Tyr Thr Arg Val Leu Ile Phe Leu Ile Ile Phe Gln Val Phe
   1               5                   10                  15
   Tyr Leu Ala Leu Gly Asp Ser Phe Asp Lys Asn Ile Tyr Thr Arg Gln
               20                  25                  30
   Asn Ser Leu Ser Thr Cys Ala Arg Asp Cys Tyr Thr Asn Ala Thr Ala
           35                  40                  45
   Asn Thr Gly Ala Leu Gly Thr Cys Ser Gln Thr Asp Asn Leu Cys Leu
       50                  55                  60
   Cys Arg Arg Asp Glu Phe Gly Asn Ser Val Lys Asp Cys Trp Asp Lys
   65                  70                  75                  80
   Cys Thr Asp Ile Glu Glu Ala Ala Ala Lys Thr Trp Phe Glu Thr Glu
                   85                  90                  95
   Cys Ala Ala His Gly Val Ala Val Arg Phe Asn Asn Val Thr Ser Ala
               100                 105                 110
   Val Ser Glu Thr Ala Ser Ser Val Ala Asn Thr Ile Asn Asn Ala Arg
           115                 120                 125
   Asn Ser Gln Pro Lys Thr Val Ser Ile Ser Val Ala Leu Ala Phe Cys
       130                 135                 140
   Ser Leu Met Leu Tyr Ile Leu Phe His Leu Ser Val Asp Leu Ser
   145                 150                 155
<210> 221
   <211> 204
   <212> DNA
   <213> Phakopsora pachyrhizi
<220>
   <221> misc_feature
   <222> (1)..(204)
   <223> ta06004.01_phapa
<400> 221
   atgggaatct tgcttttgat ttatatttat aatggcagct gttgcatttc tgacttttgg      60
   attcactcag gtttatatat catcatttta agtattatca gtagagaaag aaaggttgtt     120
   ggcgctcctg atattttagc gggcaaaaat ccagtctaca gcgggttata ccgaaggcat     180
   ggacttaatg tttcaaaggg ttaa                                             204
<210> 222
   <211> 67
   <212> PRT
   <213> Phakopsora pachyrhizi
<220>
   <221> VARIANT
   <222> (1)..(67)
   <223> ta06004.01_phapa
<400> 222
   Met Gly Ile Leu Leu Leu Ile Tyr Ile Tyr Asn Gly Ser Cys Cys Ile
   1               5                   10                  15
   Ser Asp Phe Trp Ile His Ser Gly Leu Tyr Ile Ile Ile Leu Ser Ile
               20                  25                  30
   Ile Ser Arg Glu Arg Lys Val Val Gly Ala Pro Asp Ile Leu Ala Gly
           35                  40                  45
   Lys Asn Pro Val Tyr Ser Gly Leu Tyr Arg Arg His Gly Leu Asn Val
       50                  55                  60
   Ser Lys Gly
   65
<210> 223
   <211> 315
   <212> DNA
   <213> Phakopsora pachyrhizi
<220>
   <221> misc_feature
   <222> (1)..(315)
   <223> ta00128.01_phapa
<400> 223
   atgccttggt ttggacttgt tcccaccctg aagctcttag gtcttggtgg gggaggtggt      60
   ggtggcggtt tcttgattga agtttttgca cctacagtct tgactgatgt cttactgcta     120
   gtcttggatg atgatgagct gctaagaacg ttacgtttat ctagatcttc caagaagttt     180
   gcagtttcta tgactttgga tgaacaatta gatttagaga tcggtcgggc aggtatggct     240
   ggtttcctca ctccagtctt ctttggcggt atcggtggtg gtgatgatgg ggctgagatc     300
   gttgatcttt tctga                                                      315
<210> 224
   <211> 104
   <212> PRT
   <213> Phakopsora pachyrhizi
<220>
   <221> VARIANT
   <222> (1)..(104)
   <223> ta00128.01_phapa
<400> 224
   Met Pro Trp Phe Gly Leu Val Pro Thr Leu Lys Leu Leu Gly Leu Gly
   1               5                   10                  15
   Gly Gly Gly Gly Gly Gly Gly Phe Leu Ile Glu Val Phe Ala Pro Thr
               20                  25                  30
   Val Leu Thr Asp Val Leu Leu Leu Val Leu Asp Asp Asp Glu Leu Leu
           35                  40                  45
   Arg Thr Leu Arg Leu Ser Arg Ser Ser Lys Lys Phe Ala Val Ser Met
       50                  55                  60
   Thr Leu Asp Glu Gln Leu Asp Leu Glu Ile Gly Arg Ala Gly Met Ala
   65                  70                  75                  80
   Gly Phe Leu Thr Pro Val Phe Phe Gly Gly Ile Gly Gly Gly Asp Asp
                   85                  90                  95
   Gly Ala Glu Ile Val Asp Leu Phe
               100
<210> 225
   <211> 294
   <212> DNA
   <213> Phakopsora pachyrhizi
<220>
   <221> misc_feature
   <222> (1)..(294)
   <223> ta03398.01_phapa
<400> 225
   atgaggttct cagcttcaat cccactcttc ctagcccttg ttttcggtat gcttgcgagt       60
   gtgtccttag gtctgagtca gcaggacgaa gtaaggatgc tattgtcact tccaacgtgt      120
   atgaagaatt gttatcttca gaacttgaat gctgccccat cgtgcaaggg cagagcaaat      180
   tgtttgtgta gttcccaaat ttacacttat aaagttaagg cctgtgcaac gggttgtgac      240
   ttgagcgaac aattgcaagg tctgagttat tcaagaactg tttgtaacca gtga            294
<210> 226
   <211> 97
   <212> PRT
   <213> Phakopsora pachyrhizi
<220>
   <221> VARIANT
   <222> (1)..(97)
   <223> ta03398.01_phapa
<400> 226
   Met Arg Phe Ser Ala Ser Ile Pro Leu Phe Leu Ala Leu Val Phe Gly
   1               5                   10                  15
   Met Leu Ala Ser Val Ser Leu Gly Leu Ser Gln Gln Asp Glu Val Arg
               20                  25                  30
   Met Leu Leu Ser Leu Pro Thr Cys Met Lys Asn Cys Tyr Leu Gln Asn
           35                  40                  45
   Leu Asn Ala Ala Pro Ser Cys Lys Gly Arg Ala Asn Cys Leu Cys Ser
       50                  55                  60
   Ser Gln Ile Tyr Thr Tyr Lys Val Lys Ala Cys Ala Thr Gly Cys Asp
   65                  70                  75                  80
   Leu Ser Glu Gln Leu Gln Gly Leu Ser Tyr Ser Arg Thr Val Cys Asn
                   85                  90                  95
   Gln
<210> 227
   <211> 1077
   <212> DNA
   <213> Phakopsora pachyrhizi
<220>
   <221> misc_feature
   <222> (1)..(1077)
   <223> ta00591.01_phapa
<400> 227
   atggtacttc ttcttgtagg ccagaggttt ctcttgctaa cactttacag catttctttc       60
   acttgtattc aagcaaaaga aagttctttt atatctaata aaaggaacat tggtaccaca      120
   aattttacaa atgggagaga aatttcttta aaaaactcaa ctgtaataca tgatccatgc      180
   gctcgactgc cgctcactcc ggatctttgg aaatcactta atttaaacga ttatttgctt      240
   aattatcctg atggaaataa gctcactcta gagaattatg ctgaaaaagt caatgccacc      300
   aactttgatt gcggaatagg aaaacaatgc aacgccaacc agatctgcct ccctgttcgc      360
   gctcctgatt ggtatatcct ggtggccgct cagaattgga atgctttcac caatgaaatg      420
   tatcaagcaa cagcctttgc aatggaaatt gtacttggtc tatcttcttc aattgtgaat      480
   gaggtagcat tccatgaatt ggattatctt gcgattgaag gtacacttct cggtctcttc      540
   gctgggcttt gtggtgcaat acctggattt ctatatccac ctgcgtttgg attttttggt     600
   cctaaaatat ggccgtttat tcaaggagga accggtctga ttgccggctt ggcgtggaca      660
   tatcataata tttacgcaac gggccctgcg gatgaattct ctaagactac ggatgttcaa      720
   aacttgctag ctaaagctca atcgcaagct caggccaaga tagcggaaga cgcaagaaaa      780
   gttcttcaaa acggtattag ttcggaggat ggactttacg gtgttcttaa ggatggaatc      840
   tttctcaaca accatagatc tatctcagag ttttcagaaa gggatataca ggacgctata      900
   actatggttg ctcgagcaag gttgatagct gggatatgga aagctacgaa ctgttttgtt      960
   gttcgtggaa atgcatcttg cactcaggat ggacctgatg gaacttttcc aggcaatgat     1020
   gtcttatctt attgtgacga aaatggaata atgatgagta tcgtgcagtc caaaaaa        1077
<210> 228
   <211> 359
   <212> PRT
   <213> Phakopsora pachyrhizi
<220>
   <221> VARIANT
   <222> (1)..(359)
   <223> ta00591.01_phapa
<400> 228
   Met Val Leu Leu Leu Val Gly Gln Arg Phe Leu Leu Leu Thr Leu Tyr
   1               5                   10                  15
   Ser Ile Ser Phe Thr Cys Ile Gln Ala Lys Glu Ser Ser Phe Ile Ser
               20                  25                  30
   Asn Lys Arg Asn Ile Gly Thr Thr Asn Phe Thr Asn Gly Arg Glu Ile
               35                  40                  45
   Ser Leu Lys Asn Ser Thr Val Ile His Asp Pro Cys Ala Arg Leu Pro
       50                  55                  60
   Leu Thr Pro Asp Leu Trp Lys Ser Leu Asn Leu Asn Asp Tyr Leu Leu
   65                  70                  75                  80
   Asn Tyr Pro Asp Gly Asn Lys Leu Thr Leu Glu Asn Tyr Ala Glu Lys
                   85                  90                  95
   Val Asn Ala Thr Asn Phe Asp Cys Gly Ile Gly Lys Gln Cys Asn Ala
               100                 105                 110
   Asn Gln Ile Cys Leu Pro Val Arg Ala Pro Asp Trp Tyr Ile Leu Val
           115                 120                 125
   Ala Ala Gln Asn Trp Asn Ala Phe Thr Asn Glu Met Tyr Gln Ala Thr
       130                 135                 140
   Ala Phe Ala Met Glu Ile Val Leu Gly Leu Ser Ser Ser Ile Val Asn
   145                 150                 155                 160
   Glu Val Ala Phe His Glu Leu Asp Tyr Leu Ala Ile Glu Gly Thr Leu
                   165                 170                 175
   Leu Gly Leu Phe Ala Gly Leu Cys Gly Ala Ile Pro Gly Phe Leu Tyr
               180                 185                 190
   Pro Pro Ala Phe Gly Phe Phe Gly Pro Lys Ile Trp Pro Phe Ile Gln
           195                 200                 205
   Gly Gly Thr Gly Leu Ile Ala Gly Leu Ala Trp Thr Tyr His Asn Ile
       210                 215                 220
   Tyr Ala Thr Gly Pro Ala Asp Glu Phe Ser Lys Thr Thr Asp Val Gln
   225                 230                 235                 240
   Asn Leu Leu Ala Lys Ala Gln Ser Gln Ala Gln Ala Lys Ile Ala Glu
                   245                 250                 255
   Asp Ala Arg Lys Val Leu Gln Asn Gly Ile Ser Ser Glu Asp Gly Leu
               260                 265                 270
   Tyr Gly Val Leu Lys Asp Gly Ile Phe Leu Asn Asn His Arg Ser Ile
           275                 280                 285
   Ser Glu Phe Ser Glu Arg Asp Ile Gln Asp Ala Ile Thr Met Val Ala
       290                 295                 300
   Arg Ala Arg Leu Ile Ala Gly Ile Trp Lys Ala Thr Asn Cys Phe Val
   305                 310                 315                 320
   Val Arg Gly Asn Ala Ser Cys Thr Gln Asp Gly Pro Asp Gly Thr Phe
                   325                 330                 335
   Pro Gly Asn Asp Val Leu Ser Tyr Cys Asp Glu Asn Gly Ile Met Met
               340                 345                 350
   Ser Ile Val Gln Ser Lys Lys
           355
<210> 229
   <211> 1076
   <212> DNA
   <213> Phakopsora pachyrhizi
<220>
   <221> misc_feature
   <222> (1)..(1076)
   <223> ta11138.01
<400> 229
   atgacaattg gtcatctcaa taaaggcttc attttctatt gggttgcgtt gatatttttg       60
   ccaaagattg ttattggtag tttcacgcct aaatccaaag gtttcgaaac gatcattggt     120
   gatgaaaaca aaattaaaaa tgttcccttg aaagatctga gcaaagaatc ctctagccaa     180
   ttgaaagaat cccgagacga tgtttttggg cctggaagtt atgccatgag ctataatgct     240
   aagtctcaag ctacaaaaca tgccccagga gatgatgtca gtaaatcaga ggctggagtg     300
   tcatacaata ttgcaagaga tgtacaagat tccaagcgaa gcaagtcaga aggaaggtat     360
   gacgaattgc atatcaagct tcgaaattca agcagaaaat ttcaaaaccc aaaagataaa     420
   gaagtttatc gtagccttca gagcttgctt ttgcgcttaa aagattatct atccaaaaac     480
   gttaatcaac taaatcccac tgataaagct agtttaatac ctcataagga atttgaagat     540
   ttgatcttgg gagactttga cgaacttttt gggcgcatgt ttcaaaattt ttatcttaat     600
   ttttatttta ataaatcttt acttcttgcg tttgatgtca tcgattggct cgctttgaat     660
   gagtcagaca aaggaatcct tttaaataag ctattgataa gtgaaaaggt gattgaggga     720
   ctagaccttt acatctcctc aaaaattatt cttgaaggag gttatatcga aatttttaag     780
   gcggatgata ttcaacgcta tctgttaaaa catcaaaact tggaggaaat aagaaactta     840
   ttaaattgtc tggaaaaaaa tcactggaaa agggttgaac ttggattcat tagggctcat     900
   ctgactccat catttagtat aaatgtaaaa tattcaatca cagctacgaa cactgtgatt     960
   ggctttgagg aaattcttaa ttcaatagaa aaagcccaca gctttaactc tggatggctc    1020
   aaagttctaa gcatggataa agaaacagaa aatgctcttt tattttttgt agaaaa       1076
<210> 230
   <211> 358
   <212> PRT
   <213> Phakopsora pachyrhizi
<220>
   <221> VARIANT
   <222> (1)..(358)
   <223> ta11138.01_phapa
<400> 230
   Met Thr Ile Gly His Leu Asn Lys Gly Phe Ile Phe Tyr Trp Val Ala
   1               5                   10                  15
   Leu Ile Phe Leu Pro Lys Ile Val Ile Gly Ser Phe Thr Pro Lys Ser
               20                  25                  30
   Lys Gly Phe Glu Thr Ile Ile Gly Asp Glu Asn Lys Ile Lys Asn Val
           35                  40                  45
   Pro Leu Lys Asp Leu Ser Lys Glu Ser Ser Ser Gln Leu Lys Glu Ser
       50                  55                  60
   Arg Asp Asp Val Phe Gly Pro Gly Ser Tyr Ala Met Ser Tyr Asn Ala
   65                  70                  75                  80
   Lys Ser Gln Ala Thr Lys His Ala Pro Gly Asp Asp Val Ser Lys Ser
                   85                  90                  95
   Glu Ala Gly Val Ser Tyr Asn Ile Ala Arg Asp Val Gln Asp Ser Lys
               100                 105                 110
   Arg Ser Lys Ser Glu Gly Arg Tyr Asp Glu Leu His Ile Lys Leu Arg
           115                 120                 125
   Asn Ser Ser Arg Lys Phe Gln Asn Pro Lys Asp Lys Glu Val Tyr Arg
       130                 135                 140
   Ser Leu Gln Ser Leu Leu Leu Arg Leu Lys Asp Tyr Leu Ser Lys Asn
   145                 150                 155                 160
   Val Asn Gln Leu Asn Pro Thr Asp Lys Ala Ser Leu Ile Pro His Lys
                   165                 170                 175
   Glu Phe Glu Asp Leu Ile Leu Gly Asp Phe Asp Glu Leu Phe Gly Arg
               180                 185                 190
   Met Phe Gln Asn Phe Tyr Leu Asn Phe Tyr Phe Asn Lys Ser Leu Leu
           195                 200                 205
   Leu Ala Phe Asp Val Ile Asp Trp Leu Ala Leu Asn Glu Ser Asp Lys
       210                 215                 220
   Gly Ile Leu Leu Asn Lys Leu Leu Ile Ser Glu Lys Val Ile Glu Gly
   225                 230                 235                 240
   Leu Asp Leu Tyr Ile Ser Ser Lys Ile Ile Leu Glu Gly Gly Tyr Ile
                   245                 250                 255
   Glu Ile Phe Lys Ala Asp Asp Ile Gln Arg Tyr Leu Leu Lys His Gln
               260                 265                 270
   Asn Leu Glu Glu Ile Arg Asn Leu Leu Asn Cys Leu Glu Lys Asn His
           275                 280                 285
   Trp Lys Arg Val Glu Leu Gly Phe Ile Arg Ala His Leu Thr Pro Ser
       290                 295                 300
   Phe Ser Ile Asn Val Lys Tyr Ser Ile Thr Ala Thr Asn Thr Val Ile
   305                 310                 315                 320
   Gly Phe Glu Glu Ile Leu Asn Ser Ile Glu Lys Ala His Ser Phe Asn
                   325                 330                 335
   Ser Gly Trp Leu Lys Val Leu Ser Met Asp Lys Glu Thr Glu Asn Ala
               340                 345                 350
   Leu Leu Phe Phe Val Glu
                           355
<210> 231
   <211> 1056
   <212> DNA
   <213> Phakopsora pachyrhizi
<220>
   <221> misc_feature
   <222> (1)..(1056)
   <223> ta07651.03_phapa
<400> 231
   atgatgataa gttttaagta tagaattaaa tcatcagtcg tgattctctt tacgatcctc      60
   ctatcctgct tgctcataga gataatcagt tctactcagt cgccctcgac aatacaagca     120
   agaagcgctg tgaagaagat tgaaaaggat catcaacaga ggcggttgaa gataaggaag     180
   agacaagtga cagatgcacc taatacaccc aacctggatc ttaacatcac tgatgccata     240
   gccattcagg ctgatactat tccttggtac atgctggatc agtatcaaca ggaccatatt     300
   agatttcatt gttacctatc aatggcttct tatggagatt acaagacatt atgccccagc     360
   acgtttgcag tcgagggaaa agattttgag gtgattcagg agttccggac cgatctaggt     420
   caaggggtgt ttgtagccag ggttccgttg atggacaaaa tcgtgatagt cttccaagga     480
   tattccacag aaattcctct atcctgggaa cccgttcaga tcgacttcgg gcgacaaatc     540
   gcaaattgta cggccaattg tactgcgggt agcggaatac tcgatctata caattcggct     600
   aggatcgcta gtaacgattg ggagctggcc aaaaaggcag tcaatacgac tggtcacaag     660
   tttagtgtca ctggtcatgg tattggggga gctgttgcta ctctggctgc actagatctg     720
   ggatctagcg gatttgttca ttacgctcac tttcaagcct cgccgagagc agtctcacca     780
   gcagcagcag cgatcttgca aaacatcttc caaggtgaat ctgcccagca ggttacggcc     840
   aataacgatt tctttgtaca tgcgataccc cgttcaagct tttaccagag ggtaggtact     900
   gccgtttgga tctttggtaa caagactgag tggatgagaa actgtaacta ttatcctgag     960
   aatctcagct gcttgggaaa tggaactagc tttgctgatc acttttatta cttcacacct    1020
   atgggacaat gtggatcagc tgataaagga ttttaa                              1056
<210> 232
   <211> 351
   <212> PRT
   <213> Phakopsora pachyrhizi
<220>
   <221> VARIANT
   <222> (1)..(351)
   <223> ta07651.03_phapa
<400> 232
   Met Met Ile Ser Phe Lys Tyr Arg Ile Lys Ser Ser Val Val Ile Leu
   1               5                   10                  15
   Phe Thr Ile Leu Leu Ser Cys Leu Leu Ile Glu Ile Ile Ser Ser Thr
               20                  25                  30
   Gln Ser Pro Ser Thr Ile Gln Ala Arg Ser Ala Val Lys Lys Ile Glu
           35                  40                  45
   Lys Asp His Gln Gln Arg Arg Leu Lys Ile Arg Lys Arg Gln Val Thr
       50                  55                  60
   Asp Ala Pro Asn Thr Pro Asn Leu Asp Leu Asn Ile Thr Asp Ala Ile
   65                  70                  75                  80
   Ala Ile Gln Ala Asp Thr Ile Pro Trp Tyr Met Leu Asp Gln Tyr Gln
                   85                  90                  95
   Gln Asp His Ile Arg Phe His Cys Tyr Leu Ser Met Ala Ser Tyr Gly
               100                 105                 110
   Asp Tyr Lys Thr Leu Cys Pro Ser Thr Phe Ala Val Glu Gly Lys Asp
           115                 120                 125
   Phe Glu Val Ile Gln Glu Phe Arg Thr Asp Leu Gly Gln Gly Val Phe
       130                 135                 140
   Val Ala Arg Val Pro Leu Met Asp Lys Ile Val Ile Val Phe Gln Gly
   145                 150                 155                 160
   Tyr Ser Thr Glu Ile Pro Leu Ser Trp Glu Pro Val Gln Ile Asp Phe
                   165                 170                 175
   Gly Arg Gln Ile Ala Asn Cys Thr Ala Asn Cys Thr Ala Gly Ser Gly
                   180                 185                 190
   Ile Leu Asp Leu Tyr Asn Ser Ala Arg Ile Ala Ser Asn Asp Trp Glu
               195                 200                 205
   Leu Ala Lys Lys Ala Val Asn Thr Thr Gly His Lys Phe Ser Val Thr
       210                 215                 220
   Gly His Gly Ile Gly Gly Ala Val Ala Thr Leu Ala Ala Leu Asp Leu
   225                 230                 235                 240
   Gly Ser Ser Gly Phe Val His Tyr Ala His Phe Gln Ala Ser Pro Arg
                   245                 250                 255
   Ala Val Ser Pro Ala Ala Ala Ala Ile Leu Gln Asn Ile Phe Gln Gly
               260                 265                 270
   Glu Ser Ala Gln Gln Val Thr Ala Asn Asn Asp Phe Phe Val His Ala
           275                 280                 285
   Ile Pro Arg Ser Ser Phe Tyr Gln Arg Val Gly Thr Ala Val Trp Ile
       290                 295                 300
   Phe Gly Asn Lys Thr Glu Trp Met Arg Asn Cys Asn Tyr Tyr Pro Glu
   305                 310                 315                 320
   Asn Leu Ser Cys Leu Gly Asn Gly Thr Ser Phe Ala Asp His Phe Tyr
                   325                 330                 335
   Tyr Phe Thr Pro Met Gly Gln Cys Gly Ser Ala Asp Lys Gly Phe
               340                 345                 350
<210> 233
   <211> 1056
   <212> DNA
   <213> Phakopsora pachyrhizi
<220>
   <221> misc_feature
   <222> (1)..(1056)
   <223> ta07651.02_phapa
<400> 233
   atgatgataa gttttaagta tagaattaaa tcatcagtcg tgattctctt tacgatcctc      60
   ctatcctgct tgctcataga gataatcagt tctactcagt cgccctcgac aatacaagca     120
   agaagcgctg tgaagaagat tgaaaaggat catcaacaga ggcggttgaa gataaggaag     180
   agacaagtga cagatgcacc taatacaccc aacctggatc ttaacatcac tgatgccata     240
   gccattcagg ctgatactat tccttggtac atgctggatc agtatcaaca ggaccatatt     300
   agatttcatt gttacctatc aatggcttct tatggagatt acaagacatt atgccccagc     360
   acgtttgcag tcgagggaaa agattttgag gtgattcagg agttccggac cgatctaggt     420
   caaggggtgt ttgtagccag ggttccgttg atggacaaaa tcgtgatagt cttccaagga     480
   tattccacag aaattcctct atcctgggaa cccgttcaga tcgacttcgg gcgacaaatc     540
   gcaaattgta cggccaattg tactgcgggt agcggaatac tcgatctata caattcggct     600
   aggatcgcta gtaacgattg ggagctggcc aaaaaggcag tcaatacgac tggtcacaag     660
   tttagtgtca ctggtcatgg tattggggga gctgttgcta ctctggctgc actagatctg     720
   ggatctagcg gatttgttca ttacgctcac tttcaagcct cgccgagagc agtctcacca     780
   gcagcagcag cgatcttgca aaacatcttc caaggtgaat ctgcccagca ggttacggcc     840
   aataacgatt tctttgtaca tgcgataccc cgttcaagct tttaccagag ggtaggtact     900
   gccgtttgga tctttggtaa caagactgag tggatgagaa actgtaacta ttatcctgag     960
   aatctcagct gcttgggaaa tggaactagc tttgctgatc acttttatta cttcacacct    1020
   atgggacaat gtggatcagc tgataaagga ttttaa                              1056
<210> 234
   <211> 351
   <212> PRT
   <213> Phakopsora pachyrhizi
<220>
   <221> VARIANT
   <222> (1)..(351)
   <223> ta07651.02_phapa
<400> 234
   Met Met Ile Ser Phe Lys Tyr Arg Ile Lys Ser Ser Val Val Ile Leu
   1               5                   10                  15
   Phe Thr Ile Leu Leu Ser Cys Leu Leu Ile Glu Ile Ile Ser Ser Thr
               20                  25                  30
   Gln Ser Pro Ser Thr Ile Gln Ala Arg Ser Ala Val Lys Lys Ile Glu
           35                  40                  45
   Lys Asp His Gln Gln Arg Arg Leu Lys Ile Arg Lys Arg Gln Val Thr
       50                  55                  60
   Asp Ala Pro Asn Thr Pro Asn Leu Asp Leu Asn Ile Thr Asp Ala Ile
   65                  70                  75                  80
   Ala Ile Gln Ala Asp Thr Ile Pro Trp Tyr Met Leu Asp Gln Tyr Gln
                   85                  90                  95
   Gln Asp His Ile Arg Phe His Cys Tyr Leu Ser Met Ala Ser Tyr Gly
               100                 105                 110
   Asp Tyr Lys Thr Leu Cys Pro Ser Thr Phe Ala Val Glu Gly Lys Asp
           115                 120                 125
   Phe Glu Val Ile Gln Glu Phe Arg Thr Asp Leu Gly Gln Gly Val Phe
       130                 135                 140
   Val Ala Arg Val Pro Leu Met Asp Lys Ile Val Ile Val Phe Gln Gly
   145                 150                 155                 160
   Tyr Ser Thr Glu Ile Pro Leu Ser Trp Glu Pro Val Gln Ile Asp Phe
                   165                 170                 175
   Gly Arg Gln Ile Ala Asn Cys Thr Ala Asn Cys Thr Ala Gly Ser Gly
               180                 185                 190
   Ile Leu Asp Leu Tyr Asn Ser Ala Arg Ile Ala Ser Asn Asp Trp Glu
           195                 200                 205
   Leu Ala Lys Lys Ala Val Asn Thr Thr Gly His Lys Phe Ser Val Thr
       210                 215                 220
   Gly His Gly Ile Gly Gly Ala Val Ala Thr Leu Ala Ala Leu Asp Leu
   225                 230                 235                 240
   Gly Ser Ser Gly Phe Val His Tyr Ala His Phe Gln Ala Ser Pro Arg
                   245                 250                 255
   Ala Val Ser Pro Ala Ala Ala Ala Ile Leu Gln Asn Ile Phe Gln Gly
               260                 265                 270
   Glu Ser Ala Gln Gln Val Thr Ala Asn Asn Asp Phe Phe Val His Ala
           275                 280                 285
   Ile Pro Arg Ser Ser Phe Tyr Gln Arg Val Gly Thr Ala Val Trp Ile
       290                 295                 300
   Phe Gly Asn Lys Thr Glu Trp Met Arg Asn Cys Asn Tyr Tyr Pro Glu
   305                 310                 315                 320
   Asn Leu Ser Cys Leu Gly Asn Gly Thr Ser Phe Ala Asp His Phe Tyr
                   325                 330                 335
   Tyr Phe Thr Pro Met Gly Gln Cys Gly Ser Ala Asp Lys Gly Phe
               340                 345                 350
<210> 235
   <211> 1056
   <212> DNA
   <213> Phakopsora pachyrhizi
<220>
   <221> misc_feature
   <222> (1)..(1056)
   <223> ta07651.01_phapa
<400> 235
   atgatgataa gttttaagta tagaattaaa tcatcagtcg tgattctctt tacgatcctc      60
   ctatcctgct tgctcataga gataatcagt tctactcagt cgccctcgac aatacaagca     120
   agaagcgctg tgaagaagat tgaaaaggat catcaacaga ggcggttgaa gataaggaag     180
   agacaagtga cagatgcacc taatacaccc aacctggatc ttaacatcac tgatgccata     240
   gccattcagg ctgatactat tccttggtac atgctggatc agtatcaaca ggaccatatt     300
   agatttcatt gttacctatc aatggcttct tatggagatt acaagacatt atgccccagc     360
   acgtttgcag tcgagggaaa agattttgag gtgattcagg agttccggac cgatctaggt     420
   caaggggtgt ttgtagccag ggttccgttg atggacaaaa tcgtgatagt cttccaagga     480
   tattccacag aaattcctct atcctgggaa cccgttcaga tcgacttcgg gcgacaaatc     540
   gcaaattgta cggccaattg tactgcgggt agcggaatac tcgatctata caattcggct     600
   aggatcgcta gtaacgattg ggagctggcc aaaaaggcag tcaatacgac tggtcacaag     660
   tttagtgtca ctggtcatgg tattggggga gctgttgcta ctctggctgc actagatctg     720
   ggatctagcg gatttgttca ttacgctcac tttcaagcct cgccgagagc agtctcacca     780
   gcagcagcag cgatcttgca aaacatcttc caaggtgaat ctgcccagca ggttacggcc     840
   aataacgatt tctttgtaca tgcgataccc cgttcaagct tttaccagag ggtaggtact     900
   gccgtttgga tctttggtaa caagactgag tggatgagaa actgtaacta ttatcctgag     960
   aatctcagct gcttgggaaa tggaactagc tttgctgatc acttttatta cttcacacct    1020
   atgggacaat gtggatcagc tgataaagga ttttaa                              1056
<210> 236
   <211> 351
   <212> PRT
   <213> Phakopsora pachyrhizi
<220>
   <221> VARIANT
   <222> (1)..(351)
   <223> ta07651.01_phapa
<400> 236
   Met Met Ile Ser Phe Lys Tyr Arg Ile Lys Ser Ser Val Val Ile Leu
   1               5                   10                  15
   Phe Thr Ile Leu Leu Ser Cys Leu Leu Ile Glu Ile Ile Ser Ser Thr
               20                  25                  30
   Gln Ser Pro Ser Thr Ile Gln Ala Arg Ser Ala Val Lys Lys Ile Glu
           35                  40                  45
   Lys Asp His Gln Gln Arg Arg Leu Lys Ile Arg Lys Arg Gln Val Thr
       50                  55                  60
   Asp Ala Pro Asn Thr Pro Asn Leu Asp Leu Asn Ile Thr Asp Ala Ile
   65                  70                  75                  80
   Ala Ile Gln Ala Asp Thr Ile Pro Trp Tyr Met Leu Asp Gln Tyr Gln
                   85                  90                  95
   Gln Asp His Ile Arg Phe His Cys Tyr Leu Ser Met Ala Ser Tyr Gly
               100                 105                 110
   Asp Tyr Lys Thr Leu Cys Pro Ser Thr Phe Ala Val Glu Gly Lys Asp
           115                 120                 125
   Phe Glu Val Ile Gln Glu Phe Arg Thr Asp Leu Gly Gln Gly Val Phe
       130                 135                 140
   Val Ala Arg Val Pro Leu Met Asp Lys Ile Val Ile Val Phe Gln Gly
   145                 150                 155                 160
   Tyr Ser Thr Glu Ile Pro Leu Ser Trp Glu Pro Val Gln Ile Asp Phe
                   165                 170                 175
   Gly Arg Gln Ile Ala Asn Cys Thr Ala Asn Cys Thr Ala Gly Ser Gly
               180                 185                 190
   Ile Leu Asp Leu Tyr Asn Ser Ala Arg Ile Ala Ser Asn Asp Trp Glu
           195                 200                 205
   Leu Ala Lys Lys Ala Val Asn Thr Thr Gly His Lys Phe Ser Val Thr
       210                 215                 220
   Gly His Gly Ile Gly Gly Ala Val Ala Thr Leu Ala Ala Leu Asp Leu
   225                 230                 235                 240
   Gly Ser Ser Gly Phe Val His Tyr Ala His Phe Gln Ala Ser Pro Arg
                   245                 250                 255
   Ala Val Ser Pro Ala Ala Ala Ala Ile Leu Gln Asn Ile Phe Gln Gly
               260                 265                 270
   Glu Ser Ala Gln Gln Val Thr Ala Asn Asn Asp Phe Phe Val His Ala
           275                 280                 285
   Ile Pro Arg Ser Ser Phe Tyr Gln Arg Val Gly Thr Ala Val Trp Ile
       290                 295                 300
   Phe Gly Asn Lys Thr Glu Trp Met Arg Asn Cys Asn Tyr Tyr Pro Glu
   305                 310                 315                 320
   Asn Leu Ser Cys Leu Gly Asn Gly Thr Ser Phe Ala Asp His Phe Tyr
                   325                 330                 335
   Tyr Phe Thr Pro Met Gly Gln Cys Gly Ser Ala Asp Lys Gly Phe
               340                 345                 350
<210> 237
   <211> 1029
   <212> DNA
   <213> Phakopsora pachyrhizi
<220>
   <221> misc_feature
   <222> (1)..(1029)
   <223> ta00414.01_phapa
<400> 237
   atgatagcca tcaacattct attgatatat ctgctcgcta taggaagatt catcagggga       60
   gatgaaggac aacaagctct agccagtgat caagaaccct acaaactctc aatattccat     120
   agactttcat caaaattatc aaccggcgta gatcccaaca atcacagcaa cttctcgtcc     180
   tccttctcaa atggccagac ctggaggaaa cgtggagacc tgaagataag gagagccaac     240
   agtgattcca aaagtctgac aatctcatct tttaaaaggc ctaccatgga acacctgaag     300
   atcgatgtga agagttggat ctcatcaaaa cacttaccag actatcagct cttgatttta     360
   cctcatcaag aatctccttc gttgccattc gactatgatt ctcatggatc tatctcattc     420
   tcattgtgta attttctctc tgacagggat cagcgattca aagagtattt aaacctatgg     480
   gttgaagagg aggagattaa gagtggggcg gaaggatcga ataggatgga attaagaatt     540
   ataggattga attggggtgt agaggaacgg tcaaggtcaa tctttgaatc tcaatcgatc     600
   gataggagct gtaagataga tgatgaaatt tttaacgaca acaagattgg tgcgaagttt     660
   gagaacgagg aggaagagaa gaggctgaaa agcgtcgaag gtcgtttgaa tgaggttttt     720
   aaaagtgtag atagttggaa tacgaaagtt atgatcagaa gacctgaaac tttaccagaa     780
   ccgtttctca ggccatttca aaaccaaggt ggagcagtag gaaatagacc gttagagtac     840
   ttaccagatg gaaccattaa aaagacaaag caggagaggt tgagagagga agaggctaac     900
   aagagttggt ttgccaagta ttggatgtat attttaccct ttgccatcct ggtcatgttt     960
   ggtggaggtg gtaacagttt tgaagaagcc aaaactgaat caaagtcaac cccttcatcc    1020
   tcttcataa                                                            1029
<210> 238
   <211> 342
   <212> PRT
   <213> Phakopsora pachyrhizi
<220>
   <221> VARIANT
   <222> (1)..(342)
   <223> ta00414.01_phapa
<400> 238
   Met Ile Ala Ile Asn Ile Leu Leu Ile Tyr Leu Leu Ala Ile Gly Arg
   1               5                   10                  15
   Phe Ile Arg Gly Asp Glu Gly Gln Gln Ala Leu Ala Ser Asp Gln Glu
               20                  25                  30
   Pro Tyr Lys Leu Ser Ile Phe His Arg Leu Ser Ser Lys Leu Ser Thr
           35                  40                  45
   Gly Val Asp Pro Asn Asn His Ser Asn Phe Ser Ser Ser Phe Ser Asn
       50                  55                  60
   Gly Gln Thr Trp Arg Lys Arg Gly Asp Leu Lys Ile Arg Arg Ala Asn
   65                  70                  75                  80
   Ser Asp Ser Lys Ser Leu Thr Ile Ser Ser Phe Lys Arg Pro Thr Met
                   85                  90                  95
   Glu His Leu Lys Ile Asp Val Lys Ser Trp Ile Ser Ser Lys His Leu
               100                 105                 110
   Pro Asp Tyr Gln Leu Leu Ile Leu Pro His Gln Glu Ser Pro Ser Leu
           115                 120                 125
   Pro Phe Asp Tyr Asp Ser His Gly Ser Ile Ser Phe Ser Leu Cys Asn
       130                 135                 140
   Phe Leu Ser Asp Arg Asp Gln Arg Phe Lys Glu Tyr Leu Asn Leu Trp
   145                 150                 155                 160
   Val Glu Glu Glu Glu Ile Lys Ser Gly Ala Glu Gly Ser Asn Arg Met
                   165                 170                 175
   Glu Leu Arg Ile Ile Gly Leu Asn Trp Gly Val Glu Glu Arg Ser Arg
               180                 185                 190
   Ser Ile Phe Glu Ser Gln Ser Ile Asp Arg Ser Cys Lys Ile Asp Asp
           195                 200                 205
   Glu Ile Phe Asn Asp Asn Lys Ile Gly Ala Lys Phe Glu Asn Glu Glu
       210                 215                 220
   Glu Glu Lys Arg Leu Lys Ser Val Glu Gly Arg Leu Asn Glu Val Phe
   225                 230                 235                 240
   Lys Ser Val Asp Ser Trp Asn Thr Lys Val Met Ile Arg Arg Pro Glu
                   245                 250                 255
   Thr Leu Pro Glu Pro Phe Leu Arg Pro Phe Gln Asn Gln Gly Gly Ala
               260                 265                 270
   Val Gly Asn Arg Pro Leu Glu Tyr Leu Pro Asp Gly Thr Ile Lys Lys
           275                 280                 285
   Thr Lys Gln Glu Arg Leu Arg Glu Glu Glu Ala Asn Lys Ser Trp Phe
       290                 295                 300
   Ala Lys Tyr Trp Met Tyr Ile Leu Pro Phe Ala Ile Leu Val Met Phe
   305                 310                 315                 320
   Gly Gly Gly Gly Asn Ser Phe Glu Glu Ala Lys Thr Glu Ser Lys Ser
                   325                 330                 335
   Thr Pro Ser Ser Ser Ser
               340
<210> 239
   <211> 1011
   <212> DNA
   <213> Phakopsora pachyrhizi
<220>
   <221> misc_feature
   <222> (1)..(1011)
   <223> ta10160.01_phapa
<400> 239
   atgaagagtc tcccgaggct ccttcagcct tactactgcc taaggctatt gagctgtttg      60
   cctgccctgc tatacctctt caaactatca gagtctataa atcacttgag cgatagctca     120
   gcctcttcaa acaaccgatc catccgtgga cagcaacaga ttcatctttc catgtcgttt     180
   ctatctgatc agctgattgc aatcatgatc cccttggtcg gaataccgct attggtatct     240
   ttgaggttga ggcagatcag aaagaccttt aaggttttgg acttaagcac tgaaacgttg     300
   cttgaaagct ggtttgcaat tttgctggtg tataaccttg gtgtcttgat ctatgtcaga     360
   caatggaatt accttacagg gtacttattt tggttatcta tatcgagctg tatgttttcg     420
   aaaccaaagt atcaagggcc atcaaaactc aggacagtca ataccaagag cttttcaaat     480
   gaggttttga atcgtagctt agaatcacta gttggttttt cagctgacga tatgttgagg     540
   ataactcctg aagaactaga gagacggtca aaagcaaata agtctgcttc gtcatcaaat     600
   cccgacaaac tttggattgt atggcccctg tttgattcaa cttatcggac cagagtcgga     660
   tttatggatg ttgaagtgtt gttatctaga ctctctctta gctccagttc aaaagatttt     720
   gagatattag ttctagatgt agaaggatcg agcgagttgt gttatgaact gaagttatgg     780
   ggggaagacg attattcgaa tctaagctct aagcatcaat cggaagagaa agggatcatc     840
   ctgctggtct ttagaggcgg aaaagaattg aggagaattc cacctcgccc taccgagcac     900
   gattctgatt cttcagatgg cgattgtgaa gcgtcgaaga acttcaagag tttaggccaa     960
   aaaccttttc tctggacact tgaagcaatc agatcggcaa tccaaatcta a              1011
<210> 240
   <211> 336
   <212> PRT
   <213> Phakopsora pachyrhizi
<220>
   <221> VARIANT
   <222> (1)..(336)
   <223> >ta10160.01_phapa
<400> 240
   Met Lys Ser Leu Pro Arg Leu Leu Gln Pro Tyr Tyr Cys Leu Arg Leu
   1               5                   10                  15
   Leu Ser Cys Leu Pro Ala Leu Leu Tyr Leu Phe Lys Leu Ser Glu Ser
               20                  25                  30
   Ile Asn His Leu Ser Asp Ser Ser Ala Ser Ser Asn Asn Arg Ser Ile
           35                  40                  45
   Arg Gly Gln Gln Gln Ile His Leu Ser Met Ser Phe Leu Ser Asp Gln
       50                  55                  60
   Leu Ile Ala Ile Met Ile Pro Leu Val Gly Ile Pro Leu Leu Val Ser
   65                  70                  75                  80
   Leu Arg Leu Arg Gln Ile Arg Lys Thr Phe Lys Val Leu Asp Leu Ser
                   85                  90                  95
   Thr Glu Thr Leu Leu Glu Ser Trp Phe Ala Ile Leu Leu Val Tyr Asn
               100                 105                 110
   Leu Gly Val Leu Ile Tyr Val Arg Gln Trp Asn Tyr Leu Thr Gly Tyr
           115                 120                 125
   Leu Phe Trp Leu Ser Ile Ser Ser Cys Met Phe Ser Lys Pro Lys Tyr
       130                 135                 140
   Gln Gly Pro Ser Lys Leu Arg Thr Val Asn Thr Lys Ser Phe Ser Asn
   145                 150                 155                 160
   Glu Val Leu Asn Arg Ser Leu Glu Ser Leu Val Gly Phe Ser Ala Asp
                   165                 170                 175
   Asp Met Leu Arg Ile Thr Pro Glu Glu Leu Glu Arg Arg Ser Lys Ala
               180                 185                 190
   Asn Lys Ser Ala Ser Ser Ser Asn Pro Asp Lys Leu Trp Ile Val Trp
           195                 200                 205
   Pro Leu Phe Asp Ser Thr Tyr Arg Thr Arg Val Gly Phe Met Asp Val
       210                 215                 220
   Glu Val Leu Leu Ser Arg Leu Ser Leu Ser Ser Ser Ser Lys Asp Phe
   225                 230                 235                 240
   Glu Ile Leu Val Leu Asp Val Glu Gly Ser Ser Glu Leu Cys Tyr Glu
                   245                 250                 255
   Leu Lys Leu Trp Gly Glu Asp Asp Tyr Ser Asn Leu Ser Ser Lys His
               260                 265                 270
   Gln Ser Glu Glu Lys Gly Ile Ile Leu Leu Val Phe Arg Gly Gly Lys
           275                 280                 285
   Glu Leu Arg Arg Ile Pro Pro Arg Pro Thr Glu His Asp Ser Asp Ser
       290                 295                 300
   Ser Asp Gly Asp Cys Glu Ala Ser Lys Asn Phe Lys Ser Leu Gly Gln
   305                 310                 315                 320
   Lys Pro Phe Leu Trp Thr Leu Glu Ala Ile Arg Ser Ala Ile Gln Ile
                   325                 330                 335
<210> 241
   <211> 993
   <212> DNA
   <213> Phakopsora pachyrhizi
<220>
   <221> misc_feature
   <222> (1)..(993)
   <223> ta09254.04_phapa
<400> 241
   atgcctcacc ccctgtggat atcattcgtc ctctctgtac tgcttcaagt tatacctttg        60
   gtttgggggc actgtaagat cgttactgct gccggaaatt taaatacttc aagtcaagtc       120
   agctatggat ttggtgtgga tttacatagc aagtacccgt ggtttaagtc tcaagccggt       180
   gatgccggag cggattctga agtttttact gaatcgaaag agtttgtcgc caatcctaat       240
   cccccttgtg gcatgcgtgc gaaaatgggt gccctagatt tcgactcatc ttttagtcaa      300
   gccgaagcaa tgggcgtagg aaatacttta gaagatgggt cttttgaggc attgattttt       360
   caggtcaacc gtgatggtgg aggacaatgc acttgtgaat acaacactgt aggacaacct       420
   gacaaattta aattttgcaa gacactcatt aatccaccgg gacaaaatgg catttggcct       480
   caagatcgtg taaatcatac ggccaaattt caactaccca gagatactac ttgccgtggt       540
   ggaatgttca aagacaagtg cttgattaga atcagatgtg gagagtttct aagatttgga       600
   ggatgtttgg caataaagac gcctgccagc ccacataaac ttcaattgaa ggttgtaatt       660
   ggaggtaaaa catcaatgaa aaccaaaccg gacttaccaa gaaatgatgt ttccagcatc       720
   gctcaaaagg ttttcaacac actgaaagcc aaaggggcct tcgtgccgat ttcttccggc       780
   agatataaaa gaagtcctgc aatgcatatt catgccttgc agcatgtaag aacccttggc       840
   gttacccctt taaaatcaaa ctttgactct atggcagatg aaatttcttt caaggtgatt       900
   gaattgatgc gagccaacaa ggttttcctg gtagcgaaga aggccaaatc aatattgaag       960
   gattataggg actctacatc tgcagaaaga taa                                   993
<210> 242
   <211> 330
   <212> PRT
   <213> Phakopsora pachyrhizi
<220>
   <221> VARIANT
   <222> (1)..(330)
   <223> ta09254.04_phapa
<400> 242
   Met Pro His Pro Leu Trp Ile Ser Phe Val Leu Ser Val Leu Leu Gln
   1               5                   10                  15
   Val Ile Pro Leu Val Trp Gly His Cys Lys Ile Val Thr Ala Ala Gly
               20                  25                  30
   Asn Leu Asn Thr Ser Ser Gln Val Ser Tyr Gly Phe Gly Val Asp Leu
           35                  40                  45
   His Ser Lys Tyr Pro Trp Phe Lys Ser Gln Ala Gly Asp Ala Gly Ala
       50                  55                  60
   Asp Ser Glu Val Phe Thr Glu Ser Lys Glu Phe Val Ala Asn Pro Asn
   65                  70                  75                  80
   Pro Pro Cys Gly Met Arg Ala Lys Met Gly Ala Leu Asp Phe Asp Ser
                   85                  90                  95
   Ser Phe Ser Gln Ala Glu Ala Met Gly Val Gly Asn Thr Leu Glu Asp
               100                 105                 110
   Gly Ser Phe Glu Ala Leu Ile Phe Gln Val Asn Arg Asp Gly Gly Gly
           115                 120                 125
   Gln Cys Thr Cys Glu Tyr Asn Thr Val Gly Gln Pro Asp Lys Phe Lys
       130                 135                 140
   Phe Cys Lys Thr Leu Ile Asn Pro Pro Gly Gln Asn Gly Ile Trp Pro
   145                 150                 155                 160
   Gln Asp Arg Val Asn His Thr Ala Lys Phe Gln Leu Pro Arg Asp Thr
                   165                 170                 175
   Thr Cys Arg Gly Gly Met Phe Lys Asp Lys Cys Leu Ile Arg Ile Arg
               180                 185                 190
   Cys Gly Glu Phe Leu Arg Phe Gly Gly Cys Leu Ala Ile Lys Thr Pro
           195                 200                 205
   Ala Ser Pro His Lys Leu Gln Leu Lys Val Val Ile Gly Gly Lys Thr
       210                 215                 220
   Ser Met Lys Thr Lys Pro Asp Leu Pro Arg Asn Asp Val Ser Ser Ile
   225                 230                 235                 240
   Ala Gln Lys Val Phe Asn Thr Leu Lys Ala Lys Gly Ala Phe Val Pro
                   245                 250                 255
   Ile Ser Ser Gly Arg Tyr Lys Arg Ser Pro Ala Met His Ile His Ala
               260                 265                 270
   Leu Gln His Val Arg Thr Leu Gly Val Thr Pro Leu Lys Ser Asn Phe
           275                 280                 285
   Asp Ser Met Ala Asp Glu Ile Ser Phe Lys Val Ile Glu Leu Met Arg
       290                 295                 300
   Ala Asn Lys Val Phe Leu Val Ala Lys Lys Ala Lys Ser Ile Leu Lys
   305                 310                 315                 320
   Asp Tyr Arg Asp Ser Thr Ser Ala Glu Arg
                   325                 330
<210> 243
   <211> 993
   <212> DNA
   <213> Phakopsora pachyrhizi
<220>
   <221> misc_feature
   <222> (1)..(993)
   <223> ta09254.03_phapa
<400> 243
   atgcctcacc ccctgtggat atcattcgtc ctctctgtac tgcttcaagt tatacctttg       60
   gtttgggggc actgtaagat cgttactgct gccggaaatt taaatacttc aagtcaagtc      120
   agctatggat ttggtgtgga tttacatagc aagtacccgt ggtttaagtc tcaagccggt      180
   gatgccggag cggattctga agtttttact gaatcgaaag agtttgtcgc caatcctaat      240
   cccccttgtg gcatgcgtgc gaaaatgggt gccctagatt tcgactcatc ttttagtcaa     300
   gccgaagcaa tgggcgtagg aaatacttta gaagatgggt cttttgaggc attgattttt      360
   caggtcaacc gtgatggtgg aggacaatgc acttgtgaat acaacactgt aggacaacct      420
   gacaaattta aattttgcaa gacactcatt aatccaccgg gacaaaatgg catttggcct      480
   caagatcgtg taaatcatac ggccaaattt caactaccca gagatactac ttgccgtggt      540
   ggaatgttca aagacaagtg cttgattaga atcagatgtg gagagtttct aagatttgga      600
   ggatgtttgg caataaagac gcctgccagc ccacataaac ttcaattgaa ggttgtaatt      660
   ggaggtaaaa catcaatgaa aaccaaaccg gacttaccaa gaaatgatgt ttccagcatc      720
   gctcaaaagg ttttcaacac actgaaagcc aaaggggcct tcgtgccgat ttcttccggc      780
   agatataaaa gaagtcctgc aatgcatatt catgccttgc agcatgtaag aacccttggc      840
   gttacccctt taaaatcaaa ctttgactct atggcagatg aaatttcttt caaggtgatt      900
   gaattgatgc gagccaacaa ggttttcctg gtagcgaaga aggccaaatc aatattgaag      960
   gattataggg actctacatc tgcagaaaga taa                                   993
<210> 244
   <211> 330
   <212> PRT
   <213> Phakopsora pachyrhizi
<220>
   <221> VARIANT
   <222> (1)..(330)
   <223> ta09254.03_phapa
<400> 244
   Met Pro His Pro Leu Trp Ile Ser Phe Val Leu Ser Val Leu Leu Gln
   1               5                   10                  15
   Val Ile Pro Leu Val Trp Gly His Cys Lys Ile Val Thr Ala Ala Gly
               20                  25                  30
   Asn Leu Asn Thr Ser Ser Gln Val Ser Tyr Gly Phe Gly Val Asp Leu
           35                  40                  45
   His Ser Lys Tyr Pro Trp Phe Lys Ser Gln Ala Gly Asp Ala Gly Ala
       50                  55                  60
   Asp Ser Glu Val Phe Thr Glu Ser Lys Glu Phe Val Ala Asn Pro Asn
   65                  70                  75                  80
   Pro Pro Cys Gly Met Arg Ala Lys Met Gly Ala Leu Asp Phe Asp Ser
                   85                  90                  95
   Ser Phe Ser Gln Ala Glu Ala Met Gly Val Gly Asn Thr Leu Glu Asp
               100                 105                 110
   Gly Ser Phe Glu Ala Leu Ile Phe Gln Val Asn Arg Asp Gly Gly Gly
           115                 120                 125
   Gln Cys Thr Cys Glu Tyr Asn Thr Val Gly Gln Pro Asp Lys Phe Lys
       130                 135                 140
   Phe Cys Lys Thr Leu Ile Asn Pro Pro Gly Gln Asn Gly Ile Trp Pro
   145                 150                 155                 160
   Gln Asp Arg Val Asn His Thr Ala Lys Phe Gln Leu Pro Arg Asp Thr
                   165                 170                 175
   Thr Cys Arg Gly Gly Met Phe Lys Asp Lys Cys Leu Ile Arg Ile Arg
               180                 185                 190
   Cys Gly Glu Phe Leu Arg Phe Gly Gly Cys Leu Ala Ile Lys Thr Pro
           195                 200                 205
   Ala Ser Pro His Lys Leu Gln Leu Lys Val Val Ile Gly Gly Lys Thr
       210                 215                 220
   Ser Met Lys Thr Lys Pro Asp Leu Pro Arg Asn Asp Val Ser Ser Ile
   225                 230                 235                 240
   Ala Gln Lys Val Phe Asn Thr Leu Lys Ala Lys Gly Ala Phe Val Pro
                   245                 250                 255
   Ile Ser Ser Gly Arg Tyr Lys Arg Ser Pro Ala Met His Ile His Ala
               260                 265                 270
   Leu Gln His Val Arg Thr Leu Gly Val Thr Pro Leu Lys Ser Asn Phe
           275                 280                 285
   Asp Ser Met Ala Asp Glu Ile Ser Phe Lys Val Ile Glu Leu Met Arg
       290                 295                 300
   Ala Asn Lys Val Phe Leu Val Ala Lys Lys Ala Lys Ser Ile Leu Lys
   305                 310                 315                 320
   Asp Tyr Arg Asp Ser Thr Ser Ala Glu Arg
                   325                 330
<210> 245
   <211> 993
   <212> DNA
   <213> Phakopsora pachyrhizi
<220>
   <221> misc_feature
   <222> (1)..(993)
   <223> ta09254.05_phapa
<400> 245
   atgcctcacc ccctgtggat atcattcgtc ctctctgtac tgcttcaagt tatacctttg      60
   gtttgggggc actgtaagat cgttactgct gccggaaatt taaatacttc aagtcaagtc     120
   agctatggat ttggtgtgga tttacatagc aagtacccgt ggtttaagtc tcaagccggt     180
   gatgccggag cggattctga agtttttact gaatcgaaag agtttgtcgc caatcctaat     240
   cccccttgtg gcatgcgtgc gaaaatgggt gccctagatt tcgactcatc ttttagtcaa     300
   gccgaagcaa tgggcgtagg aaatacttta gaagatgggt cttttgaggc attgattttt     360
   caggtcaacc gtgatggtgg aggacaatgc acttgtgaat acaacactgt aggacaacct     420
   gacaaattta aattttgcaa gacactcatt aatccaccgg gacaaaatgg catttggcct     480
   caagatcgtg taaatcatac ggccaaattt caactaccca gagatactac ttgccgtggt     540
   ggaatgttca aagacaagtg cttgattaga atcagatgtg gagagtttct aagatttgga     600
   ggatgtttgg caataaagac gcctgccagc ccacataaac ttcaattgaa ggttgtaatt     660
   ggaggtaaaa catcaatgaa aaccaaaccg gacttaccaa gaaatgatgt ttccagcatc     720
   gctcaaaagg ttttcaacac actgaaagcc aaaggggcct tcgtgccgat ttcttccggc     780
   agatataaaa gaagtcctgc aatgcatatt catgccttgc agcatgtaag aacccttggc     840
   gttacccctt taaaatcaaa ctttgactct atggcagatg aaatttcttt caaggtgatt     900
   gaattgatgc gagccaacaa ggttttcctg gtagcgaaga aggccaaatc aatattgaag     960
   gattataggg actctacatc tgcagaaaga taa                                993
<210> 246
   <211> 330
   <212> PRT
   <213> Phakopsora pachyrhizi
<220>
   <221> VARIANT
   <222> (1)..(330)
   <223> ta09254.05_phapa
<400> 246
   Met Pro His Pro Leu Trp Ile Ser Phe Val Leu Ser Val Leu Leu Gln
   1               5                   10                  15
   Val Ile Pro Leu Val Trp Gly His Cys Lys Ile Val Thr Ala Ala Gly
               20                  25                  30
   Asn Leu Asn Thr Ser Ser Gln Val Ser Tyr Gly Phe Gly Val Asp Leu
           35                  40                  45
   His Ser Lys Tyr Pro Trp Phe Lys Ser Gln Ala Gly Asp Ala Gly Ala
       50                  55                  60
   Asp Ser Glu Val Phe Thr Glu Ser Lys Glu Phe Val Ala Asn Pro Asn
   65                  70                  75                  80
   Pro Pro Cys Gly Met Arg Ala Lys Met Gly Ala Leu Asp Phe Asp Ser
                   85                  90                  95
   Ser Phe Ser Gln Ala Glu Ala Met Gly Val Gly Asn Thr Leu Glu Asp
               100                 105                 110
   Gly Ser Phe Glu Ala Leu Ile Phe Gln Val Asn Arg Asp Gly Gly Gly
           115                 120                 125
   Gln Cys Thr Cys Glu Tyr Asn Thr Val Gly Gln Pro Asp Lys Phe Lys
       130                 135                 140
   Phe Cys Lys Thr Leu Ile Asn Pro Pro Gly Gln Asn Gly Ile Trp Pro
   145                 150                 155                 160
   Gln Asp Arg Val Asn His Thr Ala Lys Phe Gln Leu Pro Arg Asp Thr
                   165                 170                 175
   Thr Cys Arg Gly Gly Met Phe Lys Asp Lys Cys Leu Ile Arg Ile Arg
               180                 185                 190
   Cys Gly Glu Phe Leu Arg Phe Gly Gly Cys Leu Ala Ile Lys Thr Pro
           195                 200                 205
   Ala Ser Pro His Lys Leu Gln Leu Lys Val Val Ile Gly Gly Lys Thr
       210                 215                 220
   Ser Met Lys Thr Lys Pro Asp Leu Pro Arg Asn Asp Val Ser Ser Ile
   225                 230                 235                 240
   Ala Gln Lys Val Phe Asn Thr Leu Lys Ala Lys Gly Ala Phe Val Pro
                   245                 250                 255
   Ile Ser Ser Gly Arg Tyr Lys Arg Ser Pro Ala Met His Ile His Ala
               260                 265                 270
   Leu Gln His Val Arg Thr Leu Gly Val Thr Pro Leu Lys Ser Asn Phe
           275                 280                 285
   Asp Ser Met Ala Asp Glu Ile Ser Phe Lys Val Ile Glu Leu Met Arg
       290                 295                 300
   Ala Asn Lys Val Phe Leu Val Ala Lys Lys Ala Lys Ser Ile Leu Lys
   305                 310                 315                 320
   Asp Tyr Arg Asp Ser Thr Ser Ala Glu Arg
                   325                 330
<210> 247
   <211> 978
   <212> DNA
   <213> Phakopsora pachyrhizi
<220>
   <221> misc_feature
   <222> (1)..(978)
   <223> ta10705.02_phapa
<400> 247
   atgcaaaagc tctacattct gcttcttttt tccattataa aactctgctc ttccgggctt      60
   ataggttttg aagctgaaag atttaataat ccaaacacat ggaaaacagt tggcgaaatc     120
   cacgaaataa ctccaacaga attaagcaga caaattaagg ccggaagtcc aattatcatc     180
   agtgattttc ataaaacatt gataggcgat gaaaaggatc caaatcagtg gaaacggctt     240
   aaaaaattaa tgactaaagt caccaagcac acaagatttg tgataatctc tcgtgggttc     300
   agtgaaaaga tgattgaaaa ccttgcgcat ttgaatgggt tgtcaatttt ccaagggatg     360
   gggtcaattt tttatcacga cggacaatat attgagttgt ttcctaatgt ttttggagag     420
   ttcaatttta taaagagaaa aattattgaa cctgtactta caaaggtagg aaattcgcat     480
   agcatggacg acgtaaagtg gtacaaaatc atcgaaaaat cactcaggtt tgactttaat     540
   gaaaaatttg atgcaaacgt tccttcaaat agagcaaaac aaatcaagaa aaatatagct     600
   gaagaaattc aacgaagaat tggagtagct agggagaaag aggaaccagt gattaagcta     660
   gaatggaaag ttgatcacgt accgggtaaa ggcagtgtca gattactccc ttcaaattgt     720
   aacaagctgg atctattaaa cttttactta gaaaaaattc aagcaggaaa tcctaatttg     780
   atcagcttgg ggaatggtgc agaagatgaa gctttacatg gttttgtaaa caatcaaaaa     840
   cattttgaaa acgatgtctt tgaatcagtg ttggtgcgta aacctgatga aaaggatcca     900
   aaccgtatga gtgttgcaaa aaatcgcatg aacaactttg aagaagtaca caaattcttg     960
   gaaatttata gctcttaa                                                   978
<210> 248
   <211> 325
   <212> PRT
   <213> Phakopsora pachyrhizi
<220>
   <221> VARIANT
   <222> (1)..(325)
   <223> ta10705.02_phapa
<400> 248
   Met Gln Lys Leu Tyr Ile Leu Leu Leu Phe Ser Ile Ile Lys Leu Cys
   1               5                   10                  15
   Ser Ser Gly Leu Ile Gly Phe Glu Ala Glu Arg Phe Asn Asn Pro Asn
               20                  25                  30
   Thr Trp Lys Thr Val Gly Glu Ile His Glu Ile Thr Pro Thr Glu Leu
           35                  40                  45
   Ser Arg Gln Ile Lys Ala Gly Ser Pro Ile Ile Ile Ser Asp Phe His
       50                  55                  60
   Lys Thr Leu Ile Gly Asp Glu Lys Asp Pro Asn Gln Trp Lys Arg Leu
   65                  70                  75                  80
   Lys Lys Leu Met Thr Lys Val Thr Lys His Thr Arg Phe Val Ile Ile
                   85                  90                  95
   Ser Arg Gly Phe Ser Glu Lys Met Ile Glu Asn Leu Ala His Leu Asn
               100                 105                 110
   Gly Leu Ser Ile Phe Gln Gly Met Gly Ser Ile Phe Tyr His Asp Gly
           115                 120                 125
   Gln Tyr Ile Glu Leu Phe Pro Asn Val Phe Gly Glu Phe Asn Phe Ile
       130                 135                 140
   Lys Arg Lys Ile Ile Glu Pro Val Leu Thr Lys Val Gly Asn Ser His
   145                 150                 155                 160
   Ser Met Asp Asp Val Lys Trp Tyr Lys Ile Ile Glu Lys Ser Leu Arg
                   165                 170                 175
   Phe Asp Phe Asn Glu Lys Phe Asp Ala Asn Val Pro Ser Asn Arg Ala
               180                 185                 190
   Lys Gln Ile Lys Lys Asn Ile Ala Glu Glu Ile Gln Arg Arg Ile Gly
           195                 200                 205
   Val Ala Arg Glu Lys Glu Glu Pro Val Ile Lys Leu Glu Trp Lys Val
       210                 215                 220
   Asp His Val Pro Gly Lys Gly Ser Val Arg Leu Leu Pro Ser Asn Cys
   225                 230                 235                 240
   Asn Lys Leu Asp Leu Leu Asn Phe Tyr Leu Glu Lys Ile Gln Ala Gly
                   245                 250                 255
   Asn Pro Asn Leu Ile Ser Leu Gly Asn Gly Ala Glu Asp Glu Ala Leu
               260                 265                 270
   His Gly Phe Val Asn Asn Gln Lys His Phe Glu Asn Asp Val Phe Glu
           275                 280                 285
   Ser Val Leu Val Arg Lys Pro Asp Glu Lys Asp Pro Asn Arg Met Ser
       290                 295                 300
   Val Ala Lys Asn Arg Met Asn Asn Phe Glu Glu Val His Lys Phe Leu
   305                 310                 315                 320
   Glu Ile Tyr Ser Ser
                   325
<210> 249
   <211> 963
   <212> DNA
   <213> Phakopsora pachyrhizi
<220>
   <221> misc_feature
   <222> (1)..(963)
   <223> ta10430.01_phapa
<400> 249
   atgcagtcct gctggatttt ttttcaccat ttattattcc tgcctcggat ttggggccgt      60
   tgttatggtg caataaaatt caatgatatg aatcaatttg ggctagctgt tgaaatctgt     120
   ggtcaggtct taaattcttt aggcgcaatt tttggattgg gtctgttgcc tagccgcttg     180
   ggcaacttca aaagtatcct gattatggcg ggatattcca taaaaatccg gtcatctcga      240
   gctgcgtttc tactaaaagc aaatcctagt acagacctta agaagattga taagaacgac      300
   ctactagaag ttttaagcgc ctactatgaa tttaaaattg agagattgga ggcgggtatt      360
   gaccaagatg aaatcaacat cgaagatcaa gtaatgattc tgaatgaaat agaactcttt      420
   agagtaaaaa aggcaataga agctttgact agaaggttgt cttggtataa atctcagcct      480
   agtgaaactc atcacgcgtt tccaattttc ggggctttat ttattataat tttatctttg      540
   ataaacagca ttgctaattg tgtactttgc tactttatgt ggacatactc tgcttcgaaa      600
   aggccaatga ttgcgctttt gatctgtggc gcaatattag ttcttactgg aatttcatct      660
   gcggcattaa tttggttttt gggtgtaaag actaaaaaga aggtagagaa aattcaaggc      720
   ggattgatac caacaataga agaagacgta gaagaggaag agaacgacga tgataaatat      780
   gaagatgttg atgttgatgt aagaagagat attcaccagg gtggaaatta tgttccagga      840
   gaagagagcc aacaatttat gaataacttt gaaaacgaag aaacgcaagt tgagcaaagg      900
   gagcaacatg aagccgaaaa cgatgacggc aattgccgat caaagagcga tcgctatatg      960
   tag                                                                    963
<210> 250
   <211> 320
   <212> PRT
   <213> Phakopsora pachyrhizi
<220>
   <221> VARIANT
   <222> (1)..(320)
   <223> ta10430.01_phapa
<400> 250
   Met Gln Ser Cys Trp Ile Phe Phe His His Leu Leu Phe Leu Pro Arg
   1               5                   10                  15
   Ile Trp Gly Arg Cys Tyr Gly Ala Ile Lys Phe Asn Asp Met Asn Gln
               20                  25                  30
   Phe Gly Leu Ala Val Glu Ile Cys Gly Gln Val Leu Asn Ser Leu Gly
           35                  40                  45
   Ala Ile Phe Gly Leu Gly Leu Leu Pro Ser Arg Leu Gly Asn Phe Lys
       50                  55                  60
   Ser Ile Leu Ile Met Ala Gly Tyr Ser Ile Lys Ile Arg Ser Ser Arg
   65                  70                  75                  80
   Ala Ala Phe Leu Leu Lys Ala Asn Pro Ser Thr Asp Leu Lys Lys Ile
                   85                  90                  95
   Asp Lys Asn Asp Leu Leu Glu Val Leu Ser Ala Tyr Tyr Glu Phe Lys
               100                 105                 110
   Ile Glu Arg Leu Glu Ala Gly Ile Asp Gln Asp Glu Ile Asn Ile Glu
           115                 120                 125
   Asp Gln Val Met Ile Leu Asn Glu Ile Glu Leu Phe Arg Val Lys Lys
       130                 135                 140
   Ala Ile Glu Ala Leu Thr Arg Arg Leu Ser Trp Tyr Lys Ser Gln Pro
   145                 150                 155                 160
   Ser Glu Thr His His Ala Phe Pro Ile Phe Gly Ala Leu Phe Ile Ile
                   165                 170                 175
   Ile Leu Ser Leu Ile Asn Ser Ile Ala Asn Cys Val Leu Cys Tyr Phe
               180                 185                 190
   Met Trp Thr Tyr Ser Ala Ser Lys Arg Pro Met Ile Ala Leu Leu Ile
           195                 200                 205
   Cys Gly Ala Ile Leu Val Leu Thr Gly Ile Ser Ser Ala Ala Leu Ile
       210                 215                 220
   Trp Phe Leu Gly Val Lys Thr Lys Lys Lys Val Glu Lys Ile Gln Gly
   225                 230                 235                 240
   Gly Leu Ile Pro Thr Ile Glu Glu Asp Val Glu Glu Glu Glu Asn Asp
                   245                 250                 255
   Asp Asp Lys Tyr Glu Asp Val Asp Val Asp Val Arg Arg Asp Ile His
               260                 265                 270
   Gln Gly Gly Asn Tyr Val Pro Gly Glu Glu Ser Gln Gln Phe Met Asn
           275                 280                 285
   Asn Phe Glu Asn Glu Glu Thr Gln Val Glu Gln Arg Glu Gln His Glu
       290                 295                 300
   Ala Glu Asn Asp Asp Gly Asn Cys Arg Ser Lys Ser Asp Arg Tyr Met
   305                 310                 315                 320
<210> 251
   <211> 954
   <212> DNA
   <213> Phakopsora pachyrhizi
<220>
   <221> misc_feature
   <222> (1)..(954)
   <223> ta08910.01_phapa
<400> 251
   atgccaaaaa acctgtctgt gataatatgc ctcttagtaa gccttaatgt ttttcaagga      60
   gctggggcaa gctttttgac aaagactgta ggaagtgttg atggtgagcc cagcttcagc     120
   tcttggagcg ctgcttggat gtctaatgct aggaatgcta aggccaatat tgttaaatcg     180
   aatggtgtgc tggaaaaagc ggcatacacc ttccctaagc aattatcctt gtctgataag     240
   gagatgaaaa acctcaaaga atttaatttt catggtgaac cacaaattgc cgcccagtac     300
   agagacctta ttaatagtta ctcatcgcca gagcagataa tccttcacaa ggaaaataaa     360
   gaaagcttag ctttattaaa cagaaattta aaagagctta ccactgagga caaagaagca     420
   ctagaaaaat gggcaagcaa aaactttcgc cctgatatct tagtagaaaa tgaacgccca     480
   aacgaatttc aaaatgctaa ggctgcttta gattggaacc tcaagagagc ttctgctaaa     540
   caagatgtat ggtggaccga aaaagatctt caacattggg gtgaaaaaaa ttctgatgaa     600
   acggttatca ttaaatttgg tgatctcttt aagtttgatg aaaagagcaa agattattta     660
   tttaaagaaa accctgtcca attaggtatg aagcttttca aagcccttga aaaaccatca     720
   gatgatgtta ttttcaaaaa gcgagtagtt tggcccgtta aagaagtgca attattggga     780
   ttaatcaagg ccgaagactc agataaatct ttacttcata tgcgattgga tcaaatgttt     840
   aagagctcaa acaacttaaa aggccaaaca aattctgaaa ctggaaacat tcgtaatatt     900
   attcgttctt caatctctgg taaaactgaa actgaaaaaa atttgcacca atag           954
<210> 252
   <211> 317
   <212> PRT
   <213> Phakopsora pachyrhizi
<220>
   <221> VARIANT
   <222> (1)..(317)
   <223> ta08910.01_phapa
<400> 252
   Met Pro Lys Asn Leu Ser Val Ile Ile Cys Leu Leu Val Ser Leu Asn
   1               5                   10                  15
   Val Phe Gln Gly Ala Gly Ala Ser Phe Leu Thr Lys Thr Val Gly Ser
               20                  25                  30
   Val Asp Gly Glu Pro Ser Phe Ser Ser Trp Ser Ala Ala Trp Met Ser
           35                  40                  45
   Asn Ala Arg Asn Ala Lys Ala Asn Ile Val Lys Ser Asn Gly Val Leu
       50                  55                  60
   Glu Lys Ala Ala Tyr Thr Phe Pro Lys Gln Leu Ser Leu Ser Asp Lys
   65                  70                  75                  80
   Glu Met Lys Asn Leu Lys Glu Phe Asn Phe His Gly Glu Pro Gln Ile
                   85                  90                  95
   Ala Ala Gln Tyr Arg Asp Leu Ile Asn Ser Tyr Ser Ser Pro Glu Gln
               100                 105                 110
   Ile Ile Leu His Lys Glu Asn Lys Glu Ser Leu Ala Leu Leu Asn Arg
           115                 120                 125
   Asn Leu Lys Glu Leu Thr Thr Glu Asp Lys Glu Ala Leu Glu Lys Trp
       130                 135                 140
   Ala Ser Lys Asn Phe Arg Pro Asp Ile Leu Val Glu Asn Glu Arg Pro
   145                 150                 155                 160
   Asn Glu Phe Gln Asn Ala Lys Ala Ala Leu Asp Trp Asn Leu Lys Arg
                   165                 170                 175
   Ala Ser Ala Lys Gln Asp Val Trp Trp Thr Glu Lys Asp Leu Gln His
                   180                 185                 190
   Trp Gly Glu Lys Asn Ser Asp Glu Thr Val Ile Ile Lys Phe Gly Asp
               195                 200                 205
   Leu Phe Lys Phe Asp Glu Lys Ser Lys Asp Tyr Leu Phe Lys Glu Asn
       210                 215                 220
   Pro Val Gln Leu Gly Met Lys Leu Phe Lys Ala Leu Glu Lys Pro Ser
   225                 230                 235                 240
   Asp Asp Val Ile Phe Lys Lys Arg Val Val Trp Pro Val Lys Glu Val
                   245                 250                 255
   Gln Leu Leu Gly Leu Ile Lys Ala Glu Asp Ser Asp Lys Ser Leu Leu
               260                 265                 270
   His Met Arg Leu Asp Gln Met Phe Lys Ser Ser Asn Asn Leu Lys Gly
           275                 280                 285
   Gln Thr Asn Ser Glu Thr Gly Asn Ile Arg Asn Ile Ile Arg Ser Ser
       290                 295                 300
   Ile Ser Gly Lys Thr Glu Thr Glu Lys Asn Leu His Gln
   305                 310                 315
<210> 253
   <211> 948
   <212> DNA
   <213> Phakopsora pachyrhizi
<220>
   <221> misc_feature
   <222> (1)..(948)
   <223> ta00023.01_phapa
<400> 253
   atgccaacct tcagcttcac ctggacagac gctgcactgt tcgggctgac tgcaggattg      60
   atcgccacga tcgccctgat cggtcgcagc tcatcatcac aatctaaaaa gagcgtctta     120
   gattcatcca gaacaaacca gtcccagagc tccaagaaga gtaagaagaa gaataaacct     180
   aagaagtctc ctccccaacc cagctctcag acaacaatcg gctctaatca gaaatccgaa     240
   ccaaggcata ccgatgagag gaaggcttca ccccctaaat cttctaaccc atcacccaaa     300
   ccgatctctg agccgaagaa gaaaaagatt caacagcccc aaattcctaa actcgatgac     360
   aatgagtttc ctacccttgc aaattcgact ggaatgtcag ctgttagagc taaagaatct     420
   aaagatgtac cgattgctga gaggagacgc gcaaatgcgc caaagacagc agttgatgat     480
   atgattgatg aagaggtaga aaaacctgtt cagatggcac gagtgatggc aattgtcgag     540
   ccagaaccaa agattatact gaatcaagag agtgacccag aagatggctg ggagaaagtt     600
   cccaattcta aaaaaccgcg aggggcagga agtgctatgt ctactacatc ctcatcagta     660
   tctcgaagtg ctgtgccaaa gcaaagcaac gcagactcat ccagcgctgc ctccactaag     720
   cgacagcgac agaacgctaa gaaaaaggaa gctgcaaagt caatcaaaga ggcagaagag     780
   gcagaacgac tcagtaggct tgcatcttac aagcgtcagc aggagaatga gaggatccga     840
   agtcaagcca ctggtagtac taaacctacc ggccagaact caaacaaccc ctcaggtgct     900
   aaacaggact caaagcttgc cccgaatggg caacttattt gggaatga                  948
<210> 254
   <211> 315
   <212> PRT
   <213> Phakopsora pachyrhizi
<220>
   <221> VARIANT
   <222> (1)..(315)
   <223> ta00023.01_phapa
<400> 254
   Met Pro Thr Phe Ser Phe Thr Trp Thr Asp Ala Ala Leu Phe Gly Leu
   1               5                   10                  15
   Thr Ala Gly Leu Ile Ala Thr Ile Ala Leu Ile Gly Arg Ser Ser Ser
               20                  25                  30
   Ser Gln Ser Lys Lys Ser Val Leu Asp Ser Ser Arg Thr Asn Gln Ser
           35                  40                  45
   Gln Ser Ser Lys Lys Ser Lys Lys Lys Asn Lys Pro Lys Lys Ser Pro
       50                  55                  60
   Pro Gln Pro Ser Ser Gln Thr Thr Ile Gly Ser Asn Gln Lys Ser Glu
   65                  70                  75                  80
   Pro Arg His Thr Asp Glu Arg Lys Ala Ser Pro Pro Lys Ser Ser Asn
                   85                  90                  95
   Pro Ser Pro Lys Pro Ile Ser Glu Pro Lys Lys Lys Lys Ile Gln Gln
               100                 105                 110
   Pro Gln Ile Pro Lys Leu Asp Asp Asn Glu Phe Pro Thr Leu Ala Asn
           115                 120                 125
   Ser Thr Gly Met Ser Ala Val Arg Ala Lys Glu Ser Lys Asp Val Pro
       130                 135                 140
   Ile Ala Glu Arg Arg Arg Ala Asn Ala Pro Lys Thr Ala Val Asp Asp
   145                 150                 155                 160
   Met Ile Asp Glu Glu Val Glu Lys Pro Val Gln Met Ala Arg Val Met
                   165                 170                 175
   Ala Ile Val Glu Pro Glu Pro Lys Ile Ile Leu Asn Gln Glu Ser Asp
               180                 185                 190
   Pro Glu Asp Gly Trp Glu Lys Val Pro Asn Ser Lys Lys Pro Arg Gly
           195                 200                 205
   Ala Gly Ser Ala Met Ser Thr Thr Ser Ser Ser Val Ser Arg Ser Ala
       210                 215                 220
   Val Pro Lys Gln Ser Asn Ala Asp Ser Ser Ser Ala Ala Ser Thr Lys
   225                 230                 235                 240
   Arg Gln Arg Gln Asn Ala Lys Lys Lys Glu Ala Ala Lys Ser Ile Lys
                   245                 250                 255
   Glu Ala Glu Glu Ala Glu Arg Leu Ser Arg Leu Ala Ser Tyr Lys Arg
               260                 265                 270
   Gln Gln Glu Asn Glu Arg Ile Arg Ser Gln Ala Thr Gly Ser Thr Lys
           275                 280                 285
   Pro Thr Gly Gln Asn Ser Asn Asn Pro Ser Gly Ala Lys Gln Asp Ser
       290                 295                 300
   Lys Leu Ala Pro Asn Gly Gln Leu Ile Trp Glu
   305                 310                 315
<210> 255
   <211> 924
   <212> DNA
   <213> Phakopsora pachyrhizi
<220>
   <221> misc_feature
   <222> (1)..(924)
   <223> ta04133.01_phapa
<400> 255
   atgaaccgaa ttaggatagc catcgtctta gcttctttaa gcttggcgaa ctgccaattt      60
   ttatttgggg atcctgcagc agatgctcag aacagctcaa caaatcaggc taattcaaca     120
   ggctctaaac tttcacccac tcaaagcagt gaccaaactg ttcctttgac ttattcctca     180
   aattccagtg atacaaactc attgaactca gctaatggca cagctactaa tagctctatc     240
   ctaacaggcg gcacgaacgc ttttgacaat actggttcta atagctccat caaaacaaac     300
   ggcagcagcc tttttaacgg tactgctacc aacagctcta ttcaatccac agggctcatt     360
   ggtagctata atgggacaat ctcaccctcc aaaaacactt caatgttttc agaagtctac     420
   actttagact acagtattaa atgggatgaa agtgatttca atgtctatgg ccaaaatggt     480
   aaagttgaat ataccattag taacaaagtg gagggagtca acatgtcaaa gaaagaattt     540
   gttgtgaaag aagccaccga tggacaagca aaagttagaa ttgacgccaa taataaattc     600
   tgtggatttg gtaaaactta cacatcagat gatggggcaa gctttacaat cgacccacgc     660
   atgtttttac ctgatcgctg gtttatcagg caaagcaatg ttacgtacgt ttttaaacgc     720
   ttcgccatga gtcttaatgg agatatcttg gatgtcgaaa acaagcgcct tgtagcgcag     780
   gtcaaagtcg acaaagctaa tactacgagt gaagaaaaga agaaaaagat gatcatttta     840
   aattcagatg gttctatctc aggttgggat ttggttgcct tcatcgctgt ggttagaaat     900
   cgcatccgcc aatgcggtta ctaa                                            924
<210> 256
   <211> 307
   <212> PRT
   <213> Phakopsora pachyrhizi
<220>
   <221> VARIANT
   <222> (1)..(307)
   <223> ta04133.01_phapa
<400> 256
   Met Asn Arg Ile Arg Ile Ala Ile Val Leu Ala Ser Leu Ser Leu Ala
   1               5                   10                  15
   Asn Cys Gln Phe Leu Phe Gly Asp Pro Ala Ala Asp Ala Gln Asn Ser
               20                  25                  30
   Ser Thr Asn Gln Ala Asn Ser Thr Gly Ser Lys Leu Ser Pro Thr Gln
           35                  40                  45
   Ser Ser Asp Gln Thr Val Pro Leu Thr Tyr Ser Ser Asn Ser Ser Asp
       50                  55                  60
   Thr Asn Ser Leu Asn Ser Ala Asn Gly Thr Ala Thr Asn Ser Ser Ile
   65                  70                  75                  80
   Leu Thr Gly Gly Thr Asn Ala Phe Asp Asn Thr Gly Ser Asn Ser Ser
                   85                  90                  95
   Ile Lys Thr Asn Gly Ser Ser Leu Phe Asn Gly Thr Ala Thr Asn Ser
               100                 105                 110
   Ser Ile Gln Ser Thr Gly Leu Ile Gly Ser Tyr Asn Gly Thr Ile Ser
           115                 120                 125
   Pro Ser Lys Asn Thr Ser Met Phe Ser Glu Val Tyr Thr Leu Asp Tyr
       130                 135                 140
   Ser Ile Lys Trp Asp Glu Ser Asp Phe Asn Val Tyr Gly Gln Asn Gly
   145                 150                 155                 160
   Lys Val Glu Tyr Thr Ile Ser Asn Lys Val Glu Gly Val Asn Met Ser
                   165                 170                 175
   Lys Lys Glu Phe Val Val Lys Glu Ala Thr Asp Gly Gln Ala Lys Val
               180                 185                 190
   Arg Ile Asp Ala Asn Asn Lys Phe Cys Gly Phe Gly Lys Thr Tyr Thr
           195                 200                 205
   Ser Asp Asp Gly Ala Ser Phe Thr Ile Asp Pro Arg Met Phe Leu Pro
       210                 215                 220
   Asp Arg Trp Phe Ile Arg Gln Ser Asn Val Thr Tyr Val Phe Lys Arg
   225                 230                 235                 240
   Phe Ala Met Ser Leu Asn Gly Asp Ile Leu Asp Val Glu Asn Lys Arg
                   245                 250                 255
   Leu Val Ala Gln Val Lys Val Asp Lys Ala Asn Thr Thr Ser Glu Glu
               260                 265                 270
   Lys Lys Lys Lys Met Ile Ile Leu Asn Ser Asp Gly Ser Ile Ser Gly
           275                 280                 285
   Trp Asp Leu Val Ala Phe Ile Ala Val Val Arg Asn Arg Ile Arg Gln
       290                 295                 300
   Cys Gly Tyr
   305
<210> 257
   <211> 906
   <212> DNA
   <213> Phakopsora pachyrhizi
<220>
   <221> misc_feature
   <222> (1)..(906)
   <223> ta10945.01_phapa
<400> 257
   atgtactcaa aaatctcaaa cctttctatt ctcttagtga cttcaagttt tattttctta        60
   aattgtgcag attgctttac cacaaatttt caatcaagga ctagtgagta caagcctgct       120
   tcaaactcag tgcagaacgt aacgataaaa gattctggag ataaaaaata ttttcctgtt       180
   cgttttgtca ctgtctacgc accggcctct caacatcaag caacgtcctc tgatcaaaac       240
   attagtcaag taaccaaagg tgaaaatgta acaagtaccc cttcaaatga gaagccgaga       300
   gatactgttt acgtcaacga atcagaagtt ggtaacttga ttccgatggc aacccaagga     360
   attcttgatg aagtctatgt gatgaataga gaattggatt ggaccaataa cgagtttccg     420
   atctacaact caacaggggg aatcgcttat acgattacca ataagataaa cggctctcag     480
   ttggcagagt cacagttcgc aatcatcggt cctgaccgtt ggttagttct aacttctgat     540
   acgaaatctg gtctctgtgg tttttcaaat gagtactctt cctccgacca cgttttatac     600
   agcttaagac caagactttt catgccagat cgttggtacc tatctggtga cttggtcagt     660
   ccactgaaag atcatgctta cgaatttcgc cggggtgctc taagttttga gggtgatata     720
   ctcactttgg gaactcatac cagacatgct aagatcagca atggtaaatt ggctcaaggg     780
   tggatcgata agaagattcc gggtggccgc acaatttccg tctttactga tggtactatt     840
   cctttgccaa acttaatttc tctgatagtg ataagcgtga caagaattaa aaaatgcggt     900
   ttttag                                                                906
<210> 258
   <211> 301
   <212> PRT
   <213> Phakopsora pachyrhizi
<220>
   <221> VARIANT
   <222> (1)..(301)
   <223> ta10945.01_phapa
<400> 258
   Met Tyr Ser Lys Ile Ser Asn Leu Ser Ile Leu Leu Val Thr Ser Ser
   1               5                   10                  15
   Phe Ile Phe Leu Asn Cys Ala Asp Cys Phe Thr Thr Asn Phe Gln Ser
               20                  25                  30
   Arg Thr Ser Glu Tyr Lys Pro Ala Ser Asn Ser Val Gln Asn Val Thr
           35                  40                  45
   Ile Lys Asp Ser Gly Asp Lys Lys Tyr Phe Pro Val Arg Phe Val Thr
       50                  55                  60
   Val Tyr Ala Pro Ala Ser Gln His Gln Ala Thr Ser Ser Asp Gln Asn
   65                  70                  75                  80
   Ile Ser Gln Val Thr Lys Gly Glu Asn Val Thr Ser Thr Pro Ser Asn
                   85                  90                  95
   Glu Lys Pro Arg Asp Thr Val Tyr Val Asn Glu Ser Glu Val Gly Asn
               100                 105                 110
   Leu Ile Pro Met Ala Thr Gln Gly Ile Leu Asp Glu Val Tyr Val Met
           115                 120                 125
   Asn Arg Glu Leu Asp Trp Thr Asn Asn Glu Phe Pro Ile Tyr Asn Ser
       130                 135                 140
   Thr Gly Gly Ile Ala Tyr Thr Ile Thr Asn Lys Ile Asn Gly Ser Gln
   145                 150                 155                 160
   Leu Ala Glu Ser Gln Phe Ala Ile Ile Gly Pro Asp Arg Trp Leu Val
                   165                 170                 175
   Leu Thr Ser Asp Thr Lys Ser Gly Leu Cys Gly Phe Ser Asn Glu Tyr
               180                 185                 190
   Ser Ser Ser Asp His Val Leu Tyr Ser Leu Arg Pro Arg Leu Phe Met
           195                 200                 205
   Pro Asp Arg Trp Tyr Leu Ser Gly Asp Leu Val Ser Pro Leu Lys Asp
       210                 215                 220
   His Ala Tyr Glu Phe Arg Arg Gly Ala Leu Ser Phe Glu Gly Asp Ile
   225                 230                 235                 240
   Leu Thr Leu Gly Thr His Thr Arg His Ala Lys Ile Ser Asn Gly Lys
                   245                 250                 255
   Leu Ala Gln Gly Trp Ile Asp Lys Lys Ile Pro Gly Gly Arg Thr Ile
               260                 265                 270
   Ser Val Phe Thr Asp Gly Thr Ile Pro Leu Pro Asn Leu Ile Ser Leu
           275                 280                 285
   Ile Val Ile Ser Val Thr Arg Ile Lys Lys Cys Gly Phe
       290                 295                 300
<210> 259
   <211> 892
   <212> DNA
   <213> Phakopsora pachyrhizi
<220>
   <221> misc_feature
   <222> (1)..(892)
   <223> ta00652.01
<400> 259
   atgccaactc ttagatcaat ccgctaccga aggaccttgc tagcatgcat tggtattatg        60
   tcctcaatct cagctcagaa cattaccagt gactttccag ccagcgacac tctttcaaca       120
   gcctgtaggg attcaataaa taatttaatt aacggagaat ttgggaagtg ttcaaaaatt       180
   tcagatttaa ttcaatttgc ctactcgaat aattccaacc cgttatctag gtacagcaac       240
   tatgttaaaa atttttgtga ggctgaggtg tgctcgagtg aaattttatc caagacggtc       300
   aagtctttaa gatctacatg cctctctgac ctaaaaactg ggaatattga tgtcatcaga       360
   acttatgcca cgagctctca atactcactt ctacgtaatt ccctctgcct aagacgtgtt       420
   tccgactcta ccctgtgcct cacctcaatg ctggatgaat tacaagtgtt tgggaaggtc       480
   ccttttagtg tttctcagat tgaaaacgtt gtaataggag gattcccatc cattatatct       540
   tcacttgata acctaccaga aaaattttgt aatgactgca ttcatggtct cattacagtg       600
   ctacctagca caacagctaa cggtgacgca gccctaccac cggtcgctaa aggccttcct       660
   gatggtgtca agattcaagc ccatccaatc ttacaaaatg cgcaacctgc cacaatgacc       720
   attgctgcat tgtgtggtgc ttctgtgatt gatggaaaaa ttcctccaag tctggtcaca       780
   ggcttgggta gtcgtccagc tgcaaacgct agcttcgttt caaagacatc ggacgcttct       840
   cgcaccactt tttacacctc aaactgcaaa cgactggctt gctcagctgc at               892
<210> 260
   <211> 297
   <212> PRT
   <213> Phakopsora pachyrhizi
<220>
   <221> VARIANT
   <222> (1)..(297)
   <223> ta00652.01_phapa
<400> 260
   Met Pro Thr Leu Arg Ser Ile Arg Tyr Arg Arg Thr Leu Leu Ala Cys
   1               5                   10                  15
   Ile Gly Ile Met Ser Ser Ile Ser Ala Gln Asn Ile Thr Ser Asp Phe
               20                  25                  30
   Pro Ala Ser Asp Thr Leu Ser Thr Ala Cys Arg Asp Ser Ile Asn Asn
           35                  40                  45
   Leu Ile Asn Gly Glu Phe Gly Lys Cys Ser Lys Ile Ser Asp Leu Ile
       50                  55                  60
   Gln Phe Ala Tyr Ser Asn Asn Ser Asn Pro Leu Ser Arg Tyr Ser Asn
   65                  70                  75                  80
   Tyr Val Lys Asn Phe Cys Glu Ala Glu Val Cys Ser Ser Glu Ile Leu
                   85                  90                  95
   Ser Lys Thr Val Lys Ser Leu Arg Ser Thr Cys Leu Ser Asp Leu Lys
               100                 105                 110
   Thr Gly Asn Ile Asp Val Ile Arg Thr Tyr Ala Thr Ser Ser Gln Tyr
           115                 120                 125
   Ser Leu Leu Arg Asn Ser Leu Cys Leu Arg Arg Val Ser Asp Ser Thr
       130                 135                 140
   Leu Cys Leu Thr Ser Met Leu Asp Glu Leu Gln Val Phe Gly Lys Val
   145                 150                 155                 160
   Pro Phe Ser Val Ser Gln Ile Glu Asn Val Val Ile Gly Gly Phe Pro
                   165                 170                 175
   Ser Ile Ile Ser Ser Leu Asp Asn Leu Pro Glu Lys Phe Cys Asn Asp
               180                 185                 190
   Cys Ile His Gly Leu Ile Thr Val Leu Pro Ser Thr Thr Ala Asn Gly
           195                 200                 205
   Asp Ala Ala Leu Pro Pro Val Ala Lys Gly Leu Pro Asp Gly Val Lys
       210                 215                 220
   Ile Gln Ala His Pro Ile Leu Gln Asn Ala Gln Pro Ala Thr Met Thr
   225                 230                 235                 240
   Ile Ala Ala Leu Cys Gly Ala Ser Val Ile Asp Gly Lys Ile Pro Pro
                   245                 250                 255
   Ser Leu Val Thr Gly Leu Gly Ser Arg Pro Ala Ala Asn Ala Ser Phe
               260                 265                 270
   Val Ser Lys Thr Ser Asp Ala Ser Arg Thr Thr Phe Tyr Thr Ser Asn
           275                 280                 285
   Cys Lys Arg Leu Ala Cys Ser Ala Ala
       290                 295
<210> 261
   <211> 886
   <212> DNA
   <213> Phakopsora pachyrhizi
<220>
   <221> misc_feature
   <222> (1)..(886)
   <223> ta03308.01
<400> 261
   atgcgattgc ttttgaaact ttgttgtttt ttgacttttt tttgcaactg ggggtctacc       60
   aggcaatgct taagactatt tcagaaaata gatagcccca atgaaatttg catagtcgag      120
   aacgccaacg atttaagttt caacccaact aatgagtcgg gaagttttgc gaaattatta      180
   aaatcaccaa aacaaatgtc ctgtatccca acttcaagca ccaggtccaa aaaagtaaaa      240
   aaaagctctg ctaaaaaaaa ccccgatatt ataaattatt ttgaatctca aggagagtgg      300
   ctagagagcc aaaaagaaat agataatctc aattcacgaa agccaaaaac taaagcaaaa      360
   gacttaagat caaaatcaga gaaaagcaag gaaaaaatct ccgacatttc taaaaattta      420
   gttgttgatc gaaattctag atccttgccg ccaaaaaaat ctccaccttt gggagccaaa      480
   gctataaaat caaagtcgtt gccgcactta agcaaatata gttttgcgga agcttatgag      540
   gctttatatt taaccgattc accacggcgt cctaaaatac tccccttaaa cagccacatc      600
   gcacaaccaa aaagtaaaat atctatttca ccttccccag ctattgataa aaaccctacc      660
   gagcataatt cttatgaagt cttacaaaat ttccttcggc gaaaaagcaa aagtctttcc      720
   ccatcagtaa cttctggctc cagctcaaag cattttgaat ccaagcaggt tgacttaaat      780
   atacccgtta cacctcacaa ggcacctaaa agtgaaattg aagagggtcg tatgattagt      840
   aaattaactg atgataaaag cattggagaa ttgtccttta gattta                     886
<210> 262
   <211> 295
   <212> PRT
   <213> Phakopsora pachyrhizi
<220>
   <221> VARIANT
   <222> (1)..(295)
   <223> ta03308.01_phapa
<400> 262
   Met Arg Leu Leu Leu Lys Leu Cys Cys Phe Leu Thr Phe Phe Cys Asn
   1               5                   10                  15
   Trp Gly Ser Thr Arg Gln Cys Leu Arg Leu Phe Gln Lys Ile Asp Ser
               20                  25                  30
   Pro Asn Glu Ile Cys Ile Val Glu Asn Ala Asn Asp Leu Ser Phe Asn
           35                  40                  45
   Pro Thr Asn Glu Ser Gly Ser Phe Ala Lys Leu Leu Lys Ser Pro Lys
       50                  55                  60
   Gln Met Ser Cys Ile Pro Thr Ser Ser Thr Arg Ser Lys Lys Val Lys
   65                  70                  75                  80
   Lys Ser Ser Ala Lys Lys Asn Pro Asp Ile Ile Asn Tyr Phe Glu Ser
                   85                  90                  95
   Gln Gly Glu Trp Leu Glu Ser Gln Lys Glu Ile Asp Asn Leu Asn Ser
               100                 105                 110
   Arg Lys Pro Lys Thr Lys Ala Lys Asp Leu Arg Ser Lys Ser Glu Lys
           115                 120                 125
   Ser Lys Glu Lys Ile Ser Asp Ile Ser Lys Asn Leu Val Val Asp Arg
       130                 135                 140
   Asn Ser Arg Ser Leu Pro Pro Lys Lys Ser Pro Pro Leu Gly Ala Lys
   145                 150                 155                 160
   Ala Ile Lys Ser Lys Ser Leu Pro His Leu Ser Lys Tyr Ser Phe Ala
                   165                 170                 175
   Glu Ala Tyr Glu Ala Leu Tyr Leu Thr Asp Ser Pro Arg Arg Pro Lys
               180                 185                 190
   Ile Leu Pro Leu Asn Ser His Ile Ala Gln Pro Lys Ser Lys Ile Ser
           195                 200                 205
   Ile Ser Pro Ser Pro Ala Ile Asp Lys Asn Pro Thr Glu His Asn Ser
       210                 215                 220
   Tyr Glu Val Leu Gln Asn Phe Leu Arg Arg Lys Ser Lys Ser Leu Ser
   225                 230                 235                 240
   Pro Ser Val Thr Ser Gly Ser Ser Ser Lys His Phe Glu Ser Lys Gln
                   245                 250                 255
   Val Asp Leu Asn Ile Pro Val Thr Pro His Lys Ala Pro Lys Ser Glu
               260                 265                 270
   Ile Glu Glu Gly Arg Met Ile Ser Lys Leu Thr Asp Asp Lys Ser Ile
           275                 280                 285
   Gly Glu Leu Ser Phe Arg Phe
       290                 295
<210> 263
   <211> 882
   <212> DNA
   <213> Phakopsora pachyrhizi
<220>
   <221> misc_feature
   <222> (1)..(882)
   <223> ta01346.01_phapa
<400> 263
   atggaaagaa gacaaaatta tgcatttcta tttgtccttc tggctacatt tagtctgttt    60
   gggcttacct tatctattcc gatgaatacg atgttgattg cgacagatac agcaaaagct   120
   acacactcag cctctgaggc tggcacagcg gcaaagatag cggaaggggt aaaatttagc   180
   gatgcttcag atgtggcatt tattgacaga aatggggata gtgtgaaagg agtattagaa   240
   actgctaccg atggagggaa aactgctgag agtttaaact ttggatccaa ggcgaacatg   300
   ctcccaccac atgttatagt tgagcctatc cctcaaccca agcccagctt gtatgccaag   360
   acaaaagcat tttttgtcag gatttggcgg aaaatgatgg ggggttttag gaaaagccca   420
   ttaggtaaat taagccggaa ggtatcaaat tcaaaaccgg caaaacaaac caataattac   480
   tttcaaaagg ggaggggttg gatcaatgaa aaatggagta aatcaaaatc aatccccaag   540
   acgtctgaat ctgtgaatgt ttccccaggg gctgagcaca atattccaga actgaattcg   600
   gtgaaacccg aagcatcagc gaagcaaaat atgctggagg tcaattccgt acaaccagag   660
   gcagctgcta aacccgtaaa tactaaggtc gaggaacctg ctactattaa gactaaatcg   720
   actgcttctg tggagtcaca acctgagtca accctttctg gtaaaactga gccaaaagta   780
   aatggtgaac ctgaaaaagc cccggaaaaa gcagaaccaa aaccagaaaa ctcagatccc   840
   gtatcgaaac ctccaccgcc accaactaac caagcccaat ga                      882
<210> 264
   <211> 293
   <212> PRT
   <213> Phakopsora pachyrhizi
<220>
   <221> VARIANT
   <222> (1)..(293)
   <223> ta01346.01_phapa
<400> 264
   Met Glu Arg Arg Gln Asn Tyr Ala Phe Leu Phe Val Leu Leu Ala Thr
   1               5                   10                  15
   Phe Ser Leu Phe Gly Leu Thr Leu Ser Ile Pro Met Asn Thr Met Leu
               20                  25                  30
   Ile Ala Thr Asp Thr Ala Lys Ala Thr His Ser Ala Ser Glu Ala Gly
               35                  40                  45
   Thr Ala Ala Lys Ile Ala Glu Gly Val Lys Phe Ser Asp Ala Ser Asp
       50                  55                  60
   Val Ala Phe Ile Asp Arg Asn Gly Asp Ser Val Lys Gly Val Leu Glu
   65                  70                  75                  80
   Thr Ala Thr Asp Gly Gly Lys Thr Ala Glu Ser Leu Asn Phe Gly Ser
                   85                  90                  95
   Lys Ala Asn Met Leu Pro Pro His Val Ile Val Glu Pro Ile Pro Gln
               100                 105                 110
   Pro Lys Pro Ser Leu Tyr Ala Lys Thr Lys Ala Phe Phe Val Arg Ile
           115                 120                 125
   Trp Arg Lys Met Met Gly Gly Phe Arg Lys Ser Pro Leu Gly Lys Leu
       130                 135                 140
   Ser Arg Lys Val Ser Asn Ser Lys Pro Ala Lys Gln Thr Asn Asn Tyr
   145                 150                 155                 160
   Phe Gln Lys Gly Arg Gly Trp Ile Asn Glu Lys Trp Ser Lys Ser Lys
                   165                 170                 175
   Ser Ile Pro Lys Thr Ser Glu Ser Val Asn Val Ser Pro Gly Ala Glu
               180                 185                 190
   His Asn Ile Pro Glu Leu Asn Ser Val Lys Pro Glu Ala Ser Ala Lys
           195                 200                 205
   Gln Asn Met Leu Glu Val Asn Ser Val Gln Pro Glu Ala Ala Ala Lys
       210                 215                 220
   Pro Val Asn Thr Lys Val Glu Glu Pro Ala Thr Ile Lys Thr Lys Ser
   225                 230                 235                 240
   Thr Ala Ser Val Glu Ser Gln Pro Glu Ser Thr Leu Ser Gly Lys Thr
                   245                 250                 255
   Glu Pro Lys Val Asn Gly Glu Pro Glu Lys Ala Pro Glu Lys Ala Glu
               260                 265                 270
   Pro Lys Pro Glu Asn Ser Asp Pro Val Ser Lys Pro Pro Pro Pro Pro
           275                 280                 285
   Thr Asn Gln Ala Gln
       290
<210> 265
   <211> 876
   <212> DNA
   <213> Phakopsora pachyrhizi
<220>
   <221> misc_feature
   <222> (1)..(876)
   <223> ta03255.01_phapa
<400> 265
   atgttgcact tttctagttt aacttctcta ggaatttttg cgtgccaaac tgcttggctc    60
   gttgcagttt caaacgcctt cgtagcggaa catgcagtta tccaggacat aaccaatctt   120
   gcttttagtc ctaataagat tgtagataga acacttggac aaaaaaaccc aaattttgcc   180
   aagttcgtca atgcagaacc attactttat cgtgaaatgg ctcgcaaagc tggacgatct   240
   gttggaacag tcttaactga ctctcgagga accaatcttc ctctactcat ccgtgacctt   300
   cgagtcaccg actggattca tagcgatccg gtaattaagg gaatgccacc aaacaatcta   360
   cctcaaaatg ctattgaact tgcccagaac tccttcgcat ctcgtctagt ggacattcgg   420
   aacgcaaatg ataaagccaa gttcgaccag cacgcattaa tagcacgtaa ccacttagct   480
   aaggtactgt tttacatcat aagtgagatg catatatcta tgcttccgaa acttgatgta   540
   gtcaatgtag ctgcgtggca gaaagaaagg gcaggccgcg tcgctctttt gatgacacaa   600
   aatcctttcc tcaagcggct ttattccgtc caccaacaaa tcggaaatcc ggatcaatac   660
   attactcctg agactgcgga tgcagctttt atgaaacttt ttcctcatca aaattcaaac   720
   aaacagggtc atccttcaca atcggcaaca tctgaaaaaa tgaaaactca ccctgagggc   780
   gaaggaaact ttcctgccca tcgccctgtg gatgaaaata aagagggcat tgtccctgga   840
   actcacccta atgctgcaac tgtttcttct tcttaa                             876
<210> 266
   <211> 291
   <212> PRT
   <213> Phakopsora pachyrhizi
<220>
   <221> VARIANT
   <222> (1)..(291)
   <223> ta03255.01_phapa
<400> 266
   Met Leu His Phe Ser Ser Leu Thr Ser Leu Gly Ile Phe Ala Cys Gln
   1               5                   10                  15
   Thr Ala Trp Leu Val Ala Val Ser Asn Ala Phe Val Ala Glu His Ala
               20                  25                  30
   Val Ile Gln Asp Ile Thr Asn Leu Ala Phe Ser Pro Asn Lys Ile Val
           35                  40                  45
   Asp Arg Thr Leu Gly Gln Lys Asn Pro Asn Phe Ala Lys Phe Val Asn
       50                  55                  60
   Ala Glu Pro Leu Leu Tyr Arg Glu Met Ala Arg Lys Ala Gly Arg Ser
   65                  70                  75                  80
   Val Gly Thr Val Leu Thr Asp Ser Arg Gly Thr Asn Leu Pro Leu Leu
                   85                  90                  95
   Ile Arg Asp Leu Arg Val Thr Asp Trp Ile His Ser Asp Pro Val Ile
               100                 105                 110
   Lys Gly Met Pro Pro Asn Asn Leu Pro Gln Asn Ala Ile Glu Leu Ala
           115                 120                 125
   Gln Asn Ser Phe Ala Ser Arg Leu Val Asp Ile Arg Asn Ala Asn Asp
       130                 135                 140
   Lys Ala Lys Phe Asp Gln His Ala Leu Ile Ala Arg Asn His Leu Ala
   145                 150                 155                 160
   Lys Val Leu Phe Tyr Ile Ile Ser Glu Met His Ile Ser Met Leu Pro
                   165                 170                 175
   Lys Leu Asp Val Val Asn Val Ala Ala Trp Gln Lys Glu Arg Ala Gly
               180                 185                 190
   Arg Val Ala Leu Leu Met Thr Gln Asn Pro Phe Leu Lys Arg Leu Tyr
           195                 200                 205
   Ser Val His Gln Gln Ile Gly Asn Pro Asp Gln Tyr Ile Thr Pro Glu
       210                 215                 220
   Thr Ala Asp Ala Ala Phe Met Lys Leu Phe Pro His Gln Asn Ser Asn
   225                 230                 235                 240
   Lys Gln Gly His Pro Ser Gln Ser Ala Thr Ser Glu Lys Met Lys Thr
                   245                 250                 255
   His Pro Glu Gly Glu Gly Asn Phe Pro Ala His Arg Pro Val Asp Glu
               260                 265                 270
   Asn Lys Glu Gly Ile Val Pro Gly Thr His Pro Asn Ala Ala Thr Val
           275                 280                 285
   Ser Ser Ser
       290
<210> 267
   <211> 858
   <212> DNA
   <213> Phakopsora pachyrhizi
<220>
   <221> misc_feature
   <222> (1)..(858)
   <223> ta10969.01_phapa
<400> 267
   atgttttgga aagatttttt tttgcttgct tttgggttta ctaccctact ttacaaatct      60
   ctagctcaaa gtcaaaatta ctctggggat gtcatgcttc agattccgga cttttatccc     120
   gagaatgggg ctggtcttcc tagcaatggc aaagtatact tcagcagcct ctacaaagca     180
   agtgttatag agtatgaccc gttaaataac acttatcgag agttcaaaat ccctggcatt     240
   agtggcaatc ctatggccca cgtctctgga attgaagcaa gcaagtggtc cagtgatacg     300
   atatgggcga taatcgaccc ggcttttgtt ttcgcgacaa atggagccaa tatgaccggg     360
   ccggatgggt tagtttccat caatattact gatcagtcaa tggacatcat atacttgaaa     420
   ccagtactag aaagagctca atccattaat ggcggaactc gggtagttgg cgcacaagat      480
   ttggtgcaag ctcccgatgg aagcgtttac ctaataataa gcttcgggca ggcaataatc      540
   aagattgtgc cacaaactag ggcgctctca gttttttata gcccaaagcc agcaatctca      600
   aagatttcct atacgggaat agagctcgta tcaaatcaca cattagtagt ctggaatact      660
   gctgaaggaa gattcgagac gtttgaaatc aattcgccag agccaaaggc gaaattcgtt      720
   aaaattttaa attctaaatc gttggacagt cgaaaaattt atggagatgc actctttggt      780
   ccgagctttg ctcaaggacg ctgtctgctt ctcagcaacc ctggtgcaaa aaccatagaa      840
   gttttcacat cgaacaac                                                    858
<210> 268
   <211> 286
   <212> PRT
   <213> Phakopsora pachyrhizi
<220>
   <221> VARIANT
   <222> (1) .. (286)
   <223> ta10969.01_phapa
<400> 268
   Met Phe Trp Lys Asp Phe Phe Leu Leu Ala Phe Gly Phe Thr Thr Leu
   1               5                   10                  15
   Leu Tyr Lys Ser Leu Ala Gln Ser Gln Asn Tyr Ser Gly Asp Val Met
               20                  25                  30
   Leu Gln Ile Pro Asp Phe Tyr Pro Glu Asn Gly Ala Gly Leu Pro Ser
           35                  40                  45
   Asn Gly Lys Val Tyr Phe Ser Ser Leu Tyr Lys Ala Ser Val Ile Glu
       50                  55                  60
   Tyr Asp Pro Leu Asn Asn Thr Tyr Arg Glu Phe Lys Ile Pro Gly Ile
   65                  70                  75                  80
   Ser Gly Asn Pro Met Ala His Val Ser Gly Ile Glu Ala Ser Lys Trp
                   85                  90                  95
   Ser Ser Asp Thr Ile Trp Ala Ile Ile Asp Pro Ala Phe Val Phe Ala
               100                 105                 110
   Thr Asn Gly Ala Asn Met Thr Gly Pro Asp Gly Leu Val Ser Ile Asn
           115                 120                 125
   Ile Thr Asp Gln Ser Met Asp Ile Ile Tyr Leu Lys Pro Val Leu Glu
       130                 135                 140
   Arg Ala Gln Ser Ile Asn Gly Gly Thr Arg Val Val Gly Ala Gln Asp
   145                 150                 155                 160
   Leu Val Gln Ala Pro Asp Gly Ser Val Tyr Leu Ile Ile Ser Phe Gly
                   165                 170                 175
   Gln Ala Ile Ile Lys Ile Val Pro Gln Thr Arg Ala Leu Ser Val Phe
               180                 185                 190
   Tyr Ser Pro Lys Pro Ala Ile Ser Lys Ile Ser Tyr Thr Gly Ile Glu
           195                 200                 205
   Leu Val Ser Asn His Thr Leu Val Val Trp Asn Thr Ala Glu Gly Arg
       210                 215                 220
   Phe Glu Thr Phe Glu Ile Asn Ser Pro Glu Pro Lys Ala Lys Phe Val
   225                 230                 235                 240
   Lys Ile Leu Asn Ser Lys Ser Leu Asp Ser Arg Lys Ile Tyr Gly Asp
                   245                 250                 255
   Ala Leu Phe Gly Pro Ser Phe Ala Gln Gly Arg Cys Leu Leu Leu Ser
               260                 265                 270
   Asn Pro Gly Ala Lys Thr Ile Glu Val Phe Thr Ser Asn Asn
           275                 280                 285
<210> 269
   <211> 846
   <212> DNA
   <213> Phakopsora pachyrhizi
<220>
   <221> misc_feature
   <222> (1)..(846)
   <223> ta10991.01_phapa
<400> 269
   atgtttgctc ggtacttgtc atttattctg ctacttttaa tattctctaa tgattccaag      60
   tgcagcttca aagagggcct ttctggactc aagaggcgat tatcgtttcc agatatcaaa     120
   gatcccagtt ttgaaattcc ggctcttaat gaacccaatc cacatgcact tgattctagt     180
   gaaggatacc ctatcacctc ttatggcgta agcgaaggtg gaatcaaaag agataaggat     240
   atttcaatgc aatcacatat aaaaatagat caattggcgg aaaagaagaa agcacaaagt     300
   gattccctgg ttgatcacca acatcaaaac ttacacagaa ccaaaaagtt aaaaaccgat     360
   cagagcgaga tggaggcggg ttatgaagat cttaaaaact gtattcaaga aactaaaaaa     420
   caaatatggt ggccaagaca tatcgaaggt ttaaaaaaaa tcgaaaaaac tttagagaat     480
   gacttatcta gtattatgtt tgaagggata gatgataact gttttaaaac cttgtccgaa     540
   gttctaagca gctatgaaaa tgtattaaaa cctggtcgcc caataagcag tgtgccagta     600
   atcccactaa tatttaaaat actcgactac atcgaaaaat ttgaattgca agcgaataga     660
   atggaaagca ataaatcact aatcaaaaaa ttctttaatg aaaaggaact actcaaacag     720
   cttatctggt acatatcaac agtcttaatt cagaaatggg ggtttattgg ggtttttttg     780
   accagtgatt ttaaagaata catccgggat cataatgatt tgaatcatat aagatttttg     840
   ttaaat                                                                846
<210> 270
   <211> 282
   <212> PRT
   <213> Phakopsora pachyrhizi
<220>
   <221> VARIANT
   <222> (1)..(282)
   <223> ta10991.01_phapa
<400> 270
   Met Phe Ala Arg Tyr Leu Ser Phe Ile Leu Leu Leu Leu Ile Phe Ser
   1 5 10 15
   Asn Asp Ser Lys Cys Ser Phe Lys Glu Gly Leu Ser Gly Leu Lys Arg
   20 25 30
   Arg Leu Ser Phe Pro Asp Ile Lys Asp Pro Ser Phe Glu Ile Pro Ala
   35 40 45
   Leu Asn Glu Pro Asn Pro His Ala Leu Asp Ser Ser Glu Gly Tyr Pro
   50 55 60
   Ile Thr Ser Tyr Gly Val Ser Glu Gly Gly Ile Lys Arg Asp Lys Asp
   65 70 75 80
   Ile Ser Met Gln Ser His Ile Lys Ile Asp Gln Leu Ala Glu Lys Lys
   85 90 95
   Lys Ala Gln Ser Asp Ser Leu Val Asp His Gln His Gln Asn Leu His
   100 105 110
   Arg Thr Lys Lys Leu Lys Thr Asp Gln Ser Glu Met Glu Ala Gly Tyr
   115 120 125
   Glu Asp Leu Lys Asn Cys Ile Gln Glu Thr Lys Lys Gln Ile Trp Trp
   130 135 140
   Pro Arg His Ile Glu Gly Leu Lys Lys Ile Glu Lys Thr Leu Glu Asn
   145 150 155 160
   Asp Leu Ser Ser Ile Met Phe Glu Gly Ile Asp Asp Asn Cys Phe Lys
   165 170 175
   Thr Leu Ser Glu Val Leu Ser Ser Tyr Glu Asn Val Leu Lys Pro Gly
   180 185 190
   Arg Pro Ile Ser Ser Val Pro Val Ile Pro Leu Ile Phe Lys Ile Leu
   195 200 205
   Asp Tyr Ile Glu Lys Phe Glu Leu Gln Ala Asn Arg Met Glu Ser Asn
   210 215 220
   Lys Ser Leu Ile Lys Lys Phe Phe Asn Glu Lys Glu Leu Leu Lys Gln
   225 230 235 240
   Leu Ile Trp Tyr Ile Ser Thr Val Leu Ile Gln Lys Trp Gly Phe Ile
   245 250 255
   Gly Val Phe Leu Thr Ser Asp Phe Lys Glu Tyr Ile Arg Asp His Asn
   260 265 270
   Asp Leu Asn His Ile Arg Phe Leu Leu Asn
   275 280
<210> 271
   <211> 846
   <212> DNA
   <213> Phakopsora pachyrhizi
<220>
   <221> misc_feature
   <222> (1)..(846)
   <223> ta02873.01_phapa
<400> 271
   atgatttctc taactactct catctcgact attttactat actctatggt tatttccatc      60
   accaactctg tcgttctcga cagaggctct tatcaatctg acaactatca tcatcatcaa     120
   agcaagagat ggttgaattc tccctttgcg ccctactaca tcagtggtag ttcggtctat     180
   gggggttact acgatccgag atataacttt tcaccaagct atccgatcgg ctctgcaaag     240
   ttctacactc agggattaac taactatctg acctggtgtc caagtgggat ctacggcttt     300
   ggtgcaagct acgaattctc gaaccagaac tataggacag tacccaatcc gagcagctac     360
   tcattcttta agaaattctc acagtctgat aagagctcta agaggagcta tgtcagtcat     420
   gaacacagta acgacgagga agattatgag atcagaaagg ttagccgtaa cagccataac     480
   tttatcaagc ggggcgacca ggttcaatgt agaaatcaga agggtgaaac tatctctttc     540
   gtaaaatcgg attgtgatgc ggctgcaatt aagatggtaa accagaaaag tgcagtctca     600
   aacgttggaa gctgtggact ggttctcatt ggacctcaag gccaactgtc tagttcaaat     660
   ctacctattg agaagattca gaatgatgtt cagaacattt taaatacgtg cacgatgggt     720
   gggccttcaa ataattttaa caaccaattc acaaataaca gaaactcttt tatcaattta     780
   cccaggggtg cgaatccaaa ctacgtgatt ctattaacaa aaggagacgg aagtccagat     840
   tattag                                                                846
<210> 272
   <211> 281
   <212> PRT
   <213> Phakopsora pachyrhizi
<220>
   <221> VARIANT
   <222> (1)..(281)
   <223> ta02873.01_phapa
<400> 272
   Met Ile Ser Leu Thr Thr Leu Ile Ser Thr Ile Leu Leu Tyr Ser Met
   1               5                   10                  15
   Val Ile Ser Ile Thr Asn Ser Val Val Leu Asp Arg Gly Ser Tyr Gln
               20                  25                  30
   Ser Asp Asn Tyr His His His Gln Ser Lys Arg Trp Leu Asn Ser Pro
           35                  40                  45
   Phe Ala Pro Tyr Tyr Ile Ser Gly Ser Ser Val Tyr Gly Gly Tyr Tyr
       50                  55                  60
   Asp Pro Arg Tyr Asn Phe Ser Pro Ser Tyr Pro Ile Gly Ser Ala Lys
   65                  70                  75                  80
   Phe Tyr Thr Gln Gly Leu Thr Asn Tyr Leu Thr Trp Cys Pro Ser Gly
                   85                  90                  95
   Ile Tyr Gly Phe Gly Ala Ser Tyr Glu Phe Ser Asn Gln Asn Tyr Arg
               100                 105                 110
   Thr Val Pro Asn Pro Ser Ser Tyr Ser Phe Phe Lys Lys Phe Ser Gln
           115                 120                 125
   Ser Asp Lys Ser Ser Lys Arg Ser Tyr Val Ser His Glu His Ser Asn
       130                 135                 140
   Asp Glu Glu Asp Tyr Glu Ile Arg Lys Val Ser Arg Asn Ser His Asn
   145                 150                 155                 160
   Phe Ile Lys Arg Gly Asp Gln Val Gln Cys Arg Asn Gln Lys Gly Glu
                   165                 170                 175
   Thr Ile Ser Phe Val Lys Ser Asp Cys Asp Ala Ala Ala Ile Lys Met
               180                 185                 190
   Val Asn Gln Lys Ser Ala Val Ser Asn Val Gly Ser Cys Gly Leu Val
           195                 200                 205
   Leu Ile Gly Pro Gln Gly Gln Leu Ser Ser Ser Asn Leu Pro Ile Glu
       210                 215                 220
   Lys Ile Gln Asn Asp Val Gln Asn Ile Leu Asn Thr Cys Thr Met Gly
   225                 230                 235                 240
   Gly Pro Ser Asn Asn Phe Asn Asn Gln Phe Thr Asn Asn Arg Asn Ser
                   245                 250                 255
   Phe Ile Asn Leu Pro Arg Gly Ala Asn Pro Asn Tyr Val Ile Leu Leu
               260                 265                 270
   Thr Lys Gly Asp Gly Ser Pro Asp Tyr
           275                 280
<210> 273
   <211> 843
   <212> DNA
   <213> Phakopsora pachyrhizi
<220>
   <221> misc_feature
   <222> (1)..(843)
   <223> ta04966.01_phapa
<400> 273
   atgaagtctt ttgttttggc ctcaggcatt acaattacaa tttttacggc tcttgaaatt      60
   gggatgttaa tttttggtgg ttccgcattt gcatcaattg tggcattgat ggccttgggc     120
   gctacggcgg ttactatggg tggtgcaggt ggtgtaggaa ctaaactggc acttatgcat     180
   ccggaggaaa ctccatttga aaggtggtcc agatatgcgt tttatctctc tgaatggcag     240
   caacacgttc aaaaacgagt tgcaaatgaa gccaggacag taatcaacgc tggtattagc     300
   tcaccgaagg gattggctaa tattgttaag aatggtgtat tcttccgaga ggttccccaa     360
   ttcagtgaag cagatttgat ttcaggcctc aaaaatacga ctacggctag aatagcagtc     420
   gatatcttga ggactcaggg cgctttcgtt acaattgcaa gtgacaagtg cacaggtaaa     480
   ggtccgggtg gggcatggga aggtaatgac aaactgtctt actgctctaa gaatgggact     540
   atgatgaaca ttattcatgg ctacaagaaa aaagttaaga atacctggtt caatgcccaa     600
   ctcctagaaa agaagtacgg tatcactgtt cagtatcttg cggagcaatc gtttcactgt     660
   cagcaaaatc acaaaagctt tggattcgac ccttacaagt ctggaacctt tcctagttca     720
   ttagatgcag agtgtattgg aaatcttcct gtctgcgatt tcaggattca agaacttgcg     780
   gaggcgaaat ctgagaaagg caccatgcga gcttgtcgga gcgttggaag gttgccgatt     840
   tga                                                                   843
<210> 274
   <211> 280
   <212> PRT
   <213> Phakopsora pachyrhizi
<220>
   <221> VARIANT
   <222> (1)..(280)
   <223> ta04966.01_phapa
<400> 274
   Met Lys Ser Phe Val Leu Ala Ser Gly Ile Thr Ile Thr Ile Phe Thr
   1               5                   10                  15
   Ala Leu Glu Ile Gly Met Leu Ile Phe Gly Gly Ser Ala Phe Ala Ser
               20                  25                  30
   Ile Val Ala Leu Met Ala Leu Gly Ala Thr Ala Val Thr Met Gly Gly
           35                  40                  45
   Ala Gly Gly Val Gly Thr Lys Leu Ala Leu Met His Pro Glu Glu Thr
       50                  55                  60
   Pro Phe Glu Arg Trp Ser Arg Tyr Ala Phe Tyr Leu Ser Glu Trp Gln
   65                  70                  75                  80
   Gln His Val Gln Lys Arg Val Ala Asn Glu Ala Arg Thr Val Ile Asn
                   85                  90                  95
   Ala Gly Ile Ser Ser Pro Lys Gly Leu Ala Asn Ile Val Lys Asn Gly
               100                 105                 110
   Val Phe Phe Arg Glu Val Pro Gln Phe Ser Glu Ala Asp Leu Ile Ser
           115                 120                 125
   Gly Leu Lys Asn Thr Thr Thr Ala Arg Ile Ala Val Asp Ile Leu Arg
       130                 135                 140
   Thr Gln Gly Ala Phe Val Thr Ile Ala Ser Asp Lys Cys Thr Gly Lys
   145                 150                 155                 160
   Gly Pro Gly Gly Ala Trp Glu Gly Asn Asp Lys Leu Ser Tyr Cys Ser
                   165                 170                 175
   Lys Asn Gly Thr Met Met Asn Ile Ile His Gly Tyr Lys Lys Lys Val
               180                 185                 190
   Lys Asn Thr Trp Phe Asn Ala Gln Leu Leu Glu Lys Lys Tyr Gly Ile
           195                 200                 205
   Thr Val Gln Tyr Leu Ala Glu Gln Ser Phe His Cys Gln Gln Asn His
       210                 215                 220
   Lys Ser Phe Gly Phe Asp Pro Tyr Lys Ser Gly Thr Phe Pro Ser Ser
   225                 230                 235                 240
   Leu Asp Ala Glu Cys Ile Gly Asn Leu Pro Val Cys Asp Phe Arg Ile
                   245                 250                 255
   Gln Glu Leu Ala Glu Ala Lys Ser Glu Lys Gly Thr Met Arg Ala Cys
               260                 265                 270
   Arg Ser Val Gly Arg Leu Pro Ile
           275                 280
<210> 275
   <211> 825
   <212> DNA
   <213> Phakopsora pachyrhizi
<220>
   <221> misc_feature
   <222> (1)..(825)
   <223> ta07858.01_phapa
<400> 275
   atgaattctc acattggctc aaagaccatt ccccacattc ttttacttcc ctatttccta       60
   atccttctta cttcgattta ccattcggtt attggtacat caatagaatc acttgtcaca      120
   gcagttgatc aaaaagatgt acatgaaggt ataaatttta tgcttgatgg ctcaacacat      180
   ggtgcacggc atgaattgaa agttggtgaa gaagataaaa atgttacatt gaaggatcat      240
   ggtcaaagtc atctacttga aattgttaga aaattagaaa agaggtattc ttctcacacc      300
   acgacgattg aaaaaacgga aaattttcca aaaagttctg ctcggtccca tcattttaaa      360
   aggagtactc catcatctgg agaaatcaga agatggctta gaagtcacaa taaatttaga      420
   gcccaatact ctgcctctcc gttggtgtgg gatcagaact tggctgacaa agccaattca      480
   gagactaata cctgcgtttg gagacactct tacaacgata tatatggcga aaacattgca      540
   gccggccagg aatcgattga agaggttgtg gatgagtggg tcacaggttc agaagagaga      600
   agagtttatt caccaaataa ccctacatat tcacatttta cccaagttgt ttggggagac      660
   acaaggcgtt taggatgtgc aatgacctct tgtagaaata ttcgaggctc tggtttacca      720
   caatctcctg taaagttttg ggcttgtgaa tattatccgc ctggaaatgt agatggccag      780
   tatcgtcaaa acgtcaaagc tagatatggt ggctctcctc tataa                      825
<210> 276
   <211> 274
   <212> PRT
   <213> Phakopsora pachyrhizi
<220>
   <221> VARIANT
   <222> (1)..(274)
   <223> ta07858.01_phapa
<400> 276
   Met Asn Ser His Ile Gly Ser Lys Thr Ile Pro His Ile Leu Leu Leu
   1               5                   10                  15
   Pro Tyr Phe Leu Ile Leu Leu Thr Ser Ile Tyr His Ser Val Ile Gly
               20                  25                  30
   Thr Ser Ile Glu Ser Leu Val Thr Ala Val Asp Gln Lys Asp Val His
               35                  40                  45
   Glu Gly Ile Asn Phe Met Leu Asp Gly Ser Thr His Gly Ala Arg His
           50                  55                  60
   Glu Leu Lys Val Gly Glu Glu Asp Lys Asn Val Thr Leu Lys Asp His
   65                  70                  75                  80
   Gly Gln Ser His Leu Leu Glu Ile Val Arg Lys Leu Glu Lys Arg Tyr
                   85                  90                  95
   Ser Ser His Thr Thr Thr Ile Glu Lys Thr Glu Asn Phe Pro Lys Ser
               100                 105                 110
   Ser Ala Arg Ser His His Phe Lys Arg Ser Thr Pro Ser Ser Gly Glu
           115                 120                 125
   Ile Arg Arg Trp Leu Arg Ser His Asn Lys Phe Arg Ala Gln Tyr Ser
       130                 135                 140
   Ala Ser Pro Leu Val Trp Asp Gln Asn Leu Ala Asp Lys Ala Asn Ser
   145                 150                 155                 160
   Glu Thr Asn Thr Cys Val Trp Arg His Ser Tyr Asn Asp Ile Tyr Gly
                   165                 170                 175
   Glu Asn Ile Ala Ala Gly Gln Glu Ser Ile Glu Glu Val Val Asp Glu
               180                 185                 190
   Trp Val Thr Gly Ser Glu Glu Arg Arg Val Tyr Ser Pro Asn Asn Pro
           195                 200                 205
   Thr Tyr Ser His Phe Thr Gln Val Val Trp Gly Asp Thr Arg Arg Leu
       210                 215                 220
   Gly Cys Ala Met Thr Ser Cys Arg Asn Ile Arg Gly Ser Gly Leu Pro
   225                 230                 235                 240
   Gln Ser Pro Val Lys Phe Trp Ala Cys Glu Tyr Tyr Pro Pro Gly Asn
                   245                 250                 255
   Val Asp Gly Gln Tyr Arg Gln Asn Val Lys Ala Arg Tyr Gly Gly Ser
               260                 265                 270
   Pro Leu
<210> 277
   <211> 813
   <212> DNA
   <213> Phakopsora pachyrhizi
<220>
   <221> misc_feature
   <222> (1)..(813)
   <223> ta09549.01_phapa
<400> 277
   atgttaatgt acccgattgc atttatcttt atgatattgc ccctttcaac ttttagatta      60
   gcggcattgg cgggtcatga ttggggagtc actgcagcct cagtttgtgg ttcaatatat     120
   tgtctaagtg gctttattga tgtgctcctt tttgggacta cgcgaagcat aatttctgtt     180
   ccaatatttt cattcaagtc acatcaaaga gttcatcctc agggtgctac ggcaattatt     240
   ggcaccaatg gtatcggaat gagttctagt gggttgggca ctcgatcagt cggatttaga     300
   gttgaagtta tacaagaaac ggtgattgac ttggacgaac caaacgaggg ggatttacat     360
   gaattaaaga agaacccaaa tcccaaacat gaccatcatc gccgaatgag tgaattaaac     420
   gatgtgctcc cttcagattc aattataaaa ccggttgaag ctactcatgg tgaaaggtat     480
   gattctatgg actcacagac ggcgggtgaa aacataaatc aaacttcgaa cacaaatatt     540
   ccagagaata ccaaccttac aagcttctta aatctggaca caacctcatc agatcttaag     600
   tctgagactt cgaaaccgat gagtagatat atgtttgggg ttgccgataa ttcaagctgt     660
   gcatcaacta attcaaatag accgatgttt tgttttgaac ctgataataa tcaatctaac     720
   tatcctcaaa ttcttcatca tgagtctcaa aacttttctt ctcatcgatc tgaatttaca     780
   gaccccatac cgtggcagaa gtcaagctca taa                                 813
<210> 278
   <211> 270
   <212> PRT
   <213> Phakopsora pachyrhizi
<220>
   <221> VARIANT
   <222> (1)..(270)
   <223> ta09549.01_phapa
<400> 278
   Met Leu Met Tyr Pro Ile Ala Phe Ile Phe Met Ile Leu Pro Leu Ser
   1               5                   10                  15
   Thr Phe Arg Leu Ala Ala Leu Ala Gly His Asp Trp Gly Val Thr Ala
               20                  25                  30
   Ala Ser Val Cys Gly Ser Ile Tyr Cys Leu Ser Gly Phe Ile Asp Val
           35                  40                  45
   Leu Leu Phe Gly Thr Thr Arg Ser Ile Ile Ser Val Pro Ile Phe Ser
       50                  55                  60
   Phe Lys Ser His Gln Arg Val His Pro Gln Gly Ala Thr Ala Ile Ile
   65                  70                  75                  80
   Gly Thr Asn Gly Ile Gly Met Ser Ser Ser Gly Leu Gly Thr Arg Ser
                   85                  90                  95
   Val Gly Phe Arg Val Glu Val Ile Gln Glu Thr Val Ile Asp Leu Asp
               100                 105                 110
   Glu Pro Asn Glu Gly Asp Leu His Glu Leu Lys Lys Asn Pro Asn Pro
           115                 120                 125
   Lys His Asp His His Arg Arg Met Ser Glu Leu Asn Asp Val Leu Pro
       130                 135                 140
   Ser Asp Ser Ile Ile Lys Pro Val Glu Ala Thr His Gly Glu Arg Tyr
   145                 150                 155                 160
   Asp Ser Met Asp Ser Gln Thr Ala Gly Glu Asn Ile Asn Gln Thr Ser
                   165                 170                 175
   Asn Thr Asn Ile Pro Glu Asn Thr Asn Leu Thr Ser Phe Leu Asn Leu
               180                 185                 190
   Asp Thr Thr Ser Ser Asp Leu Lys Ser Glu Thr Ser Lys Pro Met Ser
           195                 200                 205
   Arg Tyr Met Phe Gly Val Ala Asp Asn Ser Ser Cys Ala Ser Thr Asn
       210                 215                 220
   Ser Asn Arg Pro Met Phe Cys Phe Glu Pro Asp Asn Asn Gln Ser Asn
   225                 230                 235                 240
   Tyr Pro Gln Ile Leu His His Glu Ser Gln Asn Phe Ser Ser His Arg
                   245                 250                 255
   Ser Glu Phe Thr Asp Pro Ile Pro Trp Gln Lys Ser Ser Ser
               260                 265                 270
<210> 279
   <211> 813
   <212> DNA
   <213> Phakopsora pachyrhizi
<220>
   <221> misc_feature
   <222> (1)..(813)
   <223> ta08378.02_phapa
<400> 279
   atggttaaga gtttcgaact tctgctggtc cttttgatct ttcaaacttt ttctctgaac     60
   ttctatgttt ctggatccta cgagtatcaa ctcaatggag ttaacaatca attcgcagta    120
   ctggattcta agatctcgaa gattcagtcc ctagctcaac aacaaggaca ttccgatgtt    180
   aaagaattgt gcttggaagc taaggatcac ttgtcggctg caatcgatgg gtgggaaaga    240
   atctcaaagg tttacaaaga taagatttgg ttagcttcag agtcaaagta taaggacaat    300
   gtgcaagata gcttggataa gtgtggtgaa tcaatcatga agattctaga caatgatcac    360
   gtgaagagcg tttctggacg ttacggatct caggttgaag attgtaaacg atactatgaa    420
   acttgtcaac actcttgcaa gaatatttgg gactggggac ccccgagtcc aacaccgagt    480
   ggatcttaca cttcgtacaa caagcgacaa aatgttaatt ctctgagacg tagaagcttg    540
   gaaggcgatg aaagtgatca gattcagaaa tgccccaaag gggagactgc ttgtccgatc    600
   tcagagaatt ctattggatt tgagtgcctt gatactaagc tggaactgac taactgtggt    660
   ggatgtcgta ctaagaatga aggtgaaaac tgcttggaga ttgaaggctc agttggtgtc    720
   ggttgtatga agggtaaatg tgtcgttttt tctgttcaac ctgggtatta tttaagtaag    780
   aggatcaacc ggccggtcct caagagaaaa taa                                 813
<210> 280
   <211> 270
   <212> PRT
   <213> Phakopsora pachyrhizi
<220>
   <221> VARIANT
   <222> (1)..(270)
   <223> ta08378.02_phapa
<400> 280
   Met Val Lys Ser Phe Glu Leu Leu Leu Val Leu Leu Ile Phe Gln Thr
   1               5                   10                  15
   Phe Ser Leu Asn Phe Tyr Val Ser Gly Ser Tyr Glu Tyr Gln Leu Asn
               20                  25                  30
   Gly Val Asn Asn Gln Phe Ala Val Leu Asp Ser Lys Ile Ser Lys Ile
           35                  40                  45
   Gln Ser Leu Ala Gln Gln Gln Gly His Ser Asp Val Lys Glu Leu Cys
       50                  55                  60
   Leu Glu Ala Lys Asp His Leu Ser Ala Ala Ile Asp Gly Trp Glu Arg
   65                  70                  75                  80
   Ile Ser Lys Val Tyr Lys Asp Lys Ile Trp Leu Ala Ser Glu Ser Lys
                   85                  90                  95
   Tyr Lys Asp Asn Val Gln Asp Ser Leu Asp Lys Cys Gly Glu Ser Ile
               100                 105                 110
   Met Lys Ile Leu Asp Asn Asp His Val Lys Ser Val Ser Gly Arg Tyr
           115                 120                 125
   Gly Ser Gln Val Glu Asp Cys Lys Arg Tyr Tyr Glu Thr Cys Gln His
       130                 135                 140
   Ser Cys Lys Asn Ile Trp Asp Trp Gly Pro Pro Ser Pro Thr Pro Ser
   145                 150                 155                 160
   Gly Ser Tyr Thr Ser Tyr Asn Lys Arg Gln Asn Val Asn Ser Leu Arg
                   165                 170                 175
   Arg Arg Ser Leu Glu Gly Asp Glu Ser Asp Gln Ile Gln Lys Cys Pro
               180                 185                 190
   Lys Gly Glu Thr Ala Cys Pro Ile Ser Glu Asn Ser Ile Gly Phe Glu
           195                 200                 205
   Cys Leu Asp Thr Lys Leu Glu Leu Thr Asn Cys Gly Gly Cys Arg Thr
       210                 215                 220
   Lys Asn Glu Gly Glu Asn Cys Leu Glu Ile Glu Gly Ser Val Gly Val
   225                 230                 235                 240
   Gly Cys Met Lys Gly Lys Cys Val Val Phe Ser Val Gln Pro Gly Tyr
                   245                 250                 255
   Tyr Leu Ser Lys Arg Ile Asn Arg Pro Val Leu Lys Arg Lys
               260                 265                 270
<210> 281
   <211> 813
   <212> DNA
   <213> Phakopsora pachyrhizi
<220>
   <221> misc_feature
   <222> (1)..(813)
   <223> ta08378.03_phapa
<400> 281
   atggttaaga gtttcgaact tctgctggtc cttttgatct ttcaaacttt ttctctgaac       60
   ttctatgttt ctggatccta cgagtatcaa ctcaatggag ttaacaatca attcgcagta      120
   ctggattcta agatctcgaa gattcagtcc ctagctcaac aacaaggaca ttccgatgtt      180
   aaagaattgt gcttggaagc taaggatcac ttgtcggctg caatcgatgg gtgggaaaga      240
   atctcaaagg tttacaaaga taagatttgg ttagcttcag agtcaaagta taaggacaat      300
   gtgcaagata gcttggataa gtgtggtgaa tcaatcatga agattctaga caatgatcac      360
   gtgaagagcg tttctggacg ttacggatct caggttgaag attgtaaacg atactatgaa      420
   acttgtcaac actcttgcaa gaatatttgg gactggggac ccccgagtcc aacaccgagt      480
   ggatcttaca cttcgtacaa caagcgacaa aatgttaatt ctctgagacg tagaagcttg      540
   gaaggcgatg aaagtgatca gattcagaaa tgccccaaag gggagactgc ttgtccgatc      600
   tcagagaatt ctattggatt tgagtgcctt gatactaagc tggaactgac taactgtggt      660
   ggatgtcgta ctaagaatga aggtgaaaac tgcttggaga ttgaaggctc agttggtgtc      720
   ggttgtatga agggtaaatg tgtcgttttt tctgttcaac ctgggtatta tttaagtaag      780
   aggatcaacc ggccggtcct caagagaaaa taa                                   813
<210> 282
   <211> 270
   <212> PRT
   <213> Phakopsora pachyrhizi
<220>
   <221> VARIANT
   <222> (1)..(270)
   <223> ta08378.03_phapa
<400> 282
   Met Val Lys Ser Phe Glu Leu Leu Leu Val Leu Leu Ile Phe Gln Thr
   1               5                   10                  15
   Phe Ser Leu Asn Phe Tyr Val Ser Gly Ser Tyr Glu Tyr Gln Leu Asn
               20                  25                  30
   Gly Val Asn Asn Gln Phe Ala Val Leu Asp Ser Lys Ile Ser Lys Ile
           35                  40                  45
   Gln Ser Leu Ala Gln Gln Gln Gly His Ser Asp Val Lys Glu Leu Cys
       50                  55                  60
   Leu Glu Ala Lys Asp His Leu Ser Ala Ala Ile Asp Gly Trp Glu Arg
   65                  70                  75                  80
   Ile Ser Lys Val Tyr Lys Asp Lys Ile Trp Leu Ala Ser Glu Ser Lys
                   85                  90                  95
   Tyr Lys Asp Asn Val Gln Asp Ser Leu Asp Lys Cys Gly Glu Ser Ile
               100                 105                 110
   Met Lys Ile Leu Asp Asn Asp His Val Lys Ser Val Ser Gly Arg Tyr
           115                 120                 125
   Gly Ser Gln Val Glu Asp Cys Lys Arg Tyr Tyr Glu Thr Cys Gln His
       130                 135                 140
   Ser Cys Lys Asn Ile Trp Asp Trp Gly Pro Pro Ser Pro Thr Pro Ser
   145                 150                 155                 160
   Gly Ser Tyr Thr Ser Tyr Asn Lys Arg Gln Asn Val Asn Ser Leu Arg
                   165                 170                 175
   Arg Arg Ser Leu Glu Gly Asp Glu Ser Asp Gln Ile Gln Lys Cys Pro
               180                 185                 190
   Lys Gly Glu Thr Ala Cys Pro Ile Ser Glu Asn Ser Ile Gly Phe Glu
           195                 200                 205
   Cys Leu Asp Thr Lys Leu Glu Leu Thr Asn Cys Gly Gly Cys Arg Thr
       210                 215                 220
   Lys Asn Glu Gly Glu Asn Cys Leu Glu Ile Glu Gly Ser Val Gly Val
   225                 230                 235                 240
   Gly Cys Met Lys Gly Lys Cys Val Val Phe Ser Val Gln Pro Gly Tyr
                   245                 250                 255
   Tyr Leu Ser Lys Arg Ile Asn Arg Pro Val Leu Lys Arg Lys
               260                 265                 270
<210> 283
   <211> 813
   <212> DNA
   <213> Phakopsora pachyrhizi
<220>
   <221> misc_feature
   <222> (1)..(813)
   <223> ta03276.01_phapa
<400> 283
   atgaactgtt ttcggcttct tttattcact gtctctatct acattcttta ccttgagtca      60
   gtaaccacgc acttctacat caagcaatgg aagtctagta acgacaacag gcttagacca     120
   gctcaaaagg cagatttatc acattcagcg tatcgcagtt cctccgataa tattggttgg     180
   ataggcagta agtttctgac atcaaatcat gctattgttt gtggagcttc aaacactccc     240
   aaaggaaaag tggccccagc tggtggaaga tttttctcag atgcgtctca tgctgctggc     300
   caaacgctcg aagtgaatgc aggcggtcac attacattag ttataacaaa tgggggagga     360
   acaggatatc cccactacga aggtcacatt caggcatatc taggatactg tggtgcctcg     420
   ccaattgctt gtcaatcttt cgacgcttca aaggcttatt tcttcaaaat tcaagagtca     480
   ataaatggtg ttaagagact acgacgccaa atgtcccatt ctctaggggg agatgtatgg     540
   gaggttccaa tccccgcggg agttcctagg ggcagctata tacttcggtt tgaaataata     600
   acgccccatg aatctgttgc ctccgaagga tttcaagatc aatactaccc ctcttgtggc     660
   cagatttatg ttaaatcaaa caggaactct gttcacctga accaacttcc tctcctccga     720
   cttcctgggg gctacgaaaa taggaatatg aaagcttctc aagctccggg gcctcggctt     780
   gcaagcttta attctcttcg tttacgaaac tag                                  813
<210> 284
   <211> 270
   <212> PRT
   <213> Phakopsora pachyrhizi
<220>
   <221> VARIANT
   <222> (1)..(270)
   <223> ta03276.01_phapa
<400> 284
   Met Asn Cys Phe Arg Leu Leu Leu Phe Thr Val Ser Ile Tyr Ile Leu
   1               5                   10                  15
   Tyr Leu Glu Ser Val Thr Thr His Phe Tyr Ile Lys Gln Trp Lys Ser
               20                  25                  30
   Ser Asn Asp Asn Arg Leu Arg Pro Ala Gln Lys Ala Asp Leu Ser His
           35                  40                  45
   Ser Ala Tyr Arg Ser Ser Ser Asp Asn Ile Gly Trp Ile Gly Ser Lys
       50                  55                  60
   Phe Leu Thr Ser Asn His Ala Ile Val Cys Gly Ala Ser Asn Thr Pro
   65                  70                  75                  80
   Lys Gly Lys Val Ala Pro Ala Gly Gly Arg Phe Phe Ser Asp Ala Ser
                   85                  90                  95
   His Ala Ala Gly Gln Thr Leu Glu Val Asn Ala Gly Gly His Ile Thr
               100                 105                 110
   Leu Val Ile Thr Asn Gly Gly Gly Thr Gly Tyr Pro His Tyr Glu Gly
           115                 120                 125
   His Ile Gln Ala Tyr Leu Gly Tyr Cys Gly Ala Ser Pro Ile Ala Cys
       130                 135                 140
   Gln Ser Phe Asp Ala Ser Lys Ala Tyr Phe Phe Lys Ile Gln Glu Ser
   145                 150                 155                 160
   Ile Asn Gly Val Lys Arg Leu Arg Arg Gln Met Ser His Ser Leu Gly
                   165                 170                 175
   Gly Asp Val Trp Glu Val Pro Ile Pro Ala Gly Val Pro Arg Gly Ser
               180                 185                 190
   Tyr Ile Leu Arg Phe Glu Ile Ile Thr Pro His Glu Ser Val Ala Ser
           195                 200                 205
   Glu Gly Phe Gln Asp Gln Tyr Tyr Pro Ser Cys Gly Gln Ile Tyr Val
       210                 215                 220
   Lys Ser Asn Arg Asn Ser Val His Leu Asn Gln Leu Pro Leu Leu Arg
   225                 230                 235                 240
   Leu Pro Gly Gly Tyr Glu Asn Arg Asn Met Lys Ala Ser Gln Ala Pro
                   245                 250                 255
   Gly Pro Arg Leu Ala Ser Phe Asn Ser Leu Arg Leu Arg Asn
               260                 265                 270
<210> 285
   <211> 807
   <212> DNA
   <213> Phakopsora pachyrhizi
<220>
   <221> misc_feature
   <222> (1)..(807)
   <223> ta07479.01_phapa
<400> 285
   atgcaacatt tagtctattc ttcttcatta ctggcttcaa tcaccacact ggttctttcg      60
   atatactcat tgaatgcacc agcttggatc agatttgaca ccccatcatc atcaccactt     120
   cagtattcag aaagttatgg attaaaattc aaatgtggca gatcgaacat tgccccagat     180
   tttgtctgta gaccttttcc agatcgatca agagactgtg gtgcatcctc taactctatc     240
   cttgaagatc cattttttga gaaactctta tcagcagatg atcgacaact gcccttaaat     300
   agatctatcc atcagtttct acatcgtaaa aactcatcgt caagcatcaa aacggcccca     360
   atgggtagtg gatctatcga gctgcttgaa tcagcctcat ctgttttgga tcgattaggt     420
   gctcaaagat tcgggttctg tgagaagtgg aactcggctg gattttgtgc tgagatgagt     480
   attgtcattg gagcaatttc aatcttttgt gtctcgatcg tccttctggg taatcgtcac     540
   cgtcagaaac acggatggaa gatctgtgct ggtttgatag ctattcatgc cgtttctcag     600
   ataaccacat gggttttcgt attgcagatc ttcaacactg ataacagatt ttatatagga     660
   tcaaaacttt cgacaagttt ttacatctca gtcgcctcta gcatgatcga tttgatctgt     720
   ttgactggac tagttgcagc tggaattgtc gagaacgatg atgatgatga tagtaatgac     780
   gaatcaaact atcagccaat cccttga                                         807
<210> 286
   <211> 268
   <212> PRT
   <213> Phakopsora pachyrhizi
<220>
   <221> VARIANT
   <222> (1)..(268)
   <223> ta07479.01_phapa
<400> 286
   Met Gln His Leu Val Tyr Ser Ser Ser Leu Leu Ala Ser Ile Thr Thr
   1               5                   10                  15
   Leu Val Leu Ser Ile Tyr Ser Leu Asn Ala Pro Ala Trp Ile Arg Phe
               20                  25                  30
   Asp Thr Pro Ser Ser Ser Pro Leu Gln Tyr Ser Glu Ser Tyr Gly Leu
           35                  40                  45
   Lys Phe Lys Cys Gly Arg Ser Asn Ile Ala Pro Asp Phe Val Cys Arg
       50                  55                  60
   Pro Phe Pro Asp Arg Ser Arg Asp Cys Gly Ala Ser Ser Asn Ser Ile
   65                  70                  75                  80
   Leu Glu Asp Pro Phe Phe Glu Lys Leu Leu Ser Ala Asp Asp Arg Gln
                   85                  90                  95
   Leu Pro Leu Asn Arg Ser Ile His Gln Phe Leu His Arg Lys Asn Ser
               100                 105                 110
   Ser Ser Ser Ile Lys Thr Ala Pro Met Gly Ser Gly Ser Ile Glu Leu
           115                 120                 125
   Leu Glu Ser Ala Ser Ser Val Leu Asp Arg Leu Gly Ala Gln Arg Phe
       130                 135                 140
   Gly Phe Cys Glu Lys Trp Asn Ser Ala Gly Phe Cys Ala Glu Met Ser
   145                 150                 155                 160
   Ile Val Ile Gly Ala Ile Ser Ile Phe Cys Val Ser Ile Val Leu Leu
                   165                 170                 175
   Gly Asn Arg His Arg Gln Lys His Gly Trp Lys Ile Cys Ala Gly Leu
               180                 185                 190
   Ile Ala Ile His Ala Val Ser Gln Ile Thr Thr Trp Val Phe Val Leu
           195                 200                 205
   Gln Ile Phe Asn Thr Asp Asn Arg Phe Tyr Ile Gly Ser Lys Leu Ser
       210                 215                 220
   Thr Ser Phe Tyr Ile Ser Val Ala Ser Ser Met Ile Asp Leu Ile Cys
   225                 230                 235                 240
   Leu Thr Gly Leu Val Ala Ala Gly Ile Val Glu Asn Asp Asp Asp Asp
                       245                 250                 255
   Asp Ser Asn Asp Glu Ser Asn Tyr Gln Pro Ile Pro
                   260                 265
<210> 287
   <211> 801
   <212> DNA
   <213> Phakopsora pachyrhizi
<220>
   <221> misc_feature
   <222> (1)..(801)
   <223> ta09035.01_phapa
<400> 287
   atgaggttga atttattcat ttacagtaaa atgcgcttcg tactgttggt ttttttatta      60
   tcattcgctc ttcaggcgag tgttaatggt gaggacgtgg taagactgcc acaagaaagc     120
   aatctcatca actcagtttc gcgagatcaa agatcagata aattagaccc caaagtaatt     180
   caactggctt cttcacagaa tggttctccg gatggaaacc aatctgcttc aatgacttct     240
   ttaaataatt ttattaactt ttgctcttcg aaagaagcgc tcagcgcaaa attgacaaat     300
   ggaactcaat ctcctgatga aatcacttgt aatccaattc caatgggaat gattgtaccg     360
   cttaaaaacg cgccaagttg cagattccag cagccaaaaa actttgacaa acttaaagct     420
   gacactgctt ttaaaatgat tttaaaaatt aaaaaccttg agactggtag ttttgttaac     480
   ccaaagacaa attatttctc agcacctcaa gtattatcaa aaaaaactca caacgtgatt     540
   ggacatgctc acgtcgtggc gcaaaaaatt aaaagcttaa catctacgga ggtattgaga     600
   ccagataaat ttgaattttt caaaggcata gacgtttcaa ccgatgagga tggatacagc     660
   tctgtgattt tagaaaaagg tttaccggct ggggcctatc gtgtttcaac tttattatca     720
   gccgctaatc atcaaccaat cctagctggg gttgctcaaa gaggtgcttt tgatgatgta     780
   atatatttta ccgtggaata a                                               801
<210> 288
   <211> 266
   <212> PRT
   <213> Phakopsora pachyrhizi
<220>
   <221> VARIANT
   <222> (1) .. (266)
   <223> ta09035.01_phapa
<400> 288
   Met Arg Leu Asn Leu Phe Ile Tyr Ser Lys Met Arg Phe Val Leu Leu
   1               5                   10                  15
   Val Phe Leu Leu Ser Phe Ala Leu Gln Ala Ser Val Asn Gly Glu Asp
               20                  25                  30
   Val Val Arg Leu Pro Gln Glu Ser Asn Leu Ile Asn Ser Val Ser Arg
           35                  40                  45
   Asp Gln Arg Ser Asp Lys Leu Asp Pro Lys Val Ile Gln Leu Ala Ser
       50                  55                  60
   Ser Gln Asn Gly Ser Pro Asp Gly Asn Gln Ser Ala Ser Met Thr Ser
   65                  70                  75                  80
   Leu Asn Asn Phe Ile Asn Phe Cys Ser Ser Lys Glu Ala Leu Ser Ala
                   85                  90                  95
   Lys Leu Thr Asn Gly Thr Gln Ser Pro Asp Glu Ile Thr Cys Asn Pro
               100                 105                 110
   Ile Pro Met Gly Met Ile Val Pro Leu Lys Asn Ala Pro Ser Cys Arg
           115                 120                 125
   Phe Gln Gln Pro Lys Asn Phe Asp Lys Leu Lys Ala Asp Thr Ala Phe
       130                 135                 140
   Lys Met Ile Leu Lys Ile Lys Asn Leu Glu Thr Gly Ser Phe Val Asn
   145                 150                 155                 160
   Pro Lys Thr Asn Tyr Phe Ser Ala Pro Gln Val Leu Ser Lys Lys Thr
                   165                 170                 175
   His Asn Val Ile Gly His Ala His Val Val Ala Gln Lys Ile Lys Ser
               180                 185                 190
   Leu Thr Ser Thr Glu Val Leu Arg Pro Asp Lys Phe Glu Phe Phe Lys
           195                 200                 205
   Gly Ile Asp Val Ser Thr Asp Glu Asp Gly Tyr Ser Ser Val Ile Leu
       210                 215                 220
   Glu Lys Gly Leu Pro Ala Gly Ala Tyr Arg Val Ser Thr Leu Leu Ser
   225                 230                 235                 240
   Ala Ala Asn His Gln Pro Ile Leu Ala Gly Val Ala Gln Arg Gly Ala
                   245                 250                 255
   Phe Asp Asp Val Ile Tyr Phe Thr Val Glu
               260                 265
<210> 289
   <211> 801
   <212> DNA
   <213> Phakopsora pachyrhizi
<220>
   <221> misc_feature
   <222> (1)..(801)
   <223> ta09976.04_phapa
<400> 289
   atgctctctt acgctaccat tctttgcctt ttgagcgttt ctggcttggt tcggtctggt      60
   gtcgtaccga cgaagcctgg tcccggggaa acctacaacg agggggggca atgccaaatt      120
   gcatgggcct tagataccac agggacttgg aactcatttt caattgattt gatgtctggc      180
   tccaattttg ctatgcagca agtagttaat gtgttaaaaa atcaagacgg taccaaaggg      240
   ccaggaacct acagttttcc atgccctgaa gtcactccca actcagcaat atacttttat      300
   caattttctc aacataatgc tgaaactacc tggactacgc gattcactat agcttctgct      360
   gatggccaaa cgactccccc tgctaatcca aatcagccaa acggtcagcc gattccatgg      420
   ggaattggag cacttgcctc agccaatact caaaactctt ccagtgctac acctgttgtg      480
   aatactactg cgaccgtgac tccgcccctt aatggtaacc tgactgcctt gaccacaccg      540
   gcaaacacta ccagcagcag taacaatatt accaactcta atgtaaacac caccactagt      600
   gttacataca gtaatccccc ttcatctact cttggaaaat ccggtgcttc aaactcctct      660
   tcaggtacca ccccttctgc accaaaggca actggcaccg gcaaatcttc aggatctaaa      720
   actttttcta caagcggctt ttatttcttt tcatcatcat tagggcttat gctcattgga      780
   tctatttcat tactattgta a                                               801
<210> 290
   <211> 266
   <212> PRT
   <213> Phakopsora pachyrhizi
<220>
   <221> VARIANT
   <222> (1) .. (266)
   <223> ta09976.04_phapa
<400> 290
   Met Leu Ser Tyr Ala Thr Ile Leu Cys Leu Leu Ser Val Ser Gly Leu
   1               5                   10                  15
   Val Arg Ser Gly Val Val Pro Thr Lys Pro Gly Pro Gly Glu Thr Tyr
               20                  25                  30
   Asn Glu Gly Gly Gln Cys Gln Ile Ala Trp Ala Leu Asp Thr Thr Gly
           35                  40                  45
   Thr Trp Asn Ser Phe Ser Ile Asp Leu Met Ser Gly Ser Asn Phe Ala
       50                  55                  60
   Met Gln Gln Val Val Asn Val Leu Lys Asn Gln Asp Gly Thr Lys Gly
   65                  70                  75                  80
   Pro Gly Thr Tyr Ser Phe Pro Cys Pro Glu Val Thr Pro Asn Ser Ala
                   85                  90                  95
   Ile Tyr Phe Tyr Gln Phe Ser Gln His Asn Ala Glu Thr Thr Trp Thr
               100                 105                 110
   Thr Arg Phe Thr Ile Ala Ser Ala Asp Gly Gln Thr Thr Pro Pro Ala
           115                 120                 125
   Asn Pro Asn Gln Pro Asn Gly Gln Pro Ile Pro Trp Gly Ile Gly Ala
       130                 135                 140
   Leu Ala Ser Ala Asn Thr Gln Asn Ser Ser Ser Ala Thr Pro Val Val
   145                 150                 155                 160
   Asn Thr Thr Ala Thr Val Thr Pro Pro Leu Asn Gly Asn Leu Thr Ala
                   165                 170                 175
   Leu Thr Thr Pro Ala Asn Thr Thr Ser Ser Ser Asn Asn Ile Thr Asn
               180                 185                 190
   Ser Asn Val Asn Thr Thr Thr Ser Val Thr Tyr Ser Asn Pro Pro Ser
           195                 200                 205
   Ser Thr Leu Gly Lys Ser Gly Ala Ser Asn Ser Ser Ser Gly Thr Thr
       210                 215                 220
   Pro Ser Ala Pro Lys Ala Thr Gly Thr Gly Lys Ser Ser Gly Ser Lys
   225                 230                 235                 240
   Thr Phe Ser Thr Ser Gly Phe Tyr Phe Phe Ser Ser Ser Leu Gly Leu
                   245                 250                 255
   Met Leu Ile Gly Ser Ile Ser Leu Leu Leu
               260                 265
<210> 291
   <211> 801
   <212> DNA
   <213> Phakopsora pachyrhizi
<220>
   <221> misc_feature
   <222> (1)..(801)
   <223> ta09976.03_phapa
<400> 291
   atgctctctt acgctaccat tctttgcctt ttgagcgttt ctggcttggt tcggtctggt      60
   gtcgtaccga cgaagcctgg tcccggggaa acctacaacg agggggggca atgccaaatt      120
   gcatgggcct tagataccac agggacttgg aactcatttt caattgattt gatgtctggc     180
   tccaattttg ctatgcagca agtagttaat gtgttaaaaa atcaagacgg taccaaaggg     240
   ccaggaacct acagttttcc atgccctgaa gtcactccca actcagcaat atacttttat     300
   caattttctc aacataatgc tgaaactacc tggactacgc gattcactat agcttctgct      360
   gatggccaaa cgactccccc tgctaatcca aatcagccaa acggtcagcc gattccatgg     420
   ggaattggag cacttgcctc agccaatact caaaactctt ccagtgctac acctgttgtg     480
   aatactactg cgaccgtgac tccgcccctt aatggtaacc tgactgcctt gaccacaccg     540
   gcaaacacta ccagcagcag taacaatatt accaactcta atgtaaacac caccactagt     600
   gttacataca gtaatccccc ttcatctact cttggaaaat ccggtgcttc aaactcctct     660
   tcaggtacca ccccttctgc accaaaggca actggcaccg gcaaatcttc aggatctaaa      720
   actttttcta caagcggctt ttatttcttt tcatcatcat tagggcttat gctcattgga     780
   tctatttcat tactattgta a                                               801
<210> 292
   <211> 266
   <212> PRT
   <213> Phakopsora pachyrhizi
<220>
   <221> VARIANT
   <222> (1) .. (266)
   <223> ta09976.03_phapa
<400> 292
   Met Leu Ser Tyr Ala Thr Ile Leu Cys Leu Leu Ser Val Ser Gly Leu
   1               5                   10                  15
   Val Arg Ser Gly Val Val Pro Thr Lys Pro Gly Pro Gly Glu Thr Tyr
               20                  25                  30
   Asn Glu Gly Gly Gln Cys Gln Ile Ala Trp Ala Leu Asp Thr Thr Gly
           35                  40                  45
   Thr Trp Asn Ser Phe Ser Ile Asp Leu Met Ser Gly Ser Asn Phe Ala
       50                  55                  60
   Met Gln Gln Val Val Asn Val Leu Lys Asn Gln Asp Gly Thr Lys Gly
   65                  70                  75                  80
   Pro Gly Thr Tyr Ser Phe Pro Cys Pro Glu Val Thr Pro Asn Ser Ala
                   85                  90                  95
   Ile Tyr Phe Tyr Gln Phe Ser Gln His Asn Ala Glu Thr Thr Trp Thr
               100                 105                 110
   Thr Arg Phe Thr Ile Ala Ser Ala Asp Gly Gln Thr Thr Pro Pro Ala
           115                 120                 125
   Asn Pro Asn Gln Pro Asn Gly Gln Pro Ile Pro Trp Gly Ile Gly Ala
       130                 135                 140
   Leu Ala Ser Ala Asn Thr Gln Asn Ser Ser Ser Ala Thr Pro Val Val
   145                 150                 155                 160
   Asn Thr Thr Ala Thr Val Thr Pro Pro Leu Asn Gly Asn Leu Thr Ala
                   165                 170                 175
   Leu Thr Thr Pro Ala Asn Thr Thr Ser Ser Ser Asn Asn Ile Thr Asn
               180                 185                 190
   Ser Asn Val Asn Thr Thr Thr Ser Val Thr Tyr Ser Asn Pro Pro Ser
           195                 200                 205
   Ser Thr Leu Gly Lys Ser Gly Ala Ser Asn Ser Ser Ser Gly Thr Thr
       210                 215                 220
   Pro Ser Ala Pro Lys Ala Thr Gly Thr Gly Lys Ser Ser Gly Ser Lys
   225                 230                 235                 240
   Thr Phe Ser Thr Ser Gly Phe Tyr Phe Phe Ser Ser Ser Leu Gly Leu
                   245                 250                 255
   Met Leu Ile Gly Ser Ile Ser Leu Leu Leu
               260                 265
<210> 293
   <211> 801
   <212> DNA
   <213> Phakopsora pachyrhizi
<220>
   <221> misc_feature
   <222> (1)..(801)
   <223> ta09976.01_phapa
<400> 293
   atgctctctt acgctaccat tctttgcctt ttgagcgttt ctggcttggt tcggtctggt      60
   gtcgtaccga cgaagcctgg tcccggggaa acctacaacg agggggggca atgccaaatt     120
   gcatgggcct tagataccac agggacttgg aactcatttt caattgattt gatgtctggc     180
   tccaattttg ctatgcagca agtagttaat gtgttaaaaa atcaagacgg taccaaaggg     240
   ccaggaacct acagttttcc atgccctgaa gtcactccca actcagcaat atacttttat     300
   caattttctc aacataatgc tgaaactacc tggactacgc gattcactat agcttctgct     360
   gatggccaaa cgactccccc tgctaatcca aatcagccaa acggtcagcc gattccatgg     420
   ggaattggag cacttgcctc agccaatact caaaactctt ccagtgctac acctgttgtg     480
   aatactactg cgaccgtgac tccgcccctt aatggtaacc tgactgcctt gaccacaccg     540
   gcaaacacta ccagcagcag taacaatatt accaactcta atgtaaacac caccactagt     600
   gttacataca gtaatccccc ttcatctact cttggaaaat ccggtgcttc aaactcctct     660
   tcaggtacca ccccttctgc accaaaggca actggcaccg gcaaatcttc aggatctaaa     720
   actttttcta caagcggctt ttatttcttt tcatcatcat tagggcttat gctcattgga     780
   tctatttcat tactattgta a                                               801
<210> 294
   <211> 266
   <212> PRT
   <213> Phakopsora pachyrhizi
<220>
   <221> VARIANT
   <222> (1) .. (266)
   <223> ta09976.01_phapa
<400> 294
   Met Leu Ser Tyr Ala Thr Ile Leu Cys Leu Leu Ser Val Ser Gly Leu
   1               5                   10                  15
   Val Arg Ser Gly Val Val Pro Thr Lys Pro Gly Pro Gly Glu Thr Tyr
                   20                      25                      30
   Asn Glu Gly Gly Gln Cys Gln Ile Ala Trp Ala Leu Asp Thr Thr Gly
               35                      40                      45
   Thr Trp Asn Ser Phe Ser Ile Asp Leu Met Ser Gly Ser Asn Phe Ala
       50                      55                      60
   Met Gln Gln Val Val Asn Val Leu Lys Asn Gln Asp Gly Thr Lys Gly
   65                      70                      75                      80
   Pro Gly Thr Tyr Ser Phe Pro Cys Pro Glu Val Thr Pro Asn Ser Ala
                       85                      90                      95
   Ile Tyr Phe Tyr Gln Phe Ser Gln His Asn Ala Glu Thr Thr Trp Thr
                   100                     105                     110
   Thr Arg Phe Thr Ile Ala Ser Ala Asp Gly Gln Thr Thr Pro Pro Ala
               115                     120                     125
   Asn Pro Asn Gln Pro Asn Gly Gln Pro Ile Pro Trp Gly Ile Gly Ala
       130                     135                     140
   Leu Ala Ser Ala Asn Thr Gln Asn Ser Ser Ser Ala Thr Pro Val Val
   145                     150                     155                     160
   Asn Thr Thr Ala Thr Val Thr Pro Pro Leu Asn Gly Asn Leu Thr Ala
                       165                     170                     175
   Leu Thr Thr Pro Ala Asn Thr Thr Ser Ser Ser Asn Asn Ile Thr Asn
                   180                     185                     190
   Ser Asn Val Asn Thr Thr Thr Ser Val Thr Tyr Ser Asn Pro Pro Ser
               195                     200                     205
   Ser Thr Leu Gly Lys Ser Gly Ala Ser Asn Ser Ser Ser Gly Thr Thr
       210                     215                     220
   Pro Ser Ala Pro Lys Ala Thr Gly Thr Gly Lys Ser Ser Gly Ser Lys
   225                     230                     235                     240
   Thr Phe Ser Thr Ser Gly Phe Tyr Phe Phe Ser Ser Ser Leu Gly Leu
                       245                     250                     255
   Met Leu Ile Gly Ser Ile Ser Leu Leu Leu
                   260                     265
<210> 295
   <211> 795
   <212> DNA
   <213> Phakopsora pachyrhizi
<220>
   <221> misc_feature
   <222> (1)..(795)
   <223> ta07155.01_phapa
<400> 295
   atgaactcca agtctaccct cgcgatactt gtcgtggtca ccgccagttt gttaccatac      60
   agctggggat ttaatcagga tagctcagaa actcaaacat ttgagcatcc ttcctccgcg     120
   aaggaatcac aatcttcatt cagttatgag agtaatgaaa gcaagtcatc ttactcaagc     180
   aatcaaatat cgacaaaaaa tcaatatggg cctagtagta gcttaggagg aagcactgaa     240
   gacggctttg atcctgaatt cgatccaaca gaggaagaca caattacagc agagactcca     300
   actaaaacaa tcttttgtcc aggaccaatt aataccggaa cgggagaggg tgtatggata     360
   gatggacatt gtgaaattat gtgcttcaac aatctcgtac tggatggaga tcgatgtacc     420
   tgccccccaa cctatcactt tgaccacaaa aatgtgaaat gcgtttgcag acctccactc     480
   tgtgaacaag gcggtaagtg tatcttgaag ccatcgcaat atcctggcgt ccacaactct     540
   gcccatagaa agaggtcaat gcctgcgcag cttcgattaa ccccacaagt ttataatgga     600
   aatcatgctc gaacatcttt cgatgataag cactgcattt caaatgagat cgcctgtcgc     660
   attggtagta tgactggtgg agttcaatgc gttgatccca caagtgacct ggaacactgt     720
   ggggggtgct ccaacaccac agaaggcata aactgcaatc aaattcccgg ggtagaaaac     780
   gctggatgca atcag                                                      795
<210> 296
   <211> 265
   <212> PRT
   <213> Phakopsora pachyrhizi
<220>
   <221> VARIANT
   <222> (1)..(265)
   <223> ta07155.01_phapa
<400> 296
   Met Asn Ser Lys Ser Thr Leu Ala Ile Leu Val Val Val Thr Ala Ser
   1               5                   10                  15
   Leu Leu Pro Tyr Ser Trp Gly Phe Asn Gln Asp Ser Ser Glu Thr Gln
               20                  25                  30
   Thr Phe Glu His Pro Ser Ser Ala Lys Glu Ser Gln Ser Ser Phe Ser
           35                  40                  45
   Tyr Glu Ser Asn Glu Ser Lys Ser Ser Tyr Ser Ser Asn Gln Ile Ser
       50                  55                  60
   Thr Lys Asn Gln Tyr Gly Pro Ser Ser Ser Leu Gly Gly Ser Thr Glu
   65                  70                  75                  80
   Asp Gly Phe Asp Pro Glu Phe Asp Pro Thr Glu Glu Asp Thr Ile Thr
                   85                  90                  95
   Ala Glu Thr Pro Thr Lys Thr Ile Phe Cys Pro Gly Pro Ile Asn Thr
               100                 105                 110
   Gly Thr Gly Glu Gly Val Trp Ile Asp Gly His Cys Glu Ile Met Cys
           115                 120                 125
   Phe Asn Asn Leu Val Leu Asp Gly Asp Arg Cys Thr Cys Pro Pro Thr
       130                 135                 140
   Tyr His Phe Asp His Lys Asn Val Lys Cys Val Cys Arg Pro Pro Leu
   145                 150                 155                 160
   Cys Glu Gln Gly Gly Lys Cys Ile Leu Lys Pro Ser Gln Tyr Pro Gly
                   165                 170                 175
   Val His Asn Ser Ala His Arg Lys Arg Ser Met Pro Ala Gln Leu Arg
               180                 185                 190
   Leu Thr Pro Gln Val Tyr Asn Gly Asn His Ala Arg Thr Ser Phe Asp
           195                 200                 205
   Asp Lys His Cys Ile Ser Asn Glu Ile Ala Cys Arg Ile Gly Ser Met
       210                 215                 220
   Thr Gly Gly Val Gln Cys Val Asp Pro Thr Ser Asp Leu Glu His Cys
   225                 230                 235                 240
   Gly Gly Cys Ser Asn Thr Thr Glu Gly Ile Asn Cys Asn Gln Ile Pro
                   245                 250                 255
   Gly Val Glu Asn Ala Gly Cys Asn Gln
               260                 265
<210> 297
   <211> 786
   <212> DNA
   <213> Phakopsora pachyrhizi
<220>
   <221> misc_feature
   <222> (1)..(786)
   <223> ta02824.01_phapa
<400> 297
   atgaagtttt ttctggctct ctcaatttcg gttggattac tctcagccgc cctcgtttca        60
   gctgatgtag ttgaagaagg tatctggcaa cgccgaccaa acgctgaatt aaaatcgagt       120
   cgcggtaatt caattaaagc aagaaaccca gtatcagcta aagccttgag tcgagatgaa       180
   tctagtgcgg attatggttt ggccgatgac gatgagagtg attacgtctc aggagtttat       240
   gagaaactga gttcgatgct agaaaagatt cagccttcgg ttgaccaaat cgcagcttta       300
   tctagtaacg ccaatgataa gaacgcggac gaatattctg aaaaaatatc taagcacttg       360
   gacttcatcg ttgctgctgg agattctgtt gttaatgaaa tacaaactga agaaacttct       420
   ccaattgaga ccgaagttaa taatggtatt gagccccagt ggtgtcaccg ttgccacaag       480
   aaaaaacatc acaaacaccg acactgccat gagtgcaaaa agcgcgacca cgatgaatgt       540
   ggccatcttt tgaagaagtg ggtttatgcc tgcaaagtta gcacttctgc agtcctgact       600
   tgcaagacca aaaaggtgaa agacaagtgc gaagaacacg tccctaaggt aactaaatgt       660
   gctacccaag ttgtcaacag ctgtggaaaa tacggaattt ctcaagcgtt ccgctcagtt       720
   gcacaatctg agattgatgc gttttcaaaa attgggtatg gctcctcggc catttctgag       780
   gcatag                                                                  786
<210> 298
   <211> 261
   <212> PRT
   <213> Phakopsora pachyrhizi
<220>
   <221> VARIANT
   <222> (1)..(261)
   <223> ta02824.01_phapa
<400> 298
   Met Lys Phe Phe Leu Ala Leu Ser Ile Ser Val Gly Leu Leu Ser Ala
   1               5                   10                  15
   Ala Leu Val Ser Ala Asp Val Val Glu Glu Gly Ile Trp Gln Arg Arg
               20                  25                  30
   Pro Asn Ala Glu Leu Lys Ser Ser Arg Gly Asn Ser Ile Lys Ala Arg
           35                  40                  45
   Asn Pro Val Ser Ala Lys Ala Leu Ser Arg Asp Glu Ser Ser Ala Asp
       50                  55                  60
   Tyr Gly Leu Ala Asp Asp Asp Glu Ser Asp Tyr Val Ser Gly Val Tyr
   65                  70                  75                  80
   Glu Lys Leu Ser Ser Met Leu Glu Lys Ile Gln Pro Ser Val Asp Gln
                   85                  90                  95
   Ile Ala Ala Leu Ser Ser Asn Ala Asn Asp Lys Asn Ala Asp Glu Tyr
               100                 105                 110
   Ser Glu Lys Ile Ser Lys His Leu Asp Phe Ile Val Ala Ala Gly Asp
           115                 120                 125
   Ser Val Val Asn Glu Ile Gln Thr Glu Glu Thr Ser Pro Ile Glu Thr
       130                 135                 140
   Glu Val Asn Asn Gly Ile Glu Pro Gln Trp Cys His Arg Cys His Lys
   145                 150                 155                 160
   Lys Lys His His Lys His Arg His Cys His Glu Cys Lys Lys Arg Asp
                   165                 170                 175
   His Asp Glu Cys Gly His Leu Leu Lys Lys Trp Val Tyr Ala Cys Lys
               180                 185                 190
   Val Ser Thr Ser Ala Val Leu Thr Cys Lys Thr Lys Lys Val Lys Asp
           195                 200                 205
   Lys Cys Glu Glu His Val Pro Lys Val Thr Lys Cys Ala Thr Gln Val
       210                 215                 220
   Val Asn Ser Cys Gly Lys Tyr Gly Ile Ser Gln Ala Phe Arg Ser Val
   225                 230                 235                 240
   Ala Gln Ser Glu Ile Asp Ala Phe Ser Lys Ile Gly Tyr Gly Ser Ser
                   245                 250                 255
   Ala Ile Ser Glu Ala
               260
<210> 299
   <211> 777
   <212> DNA
   <213> Phakopsora pachyrhizi
<220>
   <221> misc_feature
   <222> (1)..(777)
   <223> ta09817.01_phapa
<400> 299
   atgtttaaga agagtactat gattcttact tttctctttt tggctttaca cgggactctg      60
   tcttcaccag tgccagattc tttgacctct aatgataata acgatataaa ctttagctca     120
   gaaatatttg cgcgagctgt ccaggatgaa tatccacaag agcaggagaa ctcaaggagt     180
   ggcataccag ggaacgaatt tgaaaatagc aacgtagtca ataatgcaag agacccagca     240
   gagttgaacg agcagaatga acaaattcgc ggtgataatc aacctactac taactctaat     300
   attcaactca atcttaaatc aagcaccgag gacaagacgg caaaagattc accaagaaaa     360
   aggtctccag aatcctttga tacaactagc agcgtacaac cttctcccag agaatcaaat     420
   ggggaaatca caaatcctca agtcaatgat gtttctcaca gcgatcagat taactcaggt      480
   aactctccga ctgagggctt tcatccagga gatttgaaca cgagcggaga tcaagggttg      540
   tctaaaatct ggccaaacaa ggatgaaccc caagtgaact atgatcctca caaagatcaa      600
   acaacctcag aaagttttcc gacacagggc tctactcaag gtaattttca acctcagcca      660
   tctagggata ccactcagat taacgaaaat ccttcgattg atcaaacaaa ctcaggtaca      720
   actactccaa atccagtcaa ttctcaagat ggtaataagc cacatggtga taaatga         777
<210> 300
   <211> 258
   <212> PRT
   <213> Phakopsora pachyrhizi
<220>
   <221> VARIANT
   <222> (1)..(258)
   <223> ta09817.01_phapa
<400> 300
   Met Phe Lys Lys Ser Thr Met Ile Leu Thr Phe Leu Phe Leu Ala Leu
   1               5                   10                  15
   His Gly Thr Leu Ser Ser Pro Val Pro Asp Ser Leu Thr Ser Asn Asp
               20                  25                  30
   Asn Asn Asp Ile Asn Phe Ser Ser Glu Ile Phe Ala Arg Ala Val Gln
           35                  40                  45
   Asp Glu Tyr Pro Gln Glu Gln Glu Asn Ser Arg Ser Gly Ile Pro Gly
       50                  55                  60
   Asn Glu Phe Glu Asn Ser Asn Val Val Asn Asn Ala Arg Asp Pro Ala
   65                  70                  75                  80
   Glu Leu Asn Glu Gln Asn Glu Gln Ile Arg Gly Asp Asn Gln Pro Thr
                   85                  90                  95
   Thr Asn Ser Asn Ile Gln Leu Asn Leu Lys Ser Ser Thr Glu Asp Lys
               100                 105                 110
   Thr Ala Lys Asp Ser Pro Arg Lys Arg Ser Pro Glu Ser Phe Asp Thr
           115                 120                 125
   Thr Ser Ser Val Gln Pro Ser Pro Arg Glu Ser Asn Gly Glu Ile Thr
       130                 135                 140
   Asn Pro Gln Val Asn Asp Val Ser His Ser Asp Gln Ile Asn Ser Gly
   145                 150                 155                 160
   Asn Ser Pro Thr Glu Gly Phe His Pro Gly Asp Leu Asn Thr Ser Gly
                   165                 170                 175
   Asp Gln Gly Leu Ser Lys Ile Trp Pro Asn Lys Asp Glu Pro Gln Val
               180                 185                 190
   Asn Tyr Asp Pro His Lys Asp Gln Thr Thr Ser Glu Ser Phe Pro Thr
           195                 200                 205
   Gln Gly Ser Thr Gln Gly Asn Phe Gln Pro Gln Pro Ser Arg Asp Thr
       210                 215                 220
   Thr Gln Ile Asn Glu Asn Pro Ser Ile Asp Gln Thr Asn Ser Gly Thr
   225                 230                 235                 240
   Thr Thr Pro Asn Pro Val Asn Ser Gln Asp Gly Asn Lys Pro His Gly
                   245                 250                 255
   Asp Lys
<210> 301
   <211> 771
   <212> DNA
   <213> Phakopsora pachyrhizi
<220>
   <221> misc_feature
   <222> (1)..(771)
   <223> ta08887.01_phapa
<400> 301
   atgtcttttc gtctcatatc tacgagaagg cttcacgtac tatactcaac ccctcttttg        60
   ctcagcctag catgtataat acttatacta tcatcgctgt gcgggcttgt tccatggcat       120
   cacacctctg gttcaatttt aattgttaaa aaaacctctc aaaatagtac tcttgtcgaa       180
   aaatcaaatg aaacggcatc attttatttc ggattgcttg gttcttgtta tcgaccttca       240
   aaggcagaaa aactacagtg cacctcttca gcattcccac ctgatctatt agctactatc       300
   gagaaggggg gtagtgggat caacgctcct catctacaac tagctttgcc tgagctgccc       360
   ccagttttca tgtcctgggc agcagtttcg cttttggcac tcgtatgcga aatcctaggt       420
   agccttccat tttacattcc taggagattc ggtcattgta gtcgtttttg ctatggattt       480
   tttactgctg cactatggat tcttgggttg ggctgggctc ttggattctc ggccgttcta       540
   gccctagcgt gctttgctca gagctttggg gaagagtaca atgcattttc tgcagacttc       600
   ttttacaata cagccacgcc aggatcaata ttcgttccgc aagtaattgc gctagtcgtt       660
   caaatattaa ttggaatagc tacaattgtc aaaatcgaaa attcttcaaa aaaagactca       720
   ttcattaatt catcaacgtg gtctaacaat ctaagagtaa ctggtccata a                771
<210> 302
   <211> 256
   <212> PRT
   <213> Phakopsora pachyrhizi
<220>
   <221> VARIANT
   <222> (1) ..(256)
   <223> ta08887.01_phapa
<400> 302
   Met Ser Phe Arg Leu Ile Ser Thr Arg Arg Leu His Val Leu Tyr Ser
   1               5                   10                  15
   Thr Pro Leu Leu Leu Ser Leu Ala Cys Ile Ile Leu Ile Leu Ser Ser
               20                  25                  30
   Leu Cys Gly Leu Val Pro Trp His His Thr Ser Gly Ser Ile Leu Ile
           35                  40                  45
   Val Lys Lys Thr Ser Gln Asn Ser Thr Leu Val Glu Lys Ser Asn Glu
       50                  55                  60
   Thr Ala Ser Phe Tyr Phe Gly Leu Leu Gly Ser Cys Tyr Arg Pro Ser
   65                  70                  75                  80
   Lys Ala Glu Lys Leu Gln Cys Thr Ser Ser Ala Phe Pro Pro Asp Leu
                   85                  90                  95
   Leu Ala Thr Ile Glu Lys Gly Gly Ser Gly Ile Asn Ala Pro His Leu
               100                 105                 110
   Gln Leu Ala Leu Pro Glu Leu Pro Pro Val Phe Met Ser Trp Ala Ala
           115                 120                 125
   Val Ser Leu Leu Ala Leu Val Cys Glu Ile Leu Gly Ser Leu Pro Phe
       130                 135                 140
   Tyr Ile Pro Arg Arg Phe Gly His Cys Ser Arg Phe Cys Tyr Gly Phe
   145                 150                 155                 160
   Phe Thr Ala Ala Leu Trp Ile Leu Gly Leu Gly Trp Ala Leu Gly Phe
                   165                 170                 175
   Ser Ala Val Leu Ala Leu Ala Cys Phe Ala Gln Ser Phe Gly Glu Glu
               180                 185                 190
   Tyr Asn Ala Phe Ser Ala Asp Phe Phe Tyr Asn Thr Ala Thr Pro Gly
           195                 200                 205
   Ser Ile Phe Val Pro Gln Val Ile Ala Leu Val Val Gln Ile Leu Ile
       210                 215                 220
   Gly Ile Ala Thr Ile Val Lys Ile Glu Asn Ser Ser Lys Lys Asp Ser
   225                 230                 235                 240
   Phe Ile Asn Ser Ser Thr Trp Ser Asn Asn Leu Arg Val Thr Gly Pro
                   245                 250                 255
<210> 303
   <211> 768
   <212> DNA
   <213> Phakopsora pachyrhizi
<220>
   <221> misc_feature
   <222> (1)..(768)
   <223> ta03435.01_phapa
<400> 303
   atgttttcta aatcatttca aggattgagc ctactcgtag ttctgatggc ctctattatc      60
   tttaactgtg atgaatcaaa cgcccagagt aactttaact cttttcaaca accaggcacg     120
   aactgttacg aacagtcaaa atcctatgga atcaatttcg aacattgtga aaaagctcta     180
   aggaaaattt catacgattc taatggtaac ttggataatt catccaagac ggtttttgtc     240
   tggcataaat cttgtgtggt caaagttcaa aaggcaacat acgctcaacc atcgagacaa     300
   caagtagaat atggtgttag aaacttacta cagacttgtc ctacgagggg agggatttat     360
   atcccttcaa atgactttag gacctatgtt tactcttcaa atcgtgaaaa cgtttacaat     420
   ttgaacagcc cagtgtgcat taagcaccaa tgtcatatca accctaatga ctgtttgatg     480
   gcctttaaca acattcctct tagtactaaa ggttttttct taccatctgg ttcaacctct     540
   tctaatgtta taaagacttc tagtggtaac tgcactgtta aacttgtcac tactgatggt     600
   gcaggattca gaattaacca tcctgaaatt aactctggta tcaaaactct actctcaaaa     660
   tgtggtagca gacctggtta caactatttc ggtggtggca gtctaggaat gaatggtgat     720
   attaaaatca tcactcaaaa cagttacaac aacggtcaat gtaattaa                  768
<210> 304
   <211> 255
   <212> PRT
   <213> Phakopsora pachyrhizi
<220>
   <221> VARIANT
   <222> (1)..(255)
   <223> ta03435.01_phapa
<400> 304
   Met Phe Ser Lys Ser Phe Gln Gly Leu Ser Leu Leu Val Val Leu Met
   1               5                   10                  15
   Ala Ser Ile Ile Phe Asn Cys Asp Glu Ser Asn Ala Gln Ser Asn Phe
               20                  25                  30
   Asn Ser Phe Gln Gln Pro Gly Thr Asn Cys Tyr Glu Gln Ser Lys Ser
           35                  40                  45
   Tyr Gly Ile Asn Phe Glu His Cys Glu Lys Ala Leu Arg Lys Ile Ser
       50                  55                  60
   Tyr Asp Ser Asn Gly Asn Leu Asp Asn Ser Ser Lys Thr Val Phe Val
   65                  70                  75                  80
   Trp His Lys Ser Cys Val Val Lys Val Gln Lys Ala Thr Tyr Ala Gln
                   85                  90                  95
   Pro Ser Arg Gln Gln Val Glu Tyr Gly Val Arg Asn Leu Leu Gln Thr
               100                 105                 110
   Cys Pro Thr Arg Gly Gly Ile Tyr Ile Pro Ser Asn Asp Phe Arg Thr
           115                 120                 125
   Tyr Val Tyr Ser Ser Asn Arg Glu Asn Val Tyr Asn Leu Asn Ser Pro
       130                 135                 140
   Val Cys Ile Lys His Gln Cys His Ile Asn Pro Asn Asp Cys Leu Met
   145                 150                 155                 160
   Ala Phe Asn Asn Ile Pro Leu Ser Thr Lys Gly Phe Phe Leu Pro Ser
                   165                 170                 175
   Gly Ser Thr Ser Ser Asn Val Ile Lys Thr Ser Ser Gly Asn Cys Thr
               180                 185                 190
   Val Lys Leu Val Thr Thr Asp Gly Ala Gly Phe Arg Ile Asn His Pro
           195                 200                 205
   Glu Ile Asn Ser Gly Ile Lys Thr Leu Leu Ser Lys Cys Gly Ser Arg
       210                 215                 220
   Pro Gly Tyr Asn Tyr Phe Gly Gly Gly Ser Leu Gly Met Asn Gly Asp
   225                 230                 235                 240
   Ile Lys Ile Ile Thr Gln Asn Ser Tyr Asn Asn Gly Gln Cys Asn
                   245                 250                 255
<210> 305
   <211> 765
   <212> DNA
   <213> Phakopsora pachyrhizi
<220>
   <221> misc_feature
   <222> (1)..(765)
   <223> ta05344.01_phapa
<400> 305
   atgaacatga aacttagagt attcatttcc ttaactgttg cgctttcttt gattgctcaa        60
   tccccaagcc tagctgtaac tttaaataga gatcggcctt caaatgccca tgcgctccag       120
   gctagacagg aacaagctgc agagctagct gctgtcacgg taccggaaga acaagctgcc       180
   gctagtgctc ccgagcagaa ggctgctgct gctgcctccg ctccagagca cgctgctgct       240
   tcctccgctc ccgagcacgc tgcttctgct gccgctcctc aaagtgacct agacaaagct       300
   gccaatagat tgtacgactt aatggtaaac cttggaaatg gattggagat cgtcacaaat       360
   cttgaggcca gtccacagga tatcaaggcc cagactcaaa aagtcaaaca atattttggt       420
   gaaatgaatg agctaagaaa caagttgctc cagatgagcc ccatacattc tggaaacctc       480
   cccaaatctg ttcaagatgc caatgaagct caggaaacat tacagaaagc gttgattgct       540
   attagtgatt cggcagaaga cgcaaatgtt ctaaaaaaga actacccagt cctttcgaag       600
   acttttaaat ccgtaactac tgctggagaa gatctgttct caactttgta ccctggctat       660
   ggtgagaaaa ctcaggaagg acaagatgct caacaacaca gccagcccca agcaacgcaa       720
   gagggtgctg ctcctgtagc tgctgctgaa gcccctcaag cttaa                       765
<210> 306
   <211> 254
   <212> PRT
   <213> Phakopsora pachyrhizi
<220>
   <221> VARIANT
   <222> (1)..(254)
   <223> ta05344.01_phapa
<400> 306
   Met Asn Met Lys Leu Arg Val Phe Ile Ser Leu Thr Val Ala Leu Ser
   1               5                   10                  15
   Leu Ile Ala Gln Ser Pro Ser Leu Ala Val Thr Leu Asn Arg Asp Arg
               20                  25                  30
   Pro Ser Asn Ala His Ala Leu Gln Ala Arg Gln Glu Gln Ala Ala Glu
           35                  40                  45
   Leu Ala Ala Val Thr Val Pro Glu Glu Gln Ala Ala Ala Ser Ala Pro
       50                  55                  60
   Glu Gln Lys Ala Ala Ala Ala Ala Ser Ala Pro Glu His Ala Ala Ala
   65                  70                  75                  80
   Ser Ser Ala Pro Glu His Ala Ala Ser Ala Ala Ala Pro Gln Ser Asp
                   85                  90                  95
   Leu Asp Lys Ala Ala Asn Arg Leu Tyr Asp Leu Met Val Asn Leu Gly
               100                 105                 110
   Asn Gly Leu Glu Ile Val Thr Asn Leu Glu Ala Ser Pro Gln Asp Ile
           115                 120                 125
   Lys Ala Gln Thr Gln Lys Val Lys Gln Tyr Phe Gly Glu Met Asn Glu
       130                 135                 140
   Leu Arg Asn Lys Leu Leu Gln Met Ser Pro Ile His Ser Gly Asn Leu
   145                 150                 155                 160
   Pro Lys Ser Val Gln Asp Ala Asn Glu Ala Gln Glu Thr Leu Gln Lys
                   165                 170                 175
   Ala Leu Ile Ala Ile Ser Asp Ser Ala Glu Asp Ala Asn Val Leu Lys
               180                 185                 190
   Lys Asn Tyr Pro Val Leu Ser Lys Thr Phe Lys Ser Val Thr Thr Ala
           195                 200                 205
   Gly Glu Asp Leu Phe Ser Thr Leu Tyr Pro Gly Tyr Gly Glu Lys Thr
       210                 215                 220
   Gln Glu Gly Gln Asp Ala Gln Gln His Ser Gln Pro Gln Ala Thr Gln
   225                 230                 235                 240
   Glu Gly Ala Ala Pro Val Ala Ala Ala Glu Ala Pro Gln Ala
                   245                 250
<210> 307
   <211> 762
   <212> DNA
   <213> Phakopsora pachyrhizi
<220>
   <221> misc_feature
   <222> (1)..(762)
   <223> ta01958.01_phapa
<400> 307
   atgaatcgat tggtcaggct gatactactg ctatcatcat ctctgacgct ctggatgata      60
   aacctctttg gttcacaggt tatggcttca tccagactgt tgggtagagt gactctaaac     120
   gataaactaa cagatatcaa tctgattcct actgattcaa gagtctcgat cagtaacggt     180
   gatttaacca ctagggttca tatcagatca gatggtacat tcgtatttca agatctaaag     240
   ccaggaagat acatcctcag ggtacagtgt cgaaggttca gcttcccaat gctaaaggtc     300
   agactgtcgg atgatgacca gggctcgatt ccgatcgtca gcccatactc catctcacag     360
   gcagaacctg accctagggc aaaccacttc gaacataagc tccaacaccc gattcagata     420
   tctccaatct ctatcctcga gtactacgag attccagtag gcttcaatcc actctcgatc     480
   ttactcggaa accccatgta cctgttgatg ggaggaatgg tcatcttcat gatcctgatg     540
   cctaaactct taaacctgtt ggatcctgat gcattggccg aactccagga gaaccagtca     600
   aacatgcaca aacagatgag tctgattcaa aacatggacc taacctctgg catatctaac     660
   attctatcgc aacagtcaga ggaagatgaa aagaggacta gtactcaggg gagaatcagt     720
   agtgataaac aaaaggatgc ctcaatcaaa aggagaagat ga                        762
<210> 308
   <211> 253
   <212> PRT
   <213> Phakopsora pachyrhizi
<220>
   <221> VARIANT
   <222> (1)..(253)
   <223> ta01958.01_phapa
<400> 308
   Met Asn Arg Leu Val Arg Leu Ile Leu Leu Leu Ser Ser Ser Leu Thr
   1               5                   10                  15
   Leu Trp Met Ile Asn Leu Phe Gly Ser Gln Val Met Ala Ser Ser Arg
               20                  25                  30
   Leu Leu Gly Arg Val Thr Leu Asn Asp Lys Leu Thr Asp Ile Asn Leu
           35                  40                  45
   Ile Pro Thr Asp Ser Arg Val Ser Ile Ser Asn Gly Asp Leu Thr Thr
       50                  55                  60
   Arg Val His Ile Arg Ser Asp Gly Thr Phe Val Phe Gln Asp Leu Lys
   65                  70                  75                  80
   Pro Gly Arg Tyr Ile Leu Arg Val Gln Cys Arg Arg Phe Ser Phe Pro
                   85                  90                  95
   Met Leu Lys Val Arg Leu Ser Asp Asp Asp Gln Gly Ser Ile Pro Ile
               100                 105                 110
   Val Ser Pro Tyr Ser Ile Ser Gln Ala Glu Pro Asp Pro Arg Ala Asn
           115                 120                 125
   His Phe Glu His Lys Leu Gln His Pro Ile Gln Ile Ser Pro Ile Ser
       130                 135                 140
   Ile Leu Glu Tyr Tyr Glu Ile Pro Val Gly Phe Asn Pro Leu Ser Ile
   145                 150                 155                 160
   Leu Leu Gly Asn Pro Met Tyr Leu Leu Met Gly Gly Met Val Ile Phe
                   165                 170                 175
   Met Ile Leu Met Pro Lys Leu Leu Asn Leu Leu Asp Pro Asp Ala Leu
               180                 185                 190
   Ala Glu Leu Gln Glu Asn Gln Ser Asn Met His Lys Gln Met Ser Leu
           195                 200                 205
   Ile Gln Asn Met Asp Leu Thr Ser Gly Ile Ser Asn Ile Leu Ser Gln
       210                 215                 220
   Gln Ser Glu Glu Asp Glu Lys Arg Thr Ser Thr Gln Gly Arg Ile Ser
   225                 230                 235                 240
   Ser Asp Lys Gln Lys Asp Ala Ser Ile Lys Arg Arg Arg
                   245                 250
<210> 309
   <211> 750
   <212> DNA
   <213> Phakopsora pachyrhizi
<220>
   <221> misc_feature
   <222> (1)..(750)
   <223> ta10330.02_phapa
<400> 309
   atgaagtgca ctcttagctc tgtaatattt ataggattta tattgtttac agcgactgga      60
   tgtgcacaca ctgcggtaca aaattctcat ttcgataaat ttagagacgc attcagcgcc     120
   agtgggttgg ttcctaattt gattccaagt tttgatccca aggcaataat gattcttcga     180
   ttcgaagatg gaactaatgt ccgtcatcct gatagcagaa tcaaaacact ctcagcccac     240
   acgccacctg ttattgaagt tcatccgtac gatttcggaa ggctaagaga tgatcaaaaa     300
   tttaccctct taattgtcag cacaaattac tcggtccgcc caagctcatt gggggtcaag     360
   ctacatcttc tggggactaa ttttgccctt cgacgaatag aatcaccgga ggagatttac     420
   aacttagtga acgagactaa atttgctgtt gaatatcgag ctcctgattc aacagaatca     480
   aggcaataca atgttctttt gtacgatggg gttgcatctt cctcacctct tcaggaagtt     540
   caggaccata aagtagatca taacaatttt aatgtgacac atttcacatc taatgcgggc     600
   ctatcaccct cgccgtattc tggggcaatc tttggactaa gtgaaaatgc tcagaactct     660
   gccaatgaag catcaagagc tcaaggactc gcaaaatcaa gtataatctt ggttttatca     720
   ttcataaatc tcttcatgtt tggaaattga                                      750
<210> 310
   <211> 249
   <212> PRT
   <213> Phakopsora pachyrhizi
<220>
   <221> VARIANT
   <222> (1) .. (249)
   <223> ta10330.02_phapa
<400> 310
   Met Lys Cys Thr Leu Ser Ser Val Ile Phe Ile Gly Phe Ile Leu Phe
   1               5                   10                  15
   Thr Ala Thr Gly Cys Ala His Thr Ala Val Gln Asn Ser His Phe Asp
               20                  25                  30
   Lys Phe Arg Asp Ala Phe Ser Ala Ser Gly Leu Val Pro Asn Leu Ile
           35                  40                  45
   Pro Ser Phe Asp Pro Lys Ala Ile Met Ile Leu Arg Phe Glu Asp Gly
       50                  55                  60
   Thr Asn Val Arg His Pro Asp Ser Arg Ile Lys Thr Leu Ser Ala His
   65                  70                  75                  80
   Thr Pro Pro Val Ile Glu Val His Pro Tyr Asp Phe Gly Arg Leu Arg
                   85                  90                  95
   Asp Asp Gln Lys Phe Thr Leu Leu Ile Val Ser Thr Asn Tyr Ser Val
               100                 105                 110
   Arg Pro Ser Ser Leu Gly Val Lys Leu His Leu Leu Gly Thr Asn Phe
           115                 120                 125
   Ala Leu Arg Arg Ile Glu Ser Pro Glu Glu Ile Tyr Asn Leu Val Asn
       130                 135                 140
   Glu Thr Lys Phe Ala Val Glu Tyr Arg Ala Pro Asp Ser Thr Glu Ser
   145                 150                 155                 160
   Arg Gln Tyr Asn Val Leu Leu Tyr Asp Gly Val Ala Ser Ser Ser Pro
                   165                 170                 175
   Leu Gln Glu Val Gln Asp His Lys Val Asp His Asn Asn Phe Asn Val
               180                 185                 190
   Thr His Phe Thr Ser Asn Ala Gly Leu Ser Pro Ser Pro Tyr Ser Gly
           195                 200                 205
   Ala Ile Phe Gly Leu Ser Glu Asn Ala Gln Asn Ser Ala Asn Glu Ala
       210                 215                 220
   Ser Arg Ala Gln Gly Leu Ala Lys Ser Ser Ile Ile Leu Val Leu Ser
   225                 230                 235                 240
   Phe Ile Asn Leu Phe Met Phe Gly Asn
                   245
<210> 311
   <211> 726
   <212> DNA
   <213> Phakopsora pachyrhizi
<220>
   <221> misc_feature
   <222> (1)..(726)
   <223> ta00415.01_phapa
<400> 311
   atgcaattcc tcatcgctgc ttctcttgtg ttcctcgctc tccagggggt ttctgcctct      60
   gatgaaaaag ctaccgtccc aaccgtctca agaaagccga atacgttacc tcacacgttt     120
   tgttataatt tttttttaca aaaagactcg tgtgtcattg gttctccaat taaagaagac      180
   cgatgtgatc cagataaaaa ggacccaaag aaatctgaga aatgtaacaa agttgaacac     240
   ccaaacaagc gtcatgctaa acagcgtgtg ctaacgaaaa gatacgatga cgaaggtagt     300
   tcttttttca taaacagtgg aagtgggatc tgtggtgttt atgacagtaa ccaacctgga      360
   gcttgcctat ttagtggtta cgatgatagc ggagcaaact cagctctagc aggatggttg     420
   aacggaaatc aaacctccaa ctgcggaaag caaatctata taatgaggca gtcggatgcc     480
   aatttagcta aagaaaagag gaatgttcaa tacgcgcccg ttgttgatgg ctgtagtttt     540
   aaccttggcg ggaattcggc taagaagaat ggagacggct gtttcagaat tggtgtcaca     600
   aatcatactt tctttgccct taaccccact gcgggagaga ttcaaaacgg aacaatctcc     660
   caactccttt gggattttga tgctgaatcc agcgacgata aggcaaagga gaacggacct     720
   ttttaa                                                                726
<210> 312
   <211> 241
   <212> PRT
   <213> Phakopsora pachyrhizi
<220>
   <221> VARIANT
   <222> (1)..(241)
   <223> ta00415.01_phapa
<400> 312
   Met Gln Phe Leu Ile Ala Ala Ser Leu Val Phe Leu Ala Leu Gln Gly
   1               5                   10                  15
   Val Ser Ala Ser Asp Glu Lys Ala Thr Val Pro Thr Val Ser Arg Lys
               20                  25                  30
   Pro Asn Thr Leu Pro His Thr Phe Cys Tyr Asn Phe Phe Leu Gln Lys
           35                  40                  45
   Asp Ser Cys Val Ile Gly Ser Pro Ile Lys Glu Asp Arg Cys Asp Pro
       50                  55                  60
   Asp Lys Lys Asp Pro Lys Lys Ser Glu Lys Cys Asn Lys Val Glu His
   65                  70                  75                  80
   Pro Asn Lys Arg His Ala Lys Gln Arg Val Leu Thr Lys Arg Tyr Asp
                   85                  90                  95
   Asp Glu Gly Ser Ser Phe Phe Ile Asn Ser Gly Ser Gly Ile Cys Gly
               100                 105                 110
   Val Tyr Asp Ser Asn Gln Pro Gly Ala Cys Leu Phe Ser Gly Tyr Asp
           115                 120                 125
   Asp Ser Gly Ala Asn Ser Ala Leu Ala Gly Trp Leu Asn Gly Asn Gln
       130                 135                 140
   Thr Ser Asn Cys Gly Lys Gln Ile Tyr Ile Met Arg Gln Ser Asp Ala
   145                 150                 155                 160
   Asn Leu Ala Lys Glu Lys Arg Asn Val Gln Tyr Ala Pro Val Val Asp
                   165                 170                 175
   Gly Cys Ser Phe Asn Leu Gly Gly Asn Ser Ala Lys Lys Asn Gly Asp
               180                 185                 190
   Gly Cys Phe Arg Ile Gly Val Thr Asn His Thr Phe Phe Ala Leu Asn
           195                 200                 205
   Pro Thr Ala Gly Glu Ile Gln Asn Gly Thr Ile Ser Gln Leu Leu Trp
       210                 215                 220
   Asp Phe Asp Ala Glu Ser Ser Asp Asp Lys Ala Lys Glu Asn Gly Pro
   225                 230                 235                 240
   Phe
<210> 313
   <211> 717
   <212> DNA
   <213> Phakopsora pachyrhizi
<220>
   <221> misc_feature
   <222> (1)..(717)
   <223> ta04712.01_phapa
<400> 313
   atggctaata atctcacagt ttctaccttc ttactgctag ccctttcacc atttgtttct        60
   tacgctgccg ttcaccctca gctcgtcgcc ttcaaaacct tttctttccc caagggtact       120
   tcgccaagct cagaaaatgg taaaaatctt caatttttta aagcagctga ctctccagca       180
   gccactgagg tgcccagtca gggcaacaat ctggttaata gtggcctaac ttcaaccgtc       240
   tgcaaatctt ccgagggcaa ggaatttact ttcactaatc aagactgtct agatgctgct       300
   agagtcatcg ccaatgaatc cacttcatct gcacagtgcg gtaactgcgt tatcggcttg       360
   ttcgatgtca ataagaagaa agcttttaca ccacctggcc cggtgtcacc ctcagttcta       420
   gagaatcaag ttaataaggt tttatcggaa tgtagcaaca gagctagcag taccaaccca       480
   ccagtgaata ataagcgcag tcttttacct tcacaatctg gagttgaaag tgtcagcgga       540
   agtgatagta tagctaatca ccctgcagtt gcaaacgctg gtgggcataa taacttggcc       600
   actcaacctg gaactacaag ccccggtggg tcgcaacctg ggagtggaag tggaaactca       660
   gattcagcta cagttcaaat ggtcatggga tacaacccca cttcaaaagc ttgctga          717
<210> 314
   <211> 238
   <212> PRT
   <213> Phakopsora pachyrhizi
<220>
   <221> VARIANT
   <222> (1)..(238)
   <223> ta04712.01_phapa
<400> 314
   Met Ala Asn Asn Leu Thr Val Ser Thr Phe Leu Leu Leu Ala Leu Ser
   1               5                   10                  15
   Pro Phe Val Ser Tyr Ala Ala Val His Pro Gln Leu Val Ala Phe Lys
               20                  25                  30
   Thr Phe Ser Phe Pro Lys Gly Thr Ser Pro Ser Ser Glu Asn Gly Lys
           35                  40                  45
   Asn Leu Gln Phe Phe Lys Ala Ala Asp Ser Pro Ala Ala Thr Glu Val
       50                  55                  60
   Pro Ser Gln Gly Asn Asn Leu Val Asn Ser Gly Leu Thr Ser Thr Val
   65                  70                  75                  80
   Cys Lys Ser Ser Glu Gly Lys Glu Phe Thr Phe Thr Asn Gln Asp Cys
                   85                  90                  95
   Leu Asp Ala Ala Arg Val Ile Ala Asn Glu Ser Thr Ser Ser Ala Gln
               100                 105                 110
   Cys Gly Asn Cys Val Ile Gly Leu Phe Asp Val Asn Lys Lys Lys Ala
           115                 120                 125
   Phe Thr Pro Pro Gly Pro Val Ser Pro Ser Val Leu Glu Asn Gln Val
       130                 135                 140
   Asn Lys Val Leu Ser Glu Cys Ser Asn Arg Ala Ser Ser Thr Asn Pro
   145                 150                 155                 160
   Pro Val Asn Asn Lys Arg Ser Leu Leu Pro Ser Gln Ser Gly Val Glu
                   165                 170                 175
   Ser Val Ser Gly Ser Asp Ser Ile Ala Asn His Pro Ala Val Ala Asn
               180                 185                 190
   Ala Gly Gly His Asn Asn Leu Ala Thr Gln Pro Gly Thr Thr Ser Pro
           195                 200                 205
   Gly Gly Ser Gln Pro Gly Ser Gly Ser Gly Asn Ser Asp Ser Ala Thr
       210                 215                 220
   Val Gln Met Val Met Gly Tyr Asn Pro Thr Ser Lys Ala Cys
   225                 230                 235
<210> 315
   <211> 711
   <212> DNA
   <213> Phakopsora pachyrhizi
<220>
   <221> misc_feature
   <222> (1)..(711)
   <223> ta10589.01_phapa
<400> 315
   atgaagttct cttactccca attgattgtt ctgctgtctg tgatttctct gagctttgtt      60
   gcatctgaag aaactaaaag tcagagggca gaatcttccg atgagaaatg gttcggacta     120
   ggttacaaca gcttgaggtg gaactactgg aacggccttg ccggatgggg tggtatagga     180
   tcattatacc cttggctggg tgcttacagc ggtgcctatg ggattggtct atgctcagga     240
   aactacctca acagctggtt caagtctgcc caaggcgaaa agcagcgccg ctctttcgag     300
   ttcaattcag gagctcaact ccaaacccgt ggtgagccag acttattgga gactgtgacg     360
   tgcaagaaca ccaagggtga atctcagcag ttcctcacaa gcagttgttt gaaggcagca     420
   gagcagttgg tcgagaaaca aacttcatct gccacttgtg gtagctgcac tctccaactc     480
   catggtccat caggagatct atcagcaaag agcattcctg cctcagagct aaccaccgca     540
   gcctctaaca tcctgaaggc ctgctccaag gctgagaaca agattcttag cacctcagag     600
   ctccagagac gtggaaacga agctgagaac agctcttcgg acaacaatgc taaggattcg     660
   aagaactcct ttgccgttgt gctcctcaaa ggcaatggtc cagagtgcta g              711
<210> 316
   <211> 236
   <212> PRT
   <213> Phakopsora pachyrhizi
<220>
   <221> VARIANT
   <222> (1) .. (236)
   <223> ta10589.01_phapa
<400> 316
   Met Lys Phe Ser Tyr Ser Gln Leu Ile Val Leu Leu Ser Val Ile Ser
   1               5                   10                  15
   Leu Ser Phe Val Ala Ser Glu Glu Thr Lys Ser Gln Arg Ala Glu Ser
               20                  25                  30
   Ser Asp Glu Lys Trp Phe Gly Leu Gly Tyr Asn Ser Leu Arg Trp Asn
           35                  40                  45
   Tyr Trp Asn Gly Leu Ala Gly Trp Gly Gly Ile Gly Ser Leu Tyr Pro
       50                  55                  60
   Trp Leu Gly Ala Tyr Ser Gly Ala Tyr Gly Ile Gly Leu Cys Ser Gly
   65                  70                  75                  80
   Asn Tyr Leu Asn Ser Trp Phe Lys Ser Ala Gln Gly Glu Lys Gln Arg
                   85                  90                  95
   Arg Ser Phe Glu Phe Asn Ser Gly Ala Gln Leu Gln Thr Arg Gly Glu
               100                 105                 110
   Pro Asp Leu Leu Glu Thr Val Thr Cys Lys Asn Thr Lys Gly Glu Ser
           115                 120                 125
   Gln Gln Phe Leu Thr Ser Ser Cys Leu Lys Ala Ala Glu Gln Leu Val
       130                 135                 140
   Glu Lys Gln Thr Ser Ser Ala Thr Cys Gly Ser Cys Thr Leu Gln Leu
   145                 150                 155                 160
   His Gly Pro Ser Gly Asp Leu Ser Ala Lys Ser Ile Pro Ala Ser Glu
                   165                 170                 175
   Leu Thr Thr Ala Ala Ser Asn Ile Leu Lys Ala Cys Ser Lys Ala Glu
               180                 185                 190
   Asn Lys Ile Leu Ser Thr Ser Glu Leu Gln Arg Arg Gly Asn Glu Ala
           195                 200                 205
   Glu Asn Ser Ser Ser Asp Asn Asn Ala Lys Asp Ser Lys Asn Ser Phe
       210                 215                 220
   Ala Val Val Leu Leu Lys Gly Asn Gly Pro Glu Cys
   225                 230                 235
<210> 317
   <211> 705
   <212> DNA
   <213> Phakopsora pachyrhizi
<220>
   <221> misc_feature
   <222> (1)..(705)
   <223> ta00721.05_phapa
<400> 317
   atgaaattat cgtttgcaaa cttgattgct ctcctctctg ttatctcatt aactttggtg      60
   gtagctgagg atgtaagtga caagagtggc gatgtctctg acagtaagtt ctttggttat     120
   ggcctaggcg cttatggagg tagttggggc tactggggag gaggtagctg gggaggtctt     180
   ggtggatact cgtggttgaa cccatggatg ggaaatgcct acgggtttgg tctctaccgc     240
   ggattttatg gtggatggct caaatcggct gatggtcacc aagagcgtcg atcaatccag     300
   gaattgcaca acctcatgtc acgggctgat cacaccgtat catgcaagaa caagaacggt     360
   gaagttgctc atttcgaaac caagagctgc ttgagtgctg caaacaagtt agctaatcaa     420
   cacgcttcga gcgctacctg tggcgcgtgc tctctgagca tccaaggtcc caacggtgct     480
   ctatctgcta agtctattcc atcatctgag ttgacaaagg cgactcttaa cattttgaag     540
   gcttgcgcca aaggtgaaag caagatgctt gctgcttcag aacttgagcg ccgctctcca     600
   ttaccagagg aaattccatc ccaatcctcg tctagcaacg aaaagagcat caaggatgca     660
   tttgcagttg tgctactgaa aggcaacggg ccggcctgtg cttag                     705
<210> 318
   <211> 234
   <212> PRT
   <213> Phakopsora pachyrhizi
<220>
   <221> VARIANT
   <222> (1)..(234)
   <223> ta00721.05₋phapa
<400> 318
   Met Lys Leu Ser Phe Ala Asn Leu Ile Ala Leu Leu Ser Val Ile Ser
   1               5                   10                  15
   Leu Thr Leu Val Val Ala Glu Asp Val Ser Asp Lys Ser Gly Asp Val
               20                  25                  30
   Ser Asp Ser Lys Phe Phe Gly Tyr Gly Leu Gly Ala Tyr Gly Gly Ser
           35                  40                  45
   Trp Gly Tyr Trp Gly Gly Gly Ser Trp Gly Gly Leu Gly Gly Tyr Ser
       50                  55                  60
   Trp Leu Asn Pro Trp Met Gly Asn Ala Tyr Gly Phe Gly Leu Tyr Arg
   65                  70                  75                  80
   Gly Phe Tyr Gly Gly Trp Leu Lys Ser Ala Asp Gly His Gln Glu Arg
                   85                  90                  95
   Arg Ser Ile Gln Glu Leu His Asn Leu Met Ser Arg Ala Asp His Thr
               100                 105                 110
   Val Ser Cys Lys Asn Lys Asn Gly Glu Val Ala His Phe Glu Thr Lys
           115                 120                 125
   Ser Cys Leu Ser Ala Ala Asn Lys Leu Ala Asn Gln His Ala Ser Ser
       130                 135                 140
   Ala Thr Cys Gly Ala Cys Ser Leu Ser Ile Gln Gly Pro Asn Gly Ala
   145                 150                 155                 160
   Leu Ser Ala Lys Ser Ile Pro Ser Ser Glu Leu Thr Lys Ala Thr Leu
                   165                 170                 175
   Asn Ile Leu Lys Ala Cys Ala Lys Gly Glu Ser Lys Met Leu Ala Ala
               180                 185                 190
   Ser Glu Leu Glu Arg Arg Ser Pro Leu Pro Glu Glu Ile Pro Ser Gln
           195                 200                 205
   Ser Ser Ser Ser Asn Glu Lys Ser Ile Lys Asp Ala Phe Ala Val Val
       210                 215                 220
   Leu Leu Lys Gly Asn Gly Pro Ala Cys Ala
   225                 230
<210> 319
   <211> 702
   <212> DNA
   <213> Phakopsora pachyrhizi
<220>
   <221> misc_feature
   <222> (1)..(702)
   <223> ta09817.02_phapa
<400> 319
   atgtttaaga agagtactat gattcttact tttctctttt tggctttaca cgggactctg        60
   tcttcaccag tgccagattc tttgacctct aatgataata acgatataaa ctttagctca       120
   gaaatatttg cgcgagctgt ccaggatgaa tatccacaag agcaggagaa ctcaaggagt       180
   ggcataccag ggaacgaatt tgaaaatagc aacgtagtca ataatgcaag agacccagca       240
   gagttgaacg agcagaatga acaaattcgc ggtgataatc aacctactac taactctaat       300
   attcaactca atcttaaatc aagcaccgag gacaagacgg caaaagattc accaagaaaa       360
   aggtctccag aatcctttga tacaactagc agcgtacaac cttctcccag agaatcaaat       420
   ggggaaatca caaatcctca agtcaatgat gtttctcaca gcgatcagat taactcaggt       480
   aactctccga ctgagggctt tcatccagga gatttgaaca cgagcggaga tcaagggttg       540
   tctaaaatct ggccaaacaa ggatgaaccc caagtgaact atgatcctca caaagatcaa       600
   acaacctcag aaagttttcc gacacagggc tctactcaag gtacaactac tccaaatcca       660
   gtcaattctc aagatggtaa taagccacat ggtgataaat ga                          702
<210> 320
   <211> 233
   <212> PRT
   <213> Phakopsora pachyrhizi
<220>
   <221> VARIANT
   <222> (1)..(233)
   <223> ta09817.02_phapa
<400> 320
   Met Phe Lys Lys Ser Thr Met Ile Leu Thr Phe Leu Phe Leu Ala Leu
   1               5                   10                  15
   His Gly Thr Leu Ser Ser Pro Val Pro Asp Ser Leu Thr Ser Asn Asp
               20                  25                  30
   Asn Asn Asp Ile Asn Phe Ser Ser Glu Ile Phe Ala Arg Ala Val Gln
           35                  40                  45
   Asp Glu Tyr Pro Gln Glu Gln Glu Asn Ser Arg Ser Gly Ile Pro Gly
       50                  55                  60
   Asn Glu Phe Glu Asn Ser Asn Val Val Asn Asn Ala Arg Asp Pro Ala
   65                  70                  75                  80
   Glu Leu Asn Glu Gln Asn Glu Gln Ile Arg Gly Asp Asn Gln Pro Thr
                   85                  90                  95
   Thr Asn Ser Asn Ile Gln Leu Asn Leu Lys Ser Ser Thr Glu Asp Lys
               100                 105                 110
   Thr Ala Lys Asp Ser Pro Arg Lys Arg Ser Pro Glu Ser Phe Asp Thr
           115                 120                 125
   Thr Ser Ser Val Gln Pro Ser Pro Arg Glu Ser Asn Gly Glu Ile Thr
       130                 135                 140
   Asn Pro Gln Val Asn Asp Val Ser His Ser Asp Gln Ile Asn Ser Gly
   145                 150                 155                 160
   Asn Ser Pro Thr Glu Gly Phe His Pro Gly Asp Leu Asn Thr Ser Gly
                   165                 170                 175
   Asp Gln Gly Leu Ser Lys Ile Trp Pro Asn Lys Asp Glu Pro Gln Val
               180                 185                 190
   Asn Tyr Asp Pro His Lys Asp Gln Thr Thr Ser Glu Ser Phe Pro Thr
           195                 200                 205
   Gln Gly Ser Thr Gln Gly Thr Thr Thr Pro Asn Pro Val Asn Ser Gln
       210                 215                 220
   Asp Gly Asn Lys Pro His Gly Asp Lys
   225                 230
<210> 321
   <211> 699
   <212> DNA
   <213> Phakopsora pachyrhizi
<220>
   <221> misc_feature
   <222> (1)..(699)
   <223> ta01937.01_phapa
<400> 321
   atgggtgttt caaagctgtt tttcatatgg attggccttt tcaccatctc aaagctcagc      60
   acaactcttt ctgttagctt tggcaaggga ataaatccat tgtcactagc agacgagggc     120
   gagagtgtgc tgaagcagtt tcaaatcttt ggtaaaaata atgaggtcgt gaatgtcaag     180
   attgtttcaa agtttaactc tgattctaac tcattaagat tcacaaagag agacctcctg     240
   ggaattgagg atgagaagag taatgagaag ggaaacgata aggtgaagaa tcaggcccag     300
   aatagaaatt acactagagt cgatcttaca cctgctcgtc ctacttcaga aacctgttac     360
   tctggttcat tccaaggacc aaaccagagt gattgcgatg tgatattcta tgcccagaag     420
   tataactctt atggtagttt aactgctttc ccaggaacgt ttgtttatgt ttactactct     480
   tcgtgtgtgg tggcttttca aaaccccaat catagtaact attcattaga ctacaattgg     540
   gcaatgctag gtgctaaagc agagcagatt aagaacagat gcctcagaga tgaggagcag     600
   tcgattggtg gatcttttct atttgaaaac tatttgggct acacttttca aaatgtgctt     660
   ataagtcttc aaaggtatgc tggcaatata acggcgtaa                            699
<210> 322
   <211> 232
   <212> PRT
   <213> Phakopsora pachyrhizi
<220>
   <221> VARIANT
   <222> (1)..(232)
   <223> ta01937.01_phapa
<400> 322
   Met Gly Val Ser Lys Leu Phe Phe Ile Trp Ile Gly Leu Phe Thr Ile
   1               5                   10                  15
   Ser Lys Leu Ser Thr Thr Leu Ser Val Ser Phe Gly Lys Gly Ile Asn
               20                  25                  30
   Pro Leu Ser Leu Ala Asp Glu Gly Glu Ser Val Leu Lys Gln Phe Gln
           35                  40                  45
   Ile Phe Gly Lys Asn Asn Glu Val Val Asn Val Lys Ile Val Ser Lys
       50                  55                  60
   Phe Asn Ser Asp Ser Asn Ser Leu Arg Phe Thr Lys Arg Asp Leu Leu
   65                  70                  75                  80
   Gly Ile Glu Asp Glu Lys Ser Asn Glu Lys Gly Asn Asp Lys Val Lys
                   85                  90                  95
   Asn Gln Ala Gln Asn Arg Asn Tyr Thr Arg Val Asp Leu Thr Pro Ala
                   100                 105                 110
   Arg Pro Thr Ser Glu Thr Cys Tyr Ser Gly Ser Phe Gln Gly Pro Asn
           115                 120                 125
   Gln Ser Asp Cys Asp Val Ile Phe Tyr Ala Gln Lys Tyr Asn Ser Tyr
       130                 135                 140
   Gly Ser Leu Thr Ala Phe Pro Gly Thr Phe Val Tyr Val Tyr Tyr Ser
   145                 150                 155                 160
   Ser Cys Val Val Ala Phe Gln Asn Pro Asn His Ser Asn Tyr Ser Leu
                   165                 170                 175
   Asp Tyr Asn Trp Ala Met Leu Gly Ala Lys Ala Glu Gln Ile Lys Asn
               180                 185                 190
   Arg Cys Leu Arg Asp Glu Glu Gln Ser Ile Gly Gly Ser Phe Leu Phe
           195                 200                 205
   Glu Asn Tyr Leu Gly Tyr Thr Phe Gln Asn Val Leu Ile Ser Leu Gln
       210                 215                 220
   Arg Tyr Ala Gly Asn Ile Thr Ala
   225                 230
<210> 323
   <211> 699
   <212> DNA
   <213> Phakopsora pachyrhizi
<220>
   <221> misc_feature
   <222> (1)..(699)
   <223> ta01937.02_phapa
<400> 323
   atgggtgttt caaagctgtt tttcatatgg attggccttt tcaccatctc aaagctcagc      60
   acaactcttt ctgttagctt tggcaaggga ataaatccat tgtcactagc agacgagggc     120
   gagagtgtgc tgaagcagtt tcaaatcttt ggtaaaaata atgaggtcgt gaatgtcaag     180
   attgtttcaa agtttaactc tgattctaac tcattaagat tcacaaagag agacctcctg     240
   ggaattgagg atgagaagag taatgagaag ggaaacgata aggtgaagaa tcaggcccag     300
   aatagaaatt acactagagt cgatcttaca cctgctcgtc ctacttcaga aacctgttac     360
   tctggttcat tccaaggacc aaaccagagt gattgcgatg tgatattcta tgcccagaag     420
   tataactctt atggtagttt aactgctttc ccaggaacgt ttgtttatgt ttactactct     480
   tcgtgtgtgg tggcttttca aaaccccaat catagtaact attcattaga ctacaattgg     540
   gcaatgctag gtgctaaagc agagcagatt aagaacagat gcctcagaga tgaggagcag     600
   tcgattggtg gatcttttct atttgaaaac tatttgggct acacttttca aaatgtgctt     660
   ataagtcttc aaaggtatgc tggcaatata acggcgtaa                            699
<210> 324
   <211> 232
   <212> PRT
   <213> Phakopsora pachyrhizi
<220>
   <221> VARIANT
   <222> (1)..(232)
   <223> ta01937.02_phapa
<400> 324
   Met Gly Val Ser Lys Leu Phe Phe Ile Trp Ile Gly Leu Phe Thr Ile
   1               5                   10                  15
   Ser Lys Leu Ser Thr Thr Leu Ser Val Ser Phe Gly Lys Gly Ile Asn
               20                  25                  30
   Pro Leu Ser Leu Ala Asp Glu Gly Glu Ser Val Leu Lys Gln Phe Gln
           35                  40                  45
   Ile Phe Gly Lys Asn Asn Glu Val Val Asn Val Lys Ile Val Ser Lys
       50                  55                  60
   Phe Asn Ser Asp Ser Asn Ser Leu Arg Phe Thr Lys Arg Asp Leu Leu
   65                  70                  75                  80
   Gly Ile Glu Asp Glu Lys Ser Asn Glu Lys Gly Asn Asp Lys Val Lys
                   85                  90                  95
   Asn Gln Ala Gln Asn Arg Asn Tyr Thr Arg Val Asp Leu Thr Pro Ala
               100                 105                 110
   Arg Pro Thr Ser Glu Thr Cys Tyr Ser Gly Ser Phe Gln Gly Pro Asn
           115                 120                 125
   Gln Ser Asp Cys Asp Val Ile Phe Tyr Ala Gln Lys Tyr Asn Ser Tyr
       130                 135                 140
   Gly Ser Leu Thr Ala Phe Pro Gly Thr Phe Val Tyr Val Tyr Tyr Ser
   145                 150                 155                 160
   Ser Cys Val Val Ala Phe Gln Asn Pro Asn His Ser Asn Tyr Ser Leu
                   165                 170                 175
   Asp Tyr Asn Trp Ala Met Leu Gly Ala Lys Ala Glu Gln Ile Lys Asn
               180                 185                 190
   Arg Cys Leu Arg Asp Glu Glu Gln Ser Ile Gly Gly Ser Phe Leu Phe
           195                 200                 205
   Glu Asn Tyr Leu Gly Tyr Thr Phe Gln Asn Val Leu Ile Ser Leu Gln
       210                 215                 220
   Arg Tyr Ala Gly Asn Ile Thr Ala
   225                 230
<210> 325
   <211> 696
   <212> DNA
   <213> Phakopsora pachyrhizi
<220>
   <221> misc_feature
   <222> (1)..(696)
   <223> ta00721.07_phapa
<400> 325
   atgaaattgt catacgccta cttgattgtt ctcatctctg tcctgtcttc aacttttgtg      60
   gtagctgagg atgttagcga caaaaacgct gaggtttctg acagcaagtt ctttggacta     120
   ggtggacttg ggggttgggg ttactgggga ggtggtagat ttgctggtct tggtggatac    180
   tcgtggttga acccatggat gggaaatgcc tacgggtttg gtctctaccg cggattttat     240
   ggtggatggc tcaaatcggc tgatggtcac caagagcgtc gatcaatcca ggaattgcac     300
   aacctcatgt cacgggctga tcacacagtt tcatgcaaga ataagaacgg tgaagttgct     360
   caattcaaca ccaagagctg cttaagtgcc gctaacaagc tagctaatca acactcctca     420
   agtgctacct gtggggcgtg ctctctcagc attcagggtc caaatggcgc actttctgcc     480
   aagtctatcc catcatctga gttgaccaaa gcaactgtca atatcttgaa ggcttgcgcc     540
   aaaggtgaga gcaagatgct ctcagcttca gagctcgagc gccgttctcc cttaccagag     600
   gaaatccaat ctcaatcatc atctagcaac gagaagggta ataaggatgc ttttgcagtt     660
   gtgctcctca aaggtaacgg ccccgagtgc tcttaa                                696
<210> 326
   <211> 231
   <212> PRT
   <213> Phakopsora pachyrhizi
<220>
   <221> VARIANT
   <222> (1)..(231)
   <223> ta00721.07_phapa
<400> 326
   Met Lys Leu Ser Tyr Ala Tyr Leu Ile Val Leu Ile Ser Val Leu Ser
   1               5                   10                  15
   Ser Thr Phe Val Val Ala Glu Asp Val Ser Asp Lys Asn Ala Glu Val
               20                  25                  30
   Ser Asp Ser Lys Phe Phe Gly Leu Gly Gly Leu Gly Gly Trp Gly Tyr
           35                  40                  45
   Trp Gly Gly Gly Arg Phe Ala Gly Leu Gly Gly Tyr Ser Trp Leu Asn
       50                  55                  60
   Pro Trp Met Gly Asn Ala Tyr Gly Phe Gly Leu Tyr Arg Gly Phe Tyr
   65                  70                  75                  80
   Gly Gly Trp Leu Lys Ser Ala Asp Gly His Gln Glu Arg Arg Ser Ile
                   85                  90                  95
   Gln Glu Leu His Asn Leu Met Ser Arg Ala Asp His Thr Val Ser Cys
               100                 105                 110
   Lys Asn Lys Asn Gly Glu Val Ala Gln Phe Asn Thr Lys Ser Cys Leu
           115                 120                 125
   Ser Ala Ala Asn Lys Leu Ala Asn Gln His Ser Ser Ser Ala Thr Cys
       130                 135                 140
   Gly Ala Cys Ser Leu Ser Ile Gln Gly Pro Asn Gly Ala Leu Ser Ala
   145                 150                 155                 160
   Lys Ser Ile Pro Ser Ser Glu Leu Thr Lys Ala Thr Val Asn Ile Leu
                   165                 170                 175
   Lys Ala Cys Ala Lys Gly Glu Ser Lys Met Leu Ser Ala Ser Glu Leu
               180                 185                 190
   Glu Arg Arg Ser Pro Leu Pro Glu Glu Ile Gln Ser Gln Ser Ser Ser
           195                 200                 205
   Ser Asn Glu Lys Gly Asn Lys Asp Ala Phe Ala Val Val Leu Leu Lys
       210                 215                 220
   Gly Asn Gly Pro Glu Cys Ser
   225                 230
<210> 327
   <211> 696
   <212> DNA
   <213> Phakopsora pachyrhizi
<220>
   <221> misc_feature
   <222> (1)..(696)
   <223> ta10435.01_phapa
<400> 327
   atgaatatct ctttaaaaaa ctttgtgcaa gtttcaatct tgctaattct cgtgtctgga        60
   gtcatcagtc ttgatgcttc tactccaaac agccatcacc tatcaagaag acagaataag       120
   gatcaggtca aggatcagac aaagaagact caacaacagg tacaagaaat caaagatgga       180
   aaaggtggag cagcccaggg cggtactcct acagagaaag ctgcaaacca gatgatgcag       240
   atgcttatgc agattggtca aagtttagat gcagtcacca gcctggggtc cacccctgag       300
   accgtaaagt cgacagcaga ggcaatcata aaaatggttc ctgatgtcgg caaggcaaca       360
   aagtctttga tagacactat gccgaacaaa gcacaacttg agggcgtcgc tgataaagcc       420
   actcaatcag cggaacagct aggaaaagtc ctcaaaacca ttgagcaaag tccagaagat       480
   gccaacttta tcaaaaaaca ttataaagcc ctgggtgacg cctttacagg aatctttggt       540
   gctgtggatc ccgtcttcag ctctgcattt cctgatgata aaaatggtgg acaacaacaa       600
   gccggtaaaa ccgacgggag acaactaaac actcagactg aaggggccca aaaggatcaa       660
   aaagatacaa aggaaaaaaa caaaaagaaa acctag                                 696
<210> 328
   <211> 231
   <212> PRT
   <213> Phakopsora pachyrhizi
<220>
   <221> VARIANT
   <222> (1)..(231)
   <223> ta10435.01_phapa
<400> 328
   Met Asn Ile Ser Leu Lys Asn Phe Val Gln Val Ser Ile Leu Leu Ile
   1               5                   10                  15
   Leu Val Ser Gly Val Ile Ser Leu Asp Ala Ser Thr Pro Asn Ser His
               20                  25                  30
   His Leu Ser Arg Arg Gln Asn Lys Asp Gln Val Lys Asp Gln Thr Lys
           35                  40                  45
   Lys Thr Gln Gln Gln Val Gln Glu Ile Lys Asp Gly Lys Gly Gly Ala
       50                  55                  60
   Ala Gln Gly Gly Thr Pro Thr Glu Lys Ala Ala Asn Gln Met Met Gln
   65                  70                  75                  80
   Met Leu Met Gln Ile Gly Gln Ser Leu Asp Ala Val Thr Ser Leu Gly
                   85                  90                  95
   Ser Thr Pro Glu Thr Val Lys Ser Thr Ala Glu Ala Ile Ile Lys Met
               100                 105                 110
   Val Pro Asp Val Gly Lys Ala Thr Lys Ser Leu Ile Asp Thr Met Pro
           115                 120                 125
   Asn Lys Ala Gln Leu Glu Gly Val Ala Asp Lys Ala Thr Gln Ser Ala
       130                 135                 140
   Glu Gln Leu Gly Lys Val Leu Lys Thr Ile Glu Gln Ser Pro Glu Asp
   145                 150                 155                 160
   Ala Asn Phe Ile Lys Lys His Tyr Lys Ala Leu Gly Asp Ala Phe Thr
                   165                 170                 175
   Gly Ile Phe Gly Ala Val Asp Pro Val Phe Ser Ser Ala Phe Pro Asp
               180                 185                 190
   Asp Lys Asn Gly Gly Gln Gln Gln Ala Gly Lys Thr Asp Gly Arg Gln
           195                 200                 205
   Leu Asn Thr Gln Thr Glu Gly Ala Gln Lys Asp Gln Lys Asp Thr Lys
       210                 215                 220
   Glu Lys Asn Lys Lys Lys Thr
   225                 230
<210> 329
   <211> 696
   <212> DNA
   <213> Phakopsora pachyrhizi
<220>
   <221> misc_feature
   <222> (1)..(696)
   <223> ta00721.03_phapa
<400> 329
   atgaaattgt catacgccta cttgattgtt ctcatctctg tcctgtcttc aacttttgtg      60
   gtagctgagg atgttagcga caaaaacgct gaggtttctg acagcaagtt ctttggacta     120
   ggtggacttg ggggttgggg ttactgggga ggtggtagat ttgctggtct tggtggatac     180
   tcgtggttga acccatggat gggaaatgcc tacgggtttg gtctctaccg cggattttat     240
   ggtggatggc tcaaatcggc tgatggtcac caagagcgtc gatcaatcca ggaattgcac     300
   aacctcatgt cacgggctga tcacaccgta tcatgcaaga acaagaacgg tgaagttgct     360
   catttcgaaa ccaagagctg cttgagtgct gcaaacaagt tagctaatca acacgcttcg     420
   agcgctacct gtggcgcgtg ctctctgagc atccaaggtc ccaacggtgc tctatctgct     480
   aagtctattc catcatctga gttgacaaag gcgactctta acattttgaa ggcttgcgcc     540
   aaaggtgaaa gcaagatgct tgctgcttca gaacttgagc gccgctctcc attaccagag     600
   gaaattccat ctcaatcttc gtctagcaac gacaagagca ccaaggatgc gtttgcagtc     660
   gtgctactga aaggcaacgg gccagcctgt gcttaa                               696
<210> 330
   <211> 231
   <212> PRT
   <213> Phakopsora pachyrhizi
<220>
   <221> VARIANT
   <222> (1)..(231)
   <223> ta00721.03_phapa
<400> 330
   Met Lys Leu Ser Tyr Ala Tyr Leu Ile Val Leu Ile Ser Val Leu Ser
   1               5                   10                  15
   Ser Thr Phe Val Val Ala Glu Asp Val Ser Asp Lys Asn Ala Glu Val
               20                  25                  30
   Ser Asp Ser Lys Phe Phe Gly Leu Gly Gly Leu Gly Gly Trp Gly Tyr
           35                  40                  45
   Trp Gly Gly Gly Arg Phe Ala Gly Leu Gly Gly Tyr Ser Trp Leu Asn
       50                  55                  60
   Pro Trp Met Gly Asn Ala Tyr Gly Phe Gly Leu Tyr Arg Gly Phe Tyr
   65                  70                  75                  80
   Gly Gly Trp Leu Lys Ser Ala Asp Gly His Gln Glu Arg Arg Ser Ile
                   85                  90                  95
   Gln Glu Leu His Asn Leu Met Ser Arg Ala Asp His Thr Val Ser Cys
               100                 105                 110
   Lys Asn Lys Asn Gly Glu Val Ala His Phe Glu Thr Lys Ser Cys Leu
           115                 120                 125
   Ser Ala Ala Asn Lys Leu Ala Asn Gln His Ala Ser Ser Ala Thr Cys
       130                 135                 140
   Gly Ala Cys Ser Leu Ser Ile Gln Gly Pro Asn Gly Ala Leu Ser Ala
   145                 150                 155                 160
   Lys Ser Ile Pro Ser Ser Glu Leu Thr Lys Ala Thr Leu Asn Ile Leu
                   165                 170                 175
   Lys Ala Cys Ala Lys Gly Glu Ser Lys Met Leu Ala Ala Ser Glu Leu
               180                 185                 190
   Glu Arg Arg Ser Pro Leu Pro Glu Glu Ile Pro Ser Gln Ser Ser Ser
           195                 200                 205
   Ser Asn Asp Lys Ser Thr Lys Asp Ala Phe Ala Val Val Leu Leu Lys
       210                 215                 220
   Gly Asn Gly Pro Ala Cys Ala
   225                 230
<210> 331
   <211> 696
   <212> DNA
   <213> Phakopsora pachyrhizi
<220>
   <221> misc_feature
   <222> (1)..(696)
   <223> ta00721.04_phapa
<400> 331
   atgaaattgt catacgccta cttgattgtt ctcatctctg tcctgtcttc aacttttgtg       60
   gtagctgagg atgttagcga caaaaacgct gaggtttctg acagcaagtt ctttggacta      120
   ggtggacttg ggggttgggg ttactgggga ggtggtagat ttgctggtct tggtggatac      180
   tcgtggttga acccatggat gggaaatgcc tacgggtttg gtctataccg cggcttatat      240
   ggcggatggc taaaatctgc tgatggccac caagagcgtc gatcaatcca agagttacaa      300
   aaactcatgg cacgggctga tcacaccgta tcatgcaaga acaagaacgg tgaagttgct      360
   catttcgaaa ccaagagctg cttgagtgct gcaaacaagt tagctaatca acacgcttcg      420
   agcgctacct gtggcgcgtg ctctctgagc atccaaggtc ccaacggtgc tctatctgct      480
   aagtctattc catcatctga gttgacaaag gcgactctta acattttgaa ggcttgcgcc      540
   aaaggtgaaa gcaagatgct tgctgcttca gaacttgagc gccgctctcc attaccagag      600
   gaaattccat cccaatcctc gtctagcaac gaaaagagca tcaaggatgc atttgcagtt      660
   gtgctactga aaggcaacgg gccggcctgt gcttag                                 696
<210> 332
   <211> 231
   <212> PRT
   <213> Phakopsora pachyrhizi
<220>
   <221> VARIANT
   <222> (1)..(231)
   <223> ta00721.04_phapa
<400> 332
   Met Lys Leu Ser Tyr Ala Tyr Leu Ile Val Leu Ile Ser Val Leu Ser
   1               5                   10                  15
   Ser Thr Phe Val Val Ala Glu Asp Val Ser Asp Lys Asn Ala Glu Val
               20                  25                  30
   Ser Asp Ser Lys Phe Phe Gly Leu Gly Gly Leu Gly Gly Trp Gly Tyr
           35                  40                  45
   Trp Gly Gly Gly Arg Phe Ala Gly Leu Gly Gly Tyr Ser Trp Leu Asn
       50                  55                  60
   Pro Trp Met Gly Asn Ala Tyr Gly Phe Gly Leu Tyr Arg Gly Leu Tyr
   65                  70                  75                  80
   Gly Gly Trp Leu Lys Ser Ala Asp Gly His Gln Glu Arg Arg Ser Ile
                   85                  90                  95
   Gln Glu Leu Gln Lys Leu Met Ala Arg Ala Asp His Thr Val Ser Cys
               100                 105                 110
   Lys Asn Lys Asn Gly Glu Val Ala His Phe Glu Thr Lys Ser Cys Leu
           115                 120                 125
   Ser Ala Ala Asn Lys Leu Ala Asn Gln His Ala Ser Ser Ala Thr Cys
       130                 135                 140
   Gly Ala Cys Ser Leu Ser Ile Gln Gly Pro Asn Gly Ala Leu Ser Ala
   145                 150                 155                 160
   Lys Ser Ile Pro Ser Ser Glu Leu Thr Lys Ala Thr Leu Asn Ile Leu
                   165                 170                 175
   Lys Ala Cys Ala Lys Gly Glu Ser Lys Met Leu Ala Ala Ser Glu Leu
               180                 185                 190
   Glu Arg Arg Ser Pro Leu Pro Glu Glu Ile Pro Ser Gln Ser Ser Ser
           195                 200                 205
   Ser Asn Glu Lys Ser Ile Lys Asp Ala Phe Ala Val Val Leu Leu Lys
       210                 215                 220
   Gly Asn Gly Pro Ala Cys Ala
   225                 230
<210> 333
   <211> 693
   <212> DNA
   <213> Phakopsora pachyrhizi
<220>
   <221> misc_feature
   <222> (1)..(693)
   <223> ta09424.01_phapa
<400> 333
   atgaagatct gtagatctgg actatcgctc gtttctatcc ttttcttcag ccttatccta      60
   actgtcgaga tcacaagctc gaccgatcaa tctaatgata atattaaact ccaaagaagg     120
   tatccctcag atagccacac cagtaataac gataatgata accccaataa taaccctcaa     180
   aaacctttaa ctgaagctgg gaatgttcat ggtggaggaa aaaagatgaa ttgcagtggt     240
   actccaaaca actgtcatcc ctcatcatca gcattaatac agaaccgcac cgatgctcga     300
   aagccaactc cgttctctca accatgtcaa gagtactaca gtgcaaacac cgatcatgcc     360
   gtttgcagag gagatcgatc aattatgtgc cactctggct gtactggagc agtagtttct     420
   caaaactgtc aagttgatga aaattctata aagacgaatc agacttgtaa tgtagcattt     480
   tcaaagacgt cgatgaattc atttctttgt actacgagtg aaggagcatt tacttgtgca     540
   ggtccattcg aaggtcaagc tatttgtaat aattgtttac cgacgaatga taatgaagaa     600
   aacgatggcg atcatagttc tgctaatcat catttcaata aaaatattat ccaaaaatta     660
   actctaatca gcttaatcat aagtcttctt taa                                  693
<210> 334
   <211> 230
   <212> PRT
   <213> Phakopsora pachyrhizi
<220>
   <221> VARIANT
   <222> (1)..(230)
   <223> ta09424.01_phapa
<400> 334
   Met Lys Ile Cys Arg Ser Gly Leu Ser Leu Val Ser Ile Leu Phe Phe
   1               5                   10                  15
   Ser Leu Ile Leu Thr Val Glu Ile Thr Ser Ser Thr Asp Gln Ser Asn
               20                  25                  30
   Asp Asn Ile Lys Leu Gln Arg Arg Tyr Pro Ser Asp Ser His Thr Ser
           35                  40                  45
   Asn Asn Asp Asn Asp Asn Pro Asn Asn Asn Pro Gln Lys Pro Leu Thr
       50                  55                  60
   Glu Ala Gly Asn Val His Gly Gly Gly Lys Lys Met Asn Cys Ser Gly
   65                  70                  75                  80
   Thr Pro Asn Asn Cys His Pro Ser Ser Ser Ala Leu Ile Gln Asn Arg
                   85                  90                  95
   Thr Asp Ala Arg Lys Pro Thr Pro Phe Ser Gln Pro Cys Gln Glu Tyr
               100                 105                 110
   Tyr Ser Ala Asn Thr Asp His Ala Val Cys Arg Gly Asp Arg Ser Ile
           115                 120                 125
   Met Cys His Ser Gly Cys Thr Gly Ala Val Val Ser Gln Asn Cys Gln
       130                 135                 140
   Val Asp Glu Asn Ser Ile Lys Thr Asn Gln Thr Cys Asn Val Ala Phe
   145                 150                 155                 160
   Ser Lys Thr Ser Met Asn Ser Phe Leu Cys Thr Thr Ser Glu Gly Ala
                   165                 170                 175
   Phe Thr Cys Ala Gly Pro Phe Glu Gly Gln Ala Ile Cys Asn Asn Cys
               180                 185                 190
   Leu Pro Thr Asn Asp Asn Glu Glu Asn Asp Gly Asp His Ser Ser Ala
           195                 200                 205
   Asn His His Phe Asn Lys Asn Ile Ile Gln Lys Leu Thr Leu Ile Ser
       210                 215                 220
   Leu Ile Ile Ser Leu Leu
   225                 230
<210> 335
   <211> 681
   <212> DNA
   <213> Phakopsora pachyrhizi
<220>
   <221> misc_feature
   <222> (1)..(681)
   <223> ta10800.02_phapa
<400> 335
   atggttcact tgatcccctt ctttgcatta gtcctcgccg gtgctattgc tgctgaaaac      60
   gacaagtcaa gtagcaaggc atttggaggc ggggccgttg gaggtggagc cttcgggggc     120
   gctgcttcag gctttagctc aagttcatct tctgctagtg tctcccacca ctccaaaagc     180
   ggctttgttg caggtggtgg gggtggattc aacttcggtg gtcttggact acccatggcc     240
   ttcagccctt ttggtggcct tattaatcag ataggagcat gtcgttcttc tttggtatct     300
   gggatctctg cacaagtggc atttagtcaa gttagccgca ttgcttcaat gtttcaacaa     360
   gcaataggtt ccttacaagg ttgtggtact tgcggccttg ttggtcctgg attgagcgct     420
   tttggcggcg ttatccaacg aattaccggc ggtataatat cgttagtcgg gagacttcag     480
   ggtatcttcc cttctatgtg gcaagggatc ctaacttccg ctttccaacc acttggtggt     540
   tctctcccag gactgtttaa cttttgcaac ggtgtcggcc ttcccgttaa caaatttttt     600
   ggtggaggat tttctcagtg ccttgctcct ctcaagattg gctctctgaa ccaatctctc      660
   tctcgtttcg gcctactttg a                                                681
<210> 336
   <211> 226
   <212> PRT
   <213> Phakopsora pachyrhizi
<220>
   <221> VARIANT
   <222> (1) .. (226)
   <223> ta10800.02_phapa
<400> 336
   Met Val His Leu Ile Pro Phe Phe Ala Leu Val Leu Ala Gly Ala Ile
   1               5                   10                  15
   Ala Ala Glu Asn Asp Lys Ser Ser Ser Lys Ala Phe Gly Gly Gly Ala
               20                  25                  30
   Val Gly Gly Gly Ala Phe Gly Gly Ala Ala Ser Gly Phe Ser Ser Ser
           35                  40                  45
   Ser Ser Ser Ala Ser Val Ser His His Ser Lys Ser Gly Phe Val Ala
       50                  55                  60
   Gly Gly Gly Gly Gly Phe Asn Phe Gly Gly Leu Gly Leu Pro Met Ala
   65                  70                  75                  80
   Phe Ser Pro Phe Gly Gly Leu Ile Asn Gln Ile Gly Ala Cys Arg Ser
                   85                  90                  95
   Ser Leu Val Ser Gly Ile Ser Ala Gln Val Ala Phe Ser Gln Val Ser
               100                 105                 110
   Arg Ile Ala Ser Met Phe Gln Gln Ala Ile Gly Ser Leu Gln Gly Cys
           115                 120                 125
   Gly Thr Cys Gly Leu Val Gly Pro Gly Leu Ser Ala Phe Gly Gly Val
       130                 135                 140
   Ile Gln Arg Ile Thr Gly Gly Ile Ile Ser Leu Val Gly Arg Leu Gln
   145                 150                 155                 160
   Gly Ile Phe Pro Ser Met Trp Gln Gly Ile Leu Thr Ser Ala Phe Gln
                   165                 170                 175
   Pro Leu Gly Gly Ser Leu Pro Gly Leu Phe Asn Phe Cys Asn Gly Val
               180                 185                 190
   Gly Leu Pro Val Asn Lys Phe Phe Gly Gly Gly Phe Ser Gln Cys Leu
           195                 200                 205
   Ala Pro Leu Lys Ile Gly Ser Leu Asn Gln Ser Leu Ser Arg Phe Gly
       210                 215                 220
   Leu Leu
   225
<210> 337
   <211> 678
   <212> DNA
   <213> Phakopsora pachyrhizi
<220>
   <221> misc_feature
   <222> (1)..(678)
   <223> ta00012.04_phapa
<400> 337
   atgtttagat ctttattttt aggaatttta gcttgttctg cagttaaact taccatttgt      60
   agtccaagtg atgaggtagt tgtcaaacct cgacagatag gttatggagc tggtcttggt     120
   gctggaggag cttcctcaag ctctttcagt agtagtacca gcgtttcttc cttcagttcg     180
   ttcgttcaag gttggagtgt aattccaact gctttcggta cctgtgcagc tgtctttcag     240
   agacaggtaa cagttgaggt ggccattcaa tctgttcagg agttacatag tacagtatct     300
   ggtgttctgg gtaattatgg aagttgttct agctgtggtg gtgcttcggc agctagctct     360
   tactcgtctc actaccaaag tataattgta aagaccttta cctcatggca atctatcatg     420
   tcagtcggtc attcactata tgacaatgtc tgggaacctc agtttgctcc tctctttcga     480
   caattcaacc cattccttac agccgttcaa cagaactcag gtttctttgg tatcaacctt     540
   ggaaacattc ttggtggtct tcatctaaac ttaaacctat tctcatcttg tggtcttaac     600
   attggtggat tacttggagg tgttttgagt acggttggtg gattgttagg tggtcttaaa     660
   gatcatgaag aagcttga                                                   678
<210> 338
   <211> 225
   <212> PRT
   <213> Phakopsora pachyrhizi
<220>
   <221> VARIANT
   <222> (1)..(225)
   <223> ta00012.04_phapa
<400> 338
   Met Phe Arg Ser Leu Phe Leu Gly Ile Leu Ala Cys Ser Ala Val Lys
   1               5                   10                  15
   Leu Thr Ile Cys Ser Pro Ser Asp Glu Val Val Val Lys Pro Arg Gln
               20                  25                  30
   Ile Gly Tyr Gly Ala Gly Leu Gly Ala Gly Gly Ala Ser Ser Ser Ser
                   35                  40                  45
   Phe Ser Ser Ser Thr Ser Val Ser Ser Phe Ser Ser Phe Val Gln Gly
       50                  55                  60
   Trp Ser Val Ile Pro Thr Ala Phe Gly Thr Cys Ala Ala Val Phe Gln
   65                  70                  75                  80
   Arg Gln Val Thr Val Glu Val Ala Ile Gln Ser Val Gln Glu Leu His
                   85                  90                  95
   Ser Thr Val Ser Gly Val Leu Gly Asn Tyr Gly Ser Cys Ser Ser Cys
               100                 105                 110
   Gly Gly Ala Ser Ala Ala Ser Ser Tyr Ser Ser His Tyr Gln Ser Ile
           115                 120                 125
   Ile Val Lys Thr Phe Thr Ser Trp Gln Ser Ile Met Ser Val Gly His
       130                 135                 140
   Ser Leu Tyr Asp Asn Val Trp Glu Pro Gln Phe Ala Pro Leu Phe Arg
   145                 150                 155                 160
   Gln Phe Asn Pro Phe Leu Thr Ala Val Gln Gln Asn Ser Gly Phe Phe
                   165                 170                 175
   Gly Ile Asn Leu Gly Asn Ile Leu Gly Gly Leu His Leu Asn Leu Asn
               180                 185                 190
   Leu Phe Ser Ser Cys Gly Leu Asn Ile Gly Gly Leu Leu Gly Gly Val
           195                 200                 205
   Leu Ser Thr Val Gly Gly Leu Leu Gly Gly Leu Lys Asp His Glu Glu
       210                 215                 220
   Ala
   225
<210> 339
   <211> 663
   <212> DNA
   <213> Phakopsora pachyrhizi
<220>
   <221> misc_feature
   <222> (1)..(663)
   <223> ta04963.01_phapa
<400> 339
   atggtttact caaaggcaac cttcaccctg gtctcactcc tacctatgat cctggcccag    60
   cagagcatca acaacagtga taccacaacc acggctgcta ccccggccag tcccaccata   120
   ggctcaactg gtactaccaa cagcagcagc tctaccacta cttccactac cacctctaac   180
   actgccagtg gagctgctca gtccttccct gcgccaacag tctcaggaac atcctgtggt   240
   acagcccagc agaactttaa ccagtgtgtt accaaagtat caaaggatat ctcctcatgt   300
   ccctctaccg ataacacctg tctttgccaa acgtatgcaa acctagccta ctgttacaac   360
   gcctgtccag atctggcatc atcaggggcc ggatacctgc agcagtccac agtgaactgt   420
   gatgcagcag gtatcaagcc aaacgctact tccaacgtca ccaccacccc tgtcacctcc   480
   accactaaca ggaacaccac caacacctca cccatctcta acacaaacaa gaacaattct   540
   acctcgggca acactgctgc tacatttgct gctggaaact ccaaggcaag tgggattgag   600
   gccccgttac tgagcgttgc tgttgttggg ctgtgtggta tcattgctag cctatttgca   660
   tga                                                                 663
<210> 340
   <211> 220
   <212> PRT
   <213> Phakopsora pachyrhizi
<220>
   <221> VARIANT
   <222> (1)..(220)
   <223> ta04963.01_phapa
<400> 340
   Met Val Tyr Ser Lys Ala Thr Phe Thr Leu Val Ser Leu Leu Pro Met
   1               5                   10                  15
   Ile Leu Ala Gln Gln Ser Ile Asn Asn Ser Asp Thr Thr Thr Thr Ala
               20                  25                  30
   Ala Thr Pro Ala Ser Pro Thr Ile Gly Ser Thr Gly Thr Thr Asn Ser
           35                  40                  45
   Ser Ser Ser Thr Thr Thr Ser Thr Thr Thr Ser Asn Thr Ala Ser Gly
       50                  55                  60
   Ala Ala Gln Ser Phe Pro Ala Pro Thr Val Ser Gly Thr Ser Cys Gly
   65                  70                  75                  80
   Thr Ala Gln Gln Asn Phe Asn Gln Cys Val Thr Lys Val Ser Lys Asp
                   85                  90                  95
   Ile Ser Ser Cys Pro Ser Thr Asp Asn Thr Cys Leu Cys Gln Thr Tyr
               100                 105                 110
   Ala Asn Leu Ala Tyr Cys Tyr Asn Ala Cys Pro Asp Leu Ala Ser Ser
           115                 120                 125
   Gly Ala Gly Tyr Leu Gln Gln Ser Thr Val Asn Cys Asp Ala Ala Gly
       130                 135                 140
   Ile Lys Pro Asn Ala Thr Ser Asn Val Thr Thr Thr Pro Val Thr Ser
   145                 150                 155                 160
   Thr Thr Asn Arg Asn Thr Thr Asn Thr Ser Pro Ile Ser Asn Thr Asn
                   165                 170                 175
   Lys Asn Asn Ser Thr Ser Gly Asn Thr Ala Ala Thr Phe Ala Ala Gly
               180                 185                 190
   Asn Ser Lys Ala Ser Gly Ile Glu Ala Pro Leu Leu Ser Val Ala Val
           195                 200                 205
   Val Gly Leu Cys Gly Ile Ile Ala Ser Leu Phe Ala
       210                 215                 220
<210> 341
   <211> 660
   <212> DNA
   <213> Phakopsora pachyrhizi
<220>
   <221> misc_feature
   <222> (1)..(660)
   <223> ta01860.01_phapa
<400> 341
   atgaagattt cttttgcaaa cttatttgtt cttttctccg cactctctgt ggccttagtt      60
   gctaccgagg atgttcaagg tgtaaaaggc gataactcgg aagctaaatg gctatactgg     120
   ggtggtggta taggttatgg tggattagga agctattatg gtaattggtt gagcccatac     180
   tacaacaatg ccttcgggtt tggggtatac aattacttgc ctagacgctg gtaccgtcgt     240
   gctctacaaa ccagagggga tgagtcgaat gccaaagctt cggttagctc aactgttcag     300
   tgctccaacg cgcagggggt caagcaagag ttctcaactg ctagttgttt aaaggccgct     360
   accaagattt ccaaagagca aatcaccact gctacctgtg gcacatgcac catccagctt     420
   cacaataaag agggaccaat tcaatttaat gcgatccccc ctcaatctga attaaactcg     480
   gcagtaaaca acatgctaaa ggcctgcagt aaaagccagg acaaacttct cagcgcttca     540
   gagctctcga gacgctcccc agctgaaaat ggtaatggtg tggattctaa ggatacttcg     600
   agcaacaaga acgccttggc gattatatta cttaaaggca acgggccaga gtgtaactaa     660
<210> 342
   <211> 219
   <212> PRT
   <213> Phakopsora pachyrhizi
<220>
   <221> VARIANT
   <222> (1) .. (219)
   <223> ta01860.01_phapa
<400> 342
   Met Lys Ile Ser Phe Ala Asn Leu Phe Val Leu Phe Ser Ala Leu Ser
   1               5                   10                  15
   Val Ala Leu Val Ala Thr Glu Asp Val Gln Gly Val Lys Gly Asp Asn
               20                  25                  30
   Ser Glu Ala Lys Trp Leu Tyr Trp Gly Gly Gly Ile Gly Tyr Gly Gly
           35                  40                  45
   Leu Gly Ser Tyr Tyr Gly Asn Trp Leu Ser Pro Tyr Tyr Asn Asn Ala
       50                  55                  60
   Phe Gly Phe Gly Val Tyr Asn Tyr Leu Pro Arg Arg Trp Tyr Arg Arg
   65                  70                  75                  80
   Ala Leu Gln Thr Arg Gly Asp Glu Ser Asn Ala Lys Ala Ser Val Ser
                   85                  90                  95
   Ser Thr Val Gln Cys Ser Asn Ala Gln Gly Val Lys Gln Glu Phe Ser
               100                 105                 110
   Thr Ala Ser Cys Leu Lys Ala Ala Thr Lys Ile Ser Lys Glu Gln Ile
           115                 120                 125
   Thr Thr Ala Thr Cys Gly Thr Cys Thr Ile Gln Leu His Asn Lys Glu
       130                 135                 140
   Gly Pro Ile Gln Phe Asn Ala Ile Pro Pro Gln Ser Glu Leu Asn Ser
   145                 150                 155                 160
   Ala Val Asn Asn Met Leu Lys Ala Cys Ser Lys Ser Gln Asp Lys Leu
                   165                 170                 175
   Leu Ser Ala Ser Glu Leu Ser Arg Arg Ser Pro Ala Glu Asn Gly Asn
               180                 185                 190
   Gly Val Asp Ser Lys Asp Thr Ser Ser Asn Lys Asn Ala Leu Ala Ile
           195                 200                 205
   Ile Leu Leu Lys Gly Asn Gly Pro Glu Cys Asn
       210                 215
<210> 343
   <211> 657
   <212> DNA
   <213> Phakopsora pachyrhizi
<220>
   <221> misc_feature
   <222> (1)..(657)
   <223> ta09379.01_phapa
<400> 343
   atgctttttg ctacactaat agccgtttgc ttacttgctc tgggaggcaa agctgagagt    60
   gatgtccaaa gtgataccgc atctaaactt cagcgacgag gacatgatga ctctctacct   120
   cctgtgacat ttattatgag agatagcaac gagcatgtag gcggaaagct gttgatctac   180
   aattctgatg gaacactagc tttcaccttt cgcagagcag ttttgaactc tgatggcctt   240
   tccaacgttg aagtcagaga tgtgcgaaat aatttttcga taaacttaga atctaacgat   300
   gacacctgct ttaaaaagtc tcactatgtt gaaagagaaa aaaatctggg tcaattcaaa   360
   attgatccac gaggtgcaaa agcagacaga tggcatttca ctaggaaaac tccacacgga   420
   gatttcaagt atgactttca tcgcaagtat ttttcgaagg acggtaatat ttacataaaa   480
   gacacccatg tgcgagtggc ttccctgaca tccgaaattc gacatgaagc ttggctccaa   540
   ccggggaaac atggtgttcc aacattctca ttgcatcttc aatataacac tgagcctatc   600
   ttttttgtag ctttgatggg cttagatctc accagagtag acacatgtgg actttga      657
<210> 344
   <211> 218
   <212> PRT
   <213> Phakopsora pachyrhizi
<220>
   <221> VARIANT
   <222> (1)..(218)
   <223> ta09379.01_phapa
<400> 344
   Met Leu Phe Ala Thr Leu Ile Ala Val Cys Leu Leu Ala Leu Gly Gly
   1               5                   10                  15
   Lys Ala Glu Ser Asp Val Gln Ser Asp Thr Ala Ser Lys Leu Gln Arg
               20                  25                  30
   Arg Gly His Asp Asp Ser Leu Pro Pro Val Thr Phe Ile Met Arg Asp
           35                  40                  45
   Ser Asn Glu His Val Gly Gly Lys Leu Leu Ile Tyr Asn Ser Asp Gly
       50                  55                  60
   Thr Leu Ala Phe Thr Phe Arg Arg Ala Val Leu Asn Ser Asp Gly Leu
   65                  70                  75                  80
   Ser Asn Val Glu Val Arg Asp Val Arg Asn Asn Phe Ser Ile Asn Leu
                   85                  90                  95
   Glu Ser Asn Asp Asp Thr Cys Phe Lys Lys Ser His Tyr Val Glu Arg
               100                 105                 110
   Glu Lys Asn Leu Gly Gln Phe Lys Ile Asp Pro Arg Gly Ala Lys Ala
           115                 120                 125
   Asp Arg Trp His Phe Thr Arg Lys Thr Pro His Gly Asp Phe Lys Tyr
       130                 135                 140
   Asp Phe His Arg Lys Tyr Phe Ser Lys Asp Gly Asn Ile Tyr Ile Lys
   145                 150                 155                 160
   Asp Thr His Val Arg Val Ala Ser Leu Thr Ser Glu Ile Arg His Glu
                   165                 170                 175
   Ala Trp Leu Gln Pro Gly Lys His Gly Val Pro Thr Phe Ser Leu His
               180                 185                 190
   Leu Gln Tyr Asn Thr Glu Pro Ile Phe Phe Val Ala Leu Met Gly Leu
           195                 200                 205
   Asp Leu Thr Arg Val Asp Thr Cys Gly Leu
       210                 215
<210> 345
   <211> 657
   <212> DNA
   <213> Phakopsora pachyrhizi
<220>
   <221> misc_feature
   <222> (1)..(657)
   <223> ta07208.01_phapa
<400> 345
   atgagtctaa gttttaaggc tttactagtc tttctaccag tgatcttctt ctacctggtg       60
   ttggctgtaa aacaaccatc agatcaaaca actcattcta cccaaccaca gaagcataac      120
   actcatcaaa catctatcaa ctcaaagcga gaactacaac agcaactcag tcagttcaat      180
   ccacatcatc aatctttagc ttcatcaacc aggatgtcat cagtcaatga tagaacagct      240
   tcaccaatca cagatattga ctcaaacaca aacgttccaa cagctgccaa gggtgaggag      300
   gagtccacaa aacagaagct tgaagatcaa ccagcaggcg atggtacaaa ggcatcatta      360
   tcagctaacc ctggtgaatc gcaacagagt gacgagaaga atcttaaatc tcagaatgtt      420
   tccccgagtc aagaacacat gagtagatac attgtcactt tctcgcgtga gactacttct      480
   gcccagttga aggaatacgc tgataaaatc attcagagag gtggaaagat taaccatacg      540
   tatgattcag cgattcttaa aggatttgct gtttctataa gtgatagtct ggttacaacc      600
   ctggatgatg atccaaatgt aaagagtgtt gaacctgatg gtgaagtaca catctga         657
<210> 346
   <211> 218
   <212> PRT
   <213> Phakopsora pachyrhizi
<220>
   <221> VARIANT
   <222> (1)..(218)
   <223> ta07208.01_phapa
<400> 346
   Met Ser Leu Ser Phe Lys Ala Leu Leu Val Phe Leu Pro Val Ile Phe
   1               5                   10                  15
   Phe Tyr Leu Val Leu Ala Val Lys Gln Pro Ser Asp Gln Thr Thr His
               20                  25                  30
   Ser Thr Gln Pro Gln Lys His Asn Thr His Gln Thr Ser Ile Asn Ser
           35                  40                  45
   Lys Arg Glu Leu Gln Gln Gln Leu Ser Gln Phe Asn Pro His His Gln
       50                  55                  60
   Ser Leu Ala Ser Ser Thr Arg Met Ser Ser Val Asn Asp Arg Thr Ala
   65                  70                  75                  80
   Ser Pro Ile Thr Asp Ile Asp Ser Asn Thr Asn Val Pro Thr Ala Ala
                   85                  90                  95
   Lys Gly Glu Glu Glu Ser Thr Lys Gln Lys Leu Glu Asp Gln Pro Ala
               100                 105                 110
   Gly Asp Gly Thr Lys Ala Ser Leu Ser Ala Asn Pro Gly Glu Ser Gln
           115                 120                 125
   Gln Ser Asp Glu Lys Asn Leu Lys Ser Gln Asn Val Ser Pro Ser Gln
       130                 135                 140
   Glu His Met Ser Arg Tyr Ile Val Thr Phe Ser Arg Glu Thr Thr Ser
   145                 150                 155                 160
   Ala Gln Leu Lys Glu Tyr Ala Asp Lys Ile Ile Gln Arg Gly Gly Lys
                   165                 170                 175
   Ile Asn His Thr Tyr Asp Ser Ala Ile Leu Lys Gly Phe Ala Val Ser
               180                 185                 190
   Ile Ser Asp Ser Leu Val Thr Thr Leu Asp Asp Asp Pro Asn Val Lys
           195                 200                 205
   Ser Val Glu Pro Asp Gly Glu Val His Ile
       210                 215
<210> 347
   <211> 651
   <212> DNA
   <213> Phakopsora pachyrhizi
<220>
   <221> misc_feature
   <222> (1)..(651)
   <223> ta05865.01_phapa
<400> 347
   atgtttggtt taaaggctat cataatcgcc actggcttga tctatcaggt cactgcggct      60
   gccaatacct ctgccaccac tgctcccgga caggatcagg gccaaggtca aggtcctgaa     120
   tctggtggtt gtgcagccaa gtgtatggcc gcaaagctgg atgacgctgc caagtggttt     180
   ggtccaggta accttgcatc ctactgtcaa catgccgagt tcatcactgc atacgacaca     240
   tgtttgggcg ataactgtgc caaccatgaa gagctagatg ccggtaagaa aagcggtcgt     300
   gaggcttgcg ctgctgctgg agtcacctca ccgggatcga ttgccccacc cacaggacat     360
   acccctccta ccagtgggaa taactctcta gcagatttgc ctgccggtgc tactaatcat     420
   agcatgagca ctaattctac tccaagtgca actaccacca actctaccct tggtggtacc     480
   ctcaataaca cccttcatac ccctaacacc acctccaccg ctactaacag tagattttcc     540
   agtgcggtta actctgctgc ttctgctgct gccaacagta cagcttcaac actcactagc     600
   tcttctatct tgcttggtat ctcttctgta ctggtgattg ccaacctcta a              651
<210> 348
   <211> 216
   <212> PRT
   <213> Phakopsora pachyrhizi
<220>
   <221> VARIANT
   <222> (1) .. (216)
   <223> ta05865.01_phapa
<400> 348
   Met Phe Gly Leu Lys Ala Ile Ile Ile Ala Thr Gly Leu Ile Tyr Gln
   1               5                   10                  15
   Val Thr Ala Ala Ala Asn Thr Ser Ala Thr Thr Ala Pro Gly Gln Asp
               20                  25                  30
   Gln Gly Gln Gly Gln Gly Pro Glu Ser Gly Gly Cys Ala Ala Lys Cys
           35                  40                  45
   Met Ala Ala Lys Leu Asp Asp Ala Ala Lys Trp Phe Gly Pro Gly Asn
       50                  55                  60
   Leu Ala Ser Tyr Cys Gln His Ala Glu Phe Ile Thr Ala Tyr Asp Thr
   65                  70                  75                  80
   Cys Leu Gly Asp Asn Cys Ala Asn His Glu Glu Leu Asp Ala Gly Lys
                   85                  90                  95
   Lys Ser Gly Arg Glu Ala Cys Ala Ala Ala Gly Val Thr Ser Pro Gly
               100                 105                 110
   Ser Ile Ala Pro Pro Thr Gly His Thr Pro Pro Thr Ser Gly Asn Asn
           115                 120                 125
   Ser Leu Ala Asp Leu Pro Ala Gly Ala Thr Asn His Ser Met Ser Thr
       130                 135                 140
   Asn Ser Thr Pro Ser Ala Thr Thr Thr Asn Ser Thr Leu Gly Gly Thr
   145                 150                 155                 160
   Leu Asn Asn Thr Leu His Thr Pro Asn Thr Thr Ser Thr Ala Thr Asn
                   165                 170                 175
   Ser Arg Phe Ser Ser Ala Val Asn Ser Ala Ala Ser Ala Ala Ala Asn
               180                 185                 190
   Ser Thr Ala Ser Thr Leu Thr Ser Ser Ser Ile Leu Leu Gly Ile Ser
           195                 200                 205
   Ser Val Leu Val Ile Ala Asn Leu
       210                 215
<210> 349
   <211> 651
   <212> DNA
   <213> Phakopsora pachyrhizi
<220>
   <221> misc_feature
   <222> (1)..(651)
   <223> ta05865.02_phapa
<400> 349
   atgtttggtt taaaggctat cataatcgcc actggcttga tctatcaggt cactgcggct      60
   gccaatacct ctgccaccac tgctcccgga caggatcagg gccaaggtca aggtcctgaa     120
   tctggtggtt gtgcagccaa gtgtatggcc gcaaagctgg atgacgctgc caagtggttt     180
   ggtccaggta accttgcatc ctactgtcaa catgccgagt tcatcactgc atacgacaca     240
   tgtttgggcg ataactgtgc caaccatgaa gagctagatg ccggtaagaa aagcggtcgt     300
   gaggcttgcg ctgctgctgg agtcacctca ccgggatcga ttgccccacc cacaggacat     360
   acccctccta ccagtgggaa taactctcta gcagatttgc ctgccggtgc tactaatcat     420
   agcatgagca ctaattctac tccaagtgca actaccacca actctaccct tggtggtacc     480
   ctcaataaca cccttcatac ccctaacacc acctccaccg ctactaacag tagattttcc     540
   agtgcggtta actctgctgc ttctgctgct gccaacagta cagcttcaac actcactagc     600
   tcttctatct tgcttggtat ctcttctgta ctggtgattg ccaacctcta a              651
<210> 350
   <211> 216
   <212> PRT
   <213> Phakopsora pachyrhizi
<220>
   <221> VARIANT
   <222> (1) .. (216)
   <223> ta05865.02_phapa
<400> 350
   Met Phe Gly Leu Lys Ala Ile Ile Ile Ala Thr Gly Leu Ile Tyr Gln
   1               5                   10                  15
   Val Thr Ala Ala Ala Asn Thr Ser Ala Thr Thr Ala Pro Gly Gln Asp
               20                  25                  30
   Gln Gly Gln Gly Gln Gly Pro Glu Ser Gly Gly Cys Ala Ala Lys Cys
           35                  40                  45
   Met Ala Ala Lys Leu Asp Asp Ala Ala Lys Trp Phe Gly Pro Gly Asn
       50                  55                  60
   Leu Ala Ser Tyr Cys Gln His Ala Glu Phe Ile Thr Ala Tyr Asp Thr
   65                  70                  75                  80
   Cys Leu Gly Asp Asn Cys Ala Asn His Glu Glu Leu Asp Ala Gly Lys
                   85                  90                  95
   Lys Ser Gly Arg Glu Ala Cys Ala Ala Ala Gly Val Thr Ser Pro Gly
               100                 105                 110
   Ser Ile Ala Pro Pro Thr Gly His Thr Pro Pro Thr Ser Gly Asn Asn
           115                 120                 125
   Ser Leu Ala Asp Leu Pro Ala Gly Ala Thr Asn His Ser Met Ser Thr
       130                 135                 140
   Asn Ser Thr Pro Ser Ala Thr Thr Thr Asn Ser Thr Leu Gly Gly Thr
   145                 150                 155                 160
   Leu Asn Asn Thr Leu His Thr Pro Asn Thr Thr Ser Thr Ala Thr Asn
                   165                 170                 175
   Ser Arg Phe Ser Ser Ala Val Asn Ser Ala Ala Ser Ala Ala Ala Asn
               180                 185                 190
   Ser Thr Ala Ser Thr Leu Thr Ser Ser Ser Ile Leu Leu Gly Ile Ser
           195                 200                 205
   Ser Val Leu Val Ile Ala Asn Leu
       210                 215
<210> 351
   <211> 651
   <212> DNA
   <213> Phakopsora pachyrhizi
<220>
   <221> misc_feature
   <222> (1)..(651)
   <223> ta00656.02_phapa
<400> 351
   atgattgcca accccgtcaa ttttgttttc ttgttgagcg tatttctgag ctttcaaatt      60
   cagaatgcct caacaaaaag aggtagaggg ggaccgattg cagctctctg ccaaggaaag     120
   gagcaaaatg tgaaagttat cgctacttgc agtgaaggaa aaattttatg cgagggtttg     180
   gatgaaagtg gtactggaac aatgtcttgt tcagatggag gaaaactttt agaggtaaaa     240
   agtgaagaag ccgctcagtg tggcgccaat ccccgttgca agaagttggc gaagggaagt     300
   aaagttattg aattaggaaa ggttgtgggt gcagctctag ctgatggcaa gtccctcgca     360
   cctggaagca gagcagcagt ccaaacaggg ggtaaagaag gtaaagaagg taaagagggt     420
   aaagagggta aagagggtaa agatgataaa ggtggtaaag ctgttgattt ggctctgggc     480
   aagggtgatg caaaagctgc tgattcagca aacggggtta aagtagatgt aaaaggcaaa     540
   gagttggcaa atgcgggcaa ggaaaacaat aaagagaatg aggcacagaa agctacagaa     600
   cctactctca atactcaggc tgcttcaggt ggagaagagg taaaagcata g              651
<210> 352
   <211> 216
   <212> PRT
   <213> Phakopsora pachyrhizi
<220>
   <221> VARIANT
   <222> (1) .. (216)
   <223> ta00656.02_phapa
<400> 352
   Met Ile Ala Asn Pro Val Asn Phe Val Phe Leu Leu Ser Val Phe Leu
   1               5                   10                  15
   Ser Phe Gln Ile Gln Asn Ala Ser Thr Lys Arg Gly Arg Gly Gly Pro
               20                  25                  30
   Ile Ala Ala Leu Cys Gln Gly Lys Glu Gln Asn Val Lys Val Ile Ala
           35                  40                  45
   Thr Cys Ser Glu Gly Lys Ile Leu Cys Glu Gly Leu Asp Glu Ser Gly
       50                  55                  60
   Thr Gly Thr Met Ser Cys Ser Asp Gly Gly Lys Leu Leu Glu Val Lys
   65                  70                  75                  80
   Ser Glu Glu Ala Ala Gln Cys Gly Ala Asn Pro Arg Cys Lys Lys Leu
                   85                  90                  95
   Ala Lys Gly Ser Lys Val Ile Glu Leu Gly Lys Val Val Gly Ala Ala
               100                 105                 110
   Leu Ala Asp Gly Lys Ser Leu Ala Pro Gly Ser Arg Ala Ala Val Gln
           115                 120                 125
   Thr Gly Gly Lys Glu Gly Lys Glu Gly Lys Glu Gly Lys Glu Gly Lys
       130                 135                 140
   Glu Gly Lys Asp Asp Lys Gly Gly Lys Ala Val Asp Leu Ala Leu Gly
   145                 150                 155                 160
   Lys Gly Asp Ala Lys Ala Ala Asp Ser Ala Asn Gly Val Lys Val Asp
                   165                 170                 175
   Val Lys Gly Lys Glu Leu Ala Asn Ala Gly Lys Glu Asn Asn Lys Glu
               180                 185                 190
   Asn Glu Ala Gln Lys Ala Thr Glu Pro Thr Leu Asn Thr Gln Ala Ala
           195                 200                 205
   Ser Gly Gly Glu Glu Val Lys Ala
       210                 215
<210> 353
   <211> 645
   <212> DNA
   <213> Phakopsora pachyrhizi
<220>
   <221> misc_feature
   <222> (1)..(645)
   <223> ta09778.01_phapa
<400> 353
   atgcttcgct taatttcgct aggacttttg gtatgctctg ccgcaagatt gaccgtgagc      60
   agtccaagcg atctctcact aagacctcgt cagcttggct taggtgccgg cggagcagcc     120
   gcaggttcct tcagcagtag taccagcgta tcgacattta gctccttcgt tcaaggttgg     180
   agcgtcattc caactgcttt cggaacttgc gcggccacct tccaacaaag agttactgtt     240
   caagttgctg ttcagtcggt acagcagtta tacagtactg tgaatggagt tctaggacat     300
   tacggtggtt gtggtggttg cggtggtgct tctgctgctg gtgcttacgc ttctaaatat     360
   caatccatca tcacccagag tttcacttcc tggcaaacta tacttcaagt tgggcaatcc     420
   tcatacgcca atgcttggga ctctcaattt attcctctct tccgacaatt caaccctttc     480
   cttacagcgg tacaacagaa ttccggactt tttggcatca atctcggtaa ccttcttggt     540
   ggtctccacc ttaacatcaa cctcttctct tcatgtggtc ttaacgttgg tggactgcta     600
   gggggtgttt tgaatgtagt cggtggatta ttgggcggtc attga                     645
<210> 354
   <211> 214
   <212> PRT
   <213> Phakopsora pachyrhizi
<220>
   <221> VARIANT
   <222> (1)..(214)
   <223> ta09778.01_phapa
<400> 354
   Met Leu Arg Leu Ile Ser Leu Gly Leu Leu Val Cys Ser Ala Ala Arg
   1               5                   10                  15
   Leu Thr Val Ser Ser Pro Ser Asp Leu Ser Leu Arg Pro Arg Gln Leu
               20                  25                  30
   Gly Leu Gly Ala Gly Gly Ala Ala Ala Gly Ser Phe Ser Ser Ser Thr
           35                  40                  45
   Ser Val Ser Thr Phe Ser Ser Phe Val Gln Gly Trp Ser Val Ile Pro
       50                  55                  60
   Thr Ala Phe Gly Thr Cys Ala Ala Thr Phe Gln Gln Arg Val Thr Val
   65                  70                  75                  80
   Gln Val Ala Val Gln Ser Val Gln Gln Leu Tyr Ser Thr Val Asn Gly
                   85                  90                  95
   Val Leu Gly His Tyr Gly Gly Cys Gly Gly Cys Gly Gly Ala Ser Ala
               100                 105                 110
   Ala Gly Ala Tyr Ala Ser Lys Tyr Gln Ser Ile Ile Thr Gln Ser Phe
           115                 120                 125
   Thr Ser Trp Gln Thr Ile Leu Gln Val Gly Gln Ser Ser Tyr Ala Asn
       130                 135                 140
   Ala Trp Asp Ser Gln Phe Ile Pro Leu Phe Arg Gln Phe Asn Pro Phe
   145                 150                 155                 160
   Leu Thr Ala Val Gln Gln Asn Ser Gly Leu Phe Gly Ile Asn Leu Gly
                   165                 170                 175
   Asn Leu Leu Gly Gly Leu His Leu Asn Ile Asn Leu Phe Ser Ser Cys
               180                 185                 190
   Gly Leu Asn Val Gly Gly Leu Leu Gly Gly Val Leu Asn Val Val Gly
           195                 200                 205
   Gly Leu Leu Gly Gly His
       210
<210> 355
   <211> 645
   <212> DNA
   <213> Phakopsora pachyrhizi
<220>
   <221> misc_feature
   <222> (1)..(645)
   <223> ta00721.08_phapa
<400> 355
   atgaaattgt catacgccta cttgattgtt ctcatctctg tcctgtcttc aacttttgtg      60
   gtagctgagg atgttagcga caaaaacgct gaggtttctg acagcaagtt ctttggacta     120
   ggtggacttg ggggttgggg ttactgggga ggtggtagat ttgctggtct tggtggatac     180
   tcgtggttga acccatggat gggaaatgcc tacgggtttg gtctctaccg cggattttat     240
   ggtggatggc tcaaatcggc tgatggtcac caagagcgtc gatcaatcca ggaattgcac     300
   aacctcatgt cacgggctga tcacaccgta tcatgcaaga acaagaacgg tgaagttgct     360
   catttcgaaa ccaagagctg cttgagtgct gcaaacaagt tagctaatca acacgcttcg     420
   agcgctacct gtggcgcgtg ctctctgagc atccaaggtc ccaacggtgc tctatctgct     480
   aagtctattc catcatctga gttgacaaag gcgactctta acattttgaa ggcttgcgcc     540
   aaaggtgaaa gcaagatgct tgctgcttca gagcttgagc gccgctctcc attgcccgag     600
   gaaatttcca tctcaatcct catccagcaa cgaaaagagc tctaa                     645
<210> 356
   <211> 214
   <212> PRT
   <213> Phakopsora pachyrhizi
<220>
   <221> VARIANT
   <222> (1)..(214)
   <223> ta00721.08_phapa
<400> 356
   Met Lys Leu Ser Tyr Ala Tyr Leu Ile Val Leu Ile Ser Val Leu Ser
   1               5                   10                  15
   Ser Thr Phe Val Val Ala Glu Asp Val Ser Asp Lys Asn Ala Glu Val
               20                  25                  30
   Ser Asp Ser Lys Phe Phe Gly Leu Gly Gly Leu Gly Gly Trp Gly Tyr
           35                  40                  45
   Trp Gly Gly Gly Arg Phe Ala Gly Leu Gly Gly Tyr Ser Trp Leu Asn
       50                  55                  60
   Pro Trp Met Gly Asn Ala Tyr Gly Phe Gly Leu Tyr Arg Gly Phe Tyr
   65                  70                  75                  80
   Gly Gly Trp Leu Lys Ser Ala Asp Gly His Gln Glu Arg Arg Ser Ile
                   85                  90                  95
   Gln Glu Leu His Asn Leu Met Ser Arg Ala Asp His Thr Val Ser Cys
               100                 105                 110
   Lys Asn Lys Asn Gly Glu Val Ala His Phe Glu Thr Lys Ser Cys Leu
           115                 120                 125
   Ser Ala Ala Asn Lys Leu Ala Asn Gln His Ala Ser Ser Ala Thr Cys
       130                 135                 140
   Gly Ala Cys Ser Leu Ser Ile Gln Gly Pro Asn Gly Ala Leu Ser Ala
   145                 150                 155                 160
   Lys Ser Ile Pro Ser Ser Glu Leu Thr Lys Ala Thr Leu Asn Ile Leu
                   165                 170                 175
   Lys Ala Cys Ala Lys Gly Glu Ser Lys Met Leu Ala Ala Ser Glu Leu
               180                 185                 190
   Glu Arg Arg Ser Pro Leu Pro Glu Glu Ile Ser Ile Ser Ile Leu Ile
           195                 200                 205
   Gln Gln Arg Lys Glu Leu
       210
<210> 357
   <211> 642
   <212> DNA
   <213> Phakopsora pachyrhizi
<220>
   <221> misc_feature
   <222> (1)..(642)
   <223> ta04963.02_phapa
<400> 357
   atggtttact caaaggcaac cttcaccctg gtctcactcc tacctatgat cctggcccag      60
   cagagcatca acaacagtga taccacaacc acggctgcta ccccggccag tcccaccaac     120
   agcagcagct ctaccactac ttccactacc acctctaaca ctgccagtgg agctgctcag     180
   tccttccctg cgccaacagt ctcaggaaca tcctgtggta cagcccagca gaactttaac     240
   cagtgtgtta ccaaagtatc aaaggatatc tcctcatgtc cctctaccga taacacctgt     300
   ctttgccaaa cgtatgcaaa cctagcctac tgttacaacg cctgtccaga tctggcatca     360
   tcaggggccg gatacctgca gcagtccaca gtgaactgtg atgcagcagg tatcaagcca     420
   aacgctactt ccaacgtcac caccacccct gtcacctcca ccactaacag gaacaccacc     480
   aacacctcac ccatctctaa cacaaacaag aacaattcta cctcgggcaa cactgctgct     540
   acatttgctg ctggaaactc caaggcaagt gggattgagg ccccgttact gagcgttgct     600
   gttgttgggc tgtgtggtat cattgctagc ctatttgcat ga                        642
<210> 358
   <211> 213
   <212> PRT
   <213> Phakopsora pachyrhizi
<220>
   <221> VARIANT
   <222> (1)..(213)
   <223> ta04963.02_phapa
<400> 358
   Met Val Tyr Ser Lys Ala Thr Phe Thr Leu Val Ser Leu Leu Pro Met
   1               5                   10                  15
   Ile Leu Ala Gln Gln Ser Ile Asn Asn Ser Asp Thr Thr Thr Thr Ala
               20                  25                  30
   Ala Thr Pro Ala Ser Pro Thr Asn Ser Ser Ser Ser Thr Thr Thr Ser
           35                  40                  45
   Thr Thr Thr Ser Asn Thr Ala Ser Gly Ala Ala Gln Ser Phe Pro Ala
       50                  55                  60
   Pro Thr Val Ser Gly Thr Ser Cys Gly Thr Ala Gln Gln Asn Phe Asn
   65                  70                  75                  80
   Gln Cys Val Thr Lys Val Ser Lys Asp Ile Ser Ser Cys Pro Ser Thr
                   85                  90                  95
   Asp Asn Thr Cys Leu Cys Gln Thr Tyr Ala Asn Leu Ala Tyr Cys Tyr
               100                 105                 110
   Asn Ala Cys Pro Asp Leu Ala Ser Ser Gly Ala Gly Tyr Leu Gln Gln
           115                 120                 125
   Ser Thr Val Asn Cys Asp Ala Ala Gly Ile Lys Pro Asn Ala Thr Ser
       130                 135                 140
   Asn Val Thr Thr Thr Pro Val Thr Ser Thr Thr Asn Arg Asn Thr Thr
   145                 150                 155                 160
   Asn Thr Ser Pro Ile Ser Asn Thr Asn Lys Asn Asn Ser Thr Ser Gly
                   165                 170                 175
   Asn Thr Ala Ala Thr Phe Ala Ala Gly Asn Ser Lys Ala Ser Gly Ile
               180                 185                 190
   Glu Ala Pro Leu Leu Ser Val Ala Val Val Gly Leu Cys Gly Ile Ile
           195                 200                 205
   Ala Ser Leu Phe Ala
       210
<210> 359
   <211> 639
   <212> DNA
   <213> Phakopsora pachyrhizi
<220>
   <221> misc_feature
   <222> (1)..(639)
   <223> ta02061.01_phapa
<400> 359
   atgaatcaat ggttaattat tctatggatc tcacttctgg cttctcaagc cacaagaggt       60
   agcgggatca ttgaaatcaa ctcaaactct ctgtcgaacg agcttatata ttgggatagt      120
   aagaatgaag aaattttggg cactttaaag agtcgggaag atatggaaag actcaagaga      180
   ttggggcaac ggctaagagt ctcattttca tcgagtacat tgccgctagt cgaaatcaat      240
   tctacaagca gaaggtttct gaagcttact tcagaagatg aaaattttac agacctctca      300
   gtctcagggt tagctataaa tcagaaaagg ttttccaaga ggtcagaggg actggggaaa      360
   gagatcgttg atgcaatctt tccgattaaa ggaactgtta cgacgatcgc tgagacgata      420
   aatccaaaaa aatcaatctt tccaggttct aggattttta ataagccaca cgggacgatg      480
   ggaaccaatt atctggattt gacgaggaaa gatgttacaa tggcacaaat gtcaaaggcc      540
   aagccccagg gcaatgtaat tcaggatagt atcattcaag tcctgacggc cagtcaaaga      600
   caggtcagaa atcctagggt gacggttgac tccaaataa                             639
<210> 360
   <211> 212
   <212> PRT
   <213> Phakopsora pachyrhizi
<220>
   <221> VARIANT
   <222> (1)..(212)
   <223> ta02061.01_phapa
<400> 360
   Met Asn Gln Trp Leu Ile Ile Leu Trp Ile Ser Leu Leu Ala Ser Gln
   1               5                   10                  15
   Ala Thr Arg Gly Ser Gly Ile Ile Glu Ile Asn Ser Asn Ser Leu Ser
               20                  25                  30
   Asn Glu Leu Ile Tyr Trp Asp Ser Lys Asn Glu Glu Ile Leu Gly Thr
           35                  40                  45
   Leu Lys Ser Arg Glu Asp Met Glu Arg Leu Lys Arg Leu Gly Gln Arg
       50                  55                  60
   Leu Arg Val Ser Phe Ser Ser Ser Thr Leu Pro Leu Val Glu Ile Asn
   65                  70                  75                  80
   Ser Thr Ser Arg Arg Phe Leu Lys Leu Thr Ser Glu Asp Glu Asn Phe
                   85                  90                  95
   Thr Asp Leu Ser Val Ser Gly Leu Ala Ile Asn Gln Lys Arg Phe Ser
               100                 105                 110
   Lys Arg Ser Glu Gly Leu Gly Lys Glu Ile Val Asp Ala Ile Phe Pro
           115                 120                 125
   Ile Lys Gly Thr Val Thr Thr Ile Ala Glu Thr Ile Asn Pro Lys Lys
       130                 135                 140
   Ser Ile Phe Pro Gly Ser Arg Ile Phe Asn Lys Pro His Gly Thr Met
   145                 150                 155                 160
   Gly Thr Asn Tyr Leu Asp Leu Thr Arg Lys Asp Val Thr Met Ala Gln
                   165                 170                 175
   Met Ser Lys Ala Lys Pro Gln Gly Asn Val Ile Gln Asp Ser Ile Ile
               180                 185                 190
   Gln Val Leu Thr Ala Ser Gln Arg Gln Val Arg Asn Pro Arg Val Thr
           195                 200                 205
   Val Asp Ser Lys
       210
<210> 361
   <211> 633
   <212> DNA
   <213> Phakopsora pachyrhizi
<220>
   <221> misc_feature
   <222> (1)..(633)
   <223> ta08296.04_phapa
<400> 361
   atgcgcagct ttgtttattg tgccttgctc gtcttaattt gtcagcttgc tacaagctcg      60
   cctattaatg aaagatctga ggttgaggct tccaaagagc agtcttcgga gaagtttttt     120
   ccacttttcc ctcccccacc accagtgccg tttatacgcc cacctttacc accttttgga     180
   tttaaagaca atcaccaaaa tgcgaagaga tccgaggttg agactgccaa agaggagtct     240
   tcggagaaat tttttcccat tctacctccc cccccaccac caccattttt ccgcccccat     300
   ctaccaccct ttggattcaa agacagccat caaaatgaga aaaggtctga ggttgctgct     360
   caccaagaga gccagtcgac agagaaattt tttccagggt atggtttccc gatcctacca     420
   ccaccacatc cattttttta ccaccgtcgt ccttttggtt atggcttttt tccaccacca     480
   ccttttggat ttaaagacaa acaccaaaat gagaagcgat ctgaggtcga atcctctaag     540
   gaaaagtcaa ctgagaagtt ttttcccagg tctcttaccg attttgccac cttttccccc     600
   atttggcttc ctaaaagatg ccaagagcaa tga                                  633
<210> 362
   <211> 210
   <212> PRT
   <213> Phakopsora pachyrhizi
<220>
   <221> VARIANT
   <222> (1)..(210)
   <223> ta08296.04_phapa
<400> 362
   Met Arg Ser Phe Val Tyr Cys Ala Leu Leu Val Leu Ile Cys Gln Leu
   1               5                   10                  15
   Ala Thr Ser Ser Pro Ile Asn Glu Arg Ser Glu Val Glu Ala Ser Lys
               20                  25                  30
   Glu Gln Ser Ser Glu Lys Phe Phe Pro Leu Phe Pro Pro Pro Pro Pro
           35                  40                  45
   Val Pro Phe Ile Arg Pro Pro Leu Pro Pro Phe Gly Phe Lys Asp Asn
       50                  55                  60
   His Gln Asn Ala Lys Arg Ser Glu Val Glu Thr Ala Lys Glu Glu Ser
   65                  70                  75                  80
   Ser Glu Lys Phe Phe Pro Ile Leu Pro Pro Pro Pro Pro Pro Pro Phe
                   85                  90                  95
   Phe Arg Pro His Leu Pro Pro Phe Gly Phe Lys Asp Ser His Gln Asn
               100                 105                 110
   Glu Lys Arg Ser Glu Val Ala Ala His Gln Glu Ser Gln Ser Thr Glu
           115                 120                 125
   Lys Phe Phe Pro Gly Tyr Gly Phe Pro Ile Leu Pro Pro Pro His Pro
       130                 135                 140
   Phe Phe Tyr His Arg Arg Pro Phe Gly Tyr Gly Phe Phe Pro Pro Pro
   145                 150                 155                 160
   Pro Phe Gly Phe Lys Asp Lys His Gln Asn Glu Lys Arg Ser Glu Val
                   165                 170                 175
   Glu Ser Ser Lys Glu Lys Ser Thr Glu Lys Phe Phe Pro Arg Ser Leu
               180                 185                 190
   Thr Asp Phe Ala Thr Phe Ser Pro Ile Trp Leu Pro Lys Arg Cys Gln
           195                 200                 205
   Glu Gln
   210
<210> 363
   <211> 633
   <212> DNA
   <213> Phakopsora pachyrhizi
<220>
   <221> misc_feature
   <222> (1)..(633)
   <223> ta01247.01_phapa
<400> 363
   atgaatatga gaagaactgt tacaagtttt gggttaaaga gtttaacaat tttcattgga      60
   gtctggcttc aactttcaac tttctcaatc gcaacgatat gtatgagtag gaaaagcagc     120
   ttgaacccaa agatccttgc ctctgactgc aacatggtat tggacctcta caagcaatca     180
   caatttaagt cagatgaaac agttacccta caacattctg gaaaaaacaa aaagacatgc     240
   ttttcatgtc aacttgtttt cactacaagg ttagaaggag aatttcaacc aattttctct     300
   aaaacggaag cgcttgaagg tataaaaagt gtcttggata catgtggagg aggacctggc     360
   ctattcatga ttactcaagt cataccagat aactcaaacc caattctaaa catcactcaa     420
   ccgttagtga tccaagtacg aaaagggagc ggaaaggatt gcaaggcaaa gattaagagc     480
   aaaaattccc aatcaaacga aatagatgct tctttgttgg aatcaaacaa taccggattc     540
   attctctcaa actctggagt ttcagatgag accgataaga aagatcccag gaatcctact     600
   cgcagctttt ttgttccaat cataaggagt taa                                  633
<210> 364
   <211> 210
   <212> PRT
   <213> Phakopsora pachyrhizi
<220>
   <221> VARIANT
   <222> (1)..(210)
   <223> ta01247.01_phapa
<400> 364
   Met Asn Met Arg Arg Thr Val Thr Ser Phe Gly Leu Lys Ser Leu Thr
   1               5                   10                  15
   Ile Phe Ile Gly Val Trp Leu Gln Leu Ser Thr Phe Ser Ile Ala Thr
               20                  25                  30
   Ile Cys Met Ser Arg Lys Ser Ser Leu Asn Pro Lys Ile Leu Ala Ser
           35                  40                  45
   Asp Cys Asn Met Val Leu Asp Leu Tyr Lys Gln Ser Gln Phe Lys Ser
       50                  55                  60
   Asp Glu Thr Val Thr Leu Gln His Ser Gly Lys Asn Lys Lys Thr Cys
   65                  70                  75                  80
   Phe Ser Cys Gln Leu Val Phe Thr Thr Arg Leu Glu Gly Glu Phe Gln
                   85                  90                  95
   Pro Ile Phe Ser Lys Thr Glu Ala Leu Glu Gly Ile Lys Ser Val Leu
               100                 105                 110
   Asp Thr Cys Gly Gly Gly Pro Gly Leu Phe Met Ile Thr Gln Val Ile
           115                 120                 125
   Pro Asp Asn Ser Asn Pro Ile Leu Asn Ile Thr Gln Pro Leu Val Ile
       130                 135                 140
   Gln Val Arg Lys Gly Ser Gly Lys Asp Cys Lys Ala Lys Ile Lys Ser
   145                 150                 155                 160
   Lys Asn Ser Gln Ser Asn Glu Ile Asp Ala Ser Leu Leu Glu Ser Asn
                   165                 170                 175
   Asn Thr Gly Phe Ile Leu Ser Asn Ser Gly Val Ser Asp Glu Thr Asp
               180                 185                 190
   Lys Lys Asp Pro Arg Asn Pro Thr Arg Ser Phe Phe Val Pro Ile Ile
           195                 200                 205
   Arg Ser
       210
<210> 365
   <211> 633
   <212> DNA
   <213> Phakopsora pachyrhizi
<220>
   <221> misc_feature
   <222> (1)..(633)
   <223> ta00656.03_phapa
<400> 365
   atgattgcca accccgtcaa ttttgttttc ttgttgagcg tatttctgag ctttcaaatt      60
   cagaatgcct caacaaaaag aggtagaggg ggaccgattg cagctctctg ccaaggaaag     120
   gagcaaaatg tgaaagttat cgctacttgc agtgaaggaa aaattttatg cgagggtttg     180
   gatgaaagtg gtactggaac aatgtcttgt tcagatggag gaaaactttt agaggtaaaa     240
   agtgaagaag ccgctcagtg tggcgccaat ccccgttgca agaagttggc gaagggaagt     300
   aaagttattg aattaggaaa ggttgtgggt gcagctctag ctgatggcaa gtccctcgca     360
   cctggaagca gagcagcagt ccaaacaggg ggtaaagaag gtaaagaagg taaagagggt     420
   aaagatgata aaggtggtaa agctgttgat ttggctctgg gcaagggtga tgcaaaagct     480
   gctgattcag caaacggggt taaagtagat gtaaaaggca aagagttggc aaatgcgggc     540
   aaggaaaaca ataaagagaa tgaggcacag aaagctacag aacctactct caatactcag     600
   gctgcttcag gtggagaaga ggtaaaagca tag                                  633
<210> 366
   <211> 210
   <212> PRT
   <213> Phakopsora pachyrhizi
<220>
   <221> VARIANT
   <222> (1)..(210)
   <223> ta00656.03_phapa
<400> 366
   Met Ile Ala Asn Pro Val Asn Phe Val Phe Leu Leu Ser Val Phe Leu
   1               5                   10                  15
   Ser Phe Gln Ile Gln Asn Ala Ser Thr Lys Arg Gly Arg Gly Gly Pro
               20                  25                  30
   Ile Ala Ala Leu Cys Gln Gly Lys Glu Gln Asn Val Lys Val Ile Ala
           35                  40                  45
   Thr Cys Ser Glu Gly Lys Ile Leu Cys Glu Gly Leu Asp Glu Ser Gly
       50                  55                  60
   Thr Gly Thr Met Ser Cys Ser Asp Gly Gly Lys Leu Leu Glu Val Lys
   65                  70                  75                  80
   Ser Glu Glu Ala Ala Gln Cys Gly Ala Asn Pro Arg Cys Lys Lys Leu
                   85                  90                  95
   Ala Lys Gly Ser Lys Val Ile Glu Leu Gly Lys Val Val Gly Ala Ala
               100                 105                 110
   Leu Ala Asp Gly Lys Ser Leu Ala Pro Gly Ser Arg Ala Ala Val Gln
           115                 120                 125
   Thr Gly Gly Lys Glu Gly Lys Glu Gly Lys Glu Gly Lys Asp Asp Lys
       130                 135                 140
   Gly Gly Lys Ala Val Asp Leu Ala Leu Gly Lys Gly Asp Ala Lys Ala
   145                 150                 155                 160
   Ala Asp Ser Ala Asn Gly Val Lys Val Asp Val Lys Gly Lys Glu Leu
                   165                 170                 175
   Ala Asn Ala Gly Lys Glu Asn Asn Lys Glu Asn Glu Ala Gln Lys Ala
               180                 185                 190
   Thr Glu Pro Thr Leu Asn Thr Gln Ala Ala Ser Gly Gly Glu Glu Val
           195                 200                 205
   Lys Ala
       210
<210> 367
   <211> 627
   <212> DNA
   <213> Phakopsora pachyrhizi
<220>
   <221> misc_feature
   <222> (1)..(627)
   <223> ta09267.08_phapa
<400> 367
   atgcgttcac ttatcgttcc caccctttta tgcgtgctat ttattcataa atcttgcgca      60
   accgttcaca ctcagtgcta caactatttt ttacaaaagg atggatgcgt tttctctgca     120
   gctgatgaca gaaatcgatg ttctgcagat cccaagccca gtacagccgt cggagtggtt     180
   caagcaagca acacaaatgt taaacgtcac actctggcac gacgctatga tactactcta     240
   ccatctcctt ctgtgcgtgg tgaagggatt tgcggaaact ataacacagc agaagctgag     300
   ggagctagtc tttgggttgg ccctaacccg gatagcacaa accctgaaga agcaggttgg     360
   ctcaacaagg gaaagacatc caattgcaac aagcaattat atataataaa ccctcgcacg     420
   cgtaaaactg tatacgtgaa tgtcatagac ggtcacgatt ttcagacaac gcagcctgat     480
   gttggatgct tccagattgc actcacccaa aataccttcc ttcagcttga tccaacagat     540
   gaagaaaaag aaaagggttc cataggatct ctcacatggg acttcaacaa ccttaatggc     600
   gctagtcctc aggatggccc tgtctaa                                         627
<210> 368
   <211> 208
   <212> PRT
   <213> Phakopsora pachyrhizi
<220>
   <221> VARIANT
   <222> (1)..(208)
   <223> ta09267.08_phapa
<400> 368
   Met Arg Ser Leu Ile Val Pro Thr Leu Leu Cys Val Leu Phe Ile His
   1               5                   10                  15
   Lys Ser Cys Ala Thr Val His Thr Gln Cys Tyr Asn Tyr Phe Leu Gln
               20                  25                  30
   Lys Asp Gly Cys Val Phe Ser Ala Ala Asp Asp Arg Asn Arg Cys Ser
           35                  40                  45
   Ala Asp Pro Lys Pro Ser Thr Ala Val Gly Val Val Gln Ala Ser Asn
       50                  55                  60
   Thr Asn Val Lys Arg His Thr Leu Ala Arg Arg Tyr Asp Thr Thr Leu
   65                  70                  75                  80
   Pro Ser Pro Ser Val Arg Gly Glu Gly Ile Cys Gly Asn Tyr Asn Thr
                   85                  90                  95
   Ala Glu Ala Glu Gly Ala Ser Leu Trp Val Gly Pro Asn Pro Asp Ser
               100                 105                 110
   Thr Asn Pro Glu Glu Ala Gly Trp Leu Asn Lys Gly Lys Thr Ser Asn
           115                 120                 125
   Cys Asn Lys Gln Leu Tyr Ile Ile Asn Pro Arg Thr Arg Lys Thr Val
       130                 135                 140
   Tyr Val Asn Val Ile Asp Gly His Asp Phe Gln Thr Thr Gln Pro Asp
   145                 150                 155                 160
   Val Gly Cys Phe Gln Ile Ala Leu Thr Gln Asn Thr Phe Leu Gln Leu
                   165                 170                 175
   Asp Pro Thr Asp Glu Glu Lys Glu Lys Gly Ser Ile Gly Ser Leu Thr
               180                 185                 190
   Trp Asp Phe Asn Asn Leu Asn Gly Ala Ser Pro Gln Asp Gly Pro Val
           195                 200                 205
<210> 369
   <211> 627
   <212> DNA
   <213> Phakopsora pachyrhizi
<220>
   <221> misc_feature
   <222> (1)..(627)
   <223> ta09267.07_phapa
<400> 369
   atgcgctctc ttatcattcc cgccctttta tgtgtactat ttattcataa atcttgcgca      60
   accgttcaca ctcagtgcta caactatttt ttacaaaagg atggatgcgt tttctctgca     120
   gctgatgaca gaaatcgatg ttctgcagat cccaagccca gtacagccgt tggagtggtt     180
   caagaaagca acaaaaatgt taaaagacac actctggcac gccgttatga tactacttta     240
   ccatctcctt ctatacaggg tgaaggaatt tgcggacact atgacacagc aacagctgag     300
   ggagccagtc tttgggttgg tcctaaccca ggtagcacaa gacccgagga ggcaggctgg     360
   cttaacaggg gaaagacatc taattgcaat aagagattat atgtaataaa ccctcgcaca     420
   ggaaaaactg tctacgtaaa ggtcatagac ggccacgatt ttcagacaac acagcctgat     480
   gtgggatgct tccagattgc actcacccaa aagacctttc ttgagcttga tccgactgat     540
   gaagaaaagg caaaaggtgc cataggatct ctcacatggg acttcgacaa cctgcatgga     600
   attagtcctc agcaaggtcc tgtatga                                        627
<210> 370
   <211> 208
   <212> PRT
   <213> Phakopsora pachyrhizi
<220>
   <221> VARIANT
   <222> (1)..(208)
   <223> ta09267.07_phapa
<400> 370
   Met Arg Ser Leu Ile Ile Pro Ala Leu Leu Cys Val Leu Phe Ile His
   1               5                   10                  15
   Lys Ser Cys Ala Thr Val His Thr Gln Cys Tyr Asn Tyr Phe Leu Gln
               20                  25                  30
   Lys Asp Gly Cys Val Phe Ser Ala Ala Asp Asp Arg Asn Arg Cys Ser
           35                  40                  45
   Ala Asp Pro Lys Pro Ser Thr Ala Val Gly Val Val Gln Glu Ser Asn
       50                  55                  60
   Lys Asn Val Lys Arg His Thr Leu Ala Arg Arg Tyr Asp Thr Thr Leu
   65                  70                  75                  80
   Pro Ser Pro Ser Ile Gln Gly Glu Gly Ile Cys Gly His Tyr Asp Thr
                   85                  90                  95
   Ala Thr Ala Glu Gly Ala Ser Leu Trp Val Gly Pro Asn Pro Gly Ser
               100                 105                 110
   Thr Arg Pro Glu Glu Ala Gly Trp Leu Asn Arg Gly Lys Thr Ser Asn
           115                 120                 125
   Cys Asn Lys Arg Leu Tyr Val Ile Asn Pro Arg Thr Gly Lys Thr Val
       130                 135                 140
   Tyr Val Lys Val Ile Asp Gly His Asp Phe Gln Thr Thr Gln Pro Asp
   145                 150                 155                 160
   Val Gly Cys Phe Gln Ile Ala Leu Thr Gln Lys Thr Phe Leu Glu Leu
                   165                 170                 175
   Asp Pro Thr Asp Glu Glu Lys Ala Lys Gly Ala Ile Gly Ser Leu Thr
               180                 185                 190
   Trp Asp Phe Asp Asn Leu His Gly Ile Ser Pro Gln Gln Gly Pro Val
           195                 200                 205
<210> 371
   <211> 627
   <212> DNA
   <213> Phakopsora pachyrhizi
<220>
   <221> misc_feature
   <222> (1)..(627)
   <223> >ta09267.04_phapa
<400> 371
   atgcgttcac ttatcgttcc caccctttta tgcgtgctat ttattcataa atcttgcgca       60
   accgttcaca ctcagtgcta caactatttt ttacaaaagg atggatgcgt tttctctgca      120
   gctgatgaca gaaatcgatg ttctgcagat cccaagccca gtacagccgt tggagtggtt      180
   caagaaagca acaaaaatgt taaaagacac actctggcac gccgttatga tactacttta      240
   ccatctcctt ctatacaggg tgaaggaatt tgcggacact atgacacagc aacagctgag      300
   ggagccagtc tttgggttgg tcctaaccca ggtagcacaa gacccgagga ggcaggctgg      360
   cttaacaggg gaaagacatc taattgcaat aagagattat atgtaataaa ccctcgcaca      420
   ggaaaaactg tctacgtaaa ggtcatagac ggccacgatt ttcagacaac acagcctgat      480
   gtgggatgct tccagattgc actcacccaa aagacctttc ttgagcttga tccgactgat      540
   gaagaaaagg caaaaggtgc cataggatct ctcacatggg acttcgacaa cctgcatgga      600
   attagtcctc agcaaggtcc tgtatga                                          627
<210> 372
   <211> 208
   <212> PRT
   <213> Phakopsora pachyrhizi
<220>
   <221> VARIANT
   <222> (1)..(208)
   <223> ta09267.04_phapa
<400> 372
   Met Arg Ser Leu Ile Val Pro Thr Leu Leu Cys Val Leu Phe Ile His
   1               5                   10                  15
   Lys Ser Cys Ala Thr Val His Thr Gln Cys Tyr Asn Tyr Phe Leu Gln
               20                  25                  30
   Lys Asp Gly Cys Val Phe Ser Ala Ala Asp Asp Arg Asn Arg Cys Ser
           35                  40                  45
   Ala Asp Pro Lys Pro Ser Thr Ala Val Gly Val Val Gln Glu Ser Asn
       50                  55                  60
   Lys Asn Val Lys Arg His Thr Leu Ala Arg Arg Tyr Asp Thr Thr Leu
   65                  70                  75                  80
   Pro Ser Pro Ser Ile Gln Gly Glu Gly Ile Cys Gly His Tyr Asp Thr
                   85                  90                  95
   Ala Thr Ala Glu Gly Ala Ser Leu Trp Val Gly Pro Asn Pro Gly Ser
               100                 105                 110
   Thr Arg Pro Glu Glu Ala Gly Trp Leu Asn Arg Gly Lys Thr Ser Asn
           115                 120                 125
   Cys Asn Lys Arg Leu Tyr Val Ile Asn Pro Arg Thr Gly Lys Thr Val
       130                 135                 140
   Tyr Val Lys Val Ile Asp Gly His Asp Phe Gln Thr Thr Gln Pro Asp
   145                 150                 155                 160
   Val Gly Cys Phe Gln Ile Ala Leu Thr Gln Lys Thr Phe Leu Glu Leu
                   165                 170                 175
   Asp Pro Thr Asp Glu Glu Lys Ala Lys Gly Ala Ile Gly Ser Leu Thr
               180                 185                 190
   Trp Asp Phe Asp Asn Leu His Gly Ile Ser Pro Gln Gln Gly Pro Val
           195                 200                 205
<210> 373
   <211> 627
   <212> DNA
   <213> Phakopsora pachyrhizi
<220>
   <221> misc_feature
   <222> (1)..(627)
   <223> ta09267.05_phapa
<400> 373
   atgcgttcac ttatcgttcc caccctttta tgcgtgctat ttattcataa atcttgcgca      60
   accgttcaca ctcagtgcta caactatttt ttacaaaagg atggatgcgt tttctctgca      120
   gctgatgaca gaaatcgatg ttctgcagat cccaagccca gtacagccgt tggagtggtt     180
   caagaaagca acaaaaatgt taaaagacac actctggcac gccgttatga tactacttta     240
   ccatctcctt ctatacaggg tgaaggaatt tgcggacact atgacacagc aacagctgag     300
   ggagccagtc tttgggttgg tcctaaccca ggtagcacaa gacccgagga ggcaggctgg     360
   cttaacaggg gaaagacatc taattgcaat aagagattat atgtaataaa ccctcgcaca     420
   ggaaaaactg tctacgtaaa ggtcatagac ggccacgatt ttcagacaac acagcctgat     480
   gtgggatgct tccagattgc actcacccaa aagacctttc ttgagcttga tccgactgat     540
   gaagaaaagg caaaaggtgc cataggatct ctcacatggg acttcgacaa cctgcatgga     600
   attagtcctc agcaaggtcc tgtatga                                         627
<210> 374
   <211> 208
   <212> PRT
   <213> Phakopsora pachyrhizi
<220>
   <221> VARIANT
   <222> (1)..(208)
   <223> ta09267.05_phapa
<400> 374
   Met Arg Ser Leu Ile Val Pro Thr Leu Leu Cys Val Leu Phe Ile His
   1               5                   10                  15
   Lys Ser Cys Ala Thr Val His Thr Gln Cys Tyr Asn Tyr Phe Leu Gln
               20                  25                  30
   Lys Asp Gly Cys Val Phe Ser Ala Ala Asp Asp Arg Asn Arg Cys Ser
           35                  40                  45
   Ala Asp Pro Lys Pro Ser Thr Ala Val Gly Val Val Gln Glu Ser Asn
       50                  55                  60
   Lys Asn Val Lys Arg His Thr Leu Ala Arg Arg Tyr Asp Thr Thr Leu
   65                  70                  75                  80
   Pro Ser Pro Ser Ile Gln Gly Glu Gly Ile Cys Gly His Tyr Asp Thr
                   85                  90                  95
   Ala Thr Ala Glu Gly Ala Ser Leu Trp Val Gly Pro Asn Pro Gly Ser
               100                 105                 110
   Thr Arg Pro Glu Glu Ala Gly Trp Leu Asn Arg Gly Lys Thr Ser Asn
           115                 120                 125
   Cys Asn Lys Arg Leu Tyr Val Ile Asn Pro Arg Thr Gly Lys Thr Val
       130                 135                 140
   Tyr Val Lys Val Ile Asp Gly His Asp Phe Gln Thr Thr Gln Pro Asp
   145                 150                 155                 160
   Val Gly Cys Phe Gln Ile Ala Leu Thr Gln Lys Thr Phe Leu Glu Leu
                   165                 170                 175
   Asp Pro Thr Asp Glu Glu Lys Ala Lys Gly Ala Ile Gly Ser Leu Thr
               180                 185                 190
   Trp Asp Phe Asp Asn Leu His Gly Ile Ser Pro Gln Gln Gly Pro Val
           195                 200                 205
<210> 375
   <211> 627
   <212> DNA
   <213> Phakopsora pachyrhizi
<220>
   <221> misc_feature
   <222> (1)..(627)
   <223> ta09267.03_phapa
<400> 375
   atgcgttcac ttatcgttcc caccctttta tgcgtgctat ttattcataa atcttgcgca      60
   accgttcaca ctcagtgcta caactatttt ttacaaaagg atggatgcgt tttctctgca     120
   gctgatgaca gaaatcgatg ttctgcagat cccaagccca gtacagccgt tggagtggtt     180
   caagaaagca acaaaaatgt taaaagacac actctggcac gccgttatga tactacttta     240
   ccatctcctt ctatacaggg tgaaggaatt tgcggacact atgacacagc aacagctgag     300
   ggagccagtc tttgggttgg tcctaaccca ggtagcacaa gacccgagga ggcaggctgg     360
   cttaacaggg gaaagacatc taattgcaat aagagattat atgtaataaa ccctcgcaca     420
   ggaaaaactg tctacgtaaa ggtcatagac ggccacgatt ttcagacaac acagcctgat     480
   gtgggatgct tccagattgc actcacccaa aagacctttc ttgagcttga tccgactgat     540
   gaagaaaagg caaaaggtgc cataggatct ctcacatggg acttcgacaa cctgcatgga     600
   attagtcctc agcaaggtcc tgtatga                                         627
<210> 376
   <211> 208
   <212> PRT
   <213> Phakopsora pachyrhizi
<220>
   <221> VARIANT
   <222> (1)..(208)
   <223> ta09267.03_phapa
<400> 376
   Met Arg Ser Leu Ile Val Pro Thr Leu Leu Cys Val Leu Phe Ile His
   1               5                   10                  15
   Lys Ser Cys Ala Thr Val His Thr Gln Cys Tyr Asn Tyr Phe Leu Gln
               20                  25                  30
   Lys Asp Gly Cys Val Phe Ser Ala Ala Asp Asp Arg Asn Arg Cys Ser
           35                  40                  45
   Ala Asp Pro Lys Pro Ser Thr Ala Val Gly Val Val Gln Glu Ser Asn
       50                  55                  60
   Lys Asn Val Lys Arg His Thr Leu Ala Arg Arg Tyr Asp Thr Thr Leu
   65                  70                  75                  80
   Pro Ser Pro Ser Ile Gln Gly Glu Gly Ile Cys Gly His Tyr Asp Thr
                   85                  90                  95
   Ala Thr Ala Glu Gly Ala Ser Leu Trp Val Gly Pro Asn Pro Gly Ser
               100                 105                 110
   Thr Arg Pro Glu Glu Ala Gly Trp Leu Asn Arg Gly Lys Thr Ser Asn
           115                 120                 125
   Cys Asn Lys Arg Leu Tyr Val Ile Asn Pro Arg Thr Gly Lys Thr Val
       130                 135                 140
   Tyr Val Lys Val Ile Asp Gly His Asp Phe Gln Thr Thr Gln Pro Asp
   145                 150                 155                 160
   Val Gly Cys Phe Gln Ile Ala Leu Thr Gln Lys Thr Phe Leu Glu Leu
                   165                 170                 175
   Asp Pro Thr Asp Glu Glu Lys Ala Lys Gly Ala Ile Gly Ser Leu Thr
               180                 185                 190
   Trp Asp Phe Asp Asn Leu His Gly Ile Ser Pro Gln Gln Gly Pro Val
           195                 200                 205
<210> 377
   <211> 614
   <212> DNA
   <213> Phakopsora pachyrhizi
<220>
   <221> misc_feature
   <222> (1)..(614)
   <223> ta01408.01
<400> 377
   atggaaatga ggattttgct ttttgtttgt ggttgtttct tagctgactc ttctttttca      60
   tctctttggc atagttttga agcagcatca agagaagatc ttcaaggcct ttctcataac     120
   atcgaagctc caagtgatat cttggaatat aaacatttcc taaagccaaa aaccattcct     180
   acatcattcg attctggagt tataattgag caagcacctt tattatttag taaagctgac     240
   ttttcaacag aaaaatacgg tttaaatgaa aaaagcctcg cacgcagctc aggcgttgta     300
   cccaaagcag cgatgaaaga tggtgaaaag ggctataaca gcatatgtta ctcaaattcg     360
   gataaagctc aaatttcaaa aggaaaaggt caattatatt acaactgtaa caatttttta     420
   ccaactgttc ctgaaattcg ccagaattat tttgatccaa tagaacaatg cctaaatcaa     480
   cacaagttta atggagaata tttaaatagg ctgcgaaaca accaattaga aagaccaact     540
   gcacaattat cgggcattaa gcctttgatc aatagcatac ctcccggtaa caacgaagaa     600
   tctgcccctg gttt                                                       614
<210> 378
   <211> 204
   <212> PRT
   <213> Phakopsora pachyrhizi
<220>
   <221> VARIANT
   <222> (1)..(204)
   <223> ta01408.01_phapa
<400> 378
   Met Glu Met Arg Ile Leu Leu Phe Val Cys Gly Cys Phe Leu Ala Asp
   1               5                   10                  15
   Ser Ser Phe Ser Ser Leu Trp His Ser Phe Glu Ala Ala Ser Arg Glu
               20                  25                  30
   Asp Leu Gln Gly Leu Ser His Asn Ile Glu Ala Pro Ser Asp Ile Leu
           35                  40                  45
   Glu Tyr Lys His Phe Leu Lys Pro Lys Thr Ile Pro Thr Ser Phe Asp
       50                  55                  60
   Ser Gly Val Ile Ile Glu Gln Ala Pro Leu Leu Phe Ser Lys Ala Asp
   65                  70                  75                  80
   Phe Ser Thr Glu Lys Tyr Gly Leu Asn Glu Lys Ser Leu Ala Arg Ser
                   85                  90                  95
   Ser Gly Val Val Pro Lys Ala Ala Met Lys Asp Gly Glu Lys Gly Tyr
               100                 105                 110
   Asn Ser Ile Cys Tyr Ser Asn Ser Asp Lys Ala Gln Ile Ser Lys Gly
           115                 120                 125
   Lys Gly Gln Leu Tyr Tyr Asn Cys Asn Asn Phe Leu Pro Thr Val Pro
       130                 135                 140
   Glu Ile Arg Gln Asn Tyr Phe Asp Pro Ile Glu Gln Cys Leu Asn Gln
   145                 150                 155                 160
   His Lys Phe Asn Gly Glu Tyr Leu Asn Arg Leu Arg Asn Asn Gln Leu
                   165                 170                 175
   Glu Arg Pro Thr Ala Gln Leu Ser Gly Ile Lys Pro Leu Ile Asn Ser
               180                 185                 190
   Ile Pro Pro Gly Asn Asn Glu Glu Ser Ala Pro Gly
           195                 200

## Description

### REFERENCE TO SEQUENCE LISTING SUBMITTED ELECTRONICALLY

The official copy of the sequence listing is submitted electronically via EFS-Web as an ASCII formatted sequence listing with a file named "5414WOPCT_Sequence_Listing" and is filed concurrently with the specification. The sequence listing contained in this ASCII formatted document is part of the specification.

### FIELD

This disclosure relates to the field of plant biotechnology, specifically resistance to Asian Soy Rust.

### BACKGROUND

Asian soybean rust (ASR) is a serious disease caused by the fungus *Phakopsora pachyrhizi.* Soybean rust is spread by windblown spores and has caused significant crop losses in many soybean-growing regions of the world. On November 10, 2004 USDA's Animal and Plant Health Inspection Service (APHIS) announced the first confirmation of Asian soybean rust in the continental United States (Louisiana), followed by finds in 8 additional southern states. In 2005, soybean rust was confirmed on soybeans in 29 counties in Georgia, 23 counties in South Carolina, 21 counties in Alabama, 18 counties in North Carolina,12 counties in Florida, 2 counties in Mississippi, and one county in Louisiana.

Crop loss estimates range from 10-90% of infested fields depending on the growing region, time of epidemic initiation, and environmental conditions. There are currently no commercial soybeans resistant to ASR.

Thus, there is a continuing need for compositions and methods for conferring resistance to Asian Soy Rust.

The invention provides an isolated polynucleotide comprising a nucleic acid sequence selected from:
(a) the nucleic acid sequence set forth in SEQ ID NO: 75, or a complement thereof; and
(b) a nucleotide sequence encoding a polypeptide having the amino acid sequence set forth in SEQ ID NO: 76.

The invention further provides an isolated polypeptide having the amino acid sequence of SEQ ID NO: 76.

Yet further provided by the invention is an expression cassette comprising a heterologous regulatory sequence operably linked to said isolated polynucleotide.

The invention further provides a non-human host cell comprising said expression cassette.

Yet further provided by the invention is a transgenic soybean plant, soybean plant part or soybean plant cell comprising said expression vector, wherein the transgenic plant, plant part or plant cell has enhanced resistance to Asian Soy Rust compared to a corresponding non-transgenic wild type plant.

The invention further provides a method of modulating at least one protein associated with resistance to Asian Soy Rust in a transgenic plant, the method comprising expressing at least one said polynucleotide.

Yet further provided by the invention is a method of enhancing plant resistance to Asian Soy Rust (ASR) infection, the method comprising inactivation of the ASR pathogen by eliciting programmed cell death by a ASR effector polypeptide sequence, wherein the ASR polypeptide is the amino acid sequence set forth in SEQ ID NO: 76.

### BRIEF DESCRIPTION OF THE FIGURES

Figure 1 shows the β-glucuronidase activity in a Soybean leaf co-transformed with β-glucuronidase and the empty vector (panel a) and in a Soybean leaf co-transformed with β-glucuronidase and the candidate ASR effector 2p12 (SEQ ID NO: 75) (panel b).
Figure 2 shows the chalcone synthase expression relative to ubiquitin expression in soybean plants expressing the pathogen effector Avr1k, 2c11 (SEQ ID NO: 73), and the control with no effector (EV = empty vector).

### DETAILED DESCRIPTION

Disease in plants is caused by biotic and abiotic causes. Biotic causes include fungi, viruses, bacteria, and nematodes. An example of the importance of plant disease is illustrated by phytopathogenic fungi, which cause significant annual crop yield losses as well as devastating epidemics. Plant disease outbreaks have resulted in catastrophic crop failures that have triggered famines and caused major social change. All of the approximately 300,000 species of flowering plants are attacked by pathogenic fungi; however, a single plant species can be host to only a few fungal species, and similarly, most fungi usually have a limited host range. Generally, the best strategy for plant disease control is to use resistant cultivars selected or developed by plant breeders for this purpose. However, the potential for serious crop disease epidemics persists today, as evidenced by outbreaks of the Victoria blight of oats and southern corn leaf blight. Molecular methods of crop protection have the potential to implement novel mechanisms for disease resistance and can also be implemented more quickly than traditional breeding methods. Accordingly, molecular methods are needed to supplement traditional breeding methods to protect plants from pathogen attack.

A host of cellular processes enable plants to defend themselves against diseases caused by pathogenic agents. These defense mechanisms are activated by initial pathogen infection in a process known as elicitation. In elicitation, the host plant recognizes a pathogen-derived compound known as an elicitor; the plant then activates disease gene expression to limit further spread of the invading organism. It is generally believed that to overcome these plant defense mechanisms, plant pathogens must find a way to suppress elicitation as well as to overcome more physically-based barriers to infection, such as reinforcement of the plant cell wall and/or rearrangement of the actin filament networks near the cell's plasma membrane.

Thus, the present disclosure solves the need for enhancement of the plant's defensive elicitation response via a molecularly based mechanism which can be quickly incorporated into commercial crops.

Pathogens secrete protein molecules that are either localized to the plant apoplast or are taken up into the plant cell. These proteins, termed effectors, can have either an avirulence or virulence function. In the former case, recognition by the cognate plant R protein activates host defense responses, ultimately leading to programmed cell death and resistance to the pathogen.

The virulence activity of effectors is associated with the manipulation of normal host cell functions or the suppression of host defense responses by the pathogen in order to establish successful infection.

Major gene resistance which relies on the gene-for-gene relationship between pathogen avirulence and plant resistance genes, has been widely used in breeding approaches. However, such resistance is typically race-specific and easily overcome by single mutations in the pathogen *avr* gene as a consequence of diversifying selection to avoid recognition by the host. Thus the durability of such qualitative resistance is of concern. Attempts have been made to introduce novel antimicrobial/antifungal genes or to modify expression of endogenous defense-related genes in transgenic plants. However, in many cases, the effect is partial and comes at a cost to plant yield and or vigor.

In the present disclosure, the resistance strategy is focused on the inactivation of pathogen molecules that are essential to establishment of Asian soybean rust infection. Different approaches are being taken to inhibit the activity of these critical soy rust molecules, termed "effectors". Random high throughput screening of phage display or peptide antibody libraries may be performed to identify short sequences that bind the fungal effector and block its functional activity. These may be high affinity peptide sequences that prevent interaction of the effector with its host target.

Alternatively, sequences identified through this approach may exhibit tight binding with effector moieties that specify uptake into host cells. In the latter case inhibition would derive from blocking entry of pathogen factors into the host cell, while in the former, effectors that are delivered into the host cytoplasm are prevented from interacting with and interfering with host target proteins. High affinity, inhibitory peptides may be over-expressed in transgenic plants and their effectiveness tested in ASR disease assays.

In a second approach, effector inhibitors may be designed based upon the targets of those pathogen molecules. Host targets may first be identified by yeast-2-hybrid screens, and then variants generated that exhibit altered effector binding activity. Of particular interest may be host target variants that fail to bind effector, but retain normal activity in the plant, or variants with significantly enhanced binding affinity that prevent interaction of the effector with the native target. The native target and the variants may be overexpressed in transgenic plants and the sensitivity to rust infection evaluated in comparison to wild type plants.

It is expected that through such approaches, it may be possible to inactivate several pathogen effectors and thus to interfere with the ability of the pathogen to infect soybean. Such a strategy can be used alone or in combination with other strategies to produce transgenic Asian soybean rust resistant plants.

These additional strategies include but are no means limited to modified expression of endogenous plant defense molecules or ectopic expression of novel molecules from other plant, microbial or animal sources. These protein molecules can include plant immune receptors such as intracellular NB-LRR proteins or extracellular pattern recognition receptors, or antifungal proteins such as plant or microbial defensins

Sequences of *Phakopsora* candidate effectors can be introduced into plants as part of overexpression or silencing constructs. In both cases, in planta expression can provide information about functional activity of the pathogen protein or virulence activity. Overexpression of bacterial or oomycete effectors in plants has been associated with visible phenotypes such as leaf chlorosis, induction of cell death, plant stunting, etc. *In planta* expression can additionally produce an alteration in plant susceptibility to pathogen infection. In the latter case, enhanced plant resistance might be expected if in planta expression of the pathogen effector leads to elicitation of plant defense responses. Silencing constructs of pathogen genes would be expected to be processed by the plant RNAi machinery to produce small RNA molecules that are taken up by the pathogen, and interact with the RISC complex to target the pathogen mRNA for degradation. Gene silencing and a loss of function phenotype can thus give important clues to the role of the pathogen genes in determining pathogen virulence and infectivity. In addition, down regulation of critical virulence genes can lead to compromised pathogenicity and therefore, increased plant resistance.

The compositions and methods of the disclosure are useful for modulating the levels of at least one protein in a plant. By "modulate" is defined herein as an increase or decrease in the level of a protein within a genetically altered plant relative to the level of that protein from the corresponding wild-type plant (i.e., a plant not genetically altered in accordance with the methods of the present disclosure).

The terms "inhibit," "inhibition," "inhibiting", "reduced", and "reduction" as used herein refer to any decrease in the expression or function of a target gene product, including any relative decrease in expression or function up to and including complete abrogation of expression or function of the target gene product. The term "expression" as used herein in the context of a gene product refers to the biosynthesis of that gene product, including the transcription and/or translation of the gene product. Inhibition of expression or function of a target gene product (i.e., a gene product of interest) can be in the context of a comparison between any two plants, for example, expression or function of a target gene product in a genetically altered plant versus the expression or function of that target gene product in a corresponding wild-type plant. Alternatively, inhibition of expression or function of the target gene product can be in the context of a comparison between plant cells, organelles, organs, tissues, or plant parts within the same plant or between plants, and includes comparisons between developmental or temporal stages within the same plant or between plants. Any method or composition that down-regulates expression of a target gene product, either at the level of transcription or translation, or down-regulates functional activity of the target gene product can be used to achieve inhibition of expression or function of the target gene product.

The term "inhibitory sequence" encompasses any polynucleotide or polypeptide sequence that is capable of inhibiting the expression of a target gene product, for example, at the level of transcription or translation, or which is capable of inhibiting the function of a target gene product. Examples of inhibitory sequences include, but are not limited to, full-length polynucleotide or polypeptide sequences, truncated polynucleotide or polypeptide sequences, fragments of polynucleotide or polypeptide sequences, variants of polynucleotide or polypeptide sequences, sense-oriented nucleotide sequences, antisense-oriented nucleotide sequences, the complement of a sense- or antisense-oriented nucleotide sequence, inverted regions of nucleotide sequences, hairpins of nucleotide sequences, double-stranded nucleotide sequences, single-stranded nucleotide sequences, and combinations thereof. The term "polynucleotide sequence" includes sequences of RNA, DNA, chemically modified nucleic acids, nucleic acid analogs, and combinations thereof.

Methods for inhibiting gene expression are well known in the art. Although any method known in the art for reducing the level of protein in a plant could be used, possible methods for reducing protein include, but are not limited to, homology-dependent gene silencing, antisense technology, co-suppression including, for example, RNA interference (RNAi) and micro RNA, site-specific recombination, site-specific integration, mutagenesis including transposon tagging, and biosynthetic competition, homologous recombination, and gene targeting, alone or in combination. Depending upon the intended goal, the level of at least one protein may be increased, decreased, or eliminated entirely as described below.

The disclosure encompasses isolated or substantially purified nucleic acid or protein compositions. An "isolated" or "purified" nucleic acid molecule or protein, or biologically active portion thereof, is substantially or essentially free from components that normally accompany or interact with the nucleic acid molecule or protein as found in its naturally occurring environment. Thus, an isolated or purified nucleic acid molecule or protein is substantially free of other cellular material, or culture medium when produced by recombinant techniques, or substantially free of chemical precursors or other chemicals when chemically synthesized. Preferably, an "isolated" nucleic acid is free of sequences (preferably protein encoding sequences) that naturally flank the nucleic acid (i.e., sequences located at the 5' and 3' ends of the nucleic acid) in the genomic DNA of the organism from which the nucleic acid is derived. For example, in various aspects, the isolated nucleic acid molecule can contain less than about 5 kb, 4 kb, 3 kb, 2 kb, 1 kb, 0.5 kb, or 0.1 kb of nucleotide sequences that naturally flank the nucleic acid molecule in genomic DNA of the cell from which the nucleic acid is derived. A protein that is substantially free of cellular material includes preparations of protein having less than about 30%, 20%, 10%, 5%, or 1% (by dry weight) of contaminating protein. When the protein of the disclosure or biologically active portion thereof is recombinantly produced, preferably culture medium represents less than about 30%, 20%, 10%, 5%, or 1% (by dry weight) of chemical precursors or non-protein-of-interest chemicals.

In some aspects the ASR effector polypeptide is encoded by a polynucleotide comprising the nucleic acid sequence set forth in SEQ ID NO:1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 89, 91, 93, 95, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181, 183, 185, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 289, 291, 293, 295, 297, 299, 301, 303, 305, 307, 309, 311, 313, 315, 317, 319, 321, 323, 325, 327, 329, 331, 333, 335, 337, 339, 341, 343, 345, 347, 349, 351, 353, 355, 357, 359, 361, 363, 365, 367, 369, 371, 373, 375, or 377.

In some aspects the ASR effector polypeptide comprises an amino acid sequences set forth in SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, or 378.

In some aspects the ASR effector polypeptide comprises an amino acid sequence having at least 50%, 55%, 60%, 65%, 70%, 75%, 80%, 85%, 90%, 95%, 96%, 97%, 98%, 99% or great sequence identity to SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, or 378.

In some aspects the nucleotide sequences encodes an ASR effector polypeptide comprising an amino acid sequences set forth in SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, or 378.

In some aspects the nucleotide sequences encodes an ASR effector polypeptide comprises an amino acid sequence having at least 50%, 55%, 60%, 65%, 70%, 75%, 80%, 85%, 90%, 95%, 96%, 97%, 98%, 99% or great sequence identity to SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296, 298, 300, 302, 304, 306, 308, 310, 312, 314, 316, 318, 320, 322, 324, 326, 328, 330, 332, 334, 336, 338, 340, 342, 344, 346, 348, 350, 352, 354, 356, 358, 360, 362, 364, 366, 368, 370, 372, 374, 376, or 378.

Fragments and variants of the disclosed nucleotide sequences and proteins encoded thereby are also encompassed by the present disclosure. By "fragment" is intended a portion of the nucleotide sequence or a portion of the amino acid sequence and hence protein encoded thereby. Fragments of a nucleotide sequence may encode protein fragments that retain the biological activity of the native protein and hence have effector-like activity and thereby affect development, developmental pathways, and defense responses. Alternatively, fragments of a nucleotide sequence that are useful as hybridization probes generally do not encode fragment proteins retaining biological activity. Thus, fragments of a nucleotide sequence may range from at least about 20 nucleotides, about 50 nucleotides, about 100 nucleotides, and up to the full-length nucleotide sequence encoding the proteins of the disclosure.

The sequences of the disclosure are provided in expression cassettes or DNA constructs for expression in the plant of interest. The cassette can include 5' and 3' heterologous regulatory sequences operably linked to a sequence of the disclosure. By "operably linked" a functional linkage between a heterologous regulatory sequence and a second sequence, such as a promoter sequence that initiates and mediates transcription of the DNA sequence corresponding to the second sequence is intended. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the nucleotide sequence to be under the transcriptional regulation of the regulatory regions. The expression cassette may additionally contain selectable marker genes.

The expression cassette can include in the 5'-3' direction of transcription, a transcriptional and translational initiation region, a DNA sequence of the disclosure, and a transcriptional and translational termination region functional in plants. The transcriptional initiation region, the promoter, may be native or analogous or foreign or heterologous to the plant host. Additionally, the promoter may be the natural sequence or alternatively a synthetic sequence. By "foreign" is intended that the transcriptional initiation region is not found in the native plant into which the transcriptional initiation region is introduced. As used herein, a chimeric gene comprises a coding sequence operably linked to a transcription initiation region that is heterologous to the coding sequence.

While it may be preferable to express the sequences using heterologous promoters, native promoter sequences may be used. Such constructs would change expression levels in the host cell (i.e., plant or plant cell). Thus, the phenotype of the host cell (i.e., plant or plant cell) is altered.

The termination region may be native with the transcriptional initiation region, may be native with the operably linked DNA sequence of interest, or may be derived from another source. Convenient termination regions are available from the Ti-plasmid of A. tumefaciens, such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed plant. That is, the genes can be synthesized using plant-preferred codons for improved expression. See, for example, Campbell and Gowri (1990) Plant Physiol. 92:1-11 for a discussion of host-preferred codon usage. Methods are available in the art for synthesizing plant-preferred genes. See, for example, U.S. Patent Nos. 5,380,831, 5,436,391, and Murray et al. (1989) Nucleic Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

The expression cassettes may additionally contain 5' leader sequences in the expression cassette construct. Such leader sequences can act to enhance translation. Translation leaders are known in the art and include: picornavirus leaders, for example, EMCV leader (Encephalomyocarditis 5' noncoding region) (Elroy Stein et al. (1989) PNAS USA 86:6126 6130); potyvirus leaders, for example, TEV leader (Tobacco Etch Virus) (Allison et al. (1986); MDMV leader (Maize Dwarf Mosaic Virus); Virology 154:9 20), and human immunoglobulin heavy chain binding protein (BiP), (Macejak et al. (1991) Nature 353:90 94); untranslated leader from the coat protein mRNA of alfalfa mosaic virus (AMV RNA 4) (Jobling et al. (1987) Nature 325:622 625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Virology 81:382 385). See also, Della Cioppa et al. (1987) Plant Physiol. 84:965 968. Other methods known to enhance translation can also be utilized, for example, introns.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, or removal of restriction sites. For this purpose, in vitro mutagenesis, primer repair, restriction, annealing, resubstitutions, e.g., transitions and transversions, may be involved.

Generally, the expression cassette can comprise a selectable marker gene for the selection of transformed cells. Selectable marker genes are utilized for the selection of transformed cells or tissues. Marker genes include genes encoding antibiotic resistance, such as those encoding neomycin phosphotransferase II (NEO) and hygromycin phosphotransferase (HPT), as well as genes conferring resistance to herbicidal compounds, such as glyphosate, glufosinate ammonium, bromoxynil, imidazolinones, and 2,4-dichlorophenoxyacetate (2,4-D). See generally, Yarranton (1992) Curr. Opin. Biotech. 3:506-511; Christopherson et al. (1992) Proc. Natl. Acad. Sci. USA 89:6314-6318; Yao et al. (1992) Cell 71:63-72; Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschle et al. (1989) Proc. Natl. Acad. Sci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Handbook of Experimental Pharmacology, Vol. 78 (Springer-Verlag, Berlin); Gill et al. (1988) Nature 334:721-724.

The above list of selectable marker genes is not meant to be limiting. Any selectable marker gene can be used in the present disclosure.

A number of promoters can be used in the practice of the disclosure. The promoters can be selected based on the desired outcome. That is, the nucleic acids can be combined with constitutive, tissue-preferred, or other promoters for expression in the host cell of interest. Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (McElroy et al. (1990) Plant Cell 2:163-171); ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); and ALS promoter (U.S. Patent No. 5,659,026). Other constitutive promoters include, for example, those disclosed in U.S. Patent Nos. 5,608,149; 5,608,144; 5,604,121; 5,569,597; 5,466,785; 5,399,680; 5,268,463; 5,608,142; and 6,177,611.

Generally, it may be beneficial to express the gene from an inducible promoter, particularly from a pathogen-inducible promoter. Such promoters include those from pathogenesis-related proteins (PR proteins), which are induced following infection by a pathogen; e.g., PR proteins, SAR proteins, beta-1,3-glucanase, chitinase, etc. See, for example, Redolfi et al. (1983) Neth. J. Plant Pathol. 89:245-254; Uknes et al. (1992) Plant Cell 4:645-656; and Van Loon (1985) Plant Mol. Virol. 4:111-116. See also WO 99/43819 published September 9, 1999.

Of interest are promoters that are expressed locally at or near the site of pathogen infection. See, for example, Marineau et al. (1987) Plant Mol. Biol. 9:335-342; Matton et al. (1989) Molecular Plant-Microbe Interactions 2:325-331; Somsisch et al. (1986) Proc. Natl. Acad. Sci. USA 83:2427-2430; Somsisch et al. (1988) Mol. Gen. Genet. 2:93-98; and Yang (1996) Proc. Natl. Acad. Sci. USA 93:14972-14977. See also, Chen et al. (1996) Plant J. 10:955-966; Zhang et al. (1994) Proc. Natl. Acad. Sci. USA 91:2507-2511; Warner et al. (1993) Plant J. 3:191-201; Siebertz et al. (1989) Plant Cell 1:961-968; U.S. Patent No. 5,750,386 (nematode-inducible); and the references cited therein. Of particular interest is the inducible promoter for the maize PRms gene, whose expression is induced by the pathogen Fusarium moniliforme (see, for example, Cordero et al. (1992) Physiol. Mol. Plant Path. 41:189-200).

Additionally, as pathogens find entry into plants through wounds or insect damage, a wound-inducible promoter may be used in the constructions of the disclosure. Such wound-inducible promoters include potato proteinase inhibitor (pin II) gene (Ryan (1990) Ann. Rev. Phytopath. 28:425-449; Duan et al. (1996) Nature Biotechnology 14:494-498); wun1 and wun2, U.S. Patent No. 5,428,148; win1 and win2 (Stanford et al. (1989) Mol. Gen. Genet. 215:200-208); systemin (McGurl et al. (1992) Science 225:1570-1573); WIP1 (Rohmeier et al. (1993) Plant Mol. Biol. 22:783-792; Eckelkamp et al. (1993) FEBS Letters 323:73-76); and MPI gene (Corderok et al. (1994) Plant J. 6(2):141-150).

Chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Chemical-inducible promoters are known in the art and include, but are not limited to, the maize In2-2 promoter, which is activated by benzenesulfonamide herbicide safeners, the maize GST promoter, which is activated by hydrophobic electrophilic compounds that are used as pre-emergent herbicides, and the tobacco PR-1a promoter, which is activated by salicylic acid. Other chemical-regulated promoters of interest include steroid-responsive promoters (see, for example, the glucocorticoid-inducible promoter in Schena et al. (1991) Proc. Natl. Acad. Sci. USA 88:10421-10425 and McNellis et al. (1998) Plant J. 14(2):247-257) and tetracycline-inducible and tetracycline-repressible promoters (see, for example, Gatz et al. (1991) Mol. Gen. Genet. 227:229-237, and U.S. Patent Nos. 5,814,618 and 5,789,156).

Tissue-preferred promoters can be utilized to target enhanced expression within a particular plant tissue. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et al. (1994) Plant Cell Physiol. 35(5):773-778; Lam (1994) Results Probl. Cell Differ. 20:181-196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression.

Leaf-specific promoters are known in the art. See, for example, Yamamoto et al. (1997) Plant J. 12(2):255-265; Kwon et al. (1994) Plant Physiol. 105:357-67; Yamamoto et al. (1994) Plant Cell Physiol. 35(5):773-778; Gotor et al. (1993) Plant J. 3:509-18; Orozco et al. (1993) Plant Mol. Biol. 23(6):1129-1138; and Matsuoka et al. (1993) Proc. Natl. Acad. Sci. USA 90(20):9586-9590.

"Seed-preferred" promoters include both "seed-specific" promoters (those promoters active during seed development such as promoters of seed storage proteins) as well as "seed-germinating" promoters (those promoters active during seed germination). See Thompson et al. (1989) BioEssays 10:108. Such seed-preferred promoters include, but are not limited to, Cim1 (cytokinin-induced message); cZ19B1 (maize 19 kDa zein); milps (myo-inositol-1-phosphate synthase); and celA (cellulose synthase) (see WO 00/11177). Gama-zein is a preferred endosperm-specific promoter. Glob-1 is a preferred embryo-specific promoter. For dicots, seed-specific promoters include, but are not limited to, bean β-phaseolin, napin, β-conglycinin, soybean lectin, and cruciferin. For monocots, seed-specific promoters include, but are not limited to, maize 15 kDa zein, 22 kDa zein, 27 kDa zein, g-zein, waxy, shrunken 1, shrunken 2, globulin 1, etc. See also WO 00/12733, where seed-preferred promoters from end1 and end2 genes are disclosed.

The method of transformation/transfection is not critical to the instant disclosure; various methods of transformation or transfection are currently available. As newer methods are available to transform crops or other host cells they may be directly applied. Accordingly, a wide variety of methods have been developed to insert a DNA sequence into the genome of a host cell to obtain the transcription and/or translation of the sequence to effect phenotypic changes in the organism. Thus, any method, which provides for effective transformation/transfection may be employed.

Transformation protocols as well as protocols for introducing nucleotide sequences into plants may vary depending on the type of plant or plant cell, i.e., monocot or dicot, targeted for transformation. Suitable methods of introducing nucleotide sequences into plant cells and subsequent insertion into the plant genome include microinjection (Crossway et al. (1986) Biotechniques 4:320 334), electroporation (Riggs et al. (1986) Proc. Natl. Acad. Sci. USA 83:5602 5606, Agrobacterium-mediated transformation (Townsend et al., U.S. Patent No. 5,563,055; Zhao et al., U.S. Patent No. 5,981,840), direct gene transfer (Paszkowski et al. (1984) EMBO J. 3:2717 2722), and ballistic particle acceleration (see, for example, Sanford et al., U.S. Patent No. 4,945,050; Tomes et al., U.S. Patent No. 5,879,918; Tomes et al., U.S. Patent No. 5,886,244; Bidney et al., U.S. Patent No. 5,932,782; McCabe et al. (1988) Biotechnology 6:923 926); and Lec1 transformation (WO 00/28058). Also see Weissinger et al. (1988) Ann. Rev. Genet. 22:421 477; Sanford et al. (1987) Particulate Science and Technology 5:27 37 (onion); Christou et al. (1988) Plant Physiol. 87:671 674 (soybean); McCabe et al. (1988) Bio/Technology 6:923 926 (soybean); Finer and McMullen (1991) In Vitro Cell Dev. Biol. 27P:175-182 (soybean); Singh et al. (1998) Theor. Appl. Genet. 96:319-324 (soybean); Datta et al. (1990) Biotechnology 8:736 740 (rice); Klein et al. (1988) Proc. Natl. Acad. Sci. USA 85:4305 4309 (maize); Klein et al. (1988) Biotechnology 6:559 563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Organ Culture: Fundamental Methods, ed. Gamborg (Springer-Verlag, Berlin) (maize); Klein et al. (1988) Plant Physiol. 91:440 444 (maize); Fromm et al. (1990) Biotechnology 8:833 839 (maize); Hooykaas-Van Slogteren et al. (1984) Nature (London) 311:763-764; Bowen et al., U.S. Patent No. 5,736,369 (cereals); Bytebier et al. (1987) Proc. Natl. Acad. Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Halluin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize via Agrobacterium tumefaciens).

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting progeny having constitutive expression of the desired phenotypic characteristic identified. Two or more generations may be grown to ensure that expression of the desired phenotypic characteristic is stably maintained and inherited and then seeds harvested to ensure that expression of the desired phenotypic characteristic has been achieved.

The present disclosure may be used for transformation of any plant species, including, but not limited to, monocots and dicots. Examples of plants of interest include, but are not limited to, corn (Zea mays), Brassica sp. (e.g., B. napus, B. rapa, B. juncea), particularly those Brassica species useful as sources of seed oil, alfalfa (Medicago sativa), rice (Oryza sativa), rye (Secale cereale), sorghum (Sorghum bicolor, Sorghum vulgare), millet (e.g., pearl millet (Pennisetum glaucum), proso millet (Panicum miliaceum), foxtail millet (Setaria italica), finger millet (Eleusine coracana)), sunflower (Helianthus annuus), safflower (Carthamus tinctorius), wheat (Triticum aestivum), soybean (Glycine max), tobacco (Nicotiana tabacum), potato (Solanum tuberosum), peanuts (Arachis hypogaea), cotton (Gossypium barbadense, Gossypium hirsutum), sweet potato (Ipomoea batatas), cassava (Manihot esculenta), coffee (Coffea spp.), coconut (Cocos nucifera), pineapple (Ananas comosus), citrus trees (Citrus spp.), cocoa (Theobroma cacao), tea (Camellia sinensis), banana (Musa spp.), avocado (Persea americana), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), sugarcane (Saccharum spp.), oats, barley, vegetables, ornamentals, and conifers.

The following examples are offered by way of illustration and not by way of limitation.

### EXAMPLES

### Example 1: Effector Identification

To isolate transcripts present in *Phakopsora pachyrhizi* haustoria infected soy leaves were harvested 6 days post inoculation and homogenized at 4°C in buffer 0.3M Sorbitol, 20mM MOPS, 0.2% PVP, 1mM, DTT (add fresh), 0.1% BSA, pH 7.2). The homogenate was passed through a 100 um Nytex filter, then a 25 um Nytex filter. The homogenate was centrifuged (3700g for 10 minutes, 4°C) and the pellet was resuspended in 3 ml of buffer (0.3M Sorbitol, 10mM MOPS, 0.2% BSA, 1 mM CaCl₂, 1 mM MnCl₂, 0.2% Protect RNA pH 5.4).

Streptavidin-paramagnetic beads which had been pre-incubated with Concanavalin A-biotin were added to the suspended homogenate and incubated on ice with rotation for 30 minutes. Beads were captured with a magnetic stand, washed 1X with suspension buffer and then the RNA captured on the beads was extracted using a standard trizol extraction method. Total RNA was quantified and a cDNA library was generated from total RNA using the SMART™ cDNA library kit and protocols (Clontech®).

5' sequence reads were obtained from these clones and clones were identified that contained an open reading frame that encoded a protein predicted to have a signal peptide by the SignalP algorithm. Clones with predicted signal peptides were fully sequenced and the predicted protein sequence was blasted against the NCBI non-redundant protein database. Predicted proteins that appeared to have clear homologs in organisms that were not within the order *Pucciniales* (Uredinales) were removed from the collection.

To isolate candidate *Phakopsora* effector sequences from soy infected with soy rust, total RNA was harvested from three samples: a soybean plant eight days after inoculation with *Phakopsora pachyrhizi;* a native soybean plant; and germinating *Phakopsora pachyrhizi* spores, using a standard trizol RNA preparation protocol. Messenger RNAs were isolated using the Qiagen® RNeasy® isolation kit for total RNA isolation, followed by mRNA isolation and fragment library construction using the Illumina TruSeq® RNA sample preparation kit (Illumina, Inc. San Diego, CA). In this method, mRNAs were isolated via oligo dT beads, fragmented, and reverse transcribed into cDNA fragments using random primers. The resulting cDNA fragments were end repaired, 3' A-Tailed, ligated with Illumina TruSeq® adapters and amplified using Illumina TruSeq® specific primers. The PCR products were purified by binding to Ampure XP® beads (Beckman Genomics, Danvers, MA) and initial library quality was assessed by the Agilent Bioanalyzer DNA 7500 chip. To reduce the presence of abundant cDNAs, the fragment libraries were denatured and re-annealed at 68°C for five hours followed by treatment with Duplex Specific Nuclease (Evrogen, Moscow, Russia) for 25 minutes. Digested libraries were purified with Ampure XP® beads (Beckman Coulter Genomics), amplified with Illumina TruSeq® primers, and checked for final quality and quantity on the Agilent Bioanalyzer DNA 7500 chip prior to sequencing on the Illumina HiSeq®2000.

After Illumina sequencing, reads from the rust-infected soy sample were searched for soy sequences which were subsequently removed. The remaining sequences were assembled into contigs using Velvet and Oasis. This set of contigs represents the *Phakopsora pachyrhizi* transcriptome.

These contigs were screened for open reading frames. These open reading frames were screened for secretory signal peptides using SignalP, and then blasted (Blastn and tblastx) against complete NA and protein databases. ORFS that did not have a signal peptide or returned significant similarity to sequences outside of the order *Pucciniales* were eliminated. The relative abundance of the remaining sequences was compared to their abundance in germinating spores. If the abundance of the transcript was higher in germinating spores, it was regarded as less likely to be a haustoria-associated effector transcript encoding an effector. The ORFs remaining were considered to be strong candidate effectors.

### Example 2: Effector Activity Assay

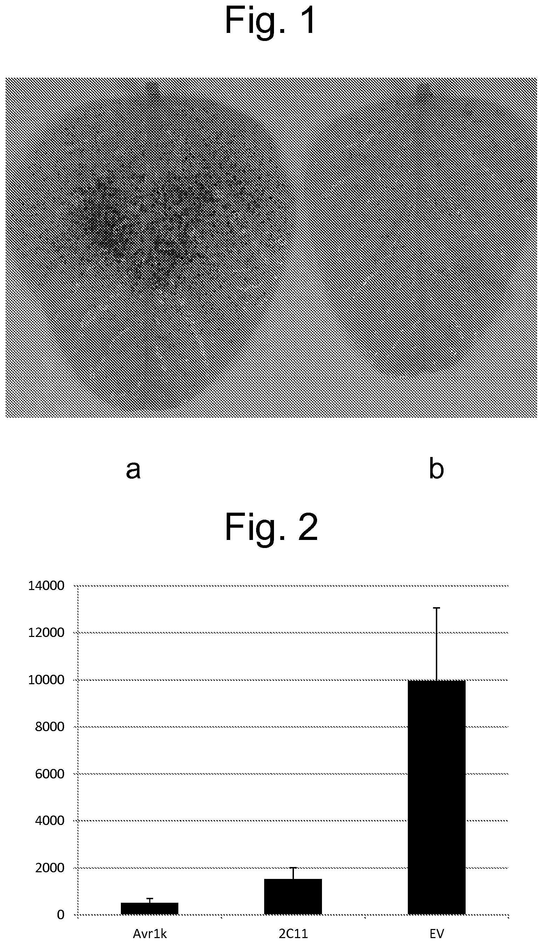

An effector can be identified by its ability to elicit programmed cell death. To assay for such cell death soybean cells were co-transformed with β-glucuronidase and the candidate effector. Plant leaves were co-bombarded biolistically with β-glucuronidase and the candidate effector, and the genes were allowed to express for two days. After two days the bombarded leaves were stained for β-glucuronidase activity using the colorometric substrate X-gluc (5-Bromo-4-chloro-3-indolyl-β-D-glucuronide). Programmed cell death (PCD) of transformed cells prevents the expression of the β-glucuronidase gene product. By comparing the intensity of staining between plant cells expressing β-glucuronidase and a candidate effector to those expressing β-glucuronidase alone it was determine that effector 2p12 (SEQ ID NO: 76) elicits cell death on soybean leaves (Figure 1) as well as on many other legumes (data not shown). After establishing that an effector elicits cell death, proteins predicted to block that effectors activity is screened by co-transforming plant cells with β-glucuronidase, 2p12 (SEQ ID NO: 75), and the candidate effector antagonist. If the PCD is blocked, restoring β-glucuronidase staining, that protein would be considered to be an effective antagonist of 2p12 (SEQ ID NO: 76), and is tested for its ability to confer resistance to ASR.

Effectors can also be identified by detecting their ability to suppress plant defense responses, which is an important function of many characterized effectors. One way to detect this suppression of plant defenses is to express a candidate effector in plant tissue via *Agrobacterium*-mediated transient expression and monitor changes in defense gene expression resulting from the presence of the candidate effector. The relative expression levels of the *Phaseolus* defense gene chalcone synthase (CHS) can be determined by semiquantitative RT-PCR (reverse transcription PCR) after infiltrating plants with *Agrobacterium* strains that transforms the leaf cells with the candidate effector of interest. Exposure to *Agrobacterium* elicits increased expression of CHS, but expression of a pathogen effector with defense gene suppression activity will interfere with this induction. Defense gene suppression activity from candidate effector 2c11 (SEQ ID NO: 74) was detected by transiently expressing 2c11 (SEQ ID NO: 73) in *Phaseolus vulgaris* and determining the reduction in the expression of the defense gene chalcone synthase (CHS). Figure 2 shows the chalcone synthase expression relative to ubiquitin expression in soybean plants expressing the pathogen effector Avr1k (gene referred to as avh331 - Dou D. et al., The Plant Cell, 20:1118-1133, 2008), 2c11 (SEQ ID NO: 73), or the control with no effector (EV = empty vector).

This assay can be used to screen candidate antagonists of the ASR effector polypeptides of the disclosure, such as 2c11 (SEQ ID NO: 74), by co-expressing both 2c11 (SEQ ID NO: 73) and the candidate antagonist. An effective antagonist would be restore the induction of CHS in plants co-infiltrated with 2c11 (SEQ ID NO: 73) and the prospective antagonist.

### Example 3: Identification of High Affinity Peptide Sequences to ASR Effectors

This example describes a procedure for identifying high affinity peptide sequences to ASR effectors using phage display libraries. Recombinant forms of the effector proteins are produced in *E*. *coli* and subjected to multiple rounds of selection against a variety of peptide display libraries constructed in the filamentous bacteriophage fd. Individual peptide display clones from the enriched pools are then tested for binding to the effector target by phage ELISA. The peptide sequences of display clones that bind specifically to the effector target are deduced by sequencing the DNA contained within the corresponding phage particles. These sequences can then be transferred to a bacterial expression vector to produce soluble peptides for further characterization of binding affinity and specificity to the effector target.

### Recombinant Effector Expression in E. coli

Effector sequences were cloned into an *E*. *coli* expression vector designed to produce soluble fusion proteins with SUMO (small ubiquitin related modifier) at the N-terminus. A T7*lac* promoter provides for high-level, IPTG-inducible expression of the fusion protein. An N-terminal polyhistidine (6x His) tag is included in the fusion construct for purification purposes. A thrombin cleavage site is located between SUMO and the effector sequence to allow removal of the 6xHis SUMO tag from the effector sequence. Located at the C-terminus of the effector sequence is a 15-amino acid tag (AviTag) that is a substrate sequence for the *E*. *coli* biotin holoenzyme synthetase, BirA. Proteins containing this sequence can be enzymatically biotinylated in vitro using BirA to transfer biotin to the epsilon amino group of lysine in the AviTag sequence.

An effector expression vector was transformed into *E*. *coli* BL21 (DE3) Gold and cultured at 37°C to an OD₆₀₀ of 0.8. Fusion protein expression was induced by adding 0.5 mM IPTG and the culture was incubated overnight at 16°C. The next day bacterial cells were harvested by centrifugation and resuspended in lysis buffer (50 mM Tris pH 8.0, 200 mM NaCl) plus 2.5 U/ml Benzonase. Cells were lysed with a homogenizer in two passes at 25kpsi. The lysate was centrifuged for 20 minutes at 16,000 rpm and the supernatant was passed through a 0.45 um filter. The cleared lysate was passed through a Ni-NTA column, washed with 4 column volumes of lysis buffer, followed by 10 column volumes with lysis buffer containing 1% Triton X-114, 15 mM imidazole. The 6xHis SUMO tag was cleaved from the effector protein by adding 1 column volume 50 mM Tris plus 10U/ml thrombin and incubating overnight at room temperature. The effector protein was eluted from the column in 50 mM Tris pH 8.0 and quantified by Bradford assay. Purified effector protein was then biotinylated in vitro using BirA enzyme according to the manufacturer's instructions (Avidity, LLC).

### Phage Display Libraries and Screening for High Affinity Sequences

Filamentous phage display libraries are constructed by inserting DNA fragments into phage or phagemid genomes such that the expressed proteins are linked to one of the phage coat proteins. This results in phage particles that display the encoded protein and also contain its gene, thus creating a direct physical link between the DNA sequences and their encoded proteins. Typically the minor coat protein (pIII) or the major coat protein (pVIII) of filamentous phage can be used for this purpose. Large collections of synthetic DNA fragments encoding random peptide sequences are cloned into a phage or phagemid vector and transformed into an *E. coli* host to produce peptide or protein display libraries.

In this example large libraries (>10⁹ members) of protein B1 domain (GB1) variants were constructed in a p3 phagemid vector and screened against a recombinant form of the effector protein. To isolate high affinity GB1 sequences from the libraries a biotinylated effector protein was captured in Neutravidin (Thermo Scientific) coated microtiter plates and then incubated at room temperature for 2h with the phage display libraries. Wells were washed six times with PT buffer (PBS, 0.05% Tween 20®) and then treated with elution buffer (0.2 M Glycine-HCl, pH 2.2, 1 mg/ml BSA) to recover phage bound to the immobilized effector target. The eluate was transferred to a microfuge tube and adjusted to neutral pH using 2M Tris. To amplify the recovered phage population for the next round of selection the eluate was added to log-phase *E*. *coli* TG1 cells and incubated for 30 min at 37°C without shaking. Infected cells were transferred to a culture tube containing 10 ml 2YT/Carb₅₀ and incubated with shaking for 1h at 37°C. M13KO7 helper phage was added to the culture and incubated for 1h at 37°C. The culture was transferred to 50 ml 2YT/Carb₅₀/Kan₂₅ in a 250 ml baffled flask and incubated overnight at 37C. The next day amplified phage were recovered from the culture supernatant by centrifugation to remove the bacterial cells. Phage were precipitated from the supernatant by adding 0.2 volumes of 20% PEG8000/2.5 M NaCl and incubating for 30 min on ice. The samples were then centrifuged at 12,000xg for 20 min at 4°C. Phage pellets were resuspended in PBS/0.05% Tween 20®/0.5% BSA and the selection process was repeated for up to five cycles to enrich for effector binders.

### Phage ELISA

A phage ELISA is used to identify individual phage clones from library selections that bind specifically to the effector target protein. Individual colonies from titer plates of phage recovered following multiple rounds of selection against an effector were picked into a 96-well deep well plate containing 1 ml 2YT/Carb₅₀. The deep well plate was incubated at 37°C for 4h in a shaker incubator. M13K07 helper phage (10⁹ pfu) was added to each well and incubated for 30 min at 37°C. Kanamycin was added to each well (25 ug/ml) and the deep well plate cultures were incubated overnight at 37C in a shaker incubator. The next day bacterial cells in the cultures were pelleted by centrifugation at 4000 rpm for 15 min at 4°C. The cleared phage containing supernatants were then transferred to a new 96-well deep well plate.

ELISA plates were prepared by coating microtiter wells with NeutrAvidin® (1 ug/well) followed by blocking with PBS/1% BSA. Biotinylated effector protein was then captured in the NeutrAvidin® coated wells. Individual phage supernatants were added to effector coated wells with an equal volume of PBT (PBS, 0.05% Tween 20®, 0.5% BSA) and incubated for 1h at room temperature. Phage supernatants were also added to corresponding control wells containing only NeutrAvidin® or only blocked with PBS/1% BSA. The microtiter plate was washed six times with PT using a BioTek plate washer. Anti-M13-HRP antibody (GE Healthcare) diluted 1:5000 in PBT was added to each well and incubated for 1h at room temperature. The plate was washed again with PT and developed by adding TMB substrate solution (BioFX Laboratories). Color development was terminated by adding Stop Solution (BioFX Laboratories) to each well and the absorbance at 405 nm was measured using a SpectraMax® plate reader. Phage clones bound to effector target that produced ELISA signals greater than 2-fold over control wells were selected for DNA sequence analysis to deduce the displayed GB1 sequences.

### Measuring Binding Affinity and Specificity of Soluble Peptides

Soluble effector binding peptides identified by phage library screening are produced by subcloning the gene from the selected phagemid display clone into an *E. coli* expression vector. In this example the effector binding peptides were fused to the C-terminus of the maltose-binding protein using the vector pMAL™-c5X (New England Biolabs). Cytoplasmically expressed fusion proteins were purified using an amylose resin column according to the manufacturer's instructions (New England Biolabs). Purified MBP peptide fusions were tested against multiple effector targets to confirm specificity by ELISA. Biotinylated effector proteins were captured on NeutrAvidin® coated microtiter wells as previously described for phage ELISA. The MBP peptide fusions were added to each well (1 ug/well) and incubated at room temperature for 1h. Wells were then washed with PT buffer and bound MBP peptide fusions were detected using an anti-MBP HRP antibody.

The binding affinity of an MBP peptide fusion to an effector protein was measured using the Octet RED384 instrument (Pall ForteBio Corp.). Real-time kinetic analysis was performed by capturing the biotinylated effector on streptavidin biosensors followed by monitoring the association and dissociation rates of the MBP peptide fusions by Bio-Layer Interferometry. The data was processed using Octet Software and was used to determine the binding affinity constant (K_{D} value) of the MBP peptide fusion for the effector protein target, which is defined as the ratio of the *k*_{d}/*k*ₐ (dissociation rate constant/association rate constant).

### Example 4: Transformation of soybean with effector antagonist plant expression vectors

Effector antagonists are tested for efficacy against ASR by transformation of a plant expression construct into soybean, followed by inoculation of transgenic plants with Phakopsora pachyrhizi and scoring for disease severity.

A suitable plant transformation construct is prepared by cloning the antagonist sequence of interest (Ex. 3) as a BamHI-HpaI DNA fragment in a suitable entry vector. The antagonist coding region fragment is ligated at the 5' end to a 1946 bp soybean ubiquitin promoter + Intronl fragment and on the 3' end to a 888 bp Arabidopsis ubiquitin terminator fragment. The entire promoter-coding region-terminator cassette is located between the attL1 and attL2 recombination sites. The plant expression construct for soybean transformation is generated by means of a Gateway® LR recombination reaction (Invitrogen, Life Technologies, Carlsbad, CA, USA) between the entry vector and a pDEST™ vector using the standard protocol provided by the manufacturer. The pDEST™ vector contains in addition to attR1 and attR2 recombination sites, a hygromycin resistance gene for bacterial selection and a herbicide resistant soybean ALS gene as a plant selectable marker.

The products of the LR recombination reaction are transformed into E.coli (TOP10), transformants are selected and pDNA isolated by standard miniprep methods. Transformants are characterized by diagnostic restriction enzyme digestions of miniprep DNA. A positive clone containing the expected pattern of digestion bands is selected for soybean transformation experiments.

Transgenic soybean lines are generated by the method of particle gun bombardment (Klein et al., Nature (London) 327:70-73 (1987); U.S. Patent No. 4,945,050) using a BIORAD Biolistic PDS1000/He instrument and either plasmid or fragment DNA. The following stock solutions and media are used for transformation and regeneration of soybean plants:
Stock solutions:
   Sulfate 100 X Stock:
      37.0 g MgSO₄.7H₂O, 1.69 g MnSO⁴.H²O, 0.86 g ZnSO⁴.7H₂O, 0.0025 g CuSO₄.5H₂O
   Halides 100 X Stock:
      30.0 g CaCl₂.2H₂O, 0.083 g Kl, 0.0025 g CoCl₂.6H2O
   P, B, Mo 100X Stock:
      18.5 g KH₂PO₄, 0.62 g H₃BO₃, 0.025 g Na₂MoO₄.2H₂O
   Fe EDTA 100X Stock:
      3.724 g Na₂EDTA, 2.784 g FeSO₄.7H₂O
   2,4 D Stock:
      10 mg/mL Vitamin
   B5 vitamins, 1000X Stock:
      100.0 g myo-inositol, 1.0 g nicotinic acid, 1.0 g pyridoxine HCl, 10 g thiamine HCL.
Media (per Liter):
   SB199 Solid Medium:
      1 package MS salts (Gibco/ BRL - Cat. No. 11117-066), 1 mL B5 vitamins 1000X stock, 30g Sucrose, 4 ml 2, 4-D (40 mg/L final concentration), pH 7.0, 2 gm Gelrite
   SB1 Solid Medium:
      1 package MS salts (Gibco/ BRL - Cat. No. 11117-066), 1 mL B5 vitamins 1000X stock, 31.5 g Glucose, 2 mL 2, 4-D (20 mg/L final concentration), pH 5.7, 8 g TC agar
   SB196:
      10 mL of each of the above stock solutions 1-4, 1 mL B5 Vitamin stock, 0.463 g (NH4)2 SO4, 2.83 g KNO₃, 1 mL 2,4 D stock, 1 g asparagine, 10 g Sucrose, pH 5.7
   SB71-4:
      Gamborg's B5 salts, 20 g sucrose, 5 g TC agar, pH 5.7.
   SB103:
      1 pk. Murashige & Skoog salts mixture, 1 mL B5 Vitamin stock, 750 mg MgCl₂ hexahydrate, 60 g maltose, 2 g gelrite™, pH 5.7.
   SB166:
      SB103 supplemented with 5 g per liter activated charcoal.

### Soybean Embryogenic Suspension Culture Initiation:

Pods with immature seeds from available soybean plants 45-55 days after planting are picked, removed from their shells and placed into a sterilized magenta box. The soybean seeds are sterilized by shaking them for 15 min in a 5% Clorox® solution with 1 drop of Ivory™ soap (i.e., 95 mL of autoclaved distilled water plus 5 mL Clorox® and 1 drop of soap, mixed well). Seeds are rinsed using 2 L sterile distilled water and those less than 3 mm are placed on individual microscope slides. The small end of the seed is cut and the cotyledons pressed out of the seed coat. Cotyledons are transferred to plates containing SB199 medium (25-30 cotyledons per plate) for 2 weeks, then transferred to SB1 for 2-4 weeks. Plates are wrapped with fiber tape. After this time, secondary embryos are cut and placed into SB196 liquid medium for 7 days.

### Culture Conditions:

Soybean embryogenic suspension cultures (cv. 93Y21) were maintained in 50 mL liquid medium SB196 on a rotary shaker, 100 - 150 rpm, 26 °C on 16:8 h day/night photoperiod at light intensity of 80-100 µE/m2/s. Cultures are subcultured every 7-14 days by inoculating up to ½ dime size quantity of tissue (clumps bulked together) into 50 mL of fresh liquid SB196.

### Preparation of DNA for Bombardment:

In particle gun bombardment procedures it is possible to use purified 1) entire plasmid DNA; or 2) DNA fragments containing only the recombinant DNA expression cassette(s) of interest. For every seventeen bombardment transformations, 85 µL of suspension is prepared containing 1 to 90 picograms (pg) of plasmid DNA per base pair of each DNA plasmid. DNA plasmids or fragments are co precipitated onto gold particles as follows. The DNAs in suspension are added to 50 µL of a 10 - 60 mg/mL 0.6 µm gold particle suspension and then combined with 50 µL CaCl2 (2.5 M) and 20 µL spermidine (0.1 M). The mixture is vortexed for 5 sec, spun in a microfuge for 5 sec, and the supernatant removed. The DNA coated particles are then washed once with 150 µL of 100% ethanol, vortexed and spun in a microfuge again, then resuspended in 85 µL of anhydrous ethanol. Five µL of the DNA coated gold particles are then loaded on each macrocarrier disk.

### Tissue Preparation and Bombardment with DNA:

Approximately 100 mg of two-week-old suspension culture is placed in an empty 60 mm X 15 mm petri plate and the residual liquid removed from the tissue using a pipette. The tissue is placed about 3.5 inches away from the retaining screen and each plate of tissue is bombarded once. Membrane rupture pressure is set at 650 psi and the chamber is evacuated to -28 inches of Hg. Following bombardment, the tissue from each plate is divided between two flasks, placed back into liquid media, and cultured as described above.

### Selection of Transformed Embryos and Plant Regeneration:

After bombardment, tissue from each bombarded plate is divided and placed into two flasks of SB196 liquid culture maintenance medium per plate of bombarded tissue. Seven days post bombardment, the liquid medium in each flask is replaced with fresh SB196 culture maintenance medium supplemented with 100 ng/ml selective agent (selection medium). For selection of transformed soybean cells the selective agent used can be a sulfonylurea (SU) compound with the chemical name, 2 chloro N ((4 methoxy 6 methy 1,3,5 triazine 2 yl)aminocarbonyl) benzenesulfonamide (common names: DPX-W4189 and chlorsulfuron). Chlorsulfuron is the active ingredient in the DuPont sulfonylurea herbicide, GLEAN®. The selection medium containing SU is replaced every two weeks for 8 weeks. After the 8 week selection period, islands of green, transformed tissue are observed growing from untransformed, necrotic embryogenic clusters. These putative transgenic events are isolated and kept in SB196 liquid medium with SU at 100 ng/ml for another 5 weeks with media changes every 1-2 weeks to generate new, clonally propagated, transformed embryogenic suspension cultures. Embryos spend a total of around 13 weeks in contact with SU. Suspension cultures are sub-cultured and maintained as clusters of immature embryos and also regenerated into whole plants by maturation and germination of individual somatic embryos.

Somatic embryos become suitable for germination after four weeks on maturation medium (1 week on SB166 followed by 3 weeks on SB103). They are then removed from the maturation medium and dried in empty petri dishes for up to seven days. The dried embryos are then planted in SB71 4 medium where they are allowed to germinate under the same light and temperature conditions as described above. Germinated embryos are transferred to potting medium and grown to maturity for seed production.

### Example 5: Transgenic testing for efficacy of antagonist constructs against Phakopsora pachyrhizi

Transgenic events that are positive for expression of the antagonist gene are identified (Western blots of epitope tagged proteins) RT-PCR of total RNA; or MS detection of predicted, diagnostic peptide fragments) and selected for evaluation by ASR infection assays.

Segregating, T1 seed of selected events are sown and allowed to grow to the Vc stage. Plants are characterized by qPCR to identify positive and nulls, and then inoculated with a suspension of *Phakopsora* spores. Inoculum may be prepared from urediniospores maintained in N₂(I) or stored in a -80°C freezer. Spores are retrieved, subjected to a heat shock at 40°C for 5 minutes and then hydrated overnight at room temperature (∼ 23C) prior to suspension in water containing 0.01% Tween 20®. Alternatively, spores may be harvested from infected leaves containing sporulating lesions by agitating leaves in 0.01% Tween 20®. Spore suspensions are thoroughly mixed, counted using a hemocytometer and adjusted to a final concentration of 0.1-1 x 10⁵ spores/ml.

Plants are spray inoculated inside a biosafety cabinet using Preval Sprayer™ attached to a bottle to deliver a fine spray of spore suspension to the plant leaves. Following inoculation, plants are maintained in a dew chamber in the dark at 100% relative humidity for approximately 16 hrs at 25°C before transfer to growth chambers (22C, 70% RH, 16 hr photoperiod). ASR infection is allowed to develop and plants are scored for disease symptoms 12-21 days after inoculation.

To evaluate efficacy of antagonist constructs against ASR, leaves are harvested, a scanned image generated and the image analyzed using appropriate software (such as Assess2.0; APS Press). Different disease parameters such as number of lesions/cm², % leaf area affected or average lesion diameter are collected. Data obtained from positive transgenic plants are compared to null plants to determine efficacy. It is expected that transgenic positive plants containing an antagonist known to interfere with the activity of an effector such as that described in Example 2 will exhibit reduced disease severity (reduced lesion number, and/or reduced lesion size, and/or reduced leaf area affected) in comparison to control plants lacking the antagonist construct.

## Claims

1. An isolated polynucleotide comprising a nucleic acid sequence selected from:
(a) the nucleic acid sequence set forth in SEQ ID NO: 75, or a complement thereof; and
(b) a nucleotide sequence encoding a polypeptide having the amino acid sequence set forth in SEQ ID NO: 76.

2. An isolated polypeptide having the amino acid sequence of SEQ ID NO: 76.

3. An expression cassette comprising a heterologous regulatory sequence operably linked to the isolated polynucleotide of claim 1.

4. A non-human host cell comprising the expression cassette of claim 3.

5. The non-human host cell of claim 4, wherein the cell is a plant cell.

6. A transgenic soybean plant, soybean plant part or soybean plant cell comprising the expression vector of claim 3, wherein the transgenic plant, plant part or plant cell has enhanced resistance to Asian Soy Rust compared to a corresponding non-transgenic wild type plant.

7. The transgenic plant of claim 6, wherein the isolated polynucleotide is introduced as an overexpression cassette.

8. A method of modulating at least one protein associated with resistance to Asian Soy Rust in a transgenic plant, the method comprising expressing at least one polynucleotide of claim 1.

9. The method of claim 8, wherein the polynucleotide is expressed in an overexpression cassette.

10. The method of claim 8 or 9, wherein the level of the protein associated with resistance to Asian Soy Rust is increased compared to the level of the protein associated with resistance to Asian Soy Rust in a corresponding non-transgenic plant.

11. A method of enhancing plant resistance to Asian Soy Rust (ASR) infection, the method comprising inactivation of the ASR pathogen by eliciting programmed cell death by a ASR effector polypeptide sequence, wherein the ASR polypeptide is the amino acid sequence set forth in SEQ ID NO: 76.

12. The method of claim 11, wherein the plant is a dicot.

13. The method of claim 12, wherein the plant is soybean.

## Patentansprüche

1. Isoliertes Polynukleotid, umfassend eine Nukleinsäuresequenz, ausgewählt aus:
(a) der in SEQ ID NR:75 dargelegten Nukleinsäuresequenz oder einem Komplement davon; und
(b) einer Nukleotidsequenz, die ein Polypeptid mit der in SEQ ID NR:76 dargelegten Aminosäuresequenz codiert.

2. Isoliertes Polypeptid mit der Aminosäuresequenz von SEQ ID NR:76.

3. Expressionskassette, umfassend eine heterologe regulatorische Sequenz, die operabel mit dem isolierten Polynukleotid nach Anspruch 1 verbunden ist.

4. Nicht-menschliche Wirtszelle, umfassend die Expressionskassette nach Anspruch 3.

5. Nicht-menschliche Wirtszelle nach Anspruch 4, wobei die Zelle eine Pflanzenzelle ist.

6. Transgene/r Sojabohnenpflanze, Sojabohnenpflanzenteil oder Sojabohnenpflanzenzelle, umfassend den Expressionsvektor nach Anspruch 3, wobei die/der transgene Pflanze, Pflanzenteil oder Pflanzenzelle eine erhöhte Resistenz gegen asiatischen Sojarost im Vergleich zu einer entsprechenden nicht-transgenen Wildtyppflanze hat.

7. Transgene Pflanze nach Anspruch 6, wobei das isolierte Polynukleotid als eine Überexpressionskassette eingeführt wird.

8. Verfahren des Modulierens von mindestens einem Protein in Verbindung mit der Resistenz gegen asiatischen Sojarost in einer transgenen Pflanze, wobei das Verfahren die Expression von mindestens einem Polynukleotid nach Anspruch 1 umfasst.

9. Verfahren nach Anspruch 8, wobei das Polynukleotid in einer Überexpressionskassette exprimiert wird.

10. Verfahren nach Anspruch 8 oder 9, wobei der Proteinspiegel in Verbindung mit der Resistenz gegen asiatischen Sojarost im Vergleich zum Proteinspiegel in Verbindung mit der Resistenz gegen Sojarost in einer entsprechenden nicht-transgenen Pflanze erhöht ist.

11. Verfahren des Erhöhens der Resistenz von Pflanzen gegen eine Infektion mit asiatischem Sojarost (ASR), das Verfahren umfassend die Inaktivierung des ASR-Pathogens durch das Hervorrufen eines programmierten Zelltods durch eine ASR-Effektor-Polypeptidsequenz, wobei das ASR-Polypeptid die in SEQ ID NR:76 dargelegte Aminosäuresequenz ist.

12. Verfahren nach Anspruch 11, wobei die Pflanze eine Dikotyle ist.

13. Verfahren nach Anspruch 11, wobei die Pflanze eine Sojabohne ist.

## Revendications

1. Polynucléotide isolé comprenant une séquence d'acides nucléiques choisie parmi :
(a) la séquence d'acides nucléiques indiquée dans la SEQ ID n° : 75 ou un complément de celle-ci ; et
(b) une séquence nucléotidique codant pour un polypeptide présentant la séquence d'acides aminés indiquée dans la SEQ ID n° : 76.

2. Polypeptide isolé présentant la séquence d'acides aminés de la SEQ ID n° : 76.

3. Cassette d'expression comprenant une séquence régulatoire hétérologue liée de manière fonctionnelle au polynucléotide isolé selon la revendication 1.

4. Cellule hôte non humaine comprenant la cassette d'expression selon la revendication 3.

5. Cellule hôte non humaine selon la revendication 4, la cellule étant une cellule végétale.

6. Plante de soja transgénique, partie de plante de soja ou cellule de plante de soja comprenant le vecteur d'expression selon la revendication 3, dans laquelle la plante transgénique, la partie de plante ou la cellule végétale présente une meilleure résistance à la rouille asiatique du soja par rapport à une plante de type sauvage non transgénique correspondante.

7. Plante transgénique selon la revendication 6, dans laquelle le polynucléotide isolé est introduit en tant que cassette de surexpression.

8. Procédé de modulation d'au moins une protéine associée à la résistance à la rouille asiatique du soja dans une plante transgénique, le procédé comprenant l'expression d'au moins un polynucléotide selon la revendication 1.

9. Procédé selon la revendication 8, dans lequel le polynucléotide est exprimé dans une cassette de surexpression.

10. Procédé selon la revendication 8 ou 9, dans lequel le niveau de la protéine associé à la résistance à la rouille asiatique du soja est augmenté par rapport au niveau de la protéine associé à la résistance à la rouille asiatique du soja dans une plante non transgénique correspondante.

11. Procédé d'amélioration de la résistance des plantes à l'infection de la rouille asiatique du soja (ASR), le procédé comprenant l'inactivation du pathogène ASR en déclenchant la mort cellulaire programmée par une séquence polypeptidique effectrice d'ASR, le polypeptide d'ASR étant la séquence d'acides aminés indiquée dans la SEQ ID n° : 76.

12. Procédé selon la revendication 11, dans lequel la plante est une dicotylédone.

13. Procédé selon la revendication 12, dans lequel la plante est du soja.
